# EUROPEAN PATENT SPECIFICATION

(11) **EP 3 365 440 B1**
(45) Date of publication and mention of the grant of the patent: **14.09.2022**
(21) Application number: 16788879.1
(22) Date of filing: 17.10.2016
(51) Int. Cl.: C12N 9/22, C12N 15/01, C12N 15/82, C12N 15/90

(54) **RESTORING FUNCTION TO A NON-FUNCTIONAL GENE PRODUCT VIA GUIDED CAS SYSTEMS AND METHODS OF USE**
WIEDERHERSTELLUNG DER FUNKTION EINES NICHTFUNKTIONELLEN GENPRODUKTS ÜBER GEFÜHRTE CAS-SYSTEME UND VERFAHREN ZUR VERWENDUNG
RESTAURATION DE LA FONCTION D'UN PRODUIT GÉNIQUE NON FONCTIONNEL PAR L'INTERMÉDIAIRE DE SYSTÈMES CAS GUIDÉS ET MÉTHODES D'UTILISATION ASSOCIÉES

(30) Priority: 20.10.2015 US 201562243719 P; 16.03.2016 US 201662309033 P; 07.07.2016 US 201662359254 P
(43) Date of publication of application: 29.08.2018
(73) Proprietor: Pioneer Hi-Bred International, Inc., Johnston, Iowa 50131-1014 (US)
(72) Inventor: CIGAN, Andrew Mark, Madison, Wisconsin 53704 (US); SVITASHEV, Sergei, Johnston, Iowa 50131-0552 (US)
(74) Representative: J A Kemp LLP
(86) International application number: PCT/US2016/057279
(87) International publication number: WO 2017/070032

(56) References cited:
- WO-A1-2014/065596
- US-A1- 2015 044 772
- WENZHI JIANG ET AL: "Efficient CRISPR/Cas9-Mediated Gene Editing in Arabidopsis thaliana and Inheritance of Modified Genes in the T2 and T3 Generations", PLOS ONE, vol. 9, no. 6, 11 June 2014 (2014-06-11), page e99225, XP055219594, DOI: 10.1371/journal.pone.0099225
- W. JIANG ET AL: "Demonstration of CRISPR/Cas9/sgRNA-mediated targeted gene modification in Arabidopsis, tobacco, sorghum and rice", NUCLEIC ACIDS RESEARCH, vol. 41, no. 20, 2 September 2013 (2013-09-02), pages e188-e188, XP055219328, ISSN: 0305-1048, DOI: 10.1093/nar/gkt780
- JE WOOK WOO ET AL: "DNA-free genome editing in plants with preassembled CRISPR-Cas9 ribonucleoproteins", NATURE BIOTECHNOLOGY, vol. 33, no. 11, 19 October 2015 (2015-10-19), pages 1162-1164, XP055290196, US ISSN: 1087-0156, DOI: 10.1038/nbt.3389
- KANCHISWAMY C N ET AL: "Non-GMO genetically edited crop plants", TRENDS IN BIOTECHNOLOGY 20150901 ELSEVIER LTD GBR, vol. 33, no. 9, 1 September 2015 (2015-09-01), XP002765281, DOI: 10.1016/J.TIBTECH.2015.04.002
- LUISA BORTESI ET AL: "The CRISPR/Cas9 system for plant genome editing and beyond", BIOTECHNOLOGY ADVANCES, vol. 33, no. 1, 1 January 2015 (2015-01-01), pages 41-52, XP055217852, ISSN: 0734-9750, DOI: 10.1016/j.biotechadv.2014.12.006
- LI ZHONGSEN ET AL: "Cas9-Guide RNA Directed Genome Editing in Soybean", PLANT PHYSIOLOGY (ROCKVILLE), vol. 169, no. 2, October 2015 (2015-10), pages 960-970, XP002765282,
- BERND ZETSCHE ET AL: "Cpf1 Is a Single RNA-Guided Endonuclease of a Class 2 CRISPR-Cas System", CELL, vol. 163, no. 3, 1 October 2015 (2015-10-01), pages 759-771, XP055267511, US ISSN: 0092-8674, DOI: 10.1016/j.cell.2015.09.038
- SINKUNAS TOMAS ET AL: "Cas3 is a single-stranded DNA nuclease and ATP-dependent helicase in the CRISPR/Cas immune system", EMBO (EUROPEAN MOLECULAR BIOLOGY ORGANIZATION) JOURNAL, vol. 30, no. 7, April 2011 (2011-04), pages 1335-1342, XP002765626,
- Qiudeng Que ET AL: "Maize transformation technology development for commercial event generation", Frontiers in Plant Science, vol. 5, 5 August 2014 (2014-08-05), XP055217826, DOI: 10.3389/fpls.2014.00379
- L. Cai ET AL: "Optimization of a biolistic transformation system for transfer of antifreeze gene KN2 and the bar herbicide resistance gene in common wheat", Genetics and Molecular Research, vol. 13, no. 2, 1 January 2014 (2014-01-01), pages 3474-3485, XP055713918, DOI: 10.4238/2014.April.30.8
- MARTIN-ORTIGOSA SUSANA ET AL: "Proteolistics: a biolistic method for intracellular delivery of proteins", TRANSGENIC RESEARCH, SPRINGER NETHERLANDS, NL, vol. 23, no. 5, 5 August 2014 (2014-08-05) , pages 743-756, XP035381272, ISSN: 0962-8819, DOI: 10.1007/S11248-014-9807-Y [retrieved on 2014-08-05]

### SEQUENCE LISTING

<110> Pioneer Hi-Bred International Inc. Cigan, Andrew Mark Svitashev, Sergei
<120> RESTORING FUNCTION TO A NON-FUNCTIONAL GENE PRODUCT VIA GUIDED CAS SYSTEMS AND METHODS OF USE
<130> BB2533 PCT
<150> 62/243719
   <151> 2015-10-20
<150> 62/309033
   <151> 2016-03-16
<150> 62/359254
   <151> 2016-07-07
<160> 82
<170> PatentIn version 3.5
<210> 1
   <211> 4107
   <212> DNA
   <213> Streptococcus pyogenes M1 GAS (SF370)
<400> 1
   atggataaga aatactcaat aggcttagat atcggcacaa atagcgtcgg atgggcggtg      60
   atcactgatg aatataaggt tccgtctaaa aagttcaagg ttctgggaaa tacagaccgc     120
   cacagtatca aaaaaaatct tataggggct cttttatttg acagtggaga gacagcggaa     180
   gcgactcgtc tcaaacggac agctcgtaga aggtatacac gtcggaagaa tcgtatttgt     240
   tatctacagg agattttttc aaatgagatg gcgaaagtag atgatagttt ctttcatcga     300
   cttgaagagt cttttttggt ggaagaagac aagaagcatg aacgtcatcc tatttttgga     360
   aatatagtag atgaagttgc ttatcatgag aaatatccaa ctatctatca tctgcgaaaa     420
   aaattggtag attctactga taaagcggat ttgcgcttaa tctatttggc cttagcgcat     480
   atgattaagt ttcgtggtca ttttttgatt gagggagatt taaatcctga taatagtgat     540
   gtggacaaac tatttatcca gttggtacaa acctacaatc aattatttga agaaaaccct     600
   attaacgcaa gtggagtaga tgctaaagcg attctttctg cacgattgag taaatcaaga     660
   cgattagaaa atctcattgc tcagctcccc ggtgagaaga aaaatggctt atttgggaat     720
   ctcattgctt tgtcattggg tttgacccct aattttaaat caaattttga tttggcagaa     780
   gatgctaaat tacagctttc aaaagatact tacgatgatg atttagataa tttattggcg     840
   caaattggag atcaatatgc tgatttgttt ttggcagcta agaatttatc agatgctatt     900
   ttactttcag atatcctaag agtaaatact gaaataacta aggctcccct atcagcttca     960
   atgattaaac gctacgatga acatcatcaa gacttgactc ttttaaaagc tttagttcga    1020
   caacaacttc cagaaaagta taaagaaatc ttttttgatc aatcaaaaaa cggatatgca    1080
   ggttatattg atgggggagc tagccaagaa gaattttata aatttatcaa accaatttta    1140
   gaaaaaatgg atggtactga ggaattattg gtgaaactaa atcgtgaaga tttgctgcgc    1200
   aagcaacgga cctttgacaa cggctctatt ccccatcaaa ttcacttggg tgagctgcat    1260
   gctattttga gaagacaaga agacttttat ccatttttaa aagacaatcg tgagaagatt    1320
   gaaaaaatct tgacttttcg aattccttat tatgttggtc cattggcgcg tggcaatagt    1380
   cgttttgcat ggatgactcg gaagtctgaa gaaacaatta ccccatggaa ttttgaagaa    1440
   gttgtcgata aaggtgcttc agctcaatca tttattgaac gcatgacaaa ctttgataaa    1500
   aatcttccaa atgaaaaagt actaccaaaa catagtttgc tttatgagta ttttacggtt    1560
   tataacgaat tgacaaaggt caaatatgtt actgaaggaa tgcgaaaacc agcatttctt    1620
   tcaggtgaac agaagaaagc cattgttgat ttactcttca aaacaaatcg aaaagtaacc    1680
   gttaagcaat taaaagaaga ttatttcaaa aaaatagaat gttttgatag tgttgaaatt    1740
   tcaggagttg aagatagatt taatgcttca ttaggtacct accatgattt gctaaaaatt    1800
   attaaagata aagatttttt ggataatgaa gaaaatgaag atatcttaga ggatattgtt    1860
   ttaacattga ccttatttga agatagggag atgattgagg aaagacttaa aacatatgct    1920
   cacctctttg atgataaggt gatgaaacag cttaaacgtc gccgttatac tggttgggga    1980
   cgtttgtctc gaaaattgat taatggtatt agggataagc aatctggcaa aacaatatta 2040
   gattttttga aatcagatgg ttttgccaat cgcaatttta tgcagctgat ccatgatgat 2100
   agtttgacat ttaaagaaga cattcaaaaa gcacaagtgt ctggacaagg cgatagttta 2160
   catgaacata ttgcaaattt agctggtagc cctgctatta aaaaaggtat tttacagact 2220
   gtaaaagttg ttgatgaatt ggtcaaagta atggggcggc ataagccaga aaatatcgtt 2280
   attgaaatgg cacgtgaaaa tcagacaact caaaagggcc agaaaaattc gcgagagcgt 2340
   atgaaacgaa tcgaagaagg tatcaaagaa ttaggaagtc agattcttaa agagcatcct 2400
   gttgaaaata ctcaattgca aaatgaaaag ctctatctct attatctcca aaatggaaga 2460
   gacatgtatg tggaccaaga attagatatt aatcgtttaa gtgattatga tgtcgatcac 2520
   attgttccac aaagtttcct taaagacgat tcaatagaca ataaggtctt aacgcgttct 2580
   gataaaaatc gtggtaaatc ggataacgtt ccaagtgaag aagtagtcaa aaagatgaaa 2640
   aactattgga gacaacttct aaacgccaag ttaatcactc aacgtaagtt tgataattta 2700
   acgaaagctg aacgtggagg tttgagtgaa cttgataaag ctggttttat caaacgccaa 2760
   ttggttgaaa ctcgccaaat cactaagcat gtggcacaaa ttttggatag tcgcatgaat 2820
   actaaatacg atgaaaatga taaacttatt cgagaggtta aagtgattac cttaaaatct 2880
   aaattagttt ctgacttccg aaaagatttc caattctata aagtacgtga gattaacaat 2940
   taccatcatg cccatgatgc gtatctaaat gccgtcgttg gaactgcttt gattaagaaa 3000
   tatccaaaac ttgaatcgga gtttgtctat ggtgattata aagtttatga tgttcgtaaa 3060
   atgattgcta agtctgagca agaaataggc aaagcaaccg caaaatattt cttttactct 3120
   aatatcatga acttcttcaa aacagaaatt acacttgcaa atggagagat tcgcaaacgc 3180
   cctctaatcg aaactaatgg ggaaactgga gaaattgtct gggataaagg gcgagatttt 3240
   gccacagtgc gcaaagtatt gtccatgccc caagtcaata ttgtcaagaa aacagaagta 3300
   cagacaggcg gattctccaa ggagtcaatt ttaccaaaaa gaaattcgga caagcttatt 3360
   gctcgtaaaa aagactggga tccaaaaaaa tatggtggtt ttgatagtcc aacggtagct 3420
   tattcagtcc tagtggttgc taaggtggaa aaagggaaat cgaagaagtt aaaatccgtt 3480
   aaagagttac tagggatcac aattatggaa agaagttcct ttgaaaaaaa tccgattgac 3540
   tttttagaag ctaaaggata taaggaagtt aaaaaagact taatcattaa actacctaaa 3600
   tatagtcttt ttgagttaga aaacggtcgt aaacggatgc tggctagtgc cggagaatta 3660
   caaaaaggaa atgagctggc tctgccaagc aaatatgtga attttttata tttagctagt 3720
   cattatgaaa agttgaaggg tagtccagaa gataacgaac aaaaacaatt gtttgtggag 3780
   cagcataagc attatttaga tgagattatt gagcaaatca gtgaattttc taagcgtgtt 3840
   attttagcag atgccaattt agataaagtt cttagtgcat ataacaaaca tagagacaaa 3900
   ccaatacgtg aacaagcaga aaatattatt catttattta cgttgacgaa tcttggagct 3960
   cccgctgctt ttaaatattt tgatacaaca attgatcgta aacgatatac gtctacaaaa 4020
   gaagttttag atgccactct tatccatcaa tccatcactg gtctttatga aacacgcatt 4080
   gatttgagtc agctaggagg tgactga 4107
<210> 2
   <211> 189
   <212> DNA
   <213> Solanum tuberosum
<400> 2
   gtaagtttct gcttctacct ttgatatata tataataatt atcattaatt agtagtaata 60
   taatatttca aatatttttt tcaaaataaa agaatgtagt atatagcaat tgcttttctg 120
   tagtttataa gtgtgtatat tttaatttat aacttttcta atatatgacc aaaacatggt 180
   gatgtgcag 189
<210> 3
   <211> 9
   <212> PRT
   <213> Simian virus 40
<400> 3
   Met Ala Pro Lys Lys Lys Arg Lys Val
   1               5
<210> 4
   <211> 18
   <212> PRT
   <213> Agrobacterium tumefaciens
<400> 4
   Lys Arg Pro Arg Asp Arg His Asp Gly Glu Leu Gly Gly Arg Lys Arg
   1               5                   10                  15
   Ala Arg
<210> 5
   <211> 6717
   <212> DNA
   <213> Artificial Sequence
<220>
   <223> Maize optimized Cas9 expression cassette
<400> 5
   gtgcagcgtg acccggtcgt gcccctctct agagataatg agcattgcat gtctaagtta 60
   taaaaaatta ccacatattt tttttgtcac acttgtttga agtgcagttt atctatcttt 120
   atacatatat ttaaacttta ctctacgaat aatataatct atagtactac aataatatca 180
   gtgttttaga gaatcatata aatgaacagt tagacatggt ctaaaggaca attgagtatt 240
   ttgacaacag gactctacag ttttatcttt ttagtgtgca tgtgttctcc tttttttttg 300
   caaatagctt cacctatata atacttcatc cattttatta gtacatccat ttagggttta 360
   gggttaatgg tttttataga ctaatttttt tagtacatct attttattct attttagcct 420
   ctaaattaag aaaactaaaa ctctatttta gtttttttat ttaataattt agatataaaa 480
   tagaataaaa taaagtgact aaaaattaaa caaataccct ttaagaaatt aaaaaaacta 540
   aggaaacatt tttcttgttt cgagtagata atgccagcct gttaaacgcc gtcgacgagt 600
   ctaacggaca ccaaccagcg aaccagcagc gtcgcgtcgg gccaagcgaa gcagacggca 660
   cggcatctct gtcgctgcct ctggacccct ctcgagagtt ccgctccacc gttggacttg 720
   ctccgctgtc ggcatccaga aattgcgtgg cggagcggca gacgtgagcc ggcacggcag 780
   gcggcctcct cctcctctca cggcaccggc agctacgggg gattcctttc ccaccgctcc 840
   ttcgctttcc cttcctcgcc cgccgtaata aatagacacc ccctccacac cctctttccc 900
   caacctcgtg ttgttcggag cgcacacaca cacaaccaga tctcccccaa atccacccgt 960
   cggcacctcc gcttcaaggt acgccgctcg tcctcccccc cccccctctc taccttctct 1020
   agatcggcgt tccggtccat gcatggttag ggcccggtag ttctacttct gttcatgttt 1080
   gtgttagatc cgtgtttgtg ttagatccgt gctgctagcg ttcgtacacg gatgcgacct 1140
   gtacgtcaga cacgttctga ttgctaactt gccagtgttt ctctttgggg aatcctggga 1200
   tggctctagc cgttccgcag acgggatcga tttcatgatt ttttttgttt cgttgcatag 1260
   ggtttggttt gcccttttcc tttatttcaa tatatgccgt gcacttgttt gtcgggtcat 1320
   cttttcatgc ttttttttgt cttggttgtg atgatgtggt ctggttgggc ggtcgttcta 1380
   gatcggagta gaattctgtt tcaaactacc tggtggattt attaattttg gatctgtatg 1440
   tgtgtgccat acatattcat agttacgaat tgaagatgat ggatggaaat atcgatctag 1500
   gataggtata catgttgatg cgggttttac tgatgcatat acagagatgc tttttgttcg 1560
   cttggttgtg atgatgtggt gtggttgggc ggtcgttcat tcgttctaga tcggagtaga 1620
   atactgtttc aaactacctg gtgtatttat taattttgga actgtatgtg tgtgtcatac 1680
   atcttcatag ttacgagttt aagatggatg gaaatatcga tctaggatag gtatacatgt 1740
   tgatgtgggt tttactgatg catatacatg atggcatatg cagcatctat tcatatgctc 1800
   taaccttgag tacctatcta ttataataaa caagtatgtt ttataattat tttgatcttg 1860
   atatacttgg atgatggcat atgcagcagc tatatgtgga tttttttagc cctgccttca 1920
   tacgctattt atttgcttgg tactgtttct tttgtcgatg ctcaccctgt tgtttggtgt 1980
   tacttctgca ggtcgactct agaggatcca tggcaccgaa gaagaagcgc aaggtgatgg 2040
   acaagaagta cagcatcggc ctcgacatcg gcaccaactc ggtgggctgg gccgtcatca 2100
   cggacgaata taaggtcccg tcgaagaagt tcaaggtcct cggcaataca gaccgccaca 2160
   gcatcaagaa aaacttgatc ggcgccctcc tgttcgatag cggcgagacc gcggaggcga 2220
   ccaggctcaa gaggaccgcc aggagacggt acactaggcg caagaacagg atctgctacc 2280
   tgcaggagat cttcagcaac gagatggcga aggtggacga ctccttcttc caccgcctgg 2340
   aggaatcatt cctggtggag gaggacaaga agcatgagcg gcacccaatc ttcggcaaca 2400
   tcgtcgacga ggtaagtttc tgcttctacc tttgatatat atataataat tatcattaat 2460
   tagtagtaat ataatatttc aaatattttt ttcaaaataa aagaatgtag tatatagcaa 2520
   ttgcttttct gtagtttata agtgtgtata ttttaattta taacttttct aatatatgac 2580
   caaaacatgg tgatgtgcag gtggcctacc acgagaagta cccgacaatc taccacctcc 2640
   ggaagaaact ggtggacagc acagacaagg cggacctccg gctcatctac cttgccctcg 2700
   cgcatatgat caagttccgc ggccacttcc tcatcgaggg cgacctgaac ccggacaact 2760
   ccgacgtgga caagctgttc atccagctcg tgcagacgta caatcaactg ttcgaggaga 2820
   accccataaa cgctagcggc gtggacgcca aggccatcct ctcggccagg ctctcgaaat 2880
   caagaaggct ggagaacctt atcgcgcagt tgccaggcga aaagaagaac ggcctcttcg 2940
   gcaaccttat tgcgctcagc ctcggcctga cgccgaactt caaatcaaac ttcgacctcg 3000
   cggaggacgc caagctccag ctctcaaagg acacctacga cgacgacctc gacaacctcc 3060
   tggcccagat aggagaccag tacgcggacc tcttcctcgc cgccaagaac ctctccgacg 3120
   ctatcctgct cagcgacatc cttcgggtca acaccgaaat taccaaggca ccgctgtccg 3180
   ccagcatgat taaacgctac gacgagcacc atcaggacct cacgctgctc aaggcactcg 3240
   tccgccagca gctccccgag aagtacaagg agatcttctt cgaccaatca aaaaacggct 3300
   acgcgggata tatcgacggc ggtgccagcc aggaagagtt ctacaagttc atcaaaccaa 3360
   tcctggagaa gatggacggc accgaggagt tgctggtcaa gctcaacagg gaggacctcc 3420
   tcaggaagca gaggaccttc gacaacggct ccatcccgca tcagatccac ctgggcgaac 3480
   tgcatgccat cctgcggcgc caggaggact tctacccgtt cctgaaggat aaccgggaga 3540
   agatcgagaa gatcttgacg ttccgcatcc catactacgt gggcccgctg gctcgcggca 3600
   actcccggtt cgcctggatg acccggaagt cggaggagac catcacaccc tggaactttg 3660
   aggaggtggt cgataagggc gctagcgctc agagcttcat cgagcgcatg accaacttcg 3720
   ataaaaacct gcccaatgaa aaagtcctcc ccaagcactc gctgctctac gagtacttca 3780
   ccgtgtacaa cgagctcacc aaggtcaaat acgtcaccga gggcatgcgg aagccggcgt 3840
   tcctgagcgg cgagcagaag aaggcgatag tggacctcct cttcaagacc aacaggaagg 3900
   tgaccgtgaa gcaattaaaa gaggactact tcaagaaaat agagtgcttc gactccgtgg 3960
   agatctcggg cgtggaggat cggttcaacg cctcactcgg cacgtatcac gacctcctca 4020
   agatcattaa agacaaggac ttcctcgaca acgaggagaa cgaggacatc ctcgaggaca 4080
   tcgtcctcac cctgaccctg ttcgaggacc gcgaaatgat cgaggagagg ctgaagacct 4140
   acgcgcacct gttcgacgac aaggtcatga aacagctcaa gaggcgccgc tacactggtt 4200
   ggggaaggct gtcccgcaag ctcattaatg gcatcaggga caagcagagc ggcaagacca 4260
   tcctggactt cctcaagtcc gacgggttcg ccaaccgcaa cttcatgcag ctcattcacg 4320
   acgactcgct cacgttcaag gaagacatcc agaaggcaca ggtgagcggg cagggtgact 4380
   ccctccacga acacatcgcc aacctggccg gctcgccggc cattaaaaag ggcatcctgc 4440
   agacggtcaa ggtcgtcgac gagctcgtga aggtgatggg ccggcacaag cccgaaaata 4500
   tcgtcataga gatggccagg gagaaccaga ccacccaaaa agggcagaag aactcgcgcg 4560
   agcggatgaa acggatcgag gagggcatta aagagctcgg gtcccagatc ctgaaggagc 4620
   accccgtgga aaatacccag ctccagaatg aaaagctcta cctctactac ctgcagaacg 4680
   gccgcgacat gtacgtggac caggagctgg acattaatcg gctatcggac tacgacgtcg 4740
   accacatcgt gccgcagtcg ttcctcaagg acgatagcat cgacaacaag gtgctcaccc 4800
   ggtcggataa aaatcggggc aagagcgaca acgtgcccag cgaggaggtc gtgaagaaga 4860
   tgaaaaacta ctggcgccag ctcctcaacg cgaaactgat cacccagcgc aagttcgaca 4920
   acctgacgaa ggcggaacgc ggtggcttga gcgaactcga taaggcgggc ttcataaaaa 4980
   ggcagctggt cgagacgcgc cagatcacga agcatgtcgc ccagatcctg gacagccgca 5040
   tgaatactaa gtacgatgaa aacgacaagc tgatccggga ggtgaaggtg atcacgctga 5100
   agtccaagct cgtgtcggac ttccgcaagg acttccagtt ctacaaggtc cgcgagatca 5160
   acaactacca ccacgcccac gacgcctacc tgaatgcggt ggtcgggacc gccctgatca 5220
   agaagtaccc gaagctggag tcggagttcg tgtacggcga ctacaaggtc tacgacgtgc 5280
   gcaaaatgat cgccaagtcc gagcaggaga tcggcaaggc cacggcaaaa tacttcttct 5340
   actcgaacat catgaacttc ttcaagaccg agatcaccct cgcgaacggc gagatccgca 5400
   agcgcccgct catcgaaacc aacggcgaga cgggcgagat cgtctgggat aagggccggg 5460
   atttcgcgac ggtccgcaag gtgctctcca tgccgcaagt caatatcgtg aaaaagacgg 5520
   aggtccagac gggcgggttc agcaaggagt ccatcctccc gaagcgcaac tccgacaagc 5580
   tcatcgcgag gaagaaggat tgggacccga aaaaatatgg cggcttcgac agcccgaccg 5640
   tcgcatacag cgtcctcgtc gtggcgaagg tggagaaggg caagtcaaag aagctcaagt 5700
   ccgtgaagga gctgctcggg atcacgatta tggagcggtc ctccttcgag aagaacccga 5760
   tcgacttcct agaggccaag ggatataagg aggtcaagaa ggacctgatt attaaactgc 5820
   cgaagtactc gctcttcgag ctggaaaacg gccgcaagag gatgctcgcc tccgcaggcg 5880
   agttgcagaa gggcaacgag ctcgccctcc cgagcaaata cgtcaatttc ctgtacctcg 5940
   ctagccacta tgaaaagctc aagggcagcc cggaggacaa cgagcagaag cagctcttcg 6000
   tggagcagca caagcattac ctggacgaga tcatcgagca gatcagcgag ttctcgaagc 6060
   gggtgatcct cgccgacgcg aacctggaca aggtgctgtc ggcatataac aagcaccgcg 6120
   acaaaccaat acgcgagcag gccgaaaata tcatccacct cttcaccctc accaacctcg 6180
   gcgctccggc agccttcaag tacttcgaca ccacgattga ccggaagcgg tacacgagca 6240
   cgaaggaggt gctcgatgcg acgctgatcc accagagcat cacagggctc tatgaaacac 6300
   gcatcgacct gagccagctg ggcggagaca agagaccacg ggaccgccac gatggcgagc 6360
   tgggaggccg caagcgggca aggtaggtac cgttaaccta gacttgtcca tcttctggat 6420
   tggccaactt aattaatgta tgaaataaaa ggatgcacac atagtgacat gctaatcact 6480
   ataatgtggg catcaaagtt gtgtgttatg tgtaattact agttatctga ataaaagaga 6540
   aagagatcat ccatatttct tatcctaaat gaatgtcacg tgtctttata attctttgat 6600
   gaaccagatg catttcatta accaaatcca tatacatata aatattaatc atatataatt 6660
   aatatcaatt gggttagcaa aacaaatcta gtctaggtgt gttttgcgaa tgcggcc    6717
<210> 6
   <211> 4437
   <212> DNA
   <213> Artificial sequence
<220>
   <223> Lig-CR3 guide RNA expression vector
<400> 6
<210> 7
   <211> 27
   <212> DNA
   <213> Zea mays
<400> 7
   gtactccatc cgccccatcg agtaggg 27
<210> 8
   <211> 24
   <212> DNA
   <213> Zea mays
<400> 8
   gcacgtacgt caccatcccg ccgg 24
<210> 9
   <211> 24
   <212> DNA
   <213> Zea mays
<400> 9
   gacgtacgtg ccctactcga tggg 24
<210> 10
   <211> 24
   <212> DNA
   <213> Zea mays
<400> 10
   gtaccgtacg tgccccggcg gagg 24
<210> 11
   <211> 24
   <212> DNA
   <213> Zea mays
<400> 11
   ggaattgtac cgtacgtgcc ccgg 24
<210> 12
   <211> 20
   <212> DNA
   <213> Zea mays
<400> 12
   gcgtacgcgt acgtgtgagg 20
<210> 13
   <211> 22
   <212> DNA
   <213> Zea mays
<400> 13
   gctggccgag gtcgactacc gg 22
<210> 14
   <211> 23
   <212> DNA
   <213> Zea mays
<400> 14
   ggccgaggtc gactaccggc cgg 23
<210> 15
   <211> 23
   <212> DNA
   <213> Zea mays
<400> 15
   ggcgcgagct cgtgcttcac cgg 23
<210> 16
   <211> 21
   <212> DNA
   <213> Zea mays
<400> 16
   ggtgccaatc atgcgtcgcg g 21
<210> 17
   <211> 20
   <212> DNA
   <213> Zea mays
<400> 17
   ggtcgccatc acgggacagg 20
<210> 18
   <211> 24
   <212> DNA
   <213> Zea mays
<400> 18
   gtcgcggcac ctgtcccgtg atgg 24
<210> 19
   <211> 56
   <212> DNA
   <213> Artificial Sequence
<220>
   <223> MS26Cas-1 forward primer
<400> 19
   ctacactctt tccctacacg acgctcttcc gatctaggac cggaagctcg ccgcgt 56
<210> 20
   <211> 54
   <212> DNA
   <213> Artificial Sequence
<220>
   <223> MS26Cas-1 and MS26Cas-3 reverse primer
<400> 20
   caagcagaag acggcatacg agctcttccg atcttcctgg aggacgacgt gctg 54
<210> 21
   <211> 59
   <212> DNA
   <213> Artificial Sequence
<220>
   <223> MS26Cas-2 forward primer
<400> 21
   ctacactctt tccctacacg acgctcttcc gatctaaggt cctggaggac gacgtgctg 59
<210> 22
   <211> 51
   <212> DNA
   <213> Artificial Sequence
<220>
   <223> MS26Cas-2 reverse primer
<400> 22
   caagcagaag acggcatacg agctcttccg atctccggaa gctcgccgcg t 51
<210> 23
   <211> 56
   <212> DNA
   <213> Artificial Sequence
<220>
   <223> MS26Cas-3 forward primer
<400> 23
   ctacactctt tccctacacg acgctcttcc gatcttcctc cggaagctcg ccgcgt 56
<210> 24
   <211> 63
   <212> DNA
   <213> Artificial Sequence
<220>
   <223> LIGCas-1 forward primer
<400> 24
   ctacactctt tccctacacg acgctcttcc gatctaggac tgtaacgatt tacgcacctg 60
   ctg 63
<210> 25
   <211> 58
   <212> DNA
   <213> Artificial Sequence
<220>
   <223> LIGCas-1 and LIGCas-2 reverse primer
<400> 25
   caagcagaag acggcatacg agctcttccg atctgcaaat gagtagcagc gcacgtat 58
<210> 26
   <211> 63
   <212> DNA
   <213> Artificial Sequence
<220>
   <223> LIGCas-2 forward primer
<400> 26
   ctacactctt tccctacacg acgctcttcc gatcttcctc tgtaacgatt tacgcacctg 60
   ctg 63
<210> 27
   <211> 60
   <212> DNA
   <213> Artificial Sequence
<220>
   <223> LIGCas-3 forward primer
<400> 27
   ctacactctt tccctacacg acgctcttcc gatctaaggc gcaaatgagt agcagcgcac 60
<210> 28
   <211> 57
   <212> DNA
   <213> Artificial Sequence
<220>
   <223> LIGCas-3 reverse primer
<400> 28
   caagcagaag acggcatacg agctcttccg atctcacctg ctgggaattg taccgta 57
<210> 29
   <211> 58
   <212> DNA
   <213> Artificial Sequence
<220>
   <223> MS45Cas-1 forward primer
<400> 29
   ctacactctt tccctacacg acgctcttcc gatctaggag gacccgttcg gcctcagt 58
<210> 30
   <211> 54
   <212> DNA
   <213> Artificial Sequence
<220>
   <223> S45Cas-1, MS45Cas-2 and MS45Cas-3 reverse primer
<400> 30
   caagcagaag acggcatacg agctcttccg atctgccggc tggcattgtc tctg 54
<210> 31
   <211> 58
   <212> DNA
   <213> Artificial Sequence
<220>
   <223> MS45Cas-2 forward primer
<400> 31
   ctacactctt tccctacacg acgctcttcc gatcttcctg gacccgttcg gcctcagt 58
<210> 32
   <211> 58
   <212> DNA
   <213> Artificial Sequence
<220>
   <223> MS45Cas-3 forward primer
<400> 32
   ctacactctt tccctacacg acgctcttcc gatctgaagg gacccgttcg gcctcagt 58
<210> 33
   <211> 58
   <212> DNA
   <213> Artificial Sequence
<220>
   <223> ALSCas-1 forward primer
<400> 33
   ctacactctt tccctacacg acgctcttcc gatctaaggc gacgatgggc gtctcctg 58
<210> 34
   <211> 53
   <212> DNA
   <213> Artificial Sequence
<220>
   <223> ALSCas-1, ALSCas-2 and ALSCas-3 reverse primer
<400> 34
   caagcagaag acggcatacg agctcttccg atctgcgtct gcatcgccac ctc 53
<210> 35
   <211> 58
   <212> DNA
   <213> Artificial Sequence
<220>
   <223> ALSCas-2 forward primer
<400> 35
   ctacactctt tccctacacg acgctcttcc gatctttccc gacgatgggc gtctcctg 58
<210> 36
   <211> 58
   <212> DNA
   <213> Artificial Sequence
<220>
   <223> ALSCas-3 forward primer
<400> 36
   ctacactctt tccctacacg acgctcttcc gatctggaac gacgatgggc gtctcctg 58
<210> 37
   <211> 43
   <212> DNA
   <213> Artificial Sequence
<220>
   <223> forward primer for secondary PCR
<400> 37
   aatgatacgg cgaccaccga gatctacact ctttccctac acg 43
<210> 38
   <211> 18
   <212> DNA
   <213> Artificial Sequence
<220>
   <223> reverse primer for secondary PCR;
<400> 38
   caagcagaag acggcata 18
<210> 39
   <211> 1910
   <212> DNA
   <213> Zea mays
<220>
   <221> misc_feature
   <222> (1)..(1910)
   <223> ALS1-DNA sequence
<400> 39
   atggccaccg ccgccaccgc ggccgccgcg ctcaccggcg ccactaccgc tacgcccaag      60
   tcgaggcgcc gagcccacca cttggccacc cggcgcgccc tcgccgcgcc catcaggtgc     120
   tcagcgttgt cacgcgccac gccgacggct cccccggcca ctccgctacg tccgtggggc     180
   cccaacgagc cccgcaaggg ctccgacatc ctcgtcgagg ctctcgagcg ctgtggcgtc     240
   cgtgacgtct tcgcctaccc cggcggcgca tccatggaga tccaccaggc actcacccgc     300
   tcccccgtca tcgccaacca cctcttccgc cacgaacaag ggaggccttt cgccgcctcc     360
   ggctacgcgc gctcctcggg ccgcgttggc gtctgcatcg ccacctccgg ccccggcgcc     420
   accaacctag tctctgcgct cgcagacgcg ttgctcgact ccgtccccat tgtcgccatc     480
   acgggacagg tgccgcgacg catgattggc accgacgcct ttcaggagac gcccatcgtc     540
   gaggtcaccc gctccatcac caagcacaac tacctggtcc tcgacgtcga cgacatcccc     600
   cgcgtcgtgc aggaggcctt cttcctcgca tcctctggtc gcccggggcc ggtgcttgtt     660
   gacatcccca aggacatcca gcagcagatg gcggtgccgg cctgggacac gcccatgagt     720
   ctgcctgggt acatcgcgcg ccttcccaag cctcccgcga ctgaatttct tgagcaggtg     780
   ctgcgtcttg ttggtgaatc acggcgccct gttctttatg ttggcggtgg ctgtgcagca     840
   tcaggtgagg agttgtgccg ctttgtggag ttgactggaa tcccagtcac aactactctt     900
   atgggccttg gcaacttccc cagcgacgac ccactgtcac tgcgcatgct tggtatgcat     960
   ggcacagtgt atgcaaatta tgcagtggat aaggccgatc tgttgcttgc atttggtgtg    1020
   cggtttgatg atcgtgtgac agggaaaatt gaggcttttg caggcagagc taagattgtg    1080
   cacattgata ttgatcctgc tgagattggc aagaacaagc agccacatgt gtccatctgt    1140
   gcagatgtta agcttgcttt gcagggcatg aatactcttc tggaaggaag cacatcaaag    1200
   aagagctttg acttcggctc atggcatgat gaattggatc agcaaaagcg ggagtttccc    1260
   cttgggtata aaatcttcaa tgaggaaatc cagccacaat atgctattca ggttcttgat    1320
   gagttgacga aggggaaggc catcattgcc acaggtgttg ggcagcacca gatgtgggcg    1380
   gcacagtatt acacttacaa gcggccaagg cagtggctgt cttcagctgg tcttggggct    1440
   atgggatttg gtttgccggc tgctgctggt gctgctgtgg ccaacccagg tgtcactgtt    1500
   gttgacatcg acggagatgg tagcttcctc atgaacattc aggagctagc tatgatccgt    1560
   attgagaacc tcccagtcaa ggtctttgtg ctaaacaacc agcacctcgg gatggtggtg    1620
   cagtgggagg acaggttcta taaggccaat agagcacaca cattcttggg aaacccagag    1680
   aacgaaagtg agatatatcc agattttgtg gcaattgcca aagggttcaa cattccagca    1740
   gtccgtgtga caaagaagag cgaagtccat gcagcaatca agaagatgct tgaggctcca    1800
   gggccgtacc tcttggatat aatcgtcccg caccaggagc atgtgttgcc tatgatccct    1860
   agtggtgggg ctttcaagga tatgatcctg gatggtgatg gcaggactgt               1910
<210> 40
   <211> 1910
   <212> DNA
   <213> Zea mays
<220>
   <221> misc_feature
   <222> (1)..(1910)
   <223> ALS2-DNA sequence
<400> 40
   atggccaccg ccgccgccgc gtctaccgcg ctcactggcg ccactaccgc tgcgcccaag      60
   gcgaggcgcc gggcgcacct cctggccacc cgccgcgccc tcgccgcgcc catcaggtgc     120
   tcagcggcgt cacccgccat gccgatggct cccccggcca ccccgctccg gccgtggggc     180
   cccaccgatc cccgcaaggg cgccgacatc ctcgtcgagt ccctcgagcg ctgcggcgtc     240
   cgcgacgtct tcgcctaccc cggcggcgcg tccatggaga tccaccaggc actcacccgc     300
   tcccccgtca tcgccaacca cctcttccgc cacgagcaag gggaggcctt tgcggcctcc     360
   ggctacgcgc gctcctcggg ccgcgtcggc gtctgcatcg ccacctccgg ccccggcgcc     420
   accaaccttg tctccgcgct cgccgacgcg ctgctcgatt ccgtccccat ggtcgccatc     480
   acgggacagg tgccgcgacg catgattggc accgacgcct tccaggagac gcccatcgtc     540
   gaggtcaccc gctccatcac caagcacaac tacctggtcc tcgacgtcga cgacatcccc     600
   cgcgtcgtgc aggaggcttt cttcctcgcc tcctctggtc gaccggggcc ggtgcttgtc     660
   gacatcccca aggacatcca gcagcagatg gcggtgcctg tctgggacaa gcccatgagt     720
   ctgcctgggt acattgcgcg ccttcccaag ccccctgcga ctgagttgct tgagcaggtg     780
   ctgcgtcttg ttggtgaatc ccggcgccct gttctttatg ttggcggtgg ctgcgcagca     840
   tctggtgagg agttgcgacg ctttgtggag ctgactggaa tcccggtcac aactactctt     900
   atgggcctcg gcaacttccc cagcgacgac ccactgtctc tgcgcatgct aggtatgcat     960
   ggcacggtgt atgcaaatta tgcagtggat aaggccgatc tgttgcttgc acttggtgtg    1020
   cggtttgatg atcgtgtgac agggaagatt gaggcttttg caagcagggc taagattgtg    1080
   cacgttgata ttgatccggc tgagattggc aagaacaagc agccacatgt gtccatctgt    1140
   gcagatgtta agcttgcttt gcagggcatg aatgctcttc ttgaaggaag cacatcaaag    1200
   aagagctttg actttggctc atggaacgat gagttggatc agcagaagag ggaattcccc    1260
   cttgggtata aaacatctaa tgaggagatc cagccacaat atgctattca ggttcttgat    1320
   gagctgacga aaggcgaggc catcatcggc acaggtgttg ggcagcacca gatgtgggcg    1380
   gcacagtact acacttacaa gcggccaagg cagtggttgt cttcagctgg tcttggggct    1440
   atgggatttg gtttgccggc tgctgctggt gcttctgtgg ccaacccagg tgttactgtt    1500
   gttgacatcg atggagatgg tagctttctc atgaacgttc aggagctagc tatgatccga    1560
   attgagaacc tcccggtgaa ggtctttgtg ctaaacaacc agcacctggg gatggtggtg    1620
   cagtgggagg acaggttcta taaggccaac agagcgcaca catacttggg aaacccagag    1680
   aatgaaagtg agatatatcc agatttcgtg acgatcgcca aagggttcaa cattccagcg    1740
   gtccgtgtga caaagaagaa cgaagtccgc gcagcgataa agaagatgct cgagactcca    1800
   gggccgtacc tcttggatat aatcgtccca caccaggagc atgtgttgcc tatgatccct    1860
   agtggtgggg ctttcaagga tatgatcctg gatggtgatg gcaggactgt                1910
<210> 41
   <211> 638
   <212> PRT
   <213> Zea mays
<220>
   <221> MISC_FEATURE
   <222> (1)..(638)
   <223> full length Zm-ALS2 protein
<400> 41
   Met Ala Thr Ala Ala Ala Ala Ser Thr Ala Leu Thr Gly Ala Thr Thr
   1               5                   10                  15
   Ala Ala Pro Lys Ala Arg Arg Arg Ala His Leu Leu Ala Thr Arg Arg
               20                  25                  30
   Ala Leu Ala Ala Pro Ile Arg Cys Ser Ala Ala Ser Pro Ala Met Pro
           35                  40                  45
   Met Ala Pro Pro Ala Thr Pro Leu Arg Pro Trp Gly Pro Thr Glu Pro
       50                  55                  60
   Arg Lys Gly Ala Asp Ile Leu Val Glu Ser Leu Glu Arg Cys Gly Val
   65                  70                  75                  80
   Arg Asp Val Phe Ala Tyr Pro Gly Gly Ala Ser Met Glu Ile His Gln
                   85                  90                  95
   Ala Leu Thr Arg Ser Pro Val Ile Ala Asn His Leu Phe Arg His Glu
               100                 105                 110
   Gln Gly Glu Ala Phe Ala Ala Ser Gly Tyr Ala Arg Ser Ser Gly Arg
           115                 120                 125
   Val Gly Val Cys Ile Ala Thr Ser Gly Pro Gly Ala Thr Asn Leu Val
       130                 135                 140
   Ser Ala Leu Ala Asp Ala Leu Leu Asp Ser Val Pro Met Val Ala Ile
   145                 150                 155                 160
   Thr Gly Gln Val Pro Arg Arg Met Ile Gly Thr Asp Ala Phe Gln Glu
                   165                 170                 175
   Thr Pro Ile Val Glu Val Thr Arg Ser Ile Thr Lys His Asn Tyr Leu
               180                 185                 190
   Val Leu Asp Val Asp Asp Ile Pro Arg Val Val Gln Glu Ala Phe Phe
           195                 200                 205
   Leu Ala Ser Ser Gly Arg Pro Gly Pro Val Leu Val Asp Ile Pro Lys
       210                 215                 220
   Asp Ile Gln Gln Gln Met Ala Val Pro Val Trp Asp Lys Pro Met Ser
   225                 230                 235                 240
   Leu Pro Gly Tyr Ile Ala Arg Leu Pro Lys Pro Pro Ala Thr Glu Leu
                   245                 250                 255
   Leu Glu Gln Val Leu Arg Leu Val Gly Glu Ser Arg Arg Pro Val Leu
               260                 265                 270
   Tyr Val Gly Gly Gly Cys Ala Ala Ser Gly Glu Glu Leu Arg Arg Phe
           275                 280                 285
   Val Glu Leu Thr Gly Ile Pro Val Thr Thr Thr Leu Met Gly Leu Gly
       290                 295                 300
   Asn Phe Pro Ser Asp Asp Pro Leu Ser Leu Arg Met Leu Gly Met His
   305                 310                 315                 320
   Gly Thr Val Tyr Ala Asn Tyr Ala Val Asp Lys Ala Asp Leu Leu Leu
                   325                 330                 335
   Ala Leu Gly Val Arg Phe Asp Asp Arg Val Thr Gly Lys Ile Glu Ala
               340                 345                 350
   Phe Ala Ser Arg Ala Lys Ile Val His Val Asp Ile Asp Pro Ala Glu
           355                 360                 365
   Ile Gly Lys Asn Lys Gln Pro His Val Ser Ile Cys Ala Asp Val Lys
       370                 375                 380
   Leu Ala Leu Gln Gly Met Asn Ala Leu Leu Glu Gly Ser Thr Ser Lys
   385                 390                 395                 400
   Lys Ser Phe Asp Phe Gly Ser Trp Asn Asp Glu Leu Asp Gln Gln Lys
                   405                 410                 415
   Arg Glu Phe Pro Leu Gly Tyr Lys Thr Ser Asn Glu Glu Ile Gln Pro
               420                 425                 430
   Gln Tyr Ala Ile Gln Val Leu Asp Glu Leu Thr Lys Gly Glu Ala Ile
           435                 440                 445
   Ile Gly Thr Gly Val Gly Gln His Gln Met Trp Ala Ala Gln Tyr Tyr
       450                 455                 460
   Thr Tyr Lys Arg Pro Arg Gln Trp Leu Ser Ser Ala Gly Leu Gly Ala
   465                 470                 475                 480
   Met Gly Phe Gly Leu Pro Ala Ala Ala Gly Ala Ser Val Ala Asn Pro
                   485                 490                 495
   Gly Val Thr Val Val Asp Ile Asp Gly Asp Gly Ser Phe Leu Met Asn
               500                 505                 510
   Val Gln Glu Leu Ala Met Ile Arg Ile Glu Asn Leu Pro Val Lys Val
               515                 520                 525
   Phe Val Leu Asn Asn Gln His Leu Gly Met Val Val Gln Trp Glu Asp
           530                 535                 540
   Arg Phe Tyr Lys Ala Asn Arg Ala His Thr Tyr Leu Gly Asn Pro Glu
   545                 550                 555                 560
   Asn Glu Ser Glu Ile Tyr Pro Asp Phe Val Thr Ile Ala Lys Gly Phe
                   565                 570                 575
   Asn Ile Pro Ala Val Arg Val Thr Lys Lys Asn Glu Val Arg Ala Ala
               580                 585                 590
   Ile Lys Lys Met Leu Glu Thr Pro Gly Pro Tyr Leu Leu Asp Ile Ile
           595                 600                 605
   Val Pro His Gln Glu His Val Leu Pro Met Ile Pro Ser Gly Gly Ala
       610                 615                 620
   Phe Lys Asp Met Ile Leu Asp Gly Asp Gly Arg Thr Val Tyr
   625                 630                 635
<210> 42
   <211> 23
   <212> DNA
   <213> Zea mays
<400> 42
   gctgctcgat tccgtcccca tgg 23
<210> 43
   <211> 794
   <212> DNA
   <213> Artificial sequence
<220>
   <223> 794 bp polynucleotide modification template
<400> 43
   ttctgctcaa gcaactcagt cgcagggggc ttgggaaggc gcgcaatgta cccaggcaga      60
   ctcatgggct tgtcccagac aggcaccgcc atctgctgct ggatgtcctt ggggatgtcg     120
   acaagcaccg gccctggtcg accagaggag gcgaggaaga aagcctcctg cacgacgcgg     180
   gggatgtcgt cgacgtcgag gaccaggtag ttgtgcttgg tgatggagcg ggtgacctcg     240
   acgatgggcg tctcctggaa ggcgtcggtg ccaatcatgc gtcgcgacac ttggccggta     300
   atcgccacca tggggacgga atcgagcagc gcgtcggcga gcgcggagac aaggttggtg     360
   gcgccggggc cggaggtggc gatgcagacg ccgacgcggc ccgaggagcg cgcgtagccg     420
   gaggccgcaa aggcctcccc ttgctcgtgg cggaagaggt ggttggcgat gacgggggag     480
   cgggtgagtg cctggtggat ctccatggac gcgccgccgg ggtaggcgaa gacgtcgcgg     540
   acgccgcagc gctcgaggga ctcgacgagg atgtcggcgc ccttgcgggg atcggtgggg     600
   ccccacggcc ggagcggggt ggccggggga gccatcggca tggcgggtga cgccgctgag      660
   cacctgatgg gcgcggcgag ggcgcggcgg gtggccagga ggtgcgcccg gcgcctcgcc     720
   ttgggcgcag cggtagtggc gccagtgagc gcggtagacg cggcggcggc ggtggccatg     780
   gttgcggcgg ctgt                                                       794
<210> 44
   <211> 127
   <212> DNA
   <213> Artificial sequence
<220>
   <223> 127 bp polynucleotide modification template oligo -1
<400> 44
   aaccttgtct ccgcgctcgc cgacgcgttg ctcgactccg tccccattgt cgccatcacg       60
   ggacaggtgt cgcgacgcat gattggcacc gacgccttcc aggagacgcc catcgtcgag      120
   gtcaccc                                                                127
<210> 45
   <211> 127
   <212> DNA
   <213> artificial sequence
<220>
   <223> 127 bp polynucleotide modification template oligo-2
<400> 45
   aaccttgtct ccgcgctcgc cgacgcgttg ctggactccg tgccgatggt cgccatcacg       60
   ggacaggtgt cccgacgcat gattggcacc gacgccttcc aggagacgcc catcgtcgag      120
   gtcaccc                                                                127
<210> 46
   <211> 56493
   <212> DNA
   <213> Artificial sequence
<220>
   <223> Agrobacterium vector containing maize codon optimized Cas9 and maize UBI promoter
<400> 46
<210> 47
   <211> 55518
   <212> DNA
   <213> Artificial sequence
<220>
   <223> Agrobacterium vector containing maize codon optimized Cas9 and maize MDH promoter
<400> 47
   gtcggatcac cggaaaggac ccgtaaagtg ataatgatta tcatctacat atcacaacgt 60
   gcgtggaggc catcaaacca cgtcaaataa tcaattatga cgcaggtatc gtattaattg 120
   atctgcatca acttaacgta aaaacaactt cagacaatac aaatcagcga cactgaatac 180
   ggggcaacct catgtccccc cccccccccc ccctgcaggc atcgtggtgt cacgctcgtc 240
   gtttggtatg gcttcattca gctccggttc ccaacgatca aggcgagtta catgatcccc 300
   catgttgtgc aaaaaagcgg ttagctcctt cggtcctccg atcgttgtca gaagtaagtt 360
   ggccgcagtg ttatcactca tggttatggc agcactgcat aattctctta ctgtcatgcc 420
   atccgtaaga tgcttttctg tgactggtga gtactcaacc aagtcattct gagaatagtg 480
   tatgcggcga ccgagttgct cttgcccggc gtcaacacgg gataataccg cgccacatag 540
   cagaacttta aaagtgctca tcattggaaa acgttcttcg gggcgaaaac tctcaaggat 600
   cttaccgctg ttgagatcca gttcgatgta acccactcgt gcacccaact gatcttcagc 660
   atcttttact ttcaccagcg tttctgggtg agcaaaaaca ggaaggcaaa atgccgcaaa 720
   aaagggaata agggcgacac ggaaatgttg aatactcata ctcttccttt ttcaatatta 780
   ttgaagcatt tatcagggtt attgtctcat gagcggatac atatttgaat gtatttagaa 840
   aaataaacaa ataggggttc cgcgcacatt tccccgaaaa gtgccacctg acgtctaaga 900
   aaccattatt atcatgacat taacctataa aaataggcgt atcacgaggc cctttcgtct 960
   tcaagaattg gtcgacgatc ttgctgcgtt cggatatttt cgtggagttc ccgccacaga 1020
   cccggattga aggcgagatc cagcaactcg cgccagatca tcctgtgacg gaactttggc 1080
   gcgtgatgac tggccaggac gtcggccgaa agagcgacaa gcagatcacg cttttcgaca 1140
   gcgtcggatt tgcgatcgag gatttttcgg cgctgcgcta cgtccgcgac cgcgttgagg 1200
   gatcaagcca cagcagccca ctcgaccttc tagccgaccc agacgagcca agggatcttt 1260
   ttggaatgct gctccgtcgt caggctttcc gacgtttggg tggttgaaca gaagtcatta 1320
   tcgtacggaa tgccaagcac tcccgagggg aaccctgtgg ttggcatgca catacaaatg 1380
   gacgaacgga taaacctttt cacgcccttt taaatatccg ttattctaat aaacgctctt 1440
   ttctcttagg tttacccgcc aatatatcct gtcaaacact gatagtttaa actgaaggcg 1500
   ggaaacgaca atctgatcat gagcggagaa ttaagggagt cacgttatga cccccgccga 1560
   tgacgcggga caagccgttt tacgtttgga actgacagaa ccgcaacgtt gaaggagcca 1620
   ctcagcccaa gctggtacga ttgtaatacg actcactata gggcgaattg agcgctgttt 1680
   aaacgctctt caactggaag agcggttacc agaggccaga atggccatct cggaccgata 1740
   tcgctatcaa ctttgtatag aaaagttggg ccgaattcga gctcggtacg gccagaatgg 1800
   cccggaccgg gttaccgaat tcgagctcgg taccctggga tccgatatcg atgggccctg 1860
   gccgaagctt tttggaaggc taaggagagg aagccggcga gaaggagggg gcgttttacg 1920
   tgtcactgtc ctgtcgtgtt ggctgttgac acgaatcatt tcttccgcgc gtgggaagaa 1980
   gaagatgcac attagcggcc tgaagtagag atgtcaatgg ggaattcccc agcggggatt 2040
   aactccccag acccgtaccc atgaacatag accggccccc atccccgaac ccgaacccga 2100
   cctcgggtac gaaaatcctc ccatacccat tcccgaccgg gtactaaata cccatgggta 2160
   tccatacccg acccgattat tcaaaaatta atgggctttt tatttgttaa ccggcggacg 2220
   caatgcttgg gactctaggt ttttttactt tgttgaccgg ctggcggctg ggctttttcc 2280
   tacaggccca aagttggtcg gcagccacta ggccacacgt cacaggcagc ccacaagtaa 2340
   atgtcgttgg attgctggat ggtggaataa aaatcctaga tgctagattg ttctggttcc 2400
   gggtattttt ctccatggct aatcgggttt gggtttagcc ctcccaaacc cgaacccgcc 2460
   atacccgatg ggtaagggat ttattccaaa tctataccca tggggatttg ttttaaccca 2520
   taccttaacc ctaatagagg aattccccac gggtaatcgg gtttcggggc ccattgacat 2580
   ctctagactg aaggcgtcca actcaaatca ttaaaaagtg ttgacgcacg cgctgatgcg 2640
   ccggccgcac agcacaggct gcacagcccg tttaatcagc gatggagccc cggccgtcag 2700
   ccagccaggt ccggcgtccg ggtctgcgcc ctgcggcgtc actgctgtcg ccaccgtctc 2760
   cgatggtccc acatccatcc agcgggccgc gcgtggtaca aaaggctctt cctcgccgtc 2820
   aggtgcagct gcccaaacac cagacacaga ctccaccacc ccgcttcgat cttctgttgc 2880
   agctgaaatc tgtcagattc tgcagttcat tcctcatggc accgaagaag aagcgcaagg 2940
   tgatggacaa gaagtacagc atcggcctcg acatcggcac caactcggtg ggctgggccg 3000
   tcatcacgga cgaatataag gtcccgtcga agaagttcaa ggtcctcggc aatacagacc 3060
   gccacagcat caagaaaaac ttgatcggcg ccctcctgtt cgatagcggc gagaccgcgg 3120
   aggcgaccag gctcaagagg accgccagga gacggtacac taggcgcaag aacaggatct 3180
   gctacctgca ggagatcttc agcaacgaga tggcgaaggt ggacgactcc ttcttccacc 3240
   gcctggagga atcattcctg gtggaggagg acaagaagca tgagcggcac ccaatcttcg 3300
   gcaacatcgt cgacgaggta agtttctgct tctacctttg atatatatat aataattatc 3360
   attaattagt agtaatataa tatttcaaat atttttttca aaataaaaga atgtagtata 3420
   tagcaattgc ttttctgtag tttataagtg tgtatatttt aatttataac ttttctaata 3480
   tatgaccaaa acatggtgat gtgcaggtgg cctaccacga gaagtacccg acaatctacc 3540
   acctccggaa gaaactggtg gacagcacag acaaggcgga cctccggctc atctaccttg 3600
   ccctcgcgca tatgatcaag ttccgcggcc acttcctcat cgagggcgac ctgaacccgg 3660
   acaactccga cgtggacaag ctgttcatcc agctcgtgca gacgtacaat caactgttcg 3720
   aggagaaccc cataaacgct agcggcgtgg acgccaaggc catcctctcg gccaggctct 3780
   cgaaatcaag aaggctggag aaccttatcg cgcagttgcc aggcgaaaag aagaacggcc 3840
   tcttcggcaa ccttattgcg ctcagcctcg gcctgacgcc gaacttcaaa tcaaacttcg 3900
   acctcgcgga ggacgccaag ctccagctct caaaggacac ctacgacgac gacctcgaca 3960
   acctcctggc ccagatagga gaccagtacg cggacctctt cctcgccgcc aagaacctct 4020
   ccgacgctat cctgctcagc gacatccttc gggtcaacac cgaaattacc aaggcaccgc 4080
   tgtccgccag catgattaaa cgctacgacg agcaccatca ggacctcacg ctgctcaagg 4140
   cactcgtccg ccagcagctc cccgagaagt acaaggagat cttcttcgac caatcaaaaa 4200
   acggctacgc gggatatatc gacggcggtg ccagccagga agagttctac aagttcatca 4260
   aaccaatcct ggagaagatg gacggcaccg aggagttgct ggtcaagctc aacagggagg 4320
   acctcctcag gaagcagagg accttcgaca acggctccat cccgcatcag atccacctgg 4380
   gcgaactgca tgccatcctg cggcgccagg aggacttcta cccgttcctg aaggataacc 4440
   gggagaagat cgagaagatc ttgacgttcc gcatcccata ctacgtgggc ccgctggctc 4500
   gcggcaactc ccggttcgcc tggatgaccc ggaagtcgga ggagaccatc acaccctgga 4560
   actttgagga ggtggtcgat aagggcgcta gcgctcagag cttcatcgag cgcatgacca 4620
   acttcgataa aaacctgccc aatgaaaaag tcctccccaa gcactcgctg ctctacgagt 4680
   acttcaccgt gtacaacgag ctcaccaagg tcaaatacgt caccgagggc atgcggaagc 4740
   cggcgttcct gagcggcgag cagaagaagg cgatagtgga cctcctcttc aagaccaaca 4800
   ggaaggtgac cgtgaagcaa ttaaaagagg actacttcaa gaaaatagag tgcttcgact 4860
   ccgtggagat ctcgggcgtg gaggatcggt tcaacgcctc actcggcacg tatcacgacc 4920
   tcctcaagat cattaaagac aaggacttcc tcgacaacga ggagaacgag gacatcctcg 4980
   aggacatcgt cctcaccctg accctgttcg aggaccgcga aatgatcgag gagaggctga 5040
   agacctacgc gcacctgttc gacgacaagg tcatgaaaca gctcaagagg cgccgctaca 5100
   ctggttgggg aaggctgtcc cgcaagctca ttaatggcat cagggacaag cagagcggca 5160
   agaccatcct ggacttcctc aagtccgacg ggttcgccaa ccgcaacttc atgcagctca 5220
   ttcacgacga ctcgctcacg ttcaaggaag acatccagaa ggcacaggtg agcgggcagg 5280
   gtgactccct ccacgaacac atcgccaacc tggccggctc gccggccatt aaaaagggca 5340
   tcctgcagac ggtcaaggtc gtcgacgagc tcgtgaaggt gatgggccgg cacaagcccg 5400
   aaaatatcgt catagagatg gccagggaga accagaccac ccaaaaaggg cagaagaact 5460
   cgcgcgagcg gatgaaacgg atcgaggagg gcattaaaga gctcgggtcc cagatcctga 5520
   aggagcaccc cgtggaaaat acccagctcc agaatgaaaa gctctacctc tactacctgc 5580
   agaacggccg cgacatgtac gtggaccagg agctggacat taatcggcta tcggactacg 5640
   acgtcgacca catcgtgccg cagtcgttcc tcaaggacga tagcatcgac aacaaggtgc 5700
   tcacccggtc ggataaaaat cggggcaaga gcgacaacgt gcccagcgag gaggtcgtga 5760
   agaagatgaa aaactactgg cgccagctcc tcaacgcgaa actgatcacc cagcgcaagt 5820
   tcgacaacct gacgaaggcg gaacgcggtg gcttgagcga actcgataag gcgggcttca     5880
   taaaaaggca gctggtcgag acgcgccaga tcacgaagca tgtcgcccag atcctggaca     5940
   gccgcatgaa tactaagtac gatgaaaacg acaagctgat ccgggaggtg aaggtgatca     6000
   cgctgaagtc caagctcgtg tcggacttcc gcaaggactt ccagttctac aaggtccgcg     6060
   agatcaacaa ctaccaccac gcccacgacg cctacctgaa tgcggtggtc gggaccgccc     6120
   tgatcaagaa gtacccgaag ctggagtcgg agttcgtgta cggcgactac aaggtctacg     6180
   acgtgcgcaa aatgatcgcc aagtccgagc aggagatcgg caaggccacg gcaaaatact     6240
   tcttctactc gaacatcatg aacttcttca agaccgagat caccctcgcg aacggcgaga     6300
   tccgcaagcg cccgctcatc gaaaccaacg gcgagacggg cgagatcgtc tgggataagg     6360
   gccgggattt cgcgacggtc cgcaaggtgc tctccatgcc gcaagtcaat atcgtgaaaa     6420
   agacggaggt ccagacgggc gggttcagca aggagtccat cctcccgaag cgcaactccg     6480
   acaagctcat cgcgaggaag aaggattggg acccgaaaaa atatggcggc ttcgacagcc     6540
   cgaccgtcgc atacagcgtc ctcgtcgtgg cgaaggtgga gaagggcaag tcaaagaagc     6600
   tcaagtccgt gaaggagctg ctcgggatca cgattatgga gcggtcctcc ttcgagaaga     6660
   acccgatcga cttcctagag gccaagggat ataaggaggt caagaaggac ctgattatta     6720
   aactgccgaa gtactcgctc ttcgagctgg aaaacggccg caagaggatg ctcgcctccg     6780
   caggcgagtt gcagaagggc aacgagctcg ccctcccgag caaatacgtc aatttcctgt     6840
   acctcgctag ccactatgaa aagctcaagg gcagcccgga ggacaacgag cagaagcagc     6900
   tcttcgtgga gcagcacaag cattacctgg acgagatcat cgagcagatc agcgagttct     6960
   cgaagcgggt gatcctcgcc gacgcgaacc tggacaaggt gctgtcggca tataacaagc     7020
   accgcgacaa accaatacgc gagcaggccg aaaatatcat ccacctcttc accctcacca     7080
   acctcggcgc tccggcagcc ttcaagtact tcgacaccac gattgaccgg aagcggtaca     7140
   cgagcacgaa ggaggtgctc gatgcgacgc tgatccacca gagcatcaca gggctctatg     7200
   aaacacgcat cgacctgagc cagctgggcg gagacaagag accacgggac cgccacgatg     7260
   gcgagctggg aggccgcaag cgggcaaggt aggtaccgtt aacctagact tgtccatctt     7320
   ctggattggc caacttaatt aatgtatgaa ataaaaggat gcacacatag tgacatgcta     7380
   atcactataa tgtgggcatc aaagttgtgt gttatgtgta attactagtt atctgaataa     7440
   aagagaaaga gatcatccat atttcttatc ctaaatgaat gtcacgtgtc tttataattc     7500
   tttgatgaac cagatgcatt tcattaacca aatccatata catataaata ttaatcatat     7560
   ataattaata tcaattgggt tagcaaaaca aatctagtct aggtgtgttt tgcgaatgcg     7620
   gccgccaccg cggtggagct cgaattcgag ctcggtaccc tgggatccag cttcgcttag     7680
   tttttagttt ttggcagaaa aaatgatcaa tgtttcacaa accaaatatt tttataactt     7740
   ttgatgaaag aagatcacca cggtcatatc taggggtggt aacaaattgc gatctaaatg     7800
   tttcttcata aaaaataagg cttcttaata aattttagtt caaaataaat acgaataaag     7860
   tctgattcta atctgattcg atccttaaat tttataatgc aaaatttaga gctcattacc     7920
   acctctagtc atatgtctag tctgaggtat atccaaaaag ccctttctct aaattccaca     7980
   cccaactcag atgtttgcaa ataaatactc cgactccaaa atgtaggtga agtgcaactt     8040
   tctccatttt atatcaacat ttgttatttt ttgtttaaca tttcacactc aaaactaatt     8100
   aataaaatac gtggttgttg aacgtgcgca catgtctccc ttacattatg tttttttatt     8160
   tatgtattat tgttgttttc ctccgaacaa cttgtcaaca tatcatcatt ggtctttaat     8220
   atttatgaat atggaagcct agttatttac acttggctac acactagttg tagttttgcc     8280
   acttgtctaa catgcaactc tagtagtttt gccacttgcc tggcatgcaa ctctagtatt     8340
   gacacttgta tagcatataa tgccaatacg acacctgcct tacatgaaac attatttttg     8400
   acacttgtat accatgcaac attaccattg acatttgtcc atacacatta tatcaaatat     8460
   attgagcgca tgtcacaaac tcgatacaaa gctggatgac cctccctcac cacatctata     8520
   aaaacccgag cgctactgta aatcactcac aacacaacac atatctttta gtaacctttc     8580
   aataggcgtc ccccaagaac tagtaaccat ggccctgtcc aacaagttca tcggcgacga     8640
   catgaagatg acctaccaca tggacggctg cgtgaacggc cactacttca ccgtgaaggg     8700
   cgagggcagc ggcaagccct acgagggcac ccagacctcc accttcaagg tgaccatggc     8760
   caacggcggc cccctggcct tctccttcga catcctgtcc accgtgttca tgtacggcaa     8820
   ccgctgcttc accgcctacc ccaccagcat gcccgactac ttcaagcagg ccttccccga     8880
   cggcatgtcc tacgagagaa ccttcaccta cgaggacggc ggcgtggcca ccgccagctg     8940
   ggagatcagc ctgaagggca actgcttcga gcacaagtcc accttccacg gcgtgaactt     9000
   ccccgccgac ggccccgtga tggccaagaa gaccaccggc tgggacccct ccttcgagaa     9060
   gatgaccgtg tgcgacggca tcttgaaggg cgacgtgacc gccttcctga tgctgcaggg     9120
   cggcggcaac tacagatgcc agttccacac ctcctacaag accaagaagc ccgtgaccat     9180
   gccccccaac cacgtggtgg agcaccgcat cgccagaacc gacctggaca agggcggcaa     9240
   cagcgtgcag ctgaccgagc acgccgtggc ccacatcacc tccgtggtgc ccttctgaag     9300
   cggcccatgg atattcgaac gcgtaggtac cacatggtta acctagactt gtccatcttc     9360
   tggattggcc aacttaatta atgtatgaaa taaaaggatg cacacatagt gacatgctaa     9420
   tcactataat gtgggcatca aagttgtgtg ttatgtgtaa ttactagtta tctgaataaa     9480
   agagaaagag atcatccata tttcttatcc taaatgaatg tcacgtgtct ttataattct     9540
   ttgatgaacc agatgcattt cattaaccaa atccatatac atataaatat taatcatata     9600
   taattaatat caattgggtt agcaaaacaa atctagtcta ggtgtgtttt gcgaattccc 9660
   atggacctcg agggggggcc cgggcaccca gctttcttgt acaaagtggc cgttaacgga 9720
   tcggccagaa tggcccggac cgggttaccg aattcgagct cggtaccctg ggatcggccg 9780
   ctctagaact agtggatccc ccgggctgca ggaattccca tggagtcaaa gattcaaata 9840
   gaggacctaa cagaactcgc cgtaaagact ggcgaacagt tcatacagag tctcttacga 9900
   ctcaatgaca agaagaaaat cttcgtcaac atggtggagc acgacacgct tgtctactcc 9960
   aaaaatatca aagatacagt ctcagaagac caaagggcaa ttgagacttt tcaacaaagg 10020
   gtaatatccg gaaacctcct cggattccat tgcccagcta tctgtcactt tattgtgaag 10080
   atagtggaaa aggaaggtgg ctcctacaaa tgccatcatt gcgataaagg aaaggccatc 10140
   gttgaagatg cctctgccga cagtggtccc aaagatggac ccccacccac gaggagcatc 10200
   gtggaaaaag aagacgttcc aaccacgtct tcaaagcaag tggattgatg tgatatctcc 10260
   actgacgtaa gggatgacgc acaatcccac taagcttcgg ccggggccca tcgatctggc 10320
   gaaaggggga tgtgctgcaa ggcgattaag ttgggtaacg ccagggtttt cccagtcacg 10380
   acgttgtaaa acgacggcca gtgccaagct cagatcagct tggggctggt atcgataaat 10440
   gtttccacat agattttgca tatcataatg atgtttgtcg ttccgtatct atgtttcata 10500
   caaaattttt acgcatatcg caacacatgg gcacatacct agtgactgta taactctgca 10560
   tgtatgagtg tatgactata tgatgtagta actaataaga agggtagaca tttgagtgat 10620
   tcttttattc ctggacttgt aagacttgac atttctgcct tgagtgcgat acatcatatg 10680
   gacaggggtt atgcatacac tgcttgtttg ttgtttatgt tctaagagca tctccaacaa 10740
   cgtgacatat gaaaatgccc tacaatttaa aaatggttat attttataaa atttagggca 10800
   taaataaaac atcccgctcc aacattaaag ccttaaatct attataggga agcccactat 10860
   gatatagtat atttgaggca ctttagaggg tgccctataa ttttttgacc atttttttat 10920
   gaaatgagac actattggag tatttttttt ccgtagagca ccatatttca atttgagaca 10980
   ccaatttaag gcattgttgg agatgttcta aatgttggtt tattttgtct gtatcgttgt 11040
   ggttttgata gtggtgcctt tgcaatgtac atcttacatt gacaataata ataggtaaaa 11100
   ctctacaaat tttttatcta atggactctt gtatgaaaca ttgtacttgc acacatctga 11160
   tgtaaacact gcatactttt aacagtgaca agattctgtt tcattttagg gctagtttgg 11220
   gaaccaaatt ttattagggt ttttattttc taagaaaaag taatttattt taccttgaga 11280
   aaatataaat tacttgagaa aatagagttc caaactagct cttatctttg tcgaatcctc 11340
   ctctattcaa atgtgacatt tctggcacgt gacaactggt gatgttgtag actgtgttaa 11400
   gtaatacgtg tcattattac taaatgccat tttagtaaat gttgagtatg tactctacta 11460
   cagtaagtat tattggtgta tttacactag acagttggcg gcctggcggg taaagttatc 11520
   ctgtagaaag ttgggccagg ccaaaaccaa ccgccaaagg aaaggccttc cggcccgccc 11580
   acctttgcgc gccgaaggtc agttccttca gtctcctccc gcttcagact ctgaccacgt 11640
   cgacaatccg ggccgaaaca catctgcacc gtccacttgc gacagattga acacaccact 11700
   tctatccacg tcagcgatcc gtggcactag cccttccacc aatcagccca agttgcccct 11760
   ttcctttaaa ttcgccgcac ccattgctct tctcacggcc atagaaatcg accgagcgaa 11820
   tccctcgcat cgcattcgca gcctttgctg catcacacca ccgcgaaacc ccagcagccg 11880
   catctgcagg tcgactctag aggatccatg gcctcctccg aggacgtcat caaggagttc 11940
   atgcgcttca aggtgcgcat ggagggctcc gtgaacggcc acgagttcga gatcgagggc 12000
   gagggcgagg gccgccccta cgagggcacc cagaccgcca agctgaaggt gaccaagggc 12060
   ggccccctgc ccttcgcctg ggacatcctg tccccccagt tccagtacgg ctccaaggtg 12120
   tacgtgaagc accccgccga catccccgac tacaagaagc tgtccttccc cgagggcttc 12180
   aagtgggagc gcgtgatgaa cttcgaggac ggcggcgtgg tgacagtgac ccaggactcc 12240
   tccctgcagg acggctcctt catctacaag gtgaagttca tcggcgtgaa cttcccctcc 12300
   gacggccccg taatgcagaa gaagactatg ggctgggagg cctccaccga gcgcctgtac 12360
   ccccgcgacg gcgtgctgaa gggcgagatc cacaaggccc tgaagctgaa ggacggcggc 12420
   cacgctagcc catccaccca ctcactcact catatctgtg ctgtacgtac gagaatttct 12480
   cgaccaaccg tcgtgagacc tgcccaccgg agatcggacg caagagggtt taggcaagaa 12540
   tgtcgtgcga cagggtgagc gctgactagt atacgtgaga gaccttgaga tatacctcac 12600
   acgtacgcgt actttacatg acgtaggaca ttacgactca aacagattca cgtcagattt 12660
   cggagtttct cacgcgtgag agccttggag ggcggtatgt atgtcatact atatgttggg 12720
   atggagggag tgagtgagtg atatgtggct agcaagggcg gccccctgcc cttcgcctgg 12780
   gacatcctgt ccccccagtt ccagtacggc tccaaggtgt acgtgaagca ccccgccgac 12840
   atccccgact acaagaagct gtccttcccc gagggcttca agtgggagcg cgtgatgaac 12900
   ttcgaggacg gcggcgtggt gacagtgacc caggactcct ccctgcagga cggctccttc 12960
   atctacaagg tgaagttcat cggcgtgaac ttcccctccg acggccccgt aatgcagaag 13020
   aagactatgg gctgggaggc ctccaccgag cgcctgtacc cccgcgacgg cgtgctgaag 13080
   ggcgagatcc acaaggccct gaagctgaag gacggcggcc actacctggt ggagttcaag 13140
   tccatctaca tggccaagaa gcccgtgcag ctgcccggct actactacgt ggactccaag 13200
   ctggacatca cctcccacaa cgaggactac accatcgtgg agcagtacga gcgcgccgag 13260
   ggccgccacc acctgttcct gtagtcagga tctgagtcga aacctagact tgtccatctt 13320
   ctggattggc caacttaatt aatgtatgaa ataaaaggat gcacacatag tgacatgcta 13380
   atcactataa tgtgggcatc aaagttgtgt gttatgtgta attactagtt atctgaataa 13440
   aagagaaaga gatcatccat atttcttatc ctaaatgaat gtcacgtgtc tttataattc 13500
   tttgatgaac cagatgcatt tcattaacca aatccatata catataaata ttaatcatat 13560
   ataattaata tcaattgggt tagcaaaaca aatctagtct aggtgtgttt tgcgaatgcg 13620
   gccgccaccg cggtggagct cgaattccgg tccgggtcac ccggtccggg cctagaaggc 13680
   cagcttgcgg ccgccccggg caactttatt atacaaagtt gatagatatc ggtccgagcg 13740
   gcctagaagg cctttggtca cctttgtcca ccaagatgga actgcggccg ctcattaatt 13800
   aagtcaggcg cgcctctagt tgaagacacg ttcatgtctt catcgtaaga agacactcag 13860
   tagtcttcgg ccagaatggc ctaactcaag gccatcgtgg cctcttgctc ttcaggatga 13920
   agagctatgt ttaaacgtgc aagcgctact agacaattca gtacattaaa aacgtccgca 13980
   atgtgttatt aagttgtcta agcgtcaatt tgtttacacc acaatatatc ctgccaccag 14040
   ccagccaaca gctccccgac cggcagctcg gcacaaaatc accactcgat acaggcagcc 14100
   catcagtccg ggacggcgtc agcgggagag ccgttgtaag gcggcagact ttgctcatgt 14160
   taccgatgct attcggaaga acggcaacta agctgccggg tttgaaacac ggatgatctc 14220
   gcggagggta gcatgttgat tgtaacgatg acagagcgtt gctgcctgtg atcaaatatc 14280
   atctccctcg cagagatccg aattatcagc cttcttattc atttctcgct taaccgtgac 14340
   aggctgtcga tcttgagaac tatgccgaca taataggaaa tcgctggata aagccgctga 14400
   ggaagctgag tggcgctatt tctttagaag tgaacgttga cgatcgtcga ccgtaccccg 14460
   atgaattaat tcggacgtac gttctgaaca cagctggata cttacttggg cgattgtcat 14520
   acatgacatc aacaatgtac ccgtttgtgt aaccgtctct tggaggttcg tatgacacta 14580
   gtggttcccc tcagcttgcg actagatgtt gaggcctaac attttattag agagcaggct 14640
   agttgcttag atacatgatc ttcaggccgt tatctgtcag ggcaagcgaa aattggccat 14700
   ttatgacgac caatgccccg cagaagctcc catctttgcc gccatagacg ccgcgccccc 14760
   cttttggggt gtagaacatc cttttgccag atgtggaaaa gaagttcgtt gtcccattgt 14820
   tggcaatgac gtagtagccg gcgaaagtgc gagacccatt tgcgctatat ataagcctac 14880
   gatttccgtt gcgactattg tcgtaattgg atgaactatt atcgtagttg ctctcagagt 14940
   tgtcgtaatt tgatggacta ttgtcgtaat tgcttatgga gttgtcgtag ttgcttggag 15000
   aaatgtcgta gttggatggg gagtagtcat agggaagacg agcttcatcc actaaaacaa 15060
   ttggcaggtc agcaagtgcc tgccccgatg ccatcgcaag tacgaggctt agaaccacct 15120
   tcaacagatc gcgcatagtc ttccccagct ctctaacgct tgagttaagc cgcgccgcga 15180
   agcggcgtcg gcttgaacga attgttagac attatttgcc gactaccttg gtgatctcgc 15240
   ctttcacgta gtgaacaaat tcttccaact gatctgcgcg cgaggccaag cgatcttctt 15300
   gtccaagata agcctgccta gcttcaagta tgacgggctg atactgggcc ggcaggcgct 15360
   ccattgccca gtcggcagcg acatccttcg gcgcgatttt gccggttact gcgctgtacc 15420
   aaatgcggga caacgtaagc actacatttc gctcatcgcc agcccagtcg ggcggcgagt 15480
   tccatagcgt taaggtttca tttagcgcct caaatagatc ctgttcagga accggatcaa 15540
   agagttcctc cgccgctgga cctaccaagg caacgctatg ttctcttgct tttgtcagca 15600
   agatagccag atcaatgtcg atcgtggctg gctcgaagat acctgcaaga atgtcattgc 15660
   gctgccattc tccaaattgc agttcgcgct tagctggata acgccacgga atgatgtcgt 15720
   cgtgcacaac aatggtgact tctacagcgc ggagaatctc gctctctcca ggggaagccg 15780
   aagtttccaa aaggtcgttg atcaaagctc gccgcgttgt ttcatcaagc cttacagtca 15840
   ccgtaaccag caaatcaata tcactgtgtg gcttcaggcc gccatccact gcggagccgt 15900
   acaaatgtac ggccagcaac gtcggttcga gatggcgctc gatgacgcca actacctctg 15960
   atagttgagt cgatacttcg gcgatcaccg cttccctcat gatgtttaac tcctgaatta 16020
   agccgcgccg cgaagcggtg tcggcttgaa tgaattgtta ggcgtcatcc tgtgctcccg 16080
   agaaccagta ccagtacatc gctgtttcgt tcgagacttg aggtctagtt ttatacgtga 16140
   acaggtcaat gccgccgaga gtaaagccac attttgcgta caaattgcag gcaggtacat 16200
   tgttcgtttg tgtctctaat cgtatgccaa ggagctgtct gcttagtgcc cactttttcg 16260
   caaattcgat gagactgtgc gcgactcctt tgcctcggtg cgtgtgcgac acaacaatgt 16320
   gttcgataga ggctagatcg ttccatgttg agttgagttc aatcttcccg acaagctctt 16380
   ggtcgatgaa tgcgccatag caagcagagt cttcatcaga gtcatcatcc gagatgtaat 16440
   ccttccggta ggggctcaca cttctggtag atagttcaaa gccttggtcg gataggtgca 16500
   catcgaacac ttcacgaaca atgaaatggt tctcagcatc caatgtttcc gccacctgct 16560
   cagggatcac cgaaatcttc atatgacgcc taacgcctgg cacagcggat cgcaaacctg 16620
   gcgcggcttt tggcacaaaa ggcgtgacag gtttgcgaat ccgttgctgc cacttgttaa 16680
   cccttttgcc agatttggta actataattt atgttagagg cgaagtcttg ggtaaaaact 16740
   ggcctaaaat tgctggggat ttcaggaaag taaacatcac cttccggctc gatgtctatt 16800
   gtagatatat gtagtgtatc tacttgatcg ggggatctgc tgcctcgcgc gtttcggtga 16860
   tgacggtgaa aacctctgac acatgcagct cccggagacg gtcacagctt gtctgtaagc 16920
   ggatgccggg agcagacaag cccgtcaggg cgcgtcagcg ggtgttggcg ggtgtcgggg 16980
   cgcagccatg acccagtcac gtagcgatag cggagtgtat actggcttaa ctatgcggca 17040
   tcagagcaga ttgtactgag agtgcaccat atgcggtgtg aaataccgca cagatgcgta 17100
   aggagaaaat accgcatcag gcgctcttcc gcttcctcgc tcactgactc gctgcgctcg 17160
   gtcgttcggc tgcggcgagc ggtatcagct cactcaaagg cggtaatacg gttatccaca 17220
   gaatcagggg ataacgcagg aaagaacatg tgagcaaaag gccagcaaaa ggccaggaac 17280
   cgtaaaaagg ccgcgttgct ggcgtttttc cataggctcc gcccccctga cgagcatcac 17340
   aaaaatcgac gctcaagtca gaggtggcga aacccgacag gactataaag ataccaggcg 17400
   tttccccctg gaagctccct cgtgcgctct cctgttccga ccctgccgct taccggatac 17460
   ctgtccgcct ttctcccttc gggaagcgtg gcgctttctc atagctcacg ctgtaggtat 17520
   ctcagttcgg tgtaggtcgt tcgctccaag ctgggctgtg tgcacgaacc ccccgttcag 17580
   cccgaccgct gcgccttatc cggtaactat cgtcttgagt ccaacccggt aagacacgac 17640
   ttatcgccac tggcagcagc cactggtaac aggattagca gagcgaggta tgtaggcggt 17700
   gctacagagt tcttgaagtg gtggcctaac tacggctaca ctagaaggac agtatttggt 17760
   atctgcgctc tgctgaagcc agttaccttc ggaaaaagag ttggtagctc ttgatccggc 17820
   aaacaaacca ccgctggtag cggtggtttt tttgtttgca agcagcagat tacgcgcaga 17880
   aaaaaaggat ctcaagaaga tcctttgatc ttttctacgg ggtctgacgc tcagtggaac 17940
   gaaaactcac gttaagggat tttggtcatg agattatcaa aaaggatctt cacctagatc 18000
   cttttaaatt aaaaatgaag ttttaaatca atctaaagta tatatgagta aacttggtct 18060
   gacagttacc aatgcttaat cagtgaggca cctatctcag cgatctgtct atttcgttca 18120
   tccatagttg cctgactccc cgtcgtgtag ataactacga tacgggaggg cttaccatct 18180
   ggccccagtg ctgcaatgat accgcgagac ccacgctcac cggctccaga tttatcagca 18240
   ataaaccagc cagccggaag ggccgagcgc agaagtggtc ctgcaacttt atccgcctcc 18300
   atccagtcta ttaattgttg ccgggaagct agagtaagta gttcgccagt taatagtttg 18360
   cgcaacgttg ttgccattgc tgcagggggg gggggggggg gggacttcca ttgttcattc 18420
   cacggacaaa aacagagaaa ggaaacgaca gaggccaaaa agcctcgctt tcagcacctg 18480
   tcgtttcctt tcttttcaga gggtatttta aataaaaaca ttaagttatg acgaagaaga 18540
   acggaaacgc cttaaaccgg aaaattttca taaatagcga aaacccgcga ggtcgccgcc 18600
   ccgtaacctg tcggatcacc ggaaaggacc cgtaaagtga taatgattat catctacata 18660
   tcacaacgtg cgtggaggcc atcaaaccac gtcaaataat caattatgac gcaggtatcg 18720
   tattaattga tctgcatcaa cttaacgtaa aaacaacttc agacaataca aatcagcgac 18780
   actgaatacg gggcaacctc atgtcccccc cccccccccc cctgcaggca tcgtggtgtc 18840
   acgctcgtcg tttggtatgg cttcattcag ctccggttcc caacgatcaa ggcgagttac 18900
   atgatccccc atgttgtgca aaaaagcggt tagctccttc ggtcctccga tcgttgtcag 18960
   aagtaagttg gccgcagtgt tatcactcat ggttatggca gcactgcata attctcttac 19020
   tgtcatgcca tccgtaagat gcttttctgt gactggtgag tactcaacca agtcattctg 19080
   agaatagtgt atgcggcgac cgagttgctc ttgcccggcg tcaacacggg ataataccgc 19140
   gccacatagc agaactttaa aagtgctcat cattggaaaa cgttcttcgg ggcgaaaact 19200
   ctcaaggatc ttaccgctgt tgagatccag ttcgatgtaa cccactcgtg cacccaactg 19260
   atcttcagca tcttttactt tcaccagcgt ttctgggtga gcaaaaacag gaaggcaaaa 19320
   tgccgcaaaa aagggaataa gggcgacacg gaaatgttga atactcatac tcttcctttt 19380
   tcaatattat tgaagcattt atcagggtta ttgtctcatg agcggataca tatttgaatg 19440
   tatttagaaa aataaacaaa taggggttcc gcgcacattt ccccgaaaag tgccacctga 19500
   cgtctaagaa accattatta tcatgacatt aacctataaa aataggcgta tcacgaggcc 19560
   ctttcgtctt caagaattcg gagcttttgc cattctcacc ggattcagtc gtcactcatg 19620
   gtgatttctc acttgataac cttatttttg acgaggggaa attaataggt tgtattgatg 19680
   ttggacgagt cggaatcgca gaccgatacc aggatcttgc catcctatgg aactgcctcg 19740
   gtgagttttc tccttcatta cagaaacggc tttttcaaaa atatggtatt gataatcctg 19800
   atatgaataa attgcagttt catttgatgc tcgatgagtt tttctaatca gaattggtta 19860
   attggttgta acactggcag agcattacgc tgacttgacg ggacggcggc tttgttgaat 19920
   aaatcgaact tttgctgagt tgaaggatca gatcacgcat cttcccgaca acgcagaccg 19980
   ttccgtggca aagcaaaagt tcaaaatcac caactggtcc acctacaaca aagctctcat 20040
   caaccgtggc tccctcactt tctggctgga tgatggggcg attcaggcct ggtatgagtc 20100
   agcaacacct tcttcacgag gcagacctca gcgccagaag gccgccagag aggccgagcg 20160
   cggccgtgag gcttggacgc tagggcaggg catgaaaaag cccgtagcgg gctgctacgg 20220
   gcgtctgacg cggtggaaag gggaggggga tgttgtctac atggctctgc tgtagtgagt 20280
   gggttgcgct ccggcagcgg tcctgatcaa tcgtcaccct ttctcggtcc ttcaacgttc 20340
   ctgacaacga gcctcctttt cgccaatcca tcgacaatca ccgcgagtcc ctgctcgaac 20400
   gctgcgtccg gaccggcttc gtcgaaggcg tctatcgcgg cccgcaacag cggcgagagc 20460
   ggagcctgtt caacggtgcc gccgcgctcg ccggcatcgc tgtcgccggc ctgctcctca 20520
   agcacggccc caacagtgaa gtagctgatt gtcatcagcg cattgacggc gtccccggcc 20580
   gaaaaacccg cctcgcagag gaagcgaagc tgcgcgtcgg ccgtttccat ctgcggtgcg 20640
   cccggtcgcg tgccggcatg gatgcgcgcg ccatcgcggt aggcgagcag cgcctgcctg 20700
   aagctgcggg cattcccgat cagaaatgag cgccagtcgt cgtcggctct cggcaccgaa 20760
   tgcgtatgat tctccgccag catggcttcg gccagtgcgt cgagcagcgc ccgcttgttc 20820
   ctgaagtgcc agtaaagcgc cggctgctga acccccaacc gttccgccag tttgcgtgtc 20880
   gtcagaccgt ctacgccgac ctcgttcaac aggtccaggg cggcacggat cactgtattc 20940
   ggctgcaact ttgtcatgct tgacacttta tcactgataa acataatatg tccaccaact 21000
   tatcagtgat aaagaatccg cgcgttcaat cggaccagcg gaggctggtc cggaggccag 21060
   acgtgaaacc caacataccc ctgatcgtaa ttctgagcac tgtcgcgctc gacgctgtcg 21120
   gcatcggcct gattatgccg gtgctgccgg gcctcctgcg cgatctggtt cactcgaacg 21180
   acgtcaccgc ccactatggc attctgctgg cgctgtatgc gttggtgcaa tttgcctgcg 21240
   cacctgtgct gggcgcgctg tcggatcgtt tcgggcggcg gccaatcttg ctcgtctcgc 21300
   tggccggcgc cactgtcgac tacgccatca tggcgacagc gcctttcctt tgggttctct 21360
   atatcgggcg gatcgtggcc ggcatcaccg gggcgactgg ggcggtagcc ggcgcttata 21420
   ttgccgatat cactgatggc gatgagcgcg cgcggcactt cggcttcatg agcgcctgtt 21480
   tcgggttcgg gatggtcgcg ggacctgtgc tcggtgggct gatgggcggt ttctcccccc 21540
   acgctccgtt cttcgccgcg gcagccttga acggcctcaa tttcctgacg ggctgtttcc 21600
   ttttgccgga gtcgcacaaa ggcgaacgcc ggccgttacg ccgggaggct ctcaacccgc 21660
   tcgcttcgtt ccggtgggcc cggggcatga ccgtcgtcgc cgccctgatg gcggtcttct 21720
   tcatcatgca acttgtcgga caggtgccgg ccgcgctttg ggtcattttc ggcgaggatc 21780
   gctttcactg ggacgcgacc acgatcggca tttcgcttgc cgcatttggc attctgcatt 21840
   cactcgccca ggcaatgatc accggccctg tagccgcccg gctcggcgaa aggcgggcac 21900
   tcatgctcgg aatgattgcc gacggcacag gctacatcct gcttgccttc gcgacacggg 21960
   gatggatggc gttcccgatc atggtcctgc ttgcttcggg tggcatcgga atgccggcgc 22020
   tgcaagcaat gttgtccagg caggtggatg aggaacgtca ggggcagctg caaggctcac 22080
   tggcggcgct caccagcctg acctcgatcg tcggacccct cctcttcacg gcgatctatg 22140
   cggcttctat aacaacgtgg aacgggtggg catggattgc aggcgctgcc ctctacttgc 22200
   tctgcctgcc ggcgctgcgt cgcgggcttt ggagcggcgc agggcaacga gccgatcgct 22260
   gatcgtggaa acgataggcc tatgccatgc gggtcaaggc gacttccggc aagctatacg 22320
   cgccctagga gtgcggttgg aacgttggcc cagccagata ctcccgatca cgagcaggac 22380
   gccgatgatt tgaagcgcac tcagcgtctg atccaagaac aaccatccta gcaacacggc 22440
   ggtccccggg ctgagaaagc ccagtaagga aacaactgta ggttcgagtc gcgagatccc 22500
   ccggaaccaa aggaagtagg ttaaacccgc tccgatcagg ccgagccacg ccaggccgag 22560
   aacattggtt cctgtaggca tcgggattgg cggatcaaac actaaagcta ctggaacgag 22620
   cagaagtcct ccggccgcca gttgccaggc ggtaaaggtg agcagaggca cgggaggttg 22680
   ccacttgcgg gtcagcacgg ttccgaacgc catggaaacc gcccccgcca ggcccgctgc 22740
   gacgccgaca ggatctagcg ctgcgtttgg tgtcaacacc aacagcgcca cgcccgcagt 22800
   tccgcaaata gcccccagga ccgccatcaa tcgtatcggg ctacctagca gagcggcaga 22860
   gatgaacacg accatcagcg gctgcacagc gcctaccgtc gccgcgaccc cgcccggcag 22920
   gcggtagacc gaaataaaca acaagctcca gaatagcgaa atattaagtg cgccgaggat 22980
   gaagatgcgc atccaccaga ttcccgttgg aatctgtcgg acgatcatca cgagcaataa 23040
   acccgccggc aacgcccgca gcagcatacc ggcgacccct cggcctcgct gttcgggctc 23100
   cacgaaaacg ccggacagat gcgccttgtg agcgtccttg gggccgtcct cctgtttgaa 23160
   gaccgacagc ccaatgatct cgccgtcgat gtaggcgccg aatgccacgg catctcgcaa 23220
   ccgttcagcg aacgcctcca tgggcttttt ctcctcgtgc tcgtaaacgg acccgaacat 23280
   ctctggagct ttcttcaggg ccgacaatcg gatctcgcgg aaatcctgca cgtcggccgc 23340
   tccaagccgt cgaatctgag ccttaatcac aattgtcaat tttaatcctc tgtttatcgg 23400
   cagttcgtag agcgcgccgt gcgtcccgag cgatactgag cgaagcaagt gcgtcgagca 23460
   gtgcccgctt gttcctgaaa tgccagtaaa gcgctggctg ctgaaccccc agccggaact 23520
   gaccccacaa ggccctagcg tttgcaatgc accaggtcat cattgaccca ggcgtgttcc 23580
   accaggccgc tgcctcgcaa ctcttcgcag gcttcgccga cctgctcgcg ccacttcttc 23640
   acgcgggtgg aatccgatcc gcacatgagg cggaaggttt ccagcttgag cgggtacggc 23700
   tcccggtgcg agctgaaata gtcgaacatc cgtcgggccg tcggcgacag cttgcggtac 23760
   ttctcccata tgaatttcgt gtagtggtcg ccagcaaaca gcacgacgat ttcctcgtcg 23820
   atcaggacct ggcaacggga cgttttcttg ccacggtcca ggacgcggaa gcggtgcagc 23880
   agcgacaccg attccaggtg cccaacgcgg tcggacgtga agcccatcgc cgtcgcctgt 23940
   aggcgcgaca ggcattcctc ggccttcgtg taataccggc cattgatcga ccagcccagg 24000
   tcctggcaaa gctcgtagaa cgtgaaggtg atcggctcgc cgataggggt gcgcttcgcg 24060
   tactccaaca cctgctgcca caccagttcg tcatcgtcgg cccgcagctc gacgccggtg 24120
   taggtgatct tcacgtcctt gttgacgtgg aaaatgacct tgttttgcag cgcctcgcgc 24180
   gggattttct tgttgcgcgt ggtgaacagg gcagagcggg ccgtgtcgtt tggcatcgct 24240
   cgcatcgtgt ccggccacgg cgcaatatcg aacaaggaaa gctgcatttc cttgatctgc 24300
   tgcttcgtgt gtttcagcaa cgcggcctgc ttggcctcgc tgacctgttt tgccaggtcc 24360
   tcgccggcgg tttttcgctt cttggtcgtc atagttcctc gcgtgtcgat ggtcatcgac 24420
   ttcgccaaac ctgccgcctc ctgttcgaga cgacgcgaac gctccacggc ggccgatggc 24480
   gcgggcaggg cagggggagc cagttgcacg ctgtcgcgct cgatcttggc cgtagcttgc 24540
   tggaccatcg agccgacgga ctggaaggtt tcgcggggcg cacgcatgac ggtgcggctt 24600
   gcgatggttt cggcatcctc ggcggaaaac cccgcgtcga tcagttcttg cctgtatgcc 24660
   ttccggtcaa acgtccgatt cattcaccct ccttgcggga ttgccccgac tcacgccggg 24720
   gcaatgtgcc cttattcctg atttgacccg cctggtgcct tggtgtccag ataatccacc 24780
   ttatcggcaa tgaagtcggt cccgtagacc gtctggccgt ccttctcgta cttggtattc 24840
   cgaatcttgc cctgcacgaa taccagcgac cccttgccca aatacttgcc gtgggcctcg 24900
   gcctgagagc caaaacactt gatgcggaag aagtcggtgc gctcctgctt gtcgccggca 24960
   tcgttgcgcc actcttcatt aaccgctata tcgaaaattg cttgcggctt gttagaattg 25020
   ccatgacgta cctcggtgtc acgggtaaga ttaccgataa actggaactg attatggctc 25080
   atatcgaaag tctccttgag aaaggagact ctagtttagc taaacattgg ttccgctgtc 25140
   aagaacttta gcggctaaaa ttttgcgggc cgcgaccaaa ggtgcgaggg gcggcttccg 25200
   ctgtgtacaa ccagatattt ttcaccaaca tccttcgtct gctcgatgag cggggcatga 25260
   cgaaacatga gctgtcggag agggcagggg tttcaatttc gtttttatca gacttaacca 25320
   acggtaaggc caacccctcg ttgaaggtga tggaggccat tgccgacgcc ctggaaactc 25380
   ccctacctct tctcctggag tccaccgacc ttgaccgcga ggcactcgcg gagattgcgg 25440
   gtcatccttt caagagcagc gtgccgcccg gatacgaacg catcagtgtg gttttgccgt 25500
   cacataaggc gtttatcgta aagaaatggg gcgacgacac ccgaaaaaag ctgcgtggaa 25560
   ggctctgacg ccaagggtta gggcttgcac ttccttcttt agccgctaaa acggcccctt 25620
   ctctgcgggc cgtcggctcg cgcatcatat cgacatcctc aacggaagcc gtgccgcgaa 25680
   tggcatcggg cgggtgcgct ttgacagttg ttttctatca gaacccctac gtcgtgcggt 25740
   tcgattagct gtttgtcttg caggctaaac actttcggta tatcgtttgc ctgtgcgata 25800
   atgttgctaa tgatttgttg cgtaggggtt actgaaaagt gagcgggaaa gaagagtttc 25860
   agaccatcaa ggagcgggcc aagcgcaagc tggaacgcga catgggtgcg gacctgttgg 25920
   ccgcgctcaa cgacccgaaa accgttgaag tcatgctcaa cgcggacggc aaggtgtggc 25980
   acgaacgcct tggcgagccg atgcggtaca tctgcgacat gcggcccagc cagtcgcagg 26040
   cgattataga aacggtggcc ggattccacg gcaaagaggt cacgcggcat tcgcccatcc 26100
   tggaaggcga gttccccttg gatggcagcc gctttgccgg ccaattgccg ccggtcgtgg 26160
   ccgcgccaac ctttgcgatc cgcaagcgcg cggtcgccat cttcacgctg gaacagtacg 26220
   tcgaggcggg catcatgacc cgcgagcaat acgaggtcat taaaagcgcc gtcgcggcgc 26280
   atcgaaacat cctcgtcatt ggcggtactg gctcgggcaa gaccacgctc gtcaacgcga 26340
   tcatcaatga aatggtcgcc ttcaacccgt ctgagcgcgt cgtcatcatc gaggacaccg 26400
   gcgaaatcca gtgcgccgca gagaacgccg tccaatacca caccagcatc gacgtctcga 26460
   tgacgctgct gctcaagaca acgctgcgta tgcgccccga ccgcatcctg gtcggtgagg 26520
   tacgtggccc cgaagccctt gatctgttga tggcctggaa caccgggcat gaaggaggtg 26580
   ccgccaccct gcacgcaaac aaccccaaag cgggcctgag ccggctcgcc atgcttatca 26640
   gcatgcaccc ggattcaccg aaacccattg agccgctgat tggcgaggcg gttcatgtgg 26700
   tcgtccatat cgccaggacc cctagcggcc gtcgagtgca agaaattctc gaagttcttg 26760
   gttacgagaa cggccagtac atcaccaaaa ccctgtaagg agtatttcca atgacaacgg 26820
   ctgttccgtt ccgtctgacc atgaatcgcg gcattttgtt ctaccttgcc gtgttcttcg 26880
   ttctcgctct cgcgttatcc gcgcatccgg cgatggcctc ggaaggcacc ggcggcagct 26940
   tgccatatga gagctggctg acgaacctgc gcaactccgt aaccggcccg gtggccttcg 27000
   cgctgtccat catcggcatc gtcgtcgccg gcggcgtgct gatcttcggc ggcgaactca 27060
   acgccttctt ccgaaccctg atcttcctgg ttctggtgat ggcgctgctg gtcggcgcgc 27120
   agaacgtgat gagcaccttc ttcggtcgtg gtgccgaaat cgcggccctc ggcaacgggg 27180
   cgctgcacca ggtgcaagtc gcggcggcgg atgccgtgcg tgcggtagcg gctggacggc 27240
   tcgcctaatc atggctctgc gcacgatccc catccgtcgc gcaggcaacc gagaaaacct 27300
   gttcatgggt ggtgatcgtg aactggtgat gttctcgggc ctgatggcgt ttgcgctgat 27360
   tttcagcgcc caagagctgc gggccaccgt ggtcggtctg atcctgtggt tcggggcgct 27420
   ctatgcgttc cgaatcatgg cgaaggccga tccgaagatg cggttcgtgt acctgcgtca 27480
   ccgccggtac aagccgtatt acccggcccg ctcgaccccg ttccgcgaga acaccaatag 27540
   ccaagggaag caataccgat gatccaagca attgcgattg caatcgcggg cctcggcgcg 27600
   cttctgttgt tcatcctctt tgcccgcatc cgcgcggtcg atgccgaact gaaactgaaa 27660
   aagcatcgtt ccaaggacgc cggcctggcc gatctgctca actacgccgc tgtcgtcgat 27720
   gacggcgtaa tcgtgggcaa gaacggcagc tttatggctg cctggctgta caagggcgat 27780
   gacaacgcaa gcagcaccga ccagcagcgc gaagtagtgt ccgcccgcat caaccaggcc 27840
   ctcgcgggcc tgggaagtgg gtggatgatc catgtggacg ccgtgcggcg tcctgctccg 27900
   aactacgcgg agcggggcct gtcggcgttc cctgaccgtc tgacggcagc gattgaagaa 27960
   gagcgctcgg tcttgccttg ctcgtcggtg atgtacttca ccagctccgc gaagtcgctc 28020
   ttcttgatgg agcgcatggg gacgtgcttg gcaatcacgc gcaccccccg gccgttttag 28080
   cggctaaaaa agtcatggct ctgccctcgg gcggaccacg cccatcatga ccttgccaag 28140
   ctcgtcctgc ttctcttcga tcttcgccag cagggcgagg atcgtggcat caccgaaccg 28200
   cgccgtgcgc gggtcgtcgg tgagccagag tttcagcagg ccgcccaggc ggcccaggtc 28260
   gccattgatg cgggccagct cgcggacgtg ctcatagtcc acgacgcccg tgattttgta 28320
   gccctggccg acggccagca ggtaggccga caggctcatg ccggccgccg ccgccttttc 28380
   ctcaatcgct cttcgttcgt ctggaaggca gtacaccttg ataggtgggc tgcccttcct 28440
   ggttggcttg gtttcatcag ccatccgctt gccctcatct gttacgccgg cggtagccgg 28500
   ccagcctcgc agagcaggat tcccgttgag caccgccagg tgcgaataag ggacagtgaa 28560
   gaaggaacac ccgctcgcgg gtgggcctac ttcacctatc ctgcccggct gacgccgttg 28620
   gatacaccaa ggaaagtcta cacgaaccct ttggcaaaat cctgtatatc gtgcgaaaaa 28680
   ggatggatat accgaaaaaa tcgctataat gaccccgaag cagggttatg cagcggaaaa 28740
   gcgctgcttc cctgctgttt tgtggaatat ctaccgactg gaaacaggca aatgcaggaa 28800
   attactgaac tgaggggaca ggcgagagac gatgccaaag agctacaccg acgagctggc 28860
   cgagtgggtt gaatcccgcg cggccaagaa gcgccggcgt gatgaggctg cggttgcgtt 28920
   cctggcggtg agggcggatg tcgaggcggc gttagcgtcc ggctatgcgc tcgtcaccat 28980
   ttgggagcac atgcgggaaa cggggaaggt caagttctcc tacgagacgt tccgctcgca 29040
   cgccaggcgg cacatcaagg ccaagcccgc cgatgtgccc gcaccgcagg ccaaggctgc 29100
   ggaacccgcg ccggcaccca agacgccgga gccacggcgg ccgaagcagg ggggcaaggc 29160
   tgaaaagccg gcccccgctg cggccccgac cggcttcacc ttcaacccaa caccggacaa 29220
   aaaggatcta ctgtaatggc gaaaattcac atggttttgc agggcaaggg cggggtcggc 29280
   aagtcggcca tcgccgcgat cattgcgcag tacaagatgg acaaggggca gacacccttg 29340
   tgcatcgaca ccgacccggt gaacgcgacg ttcgagggct acaaggccct gaacgtccgc 29400
   cggctgaaca tcatggccgg cgacgaaatt aactcgcgca acttcgacac cctggtcgag 29460
   ctgattgcgc cgaccaagga tgacgtggtg atcgacaacg gtgccagctc gttcgtgcct 29520
   ctgtcgcatt acctcatcag caaccaggtg ccggctctgc tgcaagaaat ggggcatgag 29580
   ctggtcatcc ataccgtcgt caccggcggc caggctctcc tggacacggt gagcggcttc 29640
   gcccagctcg ccagccagtt cccggccgaa gcgcttttcg tggtctggct gaacccgtat 29700
   tgggggccta tcgagcatga gggcaagagc tttgagcaga tgaaggcgta cacggccaac 29760
   aaggcccgcg tgtcgtccat catccagatt ccggccctca aggaagaaac ctacggccgc 29820
   gatttcagcg acatgctgca agagcggctg acgttcgacc aggcgctggc cgatgaatcg 29880
   ctcacgatca tgacgcggca acgcctcaag atcgtgcggc gcggcctgtt tgaacagctc 29940
   gacgcggcgg ccgtgctatg agcgaccaga ttgaagagct gatccgggag attgcggcca 30000
   agcacggcat cgccgtcggc cgcgacgacc cggtgctgat cctgcatacc atcaacgccc 30060
   ggctcatggc cgacagtgcg gccaagcaag aggaaatcct tgccgcgttc aaggaagagc 30120
   tggaagggat cgcccatcgt tggggcgagg acgccaaggc caaagcggag cggatgctga 30180
   acgcggccct ggcggccagc aaggacgcaa tggcgaaggt aatgaaggac agcgccgcgc 30240
   aggcggccga agcgatccgc agggaaatcg acgacggcct tggccgccag ctcgcggcca 30300
   aggtcgcgga cgcgcggcgc gtggcgatga tgaacatgat cgccggcggc atggtgttgt 30360
   tcgcggccgc cctggtggtg tgggcctcgt tatgaatcgc agaggcgcag atgaaaaagc 30420
   ccggcgttgc cgggctttgt ttttgcgtta gctgggcttg tttgacaggc ccaagctctg 30480
   actgcgcccg cgctcgcgct cctgggcctg tttcttctcc tgctcctgct tgcgcatcag 30540
   ggcctggtgc cgtcgggctg cttcacgcat cgaatcccag tcgccggcca gctcgggatg 30600
   ctccgcgcgc atcttgcgcg tcgccagttc ctcgatcttg ggcgcgtgaa tgcccatgcc 30660
   ttccttgatt tcgcgcacca tgtccagccg cgtgtgcagg gtctgcaagc gggcttgctg 30720
   ttgggcctgc tgctgctgcc aggcggcctt tgtacgcggc agggacagca agccgggggc 30780
   attggactgt agctgctgca aacgcgcctg ctgacggtct acgagctgtt ctaggcggtc 30840
   ctcgatgcgc tccacctggt catgctttgc ctgcacgtag agcgcaaggg tctgctggta 30900
   ggtctgctcg atgggcgcgg attctaagag ggcctgctgt tccgtctcgg cctcctgggc 30960
   cgcctgtagc aaatcctcgc cgctgttgcc gctggactgc tttactgccg gggactgctg 31020
   ttgccctgct cgcgccgtcg tcgcagttcg gcttgccccc actcgattga ctgcttcatt 31080
   tcgagccgca gcgatgcgat ctcggattgc gtcaacggac ggggcagcgc ggaggtgtcc 31140
   ggcttctcct tgggtgagtc ggtcgatgcc atagccaaag gtttccttcc aaaatgcgtc 31200
   cattgctgga ccgtgtttct cattgatgcc cgcaagcatc ttcggcttga ccgccaggtc 31260
   aagcgcgcct tcatgggcgg tcatgacgga cgccgccatg accttgccgc cgttgttctc 31320
   gatgtagccg cgtaatgagg caatggtgcc gcccatcgtc agcgtgtcat cgacaacgat 31380
   gtacttctgg ccggggatca cctccccctc gaaagtcggg ttgaacgcca ggcgatgatc 31440
   tgaaccggct ccggttcggg cgaccttctc ccgctgcaca atgtccgttt cgacctcaag 31500
   gccaaggcgg tcggccagaa cgaccgccat catggccgga atcttgttgt tccccgccgc 31560
   ctcgacggcg aggactggaa cgatgcgggg cttgtcgtcg ccgatcagcg tcttgagctg 31620
   ggcaacagtg tcgtccgaaa tcaggcgctc gaccaaatta agcgccgctt ccgcgtcgcc 31680
   ctgcttcgca gcctggtatt caggctcgtt ggtcaaagaa ccaaggtcgc cgttgcgaac 31740
   caccttcggg aagtctcccc acggtgcgcg ctcggctctg ctgtagctgc tcaagacgcc 31800
   tcccttttta gccgctaaaa ctctaacgag tgcgcccgcg actcaacttg acgctttcgg 31860
   cacttacctg tgccttgcca cttgcgtcat aggtgatgct tttcgcactc ccgatttcag 31920
   gtactttatc gaaatctgac cgggcgtgca ttacaaagtt cttccccacc tgttggtaaa 31980
   tgctgccgct atctgcgtgg acgatgctgc cgtcgtggcg ctgcgactta tcggcctttt 32040
   gggccatata gatgttgtaa atgccaggtt tcagggcccc ggctttatct accttctggt 32100
   tcgtccatgc gccttggttc tcggtctgga caattctttg cccattcatg accaggaggc 32160
   ggtgtttcat tgggtgactc ctgacggttg cctctggtgt taaacgtgtc ctggtcgctt 32220
   gccggctaaa aaaaagccga cctcggcagt tcgaggccgg ctttccctag agccgggcgc 32280
   gtcaaggttg ttccatctat tttagtgaac tgcgttcgat ttatcagtta ctttcctccc     32340
   gctttgtgtt tcctcccact cgtttccgcg tctagccgac ccctcaacat agcggcctct     32400
   tcttgggctg cctttgcctc ttgccgcgct tcgtcacgct cggcttgcac cgtcgtaaag     32460
   cgctcggcct gcctggccgc ctcttgcgcc gccaacttcc tttgctcctg gtgggcctcg     32520
   gcgtcggcct gcgccttcgc tttcaccgct gccaactccg tgcgcaaact ctccgcttcg     32580
   cgcctggtgg cgtcgcgctc gccgcgaagc gcctgcattt cctggttggc cgcgtccagg     32640
   gtcttgcggc tctcttcttt gaatgcgcgg gcgtcctggt gagcgtagtc cagctcggcg     32700
   cgcagctcct gcgctcgacg ctccacctcg tcggcccgct gcgtcgccag cgcggcccgc     32760
   tgctcggctc ctgccagggc ggtgcgtgct tcggccaggg cttgccgctg gcgtgcggcc     32820
   agctcggccg cctcggcggc ctgctgctct agcaatgtaa cgcgcgcctg ggcttcttcc     32880
   agctcgcggg cctgcgcctc gaaggcgtcg gccagctccc cgcgcacggc ttccaactcg     32940
   ttgcgctcac gatcccagcc ggcttgcgct gcctgcaacg attcattggc aagggcctgg     33000
   gcggcttgcc agagggcggc cacggcctgg ttgccggcct gctgcaccgc gtccggcacc     33060
   tggactgcca gcggggcggc ctgcgccgtg cgctggcgtc gccattcgcg catgccggcg     33120
   ctggcgtcgt tcatgttgac gcgggcggcc ttacgcactg catccacggt cgggaagttc     33180
   tcccggtcgc cttgctcgaa cagctcgtcc gcagccgcaa aaatgcggtc gcgcgtctct     33240
   ttgttcagtt ccatgttggc tccggtaatt ggtaagaata ataatactct tacctacctt     33300
   atcagcgcaa gagtttagct gaacagttct cgacttaacg gcaggttttt tagcggctga     33360
   agggcaggca aaaaaagccc cgcacggtcg gcgggggcaa aggtcagcgg ggaaggggat     33420
   tagcgggcgt cgggcttctt catgcgtcgg ggccgcgctt cttgggatgg agcacgacga     33480
   agcgcgcacg cgcatcgtcc tcggccctat cggcccgcgt cgcggtcagg aacttgtcgc     33540
   gcgctaggtc ctccctggtg ggcaccaggg gcatgaactc ggcctgctcg atgtaggtcc     33600
   actccatgac cgcatcgcag tcgaggccgc gttccttcac cgtctcttgc aggtcgcggt     33660
   acgcccgctc gttgagcggc tggtaacggg ccaattggtc gtaaatggct gtcggccatg     33720
   agcggccttt cctgttgagc cagcagccga cgacgaagcc ggcaatgcag gcccctggca     33780
   caaccaggcc gacgccgggg gcaggggatg gcagcagctc gccaaccagg aaccccgccg     33840
   cgatgatgcc gatgccggtc aaccagccct tgaaactatc cggccccgaa acacccctgc     33900
   gcattgcctg gatgctgcgc cggatagctt gcaacatcag gagccgtttc ttttgttcgt     33960
   cagtcatggt ccgccctcac cagttgttcg tatcggtgtc ggacgaactg aaatcgcaag     34020
   agctgccggt atcggtccag ccgctgtccg tgtcgctgct gccgaagcac ggcgaggggt     34080
   ccgcgaacgc cgcagacggc gtatccggcc gcagcgcatc gcccagcatg gccccggtca     34140
   gcgagccgcc ggccaggtag cccagcatgg tgctgttggt cgccccggcc accagggccg     34200
   acgtgacgaa atcgccgtca ttccctctgg attgttcgct gctcggcggg gcagtgcgcc     34260
   gcgccggcgg cgtcgtggat ggctcgggtt ggctggcctg cgacggccgg cgaaaggtgc     34320
   gcagcagctc gttatcgacc ggctgcggcg tcggggccgc cgccttgcgc tgcggtcggt     34380
   gttccttctt cggctcgcgc agcttgaaca gcatgatcgc ggaaaccagc agcaacgccg     34440
   cgcctacgcc tcccgcgatg tagaacagca tcggattcat tcttcggtcc tccttgtagc     34500
   ggaaccgttg tctgtgcggc gcgggtggcc cgcgccgctg tctttgggga tcagccctcg     34560
   atgagcgcga ccagtttcac gtcggcaagg ttcgcctcga actcctggcc gtcgtcctcg     34620
   tacttcaacc aggcatagcc ttccgccggc ggccgacggt tgaggataag gcgggcaggg     34680
   cgctcgtcgt gctcgacctg gacgatggcc tttttcagct tgtccgggtc cggctccttc     34740
   gcgccctttt ccttggcgtc cttaccgtcc tggtcgccgt cctcgccgtc ctggccgtcg     34800
   ccggcctccg cgtcacgctc ggcatcagtc tggccgttga aggcatcgac ggtgttggga     34860
   tcgcggccct tctcgtccag gaactcgcgc agcagcttga ccgtgccgcg cgtgatttcc     34920
   tgggtgtcgt cgtcaagcca cgcctcgact tcctccgggc gcttcttgaa ggccgtcacc     34980
   agctcgttca ccacggtcac gtcgcgcacg cggccggtgt tgaacgcatc ggcgatcttc     35040
   tccggcaggt ccagcagcgt gacgtgctgg gtgatgaacg ccggcgactt gccgatttcc     35100
   ttggcgatat cgcctttctt cttgcccttc gccagctcgc ggccaatgaa gtcggcaatt     35160
   tcgcgcgggg tcagctcgtt gcgttgcagg ttctcgataa cctggtcggc ttcgttgtag     35220
   tcgttgtcga tgaacgccgg gatggacttc ttgccggccc acttcgagcc acggtagcgg     35280
   cgggcgccgt gattgatgat atagcggccc ggctgctcct ggttctcgcg caccgaaatg     35340
   ggtgacttca ccccgcgctc tttgatcgtg gcaccgattt ccgcgatgct ctccggggaa     35400
   aagccggggt tgtcggccgt ccgcggctga tgcggatctt cgtcgatcag gtccaggtcc     35460
   agctcgatag ggccggaacc gccctgagac gccgcaggag cgtccaggag gctcgacagg     35520
   tcgccgatgc tatccaaccc caggccggac ggctgcgccg cgcctgcggc ttcctgagcg     35580
   gccgcagcgg tgtttttctt ggtggtcttg gcttgagccg cagtcattgg gaaatctcca     35640
   tcttcgtgaa cacgtaatca gccagggcgc gaacctcttt cgatgccttg cgcgcggccg     35700
   ttttcttgat cttccagacc ggcacaccgg atgcgagggc atcggcgatg ctgctgcgca     35760
   ggccaacggt ggccggaatc atcatcttgg ggtacgcggc cagcagctcg gcttggtggc     35820
   gcgcgtggcg cggattccgc gcatcgacct tgctgggcac catgccaagg aattgcagct     35880
   tggcgttctt ctggcgcacg ttcgcaatgg tcgtgaccat cttcttgatg ccctggatgc     35940
   tgtacgcctc aagctcgatg gggacagcag catagtcggc cgcgaagagg gcggccgcca     36000
   ggccgacgcc aagggtcggg gccgtgtcga tcaggcacac gtcgaagcct tggttcgcca     36060
   gggccttgat gttcgccccg aacagctcgc gggcgtcgtc cagcgacagc cgttcggcgt 36120
   tcgccagtac cgggttggac tcgatgaggg cgaggcgcgc ggcctggccg tcgccggctg 36180
   cgggtgcggt ttcggtccag ccgccggcag ggacagcgcc gaacagcttg cttgcatgca 36240
   ggccggtagc aaagtccttg agcgtgtagg acgcattgcc ctgggggtcc aggtcgatca 36300
   cggcaacccg caagccgcgc tcgaaaaagt cgaaggcaag atgcacaagg gtcgaagtct 36360
   tgccgacgcc gcctttctgg ttggccgtga ccaaagtttt catcgtttgg tttcctgttt 36420
   tttcttggcg tccgcttccc acttccggac gatgtacgcc tgatgttccg gcagaaccgc 36480
   cgttacccgc gcgtacccct cgggcaagtt cttgtcctcg aacgcggccc acacgcgatg 36540
   caccgcttgc gacactgcgc ccctggtcag tcccagcgac gttgcgaacg tcgcctgtgg 36600
   cttcccatcg actaagacgc cccgcgctat ctcgatggtc tgctgcccca cttccagccc 36660
   ctggatcgcc tcctggaact ggctttcggt aagccgtttc ttcatggata acacccataa 36720
   tttgctccgc gccttggttg aacatagcgg tgacagccgc cagcacatga gagaagttta 36780
   gctaaacatt tctcgcacgt caacaccttt agccgctaaa actcgtcctt ggcgtaacaa 36840
   aacaaaagcc cggaaaccgg gctttcgtct cttgccgctt atggctctgc acccggctcc 36900
   atcaccaaca ggtcgcgcac gcgcttcact cggttgcgga tcgacactgc cagcccaaca 36960
   aagccggttg ccgccgccgc caggatcgcg ccgatgatgc cggccacacc ggccatcgcc 37020
   caccaggtcg ccgccttccg gttccattcc tgctggtact gcttcgcaat gctggacctc 37080
   ggctcaccat aggctgaccg ctcgatggcg tatgccgctt ctccccttgg cgtaaaaccc 37140
   agcgccgcag gcggcattgc catgctgccc gccgctttcc cgaccacgac gcgcgcacca 37200
   ggcttgcggt ccagaccttc ggccacggcg agctgcgcaa ggacataatc agccgccgac 37260
   ttggctccac gcgcctcgat cagctcttgc actcgcgcga aatccttggc ctccacggcc 37320
   gccatgaatc gcgcacgcgg cgaaggctcc gcagggccgg cgtcgtgatc gccgccgaga 37380
   atgcccttca ccaagttcga cgacacgaaa atcatgctga cggctatcac catcatgcag 37440
   acggatcgca cgaacccgct gaattgaaca cgagcacggc acccgcgacc actatgccaa 37500
   gaatgcccaa ggtaaaaatt gccggccccg ccatgaagtc cgtgaatgcc ccgacggccg 37560
   aagtgaaggg caggccgcca cccaggccgc cgccctcact gcccggcacc tggtcgctga 37620
   atgtcgatgc cagcacctgc ggcacgtcaa tgcttccggg cgtcgcgctc gggctgatcg 37680
   cccatcccgt tactgccccg atcccggcaa tggcaaggac tgccagcgct gccatttttg 37740
   gggtgaggcc gttcgcggcc gaggggcgca gcccctgggg ggatgggagg cccgcgttag 37800
   cgggccggga gggttcgaga agggggggca ccccccttcg gcgtgcgcgg tcacgcgcac 37860
   agggcgcagc cctggttaaa aacaaggttt ataaatattg gtttaaaagc aggttaaaag 37920
   acaggttagc ggtggccgaa aaacgggcgg aaacccttgc aaatgctgga ttttctgcct 37980
   gtggacagcc cctcaaatgt caataggtgc gcccctcatc tgtcagcact ctgcccctca 38040
   agtgtcaagg atcgcgcccc tcatctgtca gtagtcgcgc ccctcaagtg tcaataccgc 38100
   agggcactta tccccaggct tgtccacatc atctgtggga aactcgcgta aaatcaggcg 38160
   ttttcgccga tttgcgaggc tggccagctc cacgtcgccg gccgaaatcg agcctgcccc 38220
   tcatctgtca acgccgcgcc gggtgagtcg gcccctcaag tgtcaacgtc cgcccctcat 38280
   ctgtcagtga gggccaagtt ttccgcgagg tatccacaac gccggcggcc gcggtgtctc 38340
   gcacacggct tcgacggcgt ttctggcgcg tttgcagggc catagacggc cgccagccca 38400
   gcggcgaggg caaccagccc ggtgagcgtc ggaaaggcgc tggaagcccc gtagcgacgc 38460
   ggagaggggc gagacaagcc aagggcgcag gctcgatgcg cagcacgaca tagccggttc 38520
   tcgcaaggac gagaatttcc ctgcggtgcc cctcaagtgt caatgaaagt ttccaacgcg 38580
   agccattcgc gagagccttg agtccacgct agatgagagc tttgttgtag gtggaccagt 38640
   tggtgatttt gaacttttgc tttgccacgg aacggtctgc gttgtcggga agatgcgtga 38700
   tctgatcctt caactcagca aaagttcgat ttattcaaca aagccacgtt gtgtctcaaa 38760
   atctctgatg ttacattgca caagataaaa atatatcatc atgaacaata aaactgtctg 38820
   cttacataaa cagtaataca aggggtgtta tgagccatat tcaacgggaa acgtcttgct 38880
   cgactctaga gctcgttcct cgaggcctcg aggcctcgag gaacggtacc tgcggggaag 38940
   cttacaataa tgtgtgttgt taagtcttgt tgcctgtcat cgtctgactg actttcgtca 39000
   taaatcccgg cctccgtaac ccagctttgg gcaagctcac ggatttgatc cggcggaacg 39060
   ggaatatcga gatgccgggc tgaacgctgc agttccagct ttccctttcg ggacaggtac 39120
   tccagctgat tgattatctg ctgaagggtc ttggttccac ctcctggcac aatgcgaatg 39180
   attacttgag cgcgatcggg catccaattt tctcccgtca ggtgcgtggt caagtgctac 39240
   aaggcacctt tcagtaacga gcgaccgtcg atccgtcgcc gggatacgga caaaatggag 39300
   cgcagtagtc catcgagggc ggcgaaagcc tcgccaaaag caatacgttc atctcgcaca 39360
   gcctccagat ccgatcgagg gtcttcggcg taggcagata gaagcatgga tacattgctt 39420
   gagagtattc cgatggactg aagtatggct tccatctttt ctcgtgtgtc tgcatctatt 39480
   tcgagaaagc ccccgatgcg gcgcaccgca acgcgaattg ccatactatc cgaaagtccc 39540
   agcaggcgcg cttgatagga aaaggtttca tactcggccg atcgcagacg ggcactcacg 39600
   accttgaacc cttcaacttt cagggatcga tgctggttga tggtagtctc actcgacgtg 39660
   gctctggtgt gttttgacat agcttcctcc aaagaaagcg gaaggtctgg atactccagc 39720
   acgaaatgtg cccgggtaga cggatggaag tctagccctg ctcaatatga aatcaacagt 39780
   acatttacag tcaatactga atatacttgc tacatttgca attgtcttat aacgaatgtg 39840
   aaataaaaat agtgtaacaa cgcttttact catcgataat cacaaaaaca tttatacgaa    39900
   caaaaataca aatgcactcc ggtttcacag gataggcggg atcagaatat gcaacttttg    39960
   acgttttgtt ctttcaaagg gggtgctggc aaaaccaccg cactcatggg cctttgcgct    40020
   gctttggcaa atgacggtaa acgagtggcc ctctttgatg ccgacgaaaa ccggcctctg    40080
   acgcgatgga gagaaaacgc cttacaaagc agtactggga tcctcgctgt gaagtctatt    40140
   ccgccgacga aatgcccctt cttgaagcag cctatgaaaa tgccgagctc gaaggatttg    40200
   attatgcgtt ggccgatacg cgtggcggct cgagcgagct caacaacaca atcatcgcta    40260
   gctcaaacct gcttctgatc cccaccatgc taacgccgct cgacatcgat gaggcactat    40320
   ctacctaccg ctacgtcatc gagctgctgt tgagtgaaaa tttggcaatt cctacagctg    40380
   ttttgcgcca acgcgtcccg gtcggccgat tgacaacatc gcaacgcagg atgtcagaga    40440
   cgctagagag ccttccagtt gtaccgtctc ccatgcatga aagagatgca tttgccgcga    40500
   tgaaagaacg cggcatgttg catcttacat tactaaacac gggaactgat ccgacgatgc    40560
   gcctcataga gaggaatctt cggattgcga tggaggaagt cgtggtcatt tcgaaactga    40620
   tcagcaaaat cttggaggct tgaagatggc aattcgcaag cccgcattgt cggtcggcga    40680
   agcacggcgg cttgctggtg ctcgacccga gatccaccat cccaacccga cacttgttcc    40740
   ccagaagctg gacctccagc acttgcctga aaaagccgac gagaaagacc agcaacgtga    40800
   gcctctcgtc gccgatcaca tttacagtcc cgatcgacaa cttaagctaa ctgtggatgc    40860
   ccttagtcca cctccgtccc cgaaaaagct ccaggttttt ctttcagcgc gaccgcccgc    40920
   gcctcaagtg tcgaaaacat atgacaacct cgttcggcaa tacagtccct cgaagtcgct    40980
   acaaatgatt ttaaggcgcg cgttggacga tttcgaaagc atgctggcag atggatcatt    41040
   tcgcgtggcc ccgaaaagtt atccgatccc ttcaactaca gaaaaatccg ttctcgttca    41100
   gacctcacgc atgttcccgg ttgcgttgct cgaggtcgct cgaagtcatt ttgatccgtt    41160
   ggggttggag accgctcgag ctttcggcca caagctggct accgccgcgc tcgcgtcatt    41220
   ctttgctgga gagaagccat cgagcaattg gtgaagaggg acctatcgga acccctcacc    41280
   aaatattgag tgtaggtttg aggccgctgg ccgcgtcctc agtcaccttt tgagccagat    41340
   aattaagagc caaatgcaat tggctcaggc tgccatcgtc cccccgtgcg aaacctgcac    41400
   gtccgcgtca aagaaataac cggcacctct tgctgttttt atcagttgag ggcttgacgg    41460
   atccgcctca agtttgcggc gcagccgcaa aatgagaaca tctatactcc tgtcgtaaac    41520
   ctcctcgtcg cgtactcgac tggcaatgag aagttgctcg cgcgatagaa cgtcgcgggg    41580
   tttctctaaa aacgcgagga gaagattgaa ctcacctgcc gtaagtttca cctcaccgcc    41640
   agcttcggac atcaagcgac gttgcctgag attaagtgtc cagtcagtaa aacaaaaaga    41700
   ccgtcggtct ttggagcgga caacgttggg gcgcacgcgc aaggcaaccc gaatgcgtgc    41760
   aagaaactct ctcgtactaa acggcttagc gataaaatca cttgctccta gctcgagtgc    41820
   aacaacttta tccgtctcct caaggcggtc gccactgata attatgattg gaatatcaga    41880
   ctttgccgcc agatttcgaa cgatctcaag cccatcttca cgacctaaat ttagatcaac    41940
   aaccacgaca tcgaccgtcg cggaagagag tactctagtg aactgggtgc tgtcggctac    42000
   cgcggtcact ttgaaggcgt ggatcgtaag gtattcgata ataagatgcc gcatagcgac    42060
   atcgtcatcg ataagaagaa cgtgtttcaa cggctcacct ttcaatctaa aatctgaacc    42120
   cttgttcaca gcgcttgaga aattttcacg tgaaggatgt acaatcatct ccagctaaat    42180
   gggcagttcg tcagaattgc ggctgaccgc ggatgacgaa aatgcgaacc aagtatttca    42240
   attttatgac aaaagttctc aatcgttgtt acaagtgaaa cgcttcgagg ttacagctac    42300
   tattgattaa ggagatcgcc tatggtctcg ccccggcgtc gtgcgtccgc cgcgagccag    42360
   atctcgccta cttcataaac gtcctcatag gcacggaatg gaatgatgac atcgatcgcc    42420
   gtagagagca tgtcaatcag tgtgcgatct tccaagctag caccttgggc gctacttttg    42480
   acaagggaaa acagtttctt gaatccttgg attggattcg cgccgtgtat tgttgaaatc    42540
   gatcccggat gtcccgagac gacttcactc agataagccc atgctgcatc gtcgcgcatc    42600
   tcgccaagca atatccggtc cggccgcata cgcagacttg cttggagcaa gtgctcggcg    42660
   ctcacagcac ccagcccagc accgttcttg gagtagagta gtctaacatg attatcgtgt    42720
   ggaatgacga gttcgagcgt atcttctatg gtgattagcc tttcctgggg ggggatggcg    42780
   ctgatcaagg tcttgctcat tgttgtcttg ccgcttccgg tagggccaca tagcaacatc    42840
   gtcagtcggc tgacgacgca tgcgtgcaga aacgcttcca aatccccgtt gtcaaaatgc    42900
   tgaaggatag cttcatcatc ctgattttgg cgtttccttc gtgtctgcca ctggttccac    42960
   ctcgaagcat cataacggga ggagacttct ttaagaccag aaacacgcga gcttggccgt    43020
   cgaatggtca agctgacggt gcccgaggga acggtcggcg gcagacagat ttgtagtcgt    43080
   tcaccaccag gaagttcagt ggcgcagagg gggttacgtg gtccgacatc ctgctttctc    43140
   agcgcgcccg ctaaaatagc gatatcttca agatcatcat aagagacggg caaaggcatc    43200
   ttggtaaaaa tgccggcttg gcgcacaaat gcctctccag gtcgattgat cgcaatttct    43260
   tcagtcttcg ggtcatcgag ccattccaaa atcggcttca gaagaaagcg tagttgcgga    43320
   tccacttcca tttacaatgt atcctatctc taagcggaaa tttgaattca ttaagagcgg    43380
   cggttcctcc cccgcgtggc gccgccagtc aggcggagct ggtaaacacc aaagaaatcg    43440
   aggtcccgtg ctacgaaaat ggaaacggtg tcaccctgat tcttcttcag ggttggcggt    43500
   atgttgatgg ttgccttaag ggctgtctca gttgtctgct caccgttatt ttgaaagctg    43560
   ttgaagctca tcccgccacc cgagctgccg gcgtaggtgc tagctgcctg gaaggcgcct    43620
   tgaacaacac tcaagagcat agctccgcta aaacgctgcc agaagtggct gtcgaccgag 43680
   cccggcaatc ctgagcgacc gagttcgtcc gcgcttggcg atgttaacga gatcatcgca 43740
   tggtcaggtg tctcggcgcg atcccacaac acaaaaacgc gcccatctcc ctgttgcaag 43800
   ccacgctgta tttcgccaac aacggtggtg ccacgatcaa gaagcacgat attgttcgtt 43860
   gttccacgaa tatcctgagg caagacacac tttacatagc ctgccaaatt tgtgtcgatt 43920
   gcggtttgca agatgcacgg aattattgtc ccttgcgtta ccataaaatc ggggtgcggc 43980
   aagagcgtgg cgctgctggg ctgcagctcg gtgggtttca tacgtatcga caaatcgttc 44040
   tcgccggaca cttcgccatt cggcaaggag ttgtcgtcac gcttgccttc ttgtcttcgg 44100
   cccgtgtcgc cctgaatggc gcgtttgctg accccttgat cgccgctgct atatgcaaaa 44160
   atcggtgttt cttccggccg tggctcatgc cgctccggtt cgcccctcgg cggtagagga 44220
   gcagcaggct gaacagcctc ttgaaccgct ggaggatccg gcggcacctc aatcggagct 44280
   ggatgaaatg gcttggtgtt tgttgcgatc aaagttgacg gcgatgcgtt ctcattcacc 44340
   ttcttttggc gcccacctag ccaaatgagg cttaatgata acgcgagaac gacacctccg 44400
   acgatcaatt tctgagaccc cgaaagacgc cggcgatgtt tgtcggagac cagggatcca 44460
   gatgcatcaa cctcatgtgc cgcttgctga ctatcgttat tcatcccttc gcccccttca 44520
   ggacgcgttt cacatcgggc ctcaccgtgc ccgtttgcgg cctttggcca acgggatcgt 44580
   aagcggtgtt ccagatacat agtactgtgt ggccatccct cagacgccaa cctcgggaaa 44640
   ccgaagaaat ctcgacatcg ctccctttaa ctgaatagtt ggcaacagct tccttgccat 44700
   caggattgat ggtgtagatg gagggtatgc gtacattgcc cggaaagtgg aataccgtcg 44760
   taaatccatt gtcgaagact tcgagtggca acagcgaacg atcgccttgg gcgacgtagt 44820
   gccaattact gtccgccgca ccaagggctg tgacaggctg atccaataaa ttctcagctt 44880
   tccgttgata ttgtgcttcc gcgtgtagtc tgtccacaac agccttctgt tgtgcctccc 44940
   ttcgccgagc cgccgcatcg tcggcggggt aggcgaattg gacgctgtaa tagagatcgg 45000
   gctgctcttt atcgaggtgg gacagagtct tggaacttat actgaaaaca taacggcgca 45060
   tcccggagtc gcttgcggtt agcacgatta ctggctgagg cgtgaggacc tggcttgcct 45120
   tgaaaaatag ataatttccc cgcggtaggg ctgctagatc tttgctattt gaaacggcaa 45180
   ccgctgtcac cgtttcgttc gtggcgaatg ttacgaccaa agtagctcca accgccgtcg 45240
   agaggcgcac cacttgatcg ggattgtaag ccaaataacg catgcgcgga tctagcttgc 45300
   ccgccattgg agtgtcttca gcctccgcac cagtcgcagc ggcaaataaa catgctaaaa 45360
   tgaaaagtgc ttttctgatc atggttcgct gtggcctacg tttgaaacgg tatcttccga 45420
   tgtctgatag gaggtgacaa ccagacctgc cgggttggtt agtctcaatc tgccgggcaa 45480
   gctggtcacc ttttcgtagc gaactgtcgc ggtccacgta ctcaccacag gcattttgcc 45540
   gtcaacgacg agggtccttt tatagcgaat ttgctgcgtg cttggagtta catcatttga 45600
   agcgatgtgc tcgacctcca ccctgccgcg tttgccaaga atgacttgag gcgaactggg 45660
   attgggatag ttgaagaatt gctggtaatc ctggcgcact gttggggcac tgaagttcga 45720
   taccaggtcg taggcgtact gagcggtgtc ggcatcataa ctctcgcgca ggcgaacgta 45780
   ctcccacaat gaggcgttaa cgacggcctc ctcttgagtt gcaggcaatc gcgagacaga 45840
   cacctcgctg tcaacggtgc cgtccggccg tatccataga tatacgggca caagcctgct 45900
   caacggcacc attgtggcta tagcgaacgc ttgagcaaca tttcccaaaa tcgcgatagc 45960
   tgcgacagct gcaatgagtt tggagagacg tcgcgccgat ttcgctcgcg cggtttgaaa 46020
   ggcttctact tccttatagt gctcggcaag gctttcgcgc gccactagca tggcatattc 46080
   aggccccgtc atagcgtcca cccgaattgc cgagctgaag atctgacgga gtaggctgcc 46140
   atcgccccac attcagcggg aagatcgggc ctttgcagct cgctaatgtg tcgtttgtct 46200
   ggcagccgct caaagcgaca actaggcaca gcaggcaata cttcatagaa ttctccattg 46260
   aggcgaattt ttgcgcgacc tagcctcgct caacctgagc gaagcgacgg tacaagctgc 46320
   tggcagattg ggttgcgccg ctccagtaac tgcctccaat gttgccggcg atcgccggca 46380
   aagcgacaat gagcgcatcc cctgtcagaa aaaacatatc gagttcgtaa agaccaatga 46440
   tcttggccgc ggtcgtaccg gcgaaggtga ttacaccaag cataagggtg agcgcagtcg 46500
   cttcggttag gatgacgatc gttgccacga ggtttaagag gagaagcaag agaccgtagg 46560
   tgataagttg cccgatccac ttagctgcga tgtcccgcgt gcgatcaaaa atatatccga 46620
   cgaggatcag aggcccgatc gcgagaagca ctttcgtgag aattccaacg gcgtcgtaaa 46680
   ctccgaaggc agaccagagc gtgccgtaaa ggacccactg tgccccttgg aaagcaagga 46740
   tgtcctggtc gttcatcgga ccgatttcgg atgcgatttt ctgaaaaacg gcctgggtca 46800
   cggcgaacat tgtatccaac tgtgccggaa cagtctgcag aggcaagccg gttacactaa 46860
   actgctgaac aaagtttggg accgtctttt cgaagatgga aaccacatag tcttggtagt 46920
   tagcctgccc aacaattaga gcaacaacga tggtgaccgt gatcacccga gtgataccgc 46980
   tacgggtatc gacttcgccg cgtatgacta aaataccctg aacaataatc caaagagtga 47040
   cacaggcgat caatggcgca ctcaccgcct cctggatagt ctcaagcatc gagtccaagc 47100
   ctgtcgtgaa ggctacatcg aagatcgtat gaatggccgt aaacggcgcc ggaatcgtga 47160
   aattcatcga ttggacctga acttgactgg tttgtcgcat aatgttggat aaaatgagct 47220
   cgcattcggc gaggatgcgg gcggatgaac aaatcgccca gccttagggg agggcaccaa 47280
   agatgacagc ggtcttttga tgctccttgc gttgagcggc cgcctcttcc gcctcgtgaa 47340
   ggccggcctg cgcggtagtc atcgttaata ggcttgtcgc ctgtacattt tgaatcattg 47400
   cgtcatggat ctgcttgaga agcaaaccat tggtcacggt tgcctgcatg atattgcgag     47460
   atcgggaaag ctgagcagac gtatcagcat tcgccgtcaa gcgtttgtcc atcgtttcca     47520
   gattgtcagc cgcaatgcca gcgctgtttg cggaaccggt gatctgcgat cgcaacaggt     47580
   ccgcttcagc atcactaccc acgactgcac gatctgtatc gctggtgatc gcacgtgccg     47640
   tggtcgacat tggcattcgc ggcgaaaaca tttcattgtc taggtccttc gtcgaaggat     47700
   actgattttt ctggttgagc gaagtcagta gtccagtaac gccgtaggcc gacgtcaaca     47760
   tcgtaaccat cgctatagtc tgagtgagat tctccgcagt cgcgagcgca gtcgcgagcg     47820
   tctcagcctc cgttgccggg tcgctaacaa caaactgcgc ccgcgcgggc tgaatatata     47880
   gaaagctgca ggtcaaaact gttgcaataa gttgcgtcgt cttcatcgtt tcctacctta     47940
   tcaatcttct gcctcgtggt gacgggccat gaattcgctg agccagccag atgagttgcc     48000
   ttcttgtgcc tcgcgtagtc gagttgcaaa gcgcaccgtg ttggcacgcc ccgaaagcac     48060
   ggcgacatat tcacgcatat cccgcagatc aaattcgcag atgacgcttc cactttctcg     48120
   tttaagaaga aacttacggc tgccgaccgt catgtcttca cggatcgcct gaaattcctt     48180
   ttcggtacat ttcagtccat cgacataagc cgatcgatct gcggttggtg atggatagaa     48240
   aatcttcgtc atacattgcg caaccaagct ggctcctagc ggcgattcca gaacatgctc     48300
   tggttgctgc gttgccagta ttagcatccc gttgtttttt cgaacggtca ggaggaattt     48360
   gtcgacgaca gtcgaaaatt tagggtttaa caaataggcg cgaaactcat cgcagctcat     48420
   cacaaaacgg cggccgtcga tcatggctcc aatccgatgc aggagatatg ctgcagcggg     48480
   agcgcatact tcctcgtatt cgagaagatg cgtcatgtcg aagccggtaa tcgacggatc     48540
   taactttact tcgtcaactt cgccgtcaaa tgcccagcca agcgcatggc cccggcacca     48600
   gcgttggagc cgcgctcctg cgccttcggc gggcccatgc aacaaaaatt cacgtaaccc     48660
   cgcgattgaa cgcatttgtg gatcaaacga gagctgacga tggataccac ggaccagacg     48720
   gcggttctct tccggagaaa tcccaccccg accatcactc tcgatgagag ccacgatcca     48780
   ttcgcgcaga aaatcgtgtg aggctgctgt gttttctagg ccacgcaacg gcgccaaccc     48840
   gctgggtgtg cctctgtgaa gtgccaaata tgttcctcct gtggcgcgaa ccagcaattc     48900
   gccaccccgg tccttgtcaa agaacacgac cgtacctgca cggtcgacca tgctctgttc     48960
   gagcatggct agaacaaaca tcatgagcgt cgtcttaccc ctcccgatag gcccgaatat     49020
   tgccgtcatg ccaacatcgt gctcatgcgg gatatagtcg aaaggcgttc cgccattggt     49080
   acgaaatcgg gcaatcgcgt tgccccagtg gcctgagctg gcgccctctg gaaagttttc     49140
   gaaagagaca aaccctgcga aattgcgtga agtgattgcg ccagggcgtg tgcgccactt     49200
   aaaattcccc ggcaattggg accaataggc cgcttccata ccaatacctt cttggacaac     49260
   cacggcacct gcatccgcca ttcgtgtccg agcccgcgcg cccctgtccc caagactatt     49320
   gagatcgtct gcatagacgc aaaggctcaa atgatgtgag cccataacga attcgttgct     49380
   cgcaagtgcg tcctcagcct cggataattt gccgatttga gtcacggctt tatcgccgga     49440
   actcagcatc tggctcgatt tgaggctaag tttcgcgtgc gcttgcgggc gagtcaggaa     49500
   cgaaaaactc tgcgtgagaa caagtggaaa atcgagggat agcagcgcgt tgagcatgcc     49560
   cggccgtgtt tttgcagggt attcgcgaaa cgaatagatg gatccaacgt aactgtcttt     49620
   tggcgttctg atctcgagtc ctcgcttgcc gcaaatgact ctgtcggtat aaatcgaagc     49680
   gccgagtgag ccgctgacga ccggaaccgg tgtgaaccga ccagtcatga tcaaccgtag     49740
   cgcttcgcca atttcggtga agagcacacc ctgcttctcg cggatgccaa gacgatgcag     49800
   gccatacgct ttaagagagc cagcgacaac atgccaaaga tcttccatgt tcctgatctg     49860
   gcccgtgaga tcgttttccc tttttccgct tagcttggtg aacctcctct ttaccttccc     49920
   taaagccgcc tgtgggtaga caatcaacgt aaggaagtgt tcattgcgga ggagttggcc     49980
   ggagagcacg cgctgttcaa aagcttcgtt caggctagcg gcgaaaacac tacggaagtg     50040
   tcgcggcgcc gatgatggca cgtcggcatg acgtacgagg tgagcatata ttgacacatg     50100
   atcatcagcg atattgcgca acagcgtgtt gaacgcacga caacgcgcat tgcgcatttc     50160
   agtttcctca agctcgaatg caacgccatc aattctcgca atggtcatga tcgatccgtc     50220
   ttcaagaagg acgatatggt cgctgaggtg gccaatataa gggagataga tctcaccgga     50280
   tctttcggtc gttccactcg cgccgagcat cacaccattc ctctccctcg tgggggaacc     50340
   ctaattggat ttgggctaac agtagcgccc ccccaaactg cactatcaat gcttcttccc     50400
   gcggtccgca aaaatagcag gacgacgctc gccgcattgt agtctcgctc cacgatgagc     50460
   cgggctgcaa accataacgg cacgagaacg acttcgtaga gcgggttctg aacgataacg     50520
   atgacaaagc cggcgaacat catgaataac cctgccaatg tcagtggcac cccaagaaac     50580
   aatgcgggcc gtgtggctgc gaggtaaagg gtcgattctt ccaaacgatc agccatcaac     50640
   taccgccagt gagcgtttgg ccgaggaagc tcgccccaaa catgataaca atgccgccga     50700
   cgacgccggc aaccagccca agcgaagccc gcccgaacat ccaggagatc ccgatagcga     50760
   caatgccgag aacagcgagt gactggccga acggaccaag gataaacgtg catatattgt     50820
   taaccattgt ggcggggtca gtgccgccac ccgcagattg cgctgcggcg ggtccggatg     50880
   aggaaatgct ccatgcaatt gcaccgcaca agcttggggc gcagctcgat atcacgcgca     50940
   tcatcgcatt cgagagcgag aggcgattta gatgtaaacg gtatctctca aagcatcgca     51000
   tcaatgcgca cctccttagt ataagtcgaa taagacttga ttgtcgtctg cggatttgcc     51060
   gttgtcctgg tgtggcggtg gcggagcgat taaaccgcca gcgccatcct cctgcgagcg     51120
   gcgctgatat gaccccccaa catcccacgt ctcttcggat tttagcgcct cgtgatcgtc     51180
   ttttggaggc tcgattaacg cgggcaccag cgattgagca gctgtttcaa cttttcgcac 51240
   gtagccgttt gcaaaaccgc cgatgaaatt accggtgttg taagcggaga tcgcccgacg 51300
   aagcgcaaat tgcttctcgt caatcgtttc gccgcctgca taacgacttt tcagcatgtt 51360
   tgcagcggca gataatgatg tgcacgcctg gagcgcaccg tcaggtgtca gaccgagcat 51420
   agaaaaattt cgagagttta tttgcatgag gccaacatcc agcgaatgcc gtgcatcgag 51480
   acggtgcctg acgacttggg ttgcttggct gtgatcttgc cagtgaagcg tttcgccggt 51540
   cgtgttgtca tgaatcgcta aaggatcaaa gcgactctcc accttagcta tcgccgcaag 51600
   cgtagatgtc gcaactgatg gggcacactt gcgagcaaca tggtcaaact cagcagatga 51660
   gagtggcgtg gcaaggctcg acgaacagaa ggagaccatc aaggcaagag aaagcgaccc 51720
   cgatctctta agcatacctt atctccttag ctcgcaacta acaccgcctc tcccgttgga 51780
   agaagtgcgt tgttttatgt tgaagattat cgggagggtc ggttactcga aaattttcaa 51840
   ttgcttcttt atgatttcaa ttgaagcgag aaacctcgcc cggcgtcttg gaacgcaaca 51900
   tggaccgaga accgcgcatc catgactaag caaccggatc gacctattca ggccgcagtt 51960
   ggtcaggtca ggctcagaac gaaaatgctc ggcgaggtta cgctgtctgt aaacccattc 52020
   gatgaacggg aagcttcctt ccgattgctc ttggcaggaa tattggccca tgcctgcttg 52080
   cgctttgcaa atgctcttat cgcgttggta tcatatgcct tgtccgccag cagaaacgca 52140
   ctctaagcga ttatttgtaa aaatgtttcg gtcatgcggc ggtcatgggc ttgacccgct 52200
   gtcagcgcaa gacggatcgg tcaaccgtcg gcatcgacaa cagcgtgaat cttggtggtc 52260
   aaaccgccac gggaacgtcc catacagcca tcgtcttgat cccgctgttt cccgtcgccg 52320
   catgttggtg gacgcggaca caggaactgt caatcatgac gacattctat cgaaagcctt 52380
   ggaaatcaca ctcagaatat gatcccagac gtctgcctca cgccatcgta caaagcgatt 52440
   gtagcaggtt gtacaggaac cgtatcgatc aggaacgtct gcccagggcg ggcccgtccg 52500
   gaagcgccac aagatgacat tgatcacccg cgtcaacgcg cggcacgcga cgcggcttat 52560
   ttgggaacaa aggactgaac aacagtccat tcgaaatcgg tgacatcaaa gcggggacgg 52620
   gttatcagtg gcctccaagt caagcctcaa tgaatcaaaa tcagaccgat ttgcaaacct 52680
   gatttatgag tgtgcggcct aaatgatgaa atcgtccttc tagatcgcct ccgtggtgta 52740
   gcaacacctc gcagtatcgc cgtgctgacc ttggccaggg aattgactgg caagggtgct 52800
   ttcacatgac cgctcttttg gccgcgatag atgatttcgt tgctgctttg ggcacgtaga 52860
   aggagagaag tcatatcgga gaaattcctc ctggcgcgag agcctgctct atcgcgacgg 52920
   catcccactg tcgggaacag accggatcat tcacgaggcg aaagtcgtca acacatgcgt 52980
   tataggcatc ttcccttgaa ggatgatctt gttgctgcca atctggaggt gcggcagccg 53040
   caggcagatg cgatctcagc gcaacttgcg gcaaaacatc tcactcacct gaaaaccact 53100
   agcgagtctc gcgatcagac gaaggccttt tacttaacga cacaatatcc gatgtctgca 53160
   tcacaggcgt cgctatccca gtcaatacta aagcggtgca ggaactaaag attactgatg 53220
   acttaggcgt gccacgaggc ctgagacgac gcgcgtagac agttttttga aatcattatc 53280
   aaagtgatgg cctccgctga agcctatcac ctctgcgccg gtctgtcgga gagatgggca 53340
   agcattatta cggtcttcgc gcccgtacat gcattggacg attgcagggt caatggatct 53400
   gagatcatcc agaggattgc cgcccttacc ttccgtttcg agttggagcc agcccctaaa 53460
   tgagacgaca tagtcgactt gatgtgacaa tgccaagaga gagatttgct taacccgatt 53520
   tttttgctca agcgtaagcc tattgaagct tgccggcatg acgtccgcgc cgaaagaata 53580
   tcctacaagt aaaacattct gcacaccgaa atgcttggtg tagacatcga ttatgtgacc 53640
   aagatcctta gcagtttcgc ttggggaccg ctccgaccag aaataccgaa gtgaactgac 53700
   gccaatgaca ggaatccctt ccgtctgcag ataggtacca tcgatagatc tgctgcctcg 53760
   cgcgtttcgg tgatgacggt gaaaacctct gacacatgca gctcccggag acggtcacag 53820
   cttgtctgta agcggatgcc gggagcagac aagcccgtca gggcgcgtca gcgggtgttg 53880
   gcgggtgtcg gggcgcagcc atgacccagt cacgtagcga tagcggagtg tatactggct 53940
   taactatgcg gcatcagagc agattgtact gagagtgcac catatgcggt gtgaaatacc 54000
   gcacagatgc gtaaggagaa aataccgcat caggcgctct tccgcttcct cgctcactga 54060
   ctcgctgcgc tcggtcgttc ggctgcggcg agcggtatca gctcactcaa aggcggtaat 54120
   acggttatcc acagaatcag gggataacgc aggaaagaac atgtgagcaa aaggccagca 54180
   aaaggccagg aaccgtaaaa aggccgcgtt gctggcgttt ttccataggc tccgcccccc 54240
   tgacgagcat cacaaaaatc gacgctcaag tcagaggtgg cgaaacccga caggactata 54300
   aagataccag gcgtttcccc ctggaagctc cctcgtgcgc tctcctgttc cgaccctgcc 54360
   gcttaccgga tacctgtccg cctttctccc ttcgggaagc gtggcgcttt ctcatagctc 54420
   acgctgtagg tatctcagtt cggtgtaggt cgttcgctcc aagctgggct gtgtgcacga 54480
   accccccgtt cagcccgacc gctgcgcctt atccggtaac tatcgtcttg agtccaaccc 54540
   ggtaagacac gacttatcgc cactggcagc agccactggt aacaggatta gcagagcgag 54600
   gtatgtaggc ggtgctacag agttcttgaa gtggtggcct aactacggct acactagaag 54660
   gacagtattt ggtatctgcg ctctgctgaa gccagttacc ttcggaaaaa gagttggtag 54720
   ctcttgatcc ggcaaacaaa ccaccgctgg tagcggtggt ttttttgttt gcaagcagca 54780
   gattacgcgc agaaaaaaag gatctcaaga agatcctttg atcttttcta cggggtctga 54840
   cgctcagtgg aacgaaaact cacgttaagg gattttggtc atgagattat caaaaaggat 54900
   cttcacctag atccttttaa attaaaaatg aagttttaaa tcaatctaaa gtatatatga 54960
   gtaaacttgg tctgacagtt accaatgctt aatcagtgag gcacctatct cagcgatctg    55020
   tctatttcgt tcatccatag ttgcctgact ccccgtcgtg tagataacta cgatacggga    55080
   gggcttacca tctggcccca gtgctgcaat gataccgcga gacccacgct caccggctcc    55140
   agatttatca gcaataaacc agccagccgg aagggccgag cgcagaagtg gtcctgcaac    55200
   tttatccgcc tccatccagt ctattaattg ttgccgggaa gctagagtaa gtagttcgcc    55260
   agttaatagt ttgcgcaacg ttgttgccat tgctgcaggg gggggggggg gggggttcca    55320
   ttgttcattc cacggacaaa aacagagaaa ggaaacgaca gaggccaaaa agctcgcttt    55380
   cagcacctgt cgtttccttt cttttcagag ggtattttaa ataaaaacat taagttatga    55440
   cgaagaagaa cggaaacgcc ttaaaccgga aaattttcat aaatagcgaa aacccgcgag    55500
   gtcgccgccc cgtaacct                                                  55518
<210> 48
   <211> 61
   <212> DNA
   <213> artificial sequence
<220>
   <223> reference sequence
<400> 48
   cacgtatata tacgcgtacg cgtacgtgtg aggtatatat atcctccgcc ggggcacgta       60
   c                                                                       61
<210> 49
   <211> 62
   <212> DNA
   <213> artificial sequence
<220>
   <223> sequence on Fig. 1
<400> 49
   cacgtatata tacgcgtacg cgtacgttgt gaggtatata tatcctccgc cggggcacgt       60
   ac                                                                      62
<210> 50
   <211> 60
   <212> DNA
   <213> artificial sequence
<220>
   <223> sequence on Fig. 1
<400> 50
   cacgtatata tacgcgtacg cgtacggtga ggtatatata tcctccgccg gggcacgtac 60
<210> 51
   <211> 60
   <212> DNA
   <213> artificial sequence
<220>
   <223> sequence on Fig. 1
<400> 51
   cacgtatata tacgcgtacg cgtactgtga ggtatatata tcctccgccg gggcacgtac 60
<210> 52
   <211> 59
   <212> DNA
   <213> artificial sequence
<220>
   <223> sequence on Fig. 1
<400> 52
   cacgtatata tacgcgtacg cgtacgtgag gtatatatat cctccgccgg ggcacgtac 59
<210> 53
   <211> 32
   <212> DNA
   <213> artificial sequence
<220>
   <223> sequence on Fig. 1
<400> 53
   cacgtatata tatcctccgc cggggcacgt ac 32
<210> 54
   <211> 19
   <212> DNA
   <213> artificial sequence
<220>
   <223> sequence on Fig. 1
<400> 54
   cacgtatata tacgcgtac 19
<210> 55
   <211> 57
   <212> DNA
   <213> artificial sequence
<220>
   <223> sequence on Fig. 1
<400> 55
   cacgtatata tacgcgtacg cgtgtgaggt atatatatcc tccgccgggg cacgtac 57
<210> 56
   <211> 33
   <212> DNA
   <213> artificial sequence
<220>
   <223> sequence on Fig. 1
<400> 56
   cacgtatata tacgcgtacg ccggggcacg tac 33
<210> 57
   <211> 30
   <212> DNA
   <213> artificial sequence
<220>
   <223> sequence on Fig. 1
<400> 57
   cacgtatata tcctccgccg gggcacgtac 30
<210> 58
   <211> 59
   <212> DNA
   <213> artificial sequence
<220>
   <223> sequence on Fig. 1
<400> 58
   cacgtatata tacgcgtacg cgtatgtgag gtatatatat cctccgccgg ggcacgtac 59
<210> 59
   <211> 54
   <212> DNA
   <213> artificial sequence
<220>
   <223> sequence on Fig. 2
<400> 59
   gcgctgctcg attccgtccc catggtcgcc atcacgggac aggtgccgcg acgc 54
<210> 60
   <211> 18
   <212> PRT
   <213> artificial sequence
<220>
   <223> sequence on Fig. 2
<400> 60
   Ala Leu Leu Asp Ser Val Pro Met Val Ala Ile Thr Gly Gln Val Pro
   1               5                   10                  15
   Arg Arg
<210> 61
   <211> 54
   <212> DNA
   <213> artificial sequence
<220>
   <223> sequence on Fig. 2
<400> 61
   gcgttgctcg actccgtccc cattgtcgcc atcacgggac aggtgtcgcg acgc 54
<210> 62
   <211> 18
   <212> PRT
   <213> artificial sequence
<220>
   <223> sequence on Fig. 2
<400> 62
   Ala Leu Leu Asp Ser Val Pro Ile Val Ala Ile Thr Gly Gln Val Ser
   1               5                   10                  15
   Arg Arg
<210> 63
   <211> 54
   <212> DNA
   <213> artificial sequence
<220>
   <223> sequence on Fig. 2
<400> 63
   gcgttgctgg actccgtgcc gatggtcgcc atcacgggac aggtgtcccg acgc 54
<210> 64
   <211> 18
   <212> PRT
   <213> artificial sequence
<220>
   <223> sequence on Fig. 2
<400> 64
   Ala Leu Leu Asp Ser Val Pro Met Val Ala Ile Thr Gly Gln Val Ser
   1               5                   10                  15
   Arg Arg
<210> 65
   <211> 31
   <212> DNA
   <213> artificial sequence
<220>
   <223> sequence on Fig. 4
<400> 65
   atggctcccc cggccacccc gctccggccg t 31
<210> 66
   <211> 10
   <212> PRT
   <213> artificial sequence
<220>
   <223> sequence on Fig. 4
<400> 66
   Met Ala Pro Pro Ala Thr Pro Leu Arg Pro
   1 5 10
<210> 67
   <211> 30
   <212> DNA
   <213> artificial sequence
<220>
   <223> sequence on Fig. 4
<400> 67
   atggctcccc cggccacccc ctccggccgt 30
<210> 68
   <211> 10
   <212> PRT
   <213> artificial sequence
<220>
   <223> sequence on Fig. 4
<400> 68
   Met Ala Pro Pro Ala Thr Pro Ser Gly Arg
   1 5 10
<210> 69
   <211> 31
   <212> DNA
   <213> artificial sequence
<220>
   <223> sequence on Fig. 4
<220>
   <221> misc_feature
   <222> (21)..(21)
   <223> n is a, c, g, or t
<400> 69
   atggctcccc cggccacccc nctccggccg t 31
<210> 70
   <211> 40
   <212> DNA
   <213> artificial sequence
<220>
   <223> sequence on Fig. 5
<400> 70
   tcgactcgct caccatgtcc ggcccatgac caccgccgcc 40
<210> 71
   <211> 41
   <212> DNA
   <213> artificial sequence
<220>
   <223> sequence on Fig. 5
<220>
   <221> misc_feature
   <222> (17)..(17)
   <223> n is a, c, g, or t
<400> 71
   tcgactcgct caccatngtc cggcccatga ctcccccggc c 41
<210> 72
   <211> 24
   <212> DNA
   <213> artificial sequence
<220>
   <223> sequence on Fig. 6
<400> 72
   attcccccgg ccaccccgtc ggcc 24
<210> 73
   <211> 8
   <212> PRT
   <213> artificial sequence
<220>
   <223> sequence on Fig. 6
<400> 73
   Ile Pro Pro Ala Thr Pro Ser Ala
   1               5
<210> 74
   <211> 23
   <212> DNA
   <213> artificial sequence
<220>
   <223> sequence on Fig. 6
<400> 74
   attcccccgg ccacccgtcg gcc 23
<210> 75
   <211> 7
   <212> PRT
   <213> artificial sequence
<220>
   <223> sequence on Fig. 6
<400> 75
   Ile Pro Pro Ala Thr Arg Arg
   1 5
<210> 76
   <211> 24
   <212> DNA
   <213> artificial sequence
<220>
   <223> sequence on Fig. 6
<400> 76
   attcccccgg ccacctcgtc ggcc 24
<210> 77
   <211> 8
   <212> PRT
   <213> artificial sequence
<220>
   <223> sequence on Fig. 6
<400> 77
   Ile Pro Pro Ala Thr Ser Ser Ala
   1 5
<210> 78
   <211> 441
   <212> DNA
   <213> Artificial Sequence
<220>
   <223> IN2 promoter
<400> 78
   atccctggcc accaaacatc cctaatcatc cccaaatttt ataggaacta ctaatttctc      60
   taacttaaaa aaaatctaaa atagtatact ttagcagcct ctcaatctga tttgttcccc     120
   aaatttgaat cctggcttcg ctctgtcacc tgttgtactc tacatggtgc gcagggggag     180
   agcctaatct ttcacgactt tgtttgtaac tgttagccag accggcgtat ttgtcaatgt     240
   ataaacacgt aataaaattt acgtaccata tagtaagact ttgtatataa gacgtcacct     300
   cttacgtgca tggttatatg cgacatgtgc agtgacgtta tcagatatag ctcaccctat     360
   atatatagct ctgtccggtg tcagtagcaa tcaccattca tcagcacccc ggcaggtcga     420
   ccccgagctc cctgcacctg c                                               441
<210> 79
   <211> 21
   <212> DNA
   <213> Zea mays
<400> 79
   gctcccccgg ccaccccgct c 21
<210> 80
   <211> 20
   <212> DNA
   <213> Zea mays
<400> 80
   gctcccccgg ccaccccctc 20
<210> 81
   <211> 20
   <212> DNA
   <213> Zea mays
<400> 81
   cgccgagggc gactaccggc 20
<210> 82
   <211> 23
   <212> DNA
   <213> Zea mays
<400> 82
   cgccgagggc gactaccggc agg 23

## Description

### FIELD

The disclosure relates to the field of molecular biology, in particular, to methods for altering the genome of a cell.

### BACKGROUND

Recombinant DNA technology has made it possible to insert DNA sequences at targeted genomic locations and/or modify (edit) specific endogenous chromosomal sequences, thus altering the organism's phenotype. Site-specific integration techniques, which employ site-specific recombination systems, as well as other types of recombination technologies, have been used to generate targeted insertions of genes of interest in a variety of organism. Genome-editing techniques such as designer zinc finger nucleases (ZFNs) or transcription activator-like effector nucleases (TALENs), or homing meganucleases, are available for producing targeted genome perturbations, but these systems tends to have a low specificity and employ designed nucleases that need to be redesigned for each target site, which renders them costly and time-consuming to prepare.

Although several approaches have been developed to target a specific site for modification in the genome of an organism, there still remains a need for new genome engineering technologies that are affordable, easy to set up, scalable, and amenable to targeting multiple positions within the genome of an organism.

### BRIEF SUMMARY

The invention provides a method comprising introducing through particle mediated delivery a ribonucleoprotein complex comprising a Cas endonuclease protein and a guide RNA into a plant cell containing a plant cell wall, wherein the guide RNA comprises a polynucleotide sequence complementary to a genomic target site sequence in the genome of the plant cell; wherein the Cas endonuclease protein and the guide RNA are applied to a particle delivery matrix comprising a microparticle; and wherein the Cas endonuclease protein and the guide RNA bind to the genomic target site sequence in the genome of the plant cell.

The invention further provides a method comprising:
(a) introducing through particle mediated delivery into a plant cell containing a plant cell wall a microparticle coated with a ribonucleoprotein complex comprising a Cas endonuclease protein and a guide RNA, and
(b) incubating the plant cell to allow the ribonucleoprotein complex to bind to a target sequence in the genome of the plant cell;
wherein the ribonucleoprotein complex modifies the target sequence by the insertion of at least one nucleotide, the deletion of at least one nucleotide, or the substitution of at least one nucleotide.

Yet further provided by the invention is a composition comprising a plant cell containing a plant cell wall and a particle delivery matrix, wherein the particle delivery matrix comprises:
(a) a microparticle; and
(b) a ribonucleoprotein complex comprising a Cas endonuclease protein and a guide RNA molecule comprising a polynucleotide sequence complementary to a genomic target site sequence in the genome of the plant cell;
wherein the microparticle is associated with the ribonucleoprotein complex.

Further aspects and preferred embodiments are defined in the dependent claims. Any aspects, embodiments, and examples of the present disclosure which do not fall under the scope of the appended claims do not form part of the invention and are merely provided for illustrative purposes.

### SUMMARY OF TECHNICAL TEACHINGS

Compositions and methods are disclosed herein for restoring function to a non-functional gene product in the genome of a cell. The methods and compositions employ a guide polynucleotide /Cas endonuclease system to restore function to a non-functional gene product and to provide an effective system for modifying or altering target sites within the genome of a plant, plant cell or seed. The present disclosure also describes methods for modifying a nucleotide sequence in the genome of a cell using a restored functional selectable marker, as well as methods for editing a nucleotide sequence in the genome a cell without introducing a polynucleotide modification template into said cell. Compositions and methods are also disclosed herein for DNA free delivery of Cas endonucleases, guide RNAs and guide RNA/Cas complexes by introducing the components of the complex via mRNA molecules (guide RNA, Cas endonuclease) or protein molecules (Cas endonuclease) or by directly introducing the guide RNA/Cas ribonucleotide-protein complex (RGEN) itself into the cell.

In one aspect of the disclosure, the method comprises a method for restoring function to a non-functional gene product in the genome of a cell, the method comprising introducing a guide RNA/Cas endonuclease complex into a cell comprising a disrupted gene in its genome, wherein said complex creates a double strand break, wherein said disrupted gene does not encode a functional gene product, wherein said disrupted gene is restored without the use of a polynucleotide modification template to a non-disrupted gene capable of encoding said functional gene product. The disrupted gene can comprise a base pair deletion of the 4^{th} nucleotide upstream (5') of a PAM sequence when compared to its corresponding non-disrupted gene, wherein said base pair deletion creates an amino acid frameshift in the gene product of the disrupted gene thereby rendering the gene product of the disrupted gene non-functional. The base pair deletion can be located at the first, second or third nucleotide of a codon sequence. The restoration can be accomplished by Non-Homologous-End -Joining (NHEJ) resulting in the insertion of a single base at the double strand break site, or can be accomplished by the insertion of a single base at the double strand break site without the use of Homologous Recombination or Homology Directed Repair

In one aspect of the disclosure, the method comprises a method for modifying a nucleotide sequence in the genome of a cell, the method comprising: introducing into at least one cell comprising a target site and a disrupted selectable marker gene, a first guide RNA, a Cas endonuclease, and at least a second guide RNA, wherein said first guide RNA and Cas endonuclease can form a first complex capable of introducing a double strand break in said disrupted selectable marker gene, wherein said disrupted selectable marker gene is restored without the use of a polynucleotide modification template to a non-disrupted selectable marker gene capable of encoding a functional selectable marker protein wherein said second guide RNA and Cas endonuclease can form a second complex that is capable of recognizing, binding to, and nicking or cleaving said target site located in said nucleotide sequence; and, selecting a cell having a modification in said nucleotide sequence, wherein the selection is provided by said functional selectable marker protein. The introducing and selection step does not comprise the introduction of a selectable marker gene, such as a recombinant DNA construct comprising a selectable marker gene. The disrupted selectable marker gene can be any disrupted marker gene including a disrupted visible marker gene. The modification in the targeted nucleotide sequence can be selected from the group consisting of an insertion of at least one nucleotide, a deletion of at least one nucleotide, or a substitution of at least one nucleotide in said target site. The method can further comprise introducing a polynucleotide modification template into said cell, wherein said polynucleotide modification template comprises at least one nucleotide modification of said nucleotide sequence, or a donor DNA wherein said donor DNA comprises at least one polynucleotide of interest to be inserted into said target site.

In one aspect of the disclosure, the method comprises a method for editing a nucleotide sequence in the genome of a cell without the use of a polynucleotide modification template, the method comprising: a) introducing into at least one cell at least one guide RNA and at least one Cas endonuclease, wherein said guide RNA and Cas endonuclease can form a complex capable of introducing a double strand break in said nucleotide sequence; b) selecting a cell from (a) comprising at least one single nucleotide deletion in said nucleotide sequence, wherein said nucleotide deletion is located at a position to be edited; and, c) introducing into a cell of (b) at least one guide RNA and at least one Cas endonuclease, wherein said guide RNA and Cas endonuclease can form a complex capable of introducing a double strand break in said nucleotide sequence and insert a single nucleotide at the same position of the nucleotide deletion of (b) without the use of a polynucleotide modification template.

In one aspect of the disclosure, the method comprises a method for editing a nucleotide sequence in the genome of a plant without the use of a polynucleotide modification template or donor DNA, the method comprising: a) introducing into at least one plant cell at least one guide RNA and at least one Cas endonuclease, wherein said guide RNA and Cas endonuclease can form a complex capable of introducing a double strand break in said nucleotide sequence; b) selecting a plant cell from (a) comprising at least one single nucleotide deletion in said nucleotide sequence, wherein said nucleotide deletion is located at a position to be edited; c) regenerating a plant from the plant cell of (b); d) introducing into a cell from the plant of (c) at least one guide RNA and at least one Cas endonuclease, wherein said guide RNA and Cas endonuclease can form a complex capable of introducing a double strand break in said nucleotide sequence and inserting a single nucleotide at the same position of the nucleotide deletion of (b) without the use of a polynucleotide modification template; and, e) optimally, selecting a cell comprising the nucleotide insertion of (d).

The guide RNA and Cas endonuclease protein forming the guide RNA /Cas endonuclease complex can be introduced into the cell directly as RNA and protein, respectively, or as a ribonucleotide-protein complex. The components of the guide RNA/Cas endonuclease complex can be introduced as mRNA molecules encoding the Cas endonuclease protein and as RNA comprising the guide RNA, or as recombinant DNA molecules encoding the guide RNA and the Cas endonuclease protein.

In one aspect of the disclosure, the method comprises a DNA free method of delivering a guide RNA /Cas endonuclease complex into a cell, the method comprising combining at least one guide RNA molecule and at least one Cas endonuclease protein to form a ribonucleotide-protein and combining said ribonucleotide-protein with a particle delivery matrix to allow for said ribonucleotide-protein and matrix to bind and form a ribonucleotide-protein-matrix complex; and, introducing said ribonucleotide-protein-matrix complex into said cell.

In one aspect of the disclosure, the method comprises a DNA free method of delivering guide RNA /Cas endonuclease components into a cell, the method comprising introducing at least one guide RNA molecule and at least one Cas endonuclease protein into a cell, and growing said cell under suitable conditions to allow said guide RNA and said Cas endonuclease protein to form a complex inside said cell.

The DNA free method can further comprise introducing a polynucleotide template, wherein said polynucleotide modification template comprises at least one nucleotide modification of a nucleotide sequence in the genome of said cell, wherein said at least one nucleotide modification of said polynucleotide modification template is selected from the group consisting of (i) a replacement of at least one nucleotide, (ii) a deletion of at least one nucleotide, (iii) an insertion of at least one nucleotide, and (iv) any combination of (i) - (iii). The DNA free method can also further comprise introducing a donor DNA, wherein said donor DNA comprises at least one polynucleotide of interest. The introduction into the cell can be via a delivery system selected from the group consisting of particle mediated delivery, whisker mediated delivery, cell-penetrating peptide mediated delivery, electroporation, PEP-mediated transfection and nanoparticle mediated delivery.

Cells include human, non-human, animal, archaea, bacterial, fungal, insect, yeast, non-conventional yeast, and plant cell.

Also disclosed herein are nucleic acid constructs, cells, plants, progeny plants, microorganisms, explants, seeds and grain produced by the methods described herein. Additional aspects of the methods and compositions of the present disclosure are shown herein.

### BRIEF DESCRIPTION OF THE DRAWINGS AND THE SEQUENCE LISTING

The disclosure can be more fully understood from the following detailed description and the accompanying drawings and Sequence Listing, which form a part of this application. The sequence descriptions and sequence listing attached hereto comply with the rules governing nucleotide and amino acid sequence disclosures in patent applications as set forth in 37 C.F.R. §§1.821-1.825. The sequence descriptions contain the three letter codes for amino acids as defined in 37 C.F.R. §§ 1.821-1.825.

### Figures

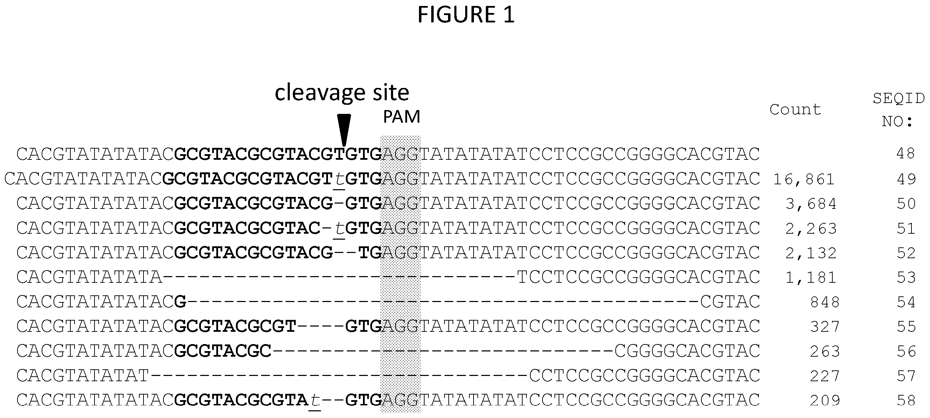

Figure 1 depicts an alignment and count of the top 10 most frequent NHEJ mutations induced by the maize optimized guide RNA/Cas endonuclease system described herein. The mutations were identified by deep sequencing. The reference sequence (SEQ ID NO: 48) represents the unmodified locus with each target site shown in bold. The PAM sequence (grey) and expected site of cleavage (arrow) are also indicated. Deletions or insertions as a result of imperfect NHEJ are shown by a "-" or an italicized underlined nucleotide, respectively. The reference and mutations 1-10 of the target site correspond to SEQ ID NOs: 49-58, respectively. In maize, for the majority of target sites, the most prevalent type of mutation generated by Cas9-gRNA system is a single nucleotide insertion (≥ 60%) (count shown as 16,861).

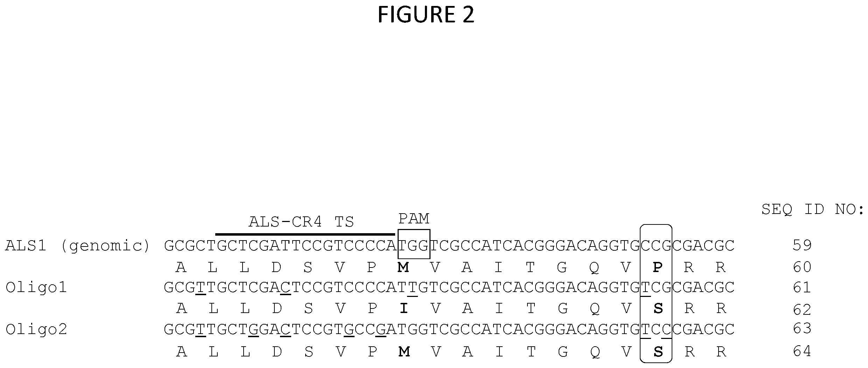

Figure 2 depicts partial nucleotide sequences ((SEQ ID NOs: 59, 61, 63) and partial amino acid sequences (SEQ ID NOs: 60, 62, 64) of the ALS2 gene and two editing repair templates (Oligo1 and Oligo2); modified nucleotides are underlined and the codon sequence targeted for gene editing (Pro to Ser) is boxed.

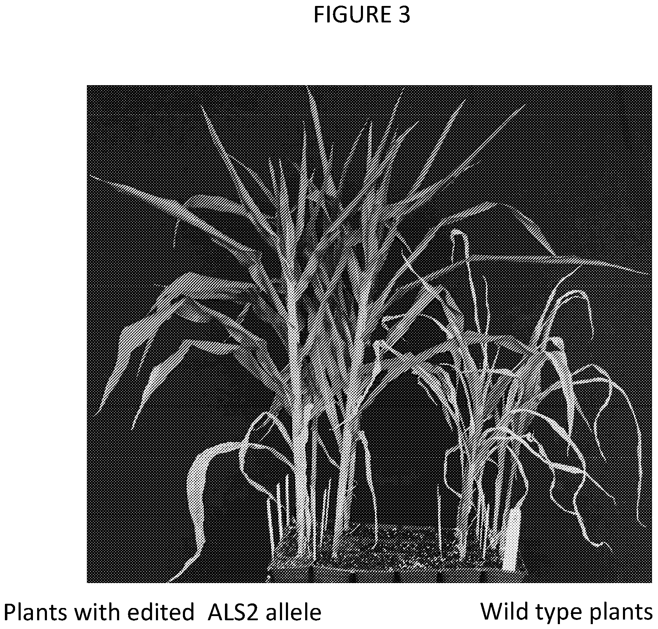

Figure 3 depicts maize plants having an edited ALS2 allele for resistance to chlorsulfuron (left) and wild type plants (right). Four-week old plants were sprayed with chlorsulfuron (100 mg/L). Plants are shown three weeks after the treatment.

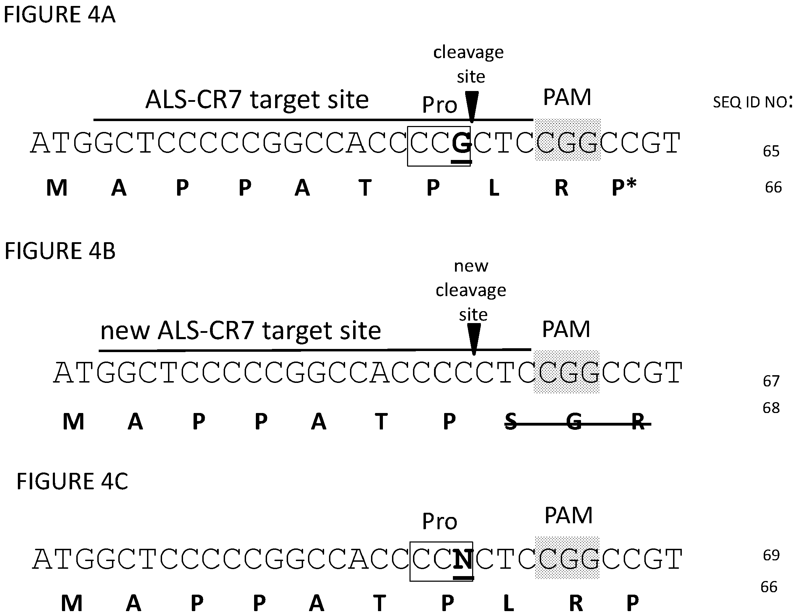

Figure 4A-4C shows a schematic of a fragment of the ALS2 gene (SEQ ID NOs: 65, 67, 69) selected for modification and use of ALS2 as a selectable marker. The encoded amino acid sequences are shown below each nucleotide sequence. (SEQ ID NOs: 66, 68). Figure 4A: A single nucleotide (G) in position 165 (bold and underlined) can be removed in order to generate a specific knock-out version of the edited for chlorsulfuron resistance ALS2 gene. Figure 4B depicts the new nucleotide sequence with a single nucleotide deletion (G removed) resulting in the translational frame shift and ALS2 gene knock-out. Figure 4C: The ALS2 gene function and chlorsulfuron resistance are restored through insertion of a single nucleotide (N, bold and underlined) during the process of DSB repair via NHEJ pathway.

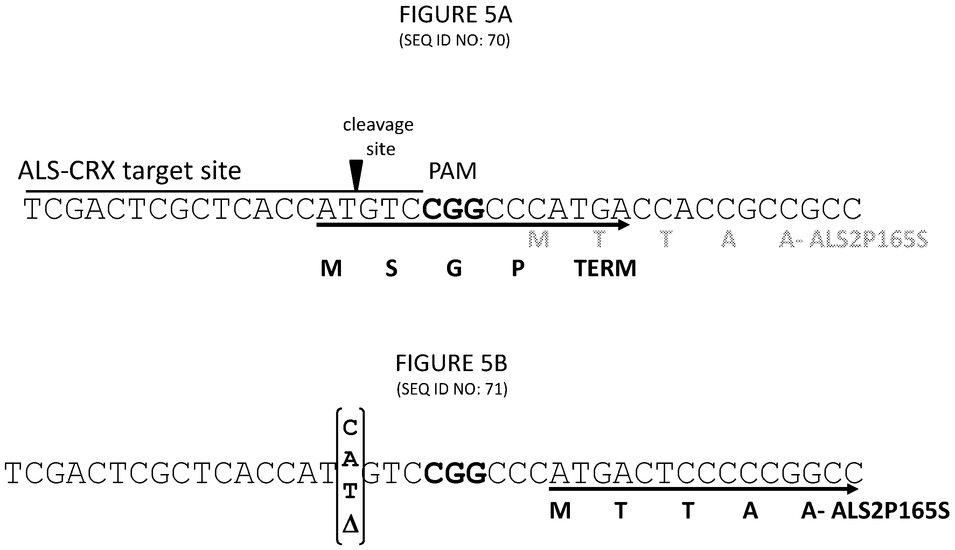

Figure 5A-5B. Re-activation of inactivated ALS2P165S as selectable marker. Figure 5A (SEQ ID NO: 70). A design of ALS 2P165S gene containing upstream out-of-frame translational start codon located 3 nucleotides 5' of PAM. Initiation of translation at the first AUG (depicted by arrow below sequence) encodes a 4 amino acid polypeptide which prevents the initiation of translation start codon of ALS2 (grey letters). Figure 5B (SEQ ID NO: 71). Single nucleotide insertion (C, A or T) or deletion (or any combination) that results in the loss of the upstream AUG allows initiation of translation at the start codon of ALS2 (depicted by arrow below sequence) restoring translation of the full-length ALS2P165S herbicide resistance gene.

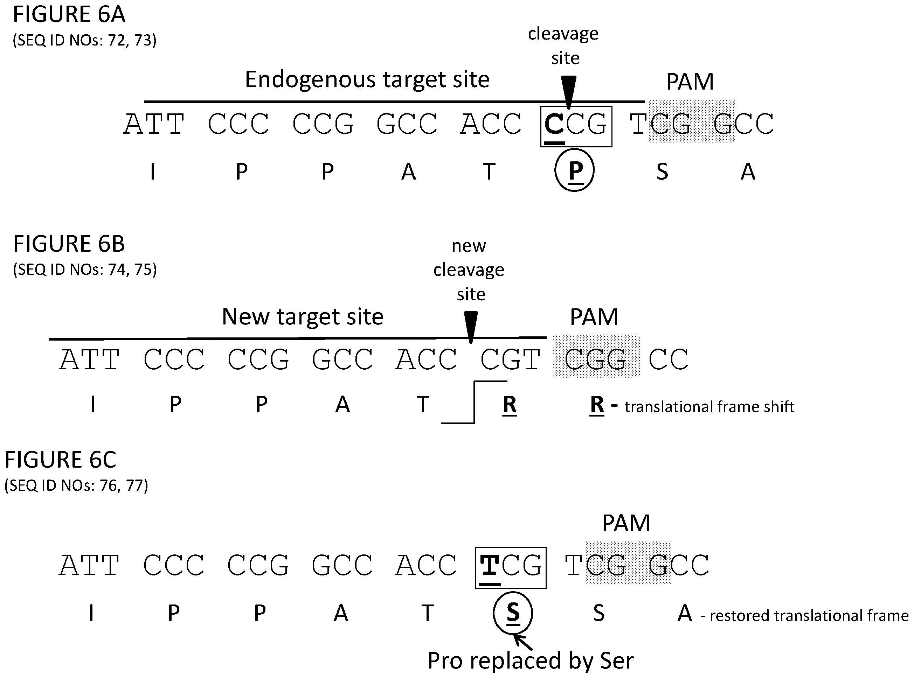

Figure 6A-6C shows a schematic of a fragment of polynucleotide of interest (SEQ ID NO: 72) comprising an endogenous target site selected for modification. The encoded amino acid sequences are shown below each nucleotide sequence. (SEQ ID NOs: 73, 75, 77). Figure 6A depicts single nucleotide (in this example C, shown in bold and underlined) located next to an endonuclease cleavage site (shown by arrow) can be removed through NHEJ. Figure 6B depicts the resulting polynucleotide of interest (SEQ ID NO:74) having a single base deleted, resulting in the creation of a new cleavage site (indicated by arrow) and translational frameshift. Figure 4C: A single nucleotide (in this example T, shown in bold and underlined) located next to an endonuclease cleavage site can be inserted through NHEJ without the use of a polynucleotide modification (repair) template, resulting in a single nucleotide edit of the polynucleotide of interest (SEQ ID NO:76). PAM sequences are highlighted in grey.

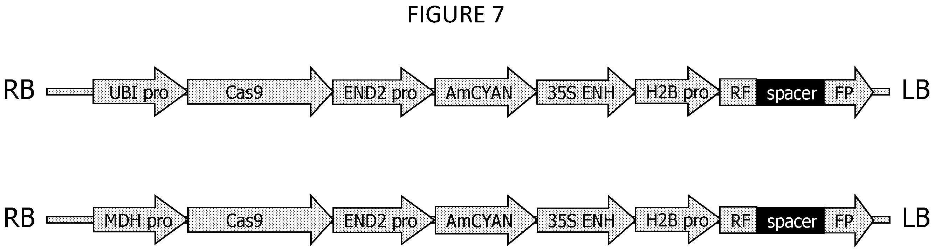

Figure 7. Top: *Agrobacterium* vector for stable integration of the UBI:Cas9 into the maize genome. Bottom: *Agrobacterium* vector for stable integration of the MDH:Cas9 into the maize genome. MDH is a temperature regulated promoter, regulating expression of the Cas9. These vectors also contain visible marker gene (END2:AmCYAN), which was used for selection of stably transformed callus sectors. Sequence of the Red Fluorescent Protein (DsRED) contained duplicated in a direct orientation 369 bp fragments separated by a 343-bp spacer, which contained sequences for recognition and targeting by two gRNAs and LIG3:4 meganuclease. H2B refers to the histone H2B gene promoter.

### Sequences

**Table 1. Summary of Nucleic Acid and Protein SEQ ID Numbers**

| Description | Nucleic acid SEQ ID NO. | Protein SEQ ID NO. |
|---|---|---|
| Cas9 coding sequence | 1 | |
| potato ST-LS1 intron | 2 | |
| SV40 NLS | | 3 |
| VirD2 NLS | 4 | |
| Maize optimized Cas9 expression cassette | 5 | |
| Lig-CR3 guide RNA expression vector | 6 | |
| Maize genomic target site MS26Cas-1 plus PAM sequence | 7 | |
| Maize genomic target site MS26Cas-2 plus PAM sequence | 8 | |
| Maize genomic target site MS26Cas-3 plus PAM sequence | 9 | |
| Maize genomic target site LIGCas-1 plus PAM sequence | 10 | |
| Maize genomic target site LIGCas-2 plus PAM sequence | 11 | |
| Maize genomic target site LIGCas-3 plus PAM sequence | 12 | |
| Maize genomic target site MS45Cas-1 plus PAM sequence | 13 | |
| Maize genomic target site MS45Cas-2 plus PAM sequence | 14 | |
| Maize genomic target site MS45Cas-3 plus PAM sequence | 15 | |
| Maize genomic target site ALSCas-1 plus PAM sequence | 16 | |
| Maize genomic target site ALSCas-2 plus PAM sequence | 17 | |
| Maize genomic target site ALSCas-3 plus PAM sequence | 18 | |
| Primer sequences | 19-38 | |
| ALS 1-DNA sequence | 39 | |
| ALS2-DNA sequence | 40 | |
| full length Zm-ALS2 protein | | 41 |
| Maize genomic target site ALSCas-4 plus PAM sequence | 42 | |
| 794 bp polynucleotide modification template | 43 | |
| 127 bp polynucleotide modification template, oligo1 | 44 | |
| 127 bp polynucleotide modification template, oligo2 | 45 | |
| Agrobacterium vector containing maize codon optimized Cas9 and maize UBI promoter | 46 | |
| Agrobacterium vector containing maize codon optimized Cas9 and maize MDH promoter | 47 | |
| *Sequences* shown in Figure 1 | 48-58 | |
| *Sequences* shown in Figure 2 | 59, 61, 63 | 60, 62, 64 |
| *Sequences* shown in Figure 4A-4C | 65, 67, 69 | 66,68 |
| *Sequences* shown in Figure 5A-5B | 70-71 | |
| *Sequences* shown in Figure 6A-6C | 72, 74, 76 | 73, 75,77 |
| IN2 promoter | 78 | |
| ALSCas7 target site | 79 | |
| ALSCas7-1 target site which is the modified ALSCas7 target site | 80 | |
| maize off target site | 81 | |

### DETAILED DESCRIPTION

The invention is defined in the appended claims. Any aspects, embodiments and examples of the present disclosure which do not fall under the scope of the appended claims do not form part of the invention and are merely provided for illustrative purposes.

Compositions and methods are disclosed herein for restoring function to a non-functional gene product in the genome of a cell. The methods and compositions employ a guide RNA/Cas endonuclease system to restore function to a non-functional gene product and to provide an effective system for modifying or altering target sites within the genome of a plant, plant cell or seed. The present disclosure also describes methods for modifying a nucleotide sequence in the genome of a cell using a restored functional selectable marker, as well as methods for editing a nucleotide sequence in the genome a cell without introducing a polynucleotide modification template into said cell. Compositions and methods are also disclosed herein for DNA free delivery of Cas9 endonucleases, sgRNAs and guide RNA/Cas complexes by introducing the components of the complex (guide RNA and Cas endonucleases) via mRNA molecules (guide RNA, Cas endonuclease) or protein molecules (Cas endonuclease) or by directly introducing the nucleotide-protein complex into the cell.

CRISPR loci (Clustered Regularly Interspaced Short Palindromic Repeats) (also known as SPIDRs--SPacer Interspersed Direct Repeats) constitute a family of DNA loci. CRISPR loci consist of short and highly conserved DNA repeats (typically 24 to 40 bp, repeated from 1 to 140 times - also referred to as CRISPR-repeats) which are partially palindromic. The repeated sequences (usually specific to a species) are interspaced by variable sequences of constant length (typically 20 to 58 by depending on the CRISPR locus (WO2007/025097, published March 1, 2007). Bacteria and archaea have evolved adaptive immune defenses termed clustered regularly interspaced short palindromic repeats (CRISPR)/CRISPR-associated (Cas) systems that use short RNA to direct degradation of foreign nucleic acids (WO2007/025097, published March 1, 2007). Multiple CRISPR-Cas systems have been described including Class 1 systems, with multisubunit effector complexes, and Class 2 systems, with single protein effectors (such as but not limiting to Cas9, Cpf1 ,C2c1,C2c2, C2c3). (Zetsche et al., 2015, Cell 163, 1-13; Shmakov et al., 2015, Molecular_Cell 60, 1-13; Makarova et al. 2015, Nature Reviews Microbiology Vol. 13:1-15, WO 2013/176772 A1 published on November 23, 2013).

The type II CRISPR/Cas system from bacteria employs a crRNA (CRISPR RNA) and tracrRNA (trans-activating CRISPR RNA) to guide the Cas endonuclease to its DNA target. The crRNA contains a spacer region complementary to one strand of the double strand DNA target and a region that base pairs with the tracrRNA (trans-activating CRISPR RNA) forming a RNA duplex that directs the Cas endonuclease to cleave the DNA target. Spacers are acquired through a not fully understood process involving Cas1 and Cas2 proteins. All type II CRISPR-Cas loci contain cas1 and cas2 genes in addition to the cas9 gene (Makarova et al. 2015, Nature Reviews Microbiology Vol. 13:1-15). Cas gene includes a gene that is generally coupled, associated or close to, or in the vicinity of flanking CRISPR loci. The terms "Cas gene", "CRISPR-associated (Cas) gene" are used interchangeably herein. A comprehensive review of the Cas protein family is presented in Haft et al. (2005) Computational Biology, PLoS Comput Biol 1(6): e60. doi:10.1371/journal.pcbi.0010060. As described therein, 41 CRISPR-associated (Cas) gene families are described, in addition to the four previously known gene families. It shows that CRISPR systems belong to different classes, with different repeat patterns, sets of genes, and species ranges. The number of Cas genes at a given CRISPR locus can vary between species (Haft et al., 2005, Computational Biology, PLoS Comput Biol 1(6): e60. doi:10.1371/journal.pcbi.0010060; Makarova et al. 2015, Nature Reviews Microbiology Vol. 13 :1-15; WO 2013/176772 A1 published on November 23, 2013).

The term "Cas endonuclease" herein refers to a protein encoded by a Cas (CRISPR-associated) gene. A Cas endonuclease, when in complex with a suitable polynucleotide component, is capable of recognizing, binding to, and optionally nicking or cleaving all or part of a specific DNA target sequence. A Cas endonuclease described herein comprises one or more nuclease domains. Cas endonucleases of the disclosure include those having a HNH or HNH-like nuclease domain and / or a RuvC or RuvC-like nuclease domain (Makarova et al. 2015, Nature Reviews Microbiology Vol. 13:1-15). A Cas includes a Cas9 protein, a Cpf1 protein, a C2c1 protein, a C2c2 protein, a C2c3 protein, Cas3, Cas3-HD, Cas 5, Cas7, Cas8, Cas10, or complexes of these.

As used herein, the terms "guide polynucleotide/Cas endonuclease complex", "guide polynucleotide/Cas endonuclease system", " guide polynucleotide/Cas complex", "guide polynucleotide/Cas system", "guided Cas system" , "PGEN" are used interchangeably herein and refer to at least one guide polynucleotide and at least one Cas endonuclease protein that are capable of forming a polynucleotide-protein complex, wherein said guide polynucleotide/Cas endonuclease complex can direct the Cas endonuclease to a DNA target site, enabling the Cas endonuclease to recognize, bind to, and optionally nick or cleave (introduce a single or double strand break) the DNA target site. A guide polynucleotide/Cas endonuclease complex herein can comprise Cas protein(s) and suitable polynucleotide component(s) of any of the four known CRISPR systems (Horvath and Barrangou, Science 327:167-170) such as a type I, II, or III CRISPR system. A Cas endonuclease unwinds the DNA duplex at the target sequence and optionally cleaves at least one DNA strand, as mediated by recognition of the target sequence by a polynucleotide (such as, but not limited to, a crRNA or guide RNA) that is in complex with the Cas protein. Such recognition and cutting of a target sequence by a Cas endonuclease typically occurs if the correct protospacer-adjacent motif (PAM) is located at or adjacent to the 3' end of the DNA target sequence. Alternatively, a Cas protein herein may lack DNA cleavage or nicking activity, but can still specifically bind to a DNA target sequence when complexed with a suitable RNA component. (See also U.S. Patent Application US 2015-0082478 A1, published on March 19, 2015 and US 2015-0059010 A1, published on February 26, 2015).

A guide polynucleotide/Cas endonuclease complex can cleave one or both strands of a DNA target sequence. A guide polynucleotide/Cas endonuclease complex that can cleave both strands of a DNA target sequence typically comprises a Cas protein that has all of its endonuclease domains in a functional state (e.g., wild type endonuclease domains or variants thereof retaining some or all activity in each endonuclease domain). Thus, a wild type Cas protein (e.g., a Cas9 protein disclosed herein), or a variant thereof retaining some or all activity in each endonuclease domain of the Cas protein, is a suitable example of a Cas endonuclease that can cleave both strands of a DNA target sequence. A Cas9 protein comprising functional RuvC and HNH nuclease domains is an example of a Cas protein that can cleave both strands of a DNA target sequence. A guide polynucleotide/Cas endonuclease complex that can cleave one strand of a DNA target sequence can be characterized herein as having nickase activity (e.g., partial cleaving capability). A Cas nickase typically comprises one functional endonuclease domain that allows the Cas to cleave only one strand (i.e., make a nick) of a DNA target sequence. For example, a Cas9 nickase may comprise (i) a mutant, dysfunctional RuvC domain and (ii) a functional HNH domain (e.g., wild type HNH domain). As another example, a Cas9 nickase may comprise (i) a functional RuvC domain (e.g., wild type RuvC domain) and (ii) a mutant, dysfunctional HNH domain. Non-limiting examples of Cas9 nickases suitable for use herein are disclosed by Gasiunas et al. (Proc. Natl. Acad. Sci. U.S.A. 109:E2579-E2586), Jinek et al. (Science 337:816-821), Sapranauskas et al. (Nucleic Acids Res. 39:9275-9282) and in U.S. Patent Appl. Publ. No. 2014/0189896.

A pair of Cas9 nickases can be used to increase the specificity of DNA targeting. In general, this can be done by introducing two Cas9 nickases that, by virtue of being associated with RNA components with different guide sequences, target and nick nearby DNA sequences on opposite strands in the region for desired targeting. Such nearby cleavage of each DNA strand creates a double strand break (i.e., a DSB with single-stranded overhangs), which is then recognized as a substrate for non-homologous-end-joining, NHEJ (prone to imperfect repair leading to mutations) or homologous recombination, HR. Each nick in these aspects of the disclosure can be at least about 5, 10, 15, 20, 30, 40, 50, 60, 70, 80, 90, or 100 (or any integer between 5 and 100) bases apart from each other, for example. One or two Cas9 nickase proteins herein can be used in a Cas9 nickase pair. For example, a Cas9 nickase with a mutant RuvC domain, but functioning HNH domain (i.e., Cas9 HNH+/RuvC-), could be used (e.g., *Streptococcus pyogenes* Cas9 HNH+/RuvC-). Each Cas9 nickase (e.g., Cas9 HNH+/RuvC-) would be directed to specific DNA sites nearby each other (up to 100 base pairs apart) by using suitable RNA components herein with guide RNA sequences targeting each nickase to each specific DNA site.

A Cas protein can be part of a fusion protein comprising one or more heterologous protein domains (e.g., 1, 2, 3, or more domains in addition to the Cas protein). Such a fusion protein may comprise any additional protein sequence, and optionally a linker sequence between any two domains, such as between Cas and a first heterologous domain. Examples of protein domains that may be fused to a Cas protein herein include, without limitation, epitope tags (e.g., histidine [His], V5, FLAG, influenza hemagglutinin [HA], myc, VSV-G, thioredoxin [Trx]), reporters (e.g., glutathione-5-transferase [GST], horseradish peroxidase [HRP], chloramphenicol acetyltransferase [CAT], beta-galactosidase, beta-glucuronidase [GUS], luciferase, green fluorescent protein [GFP], HcRed, DsRed, cyan fluorescent protein [CFP], yellow fluorescent protein [YFP], blue fluorescent protein [BFP]), and domains having one or more of the following activities: methylase activity, demethylase activity, transcription activation activity (e.g., VP16 or VP64), transcription repression activity, transcription release factor activity, histone modification activity, RNA cleavage activity and nucleic acid binding activity. A Cas protein can also be in fusion with a protein that binds DNA molecules or other molecules, such as maltose binding protein (MBP), S-tag, Lex A DNA binding domain (DBD), GAL4A DNA binding domain, and herpes simplex virus (HSV) VP16.

A Cas protein herein can be from any of the following genera: *Aeropyrum, Pyrobaculum, Sulfolobus, Archaeoglobus, Haloarcula, Methanobacteriumn, Methanococcus, Methanosarcina, Methanopyrus, Pyrococcus, Picrophilus, Thernioplasnia, Corynebacterium, Mycobacterium, Streptomyces, Aquifrx, Porphvromonas, Chlorobium, Thermus, Bacillus, Listeria, Staphylococcus, Clostridium, Thermoanaerobacter, Mycoplasma, Fusobacterium, Azarcus, Chromobacterium, Neisseria, Nitrosomonas, Desulfovibrio, Geobacter, Myrococcus, Campylobacter, Wolinella, Acinetobacter, Erwinia, Escherichia, Legionella, Methylococcus, Pasteurella, Photobacterium, Salmonella, Xanthomonas, Yersinia, Streptococcus, Treponema, Francisella,* or *Thermotoga.* Alternatively, a Cas protein herein can be encoded, for example, by any of SEQ ID NOs:462-465, 467-472, 474-477, 479-487, 489-492, 494-497, 499-503, 505-508, 510-516, or 517-521 as disclosed in U.S. Appl. Publ. No. 2010/0093617.

A guide polynucleotide/Cas endonuclease complex in certain aspects of the disclosure can bind to a DNA target site sequence, but does not cleave any strand at the target site sequence. Such a complex may comprise a Cas protein in which all of its nuclease domains are mutant, dysfunctional. For example, a Cas9 protein herein that can bind to a DNA target site sequence, but does not cleave any strand at the target site sequence, may comprise both a mutant, dysfunctional RuvC domain and a mutant, dysfunctional HNH domain. A Cas protein herein that binds, but does not cleave, a target DNA sequence can be used to modulate gene expression, for example, in which case the Cas protein could be fused with a transcription factor (or portion thereof) (e.g., a repressor or activator, such as any of those disclosed herein).

The Cas endonuclease gene can be a Type II Cas9 endonuclease , such as but not limited to, Cas9 genes listed in SEQ ID NOs: 462, 474, 489, 494, 499, 505, and 518 of WO2007/025097published March 1, 2007. In another aspect of the disclosure, the Cas endonuclease gene is a plant, maize or soybean optimized Cas9 endonuclease gene. The Cas endonuclease gene herein can be a plant or microbial codon optimized Cas9 endonuclease gene. The Cas endonuclease gene can be operably linked to a SV40 nuclear targeting signal upstream of the Cas codon region and a bipartite VirD2 nuclear localization signal (Tinland et al. (1992) Proc. Natl. Acad. Sci. USA 89:7442-6) downstream of the Cas codon region.

"Cas9" (formerly referred to as Cas5, Csn1, or Csx12) herein refers to a Cas endonuclease of a type II CRISPR system that forms a complex with a crNucleotide and a tracrNucleotide, or with a single guide polynucleotide, for specifically recognizing and cleaving all or part of a DNA target sequence. A Cas9 protein comprises a RuvC nuclease domain and an HNH (H-N-H) nuclease domain, each of which can cleave a single DNA strand at a target sequence (the concerted action of both domains leads to DNA double-strand cleavage, whereas activity of one domain leads to a nick). In general, the RuvC domain comprises subdomains I, II and III, where domain I is located near the N-terminus of Cas9 and subdomains II and III are located in the middle of the protein, flanking the HNH domain (Hsu et al, Cell 157:1262-1278). A type II CRISPR system includes a DNA cleavage system utilizing a Cas9 endonuclease in complex with at least one polynucleotide component. For example, a Cas9 can be in complex with a CRISPR RNA (crRNA) and a trans-activating CRISPR RNA (tracrRNA). In another example, a Cas9 can be in complex with a single guide RNA.

The amino acid sequence of a Cas9 protein described herein, as well as certain other Cas proteins herein, may be derived from a *Streptococcus* (e.g., *S*. *pyogenes, S. pneumoniae, S. thermophilus, S. agalactiae, S. parasanguinis, S. oralis, S. salivarius, S*. macacae, *S. dysgalactiae, S. anginosus, S. constellatus, S. pseudoporcinus, S. mutans), Listeria* (e.g., *L. innocua), Spiroplasma* (e.g., *S*. *apis, S. syrphidicola), Peptostreptococcaceae, Atopobium, Porphyromonas* (e.g., *P*. *catoniae*), *Prevotella* (e.g., *P. intermedia*)*, Veillonella, Treponema* (e.g., *T. socranskii, T. denticola*)*, Capnocytophaga, Finegoldia* (e.g., *F. magna*)*, Coriobacteriaceae* (e.g., *C. bacterium*)*, Olsenella* (e.g., *O*. *profusa*)*, Haemophilus* (e.g., *H. sputorum, H. pittmaniae*)*, Pasteurella* (e.g., *P. bettyae*)*, Olivibacter* (e.g., *O. sitiensis*)*, Epilithonimonas* (e.g., *E. tenax*)*, Mesonia* (e.g., *M. mobilis*)*, Lactobacillus* (e.g., *L. plantarum*)*, Bacillus* (e.g., *B. cereus*)*, Aquimarina* (e.g., *A. muelleri*)*, Chryseobacterium* (e.g., *C. palustre*)*, Bacteroides* (e.g., *B. graminisolvens*)*, Neisseria* (e.g., *N. meningitidis*)*, Francisella* (e.g., *F. novicida*)*,* or *Flavobacterium* (e.g., *F. frigidarium, F. soli*) species, for example. As another example, a Cas9 protein can be any of the Cas9 proteins disclosed in Chylinski et al. (RNA Biology 10:726-737 and US patent application 62/162377, filed May 15, 2015).

Accordingly, the sequence of a Cas9 protein herein can comprise, for example, any of the Cas9 amino acid sequences disclosed in GenBank Accession Nos. G3ECR1 (*S. thermophilus*)*,* WP_026709422, WP_027202655, WP_027318179, WP_027347504, WP_027376815, WP_027414302, WP_027821588, WP_027886314, WP_027963583, WP_028123848, WP_028298935, Q03JI6 (S. *thermophilus*)*,* EGP66723, EGS38969, EGV05092, EHI65578 (S. *pseudoporcinus*)*,* EIC75614 (S. *oralis),* EID22027 (S. *constellatus*)*,* EIJ69711, EJP22331 (S. *oralis),* EJP26004 (S. *anginosus*)*,* EJP30321, EPZ44001 (S. *pyogenes),* EPZ46028 (S. *pyogenes),* EQL78043 (S. *pyogenes),* EQL78548 (S. *pyogenes),* ERL10511, ERL12345, ERL19088 (S. *pyogenes),* ESA57807 (S. *pyogenes),* ESA59254 (S. *pyogenes),* ESU85303 (S. *pyogenes),* ETS96804, UC75522, EGR87316 (S. *dysgalactiae*)*,* EGS33732, EGV01468 (S. o*ralis*), EHJ52063 (S. macacae), EID26207 (S. *oralis*), EID33364, EIG27013 (S. *parasanguinis*)*,* EJF37476, EJO19166 *(Streptococcus* sp. BS35b), EJU16049, EJU32481, YP_006298249, ERF61304, ERK04546, ETJ95568 (S. *agalactiae*)*,* TS89875, ETS90967 *(Streptococcus* sp. SR4), ETS92439, EUB27844 *(Streptococcus* sp. BS21), AFJ08616, EUC82735 *(Streptococcus* sp. CM6), EWC92088, EWC94390, EJP25691, YP_008027038, YP_008868573, AGM26527, AHK22391, AHB36273, Q927P4, G3ECR1, or Q99ZW2 (S. *pyogenes).* A variant of any of these Cas9 protein sequences may be used, but should have specific binding activity, and optionally endonucleolytic activity, toward DNA when associated with an RNA component herein. Such a variant may comprise an amino acid sequence that is at least about 80%, 81%, 82%, 83%, 84%, 85%, 86%, 87%, 88%, 89%, 90%, 91%, 92%, 93%, 94%, 95%, 96%, 97%, 98%, or 99% identical to the amino acid sequence of the reference Cas9.

Alternatively, a Cas9 protein herein can be encoded by any of SEQ ID NOs:462 (S. *thermophilus*)*,* 474 (S. *thermophilus*)*,* 489 (S. *agalactiae*)*,* 494 (S. *agalactiae*)*,* 499 (S. *mutans*)*,* 505 (S. *pyogenes),* or 518 (S. *pyogenes)* as disclosed in U.S. Appl. Publ. No. 2010/0093617, for example. Alternatively still, a Cas9 protein may comprise an amino acid sequence that is at least about 80%, 81%, 82%, 83%, 84%, 85%, 86%, 87%, 88%, 89%, 90%, 91%, 92%, 93%, 94%, 95%, 96%, 97%, 98%, or 99% identical to any of the foregoing amino acid sequences, for example. Such a variant Cas9 protein should have specific binding activity, and optionally cleavage or nicking activity, toward DNA when associated with an RNA component herein.

A Cas protein herein such as a Cas9 can comprise a heterologous nuclear localization sequence (NLS). A heterologous NLS amino acid sequence herein may be of sufficient strength to drive accumulation of a Cas protein in a detectable amount in the nucleus of a yeast cell herein, for example. An NLS may comprise one (monopartite) or more (e.g., bipartite) short sequences (e.g., 2 to 20 residues) of basic, positively charged residues (e.g., lysine and/or arginine), and can be located anywhere in a Cas amino acid sequence but such that it is exposed on the protein surface. An NLS may be operably linked to the N-terminus or C-terminus of a Cas protein herein, for example. Two or more NLS sequences can be linked to a Cas protein, for example, such as on both the N- and C-termini of a Cas protein. Non-limiting examples of suitable NLS sequences herein include those disclosed in U.S. Patent Nos. 6660830 and 7309576 (e.g., Table 1 therein).

The Cas endonuclease can comprise a modified form of the Cas9 polypeptide. The modified form of the Cas9 polypeptide can include an amino acid change (e.g., deletion, insertion, or substitution) that reduces the naturally-occurring nuclease activity of the Cas9 protein. For example, in some instances, the modified form of the Cas9 protein has less than 50%, less than 40%, less than 30%, less than 20%, less than 10%, less than 5%, or less than 1% of the nuclease activity of the corresponding wild-type Cas9 polypeptide (US patent application US20140068797 A1, published on March 6, 2014). In some cases, the modified form of the Cas9 polypeptide has no substantial nuclease activity and is referred to as catalytically "inactivated Cas9" or "deactivated cas9 (dCas9)." Catalytically inactivated Cas9 variants include Cas9 variants that contain mutations in the HNH and RuvC nuclease domains. These catalytically inactivated Cas9 variants are capable of interacting with sgRNA and binding to the target site in vivo but cannot cleave either strand of the target DNA.

A catalytically inactive Cas9 can be fused to a heterologous sequence (US patent application US20140068797 A1, published on March 6, 2014). Suitable fusion partners include, but are not limited to, a polypeptide that provides an activity that indirectly increases transcription by acting directly on the target DNA or on a polypeptide (e.g., a histone or other DNA-binding protein) associated with the target DNA. Additional suitable fusion partners include, but are not limited to, a polypeptide that provides for methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, myristoylation activity, or demyristoylation activity. Further suitable fusion partners include, but are not limited to, a polypeptide that directly provides for increased transcription of the target nucleic acid (e.g., a transcription activator or a fragment thereof, a protein or fragment thereof that recruits a transcription activator, a small molecule/drug-responsive transcription regulator, etc.). A catalytically inactive Cas9 can also be fused to a Fokl nuclease to generate double strand breaks (Guilinger et al. Nature biotechnology, volume 32, number 6, June 2014).

The terms "functional fragment ", "fragment that is functionally equivalent" and "functionally equivalent fragment" of a Cas endonuclease are used interchangeably herein, and refer to a portion or subsequence of a Cas endonuclease sequence in which the ability to recognize, bind to, and optionally nick or cleave (introduce a single or double strand break in) the target site is retained.

The terms "functional variant", "Variant that is functionally equivalent" and "functionally equivalent variant" of a Cas endonuclease are used interchangeably herein, and refer to a variant of a Cas endonuclease in which the ability to recognize, bind to, and optionally nick or cleave (introduce a single or double strand break in) the target site is retained. Fragments and variants can be obtained via methods such as site-directed mutagenesis and synthetic construction.

The Cas endonuclease gene includes a plant codon optimized *Streptococcus pyogenes* Cas9 gene that can recognize any genomic sequence of the form N(12-30)NGG can in principle be targeted or a Cas9 endonuclease originated from an organism selected from the group consisting of *Brevibacillus laterosporus, Lactobacillus reuteri* Mlc3, *Lactobacillus rossiae* DSM 15814, *Pediococcus pentosaceus* SL4, *Lactobacillus nodensis* JCM 14932, *Sulfurospirillum sp.* SCADC, *Bifidobacterium thermophilum* DSM 20210, *Loktanella vestfoldensis, Sphingomonas sanxanigenens* NX02, *Epilithonimonas tenax* DSM 16811, *Sporocytophaga myxococcoides* and *Psychroflexus torquis* ATCC 700755, wherein said Cas9 endonuclease can form a guide RNA/Cas endonuclease complex capable of recognizing, binding to, and optionally nicking or cleaving all or part of a DNA target sequence. Other Cas endonuclease systems have been described in US patent applications 62/162,377 filed May 15, 2015 and 62/162,353 filed May 15, 2015.

Cas9 endonucleases can be used for targeted genome editing (via simplex and multiplex double-strand breaks and nicks) and targeted genome regulation (via tethering of epigenetic effector domains to either the Cas9 or sgRNA. Cas9 might also be engineered to function as an RNA-guided recombinase, and via RNA tethers could serve as a scaffold for the assembly of multiprotein and nucleic acid complexes (Mali et al. 2013 Nature Methods Vol. 10: 957-963.).

As used herein, the term "guide polynucleotide", relates to a polynucleotide sequence that can form a complex with a Cas endonuclease and enables the Cas endonuclease to recognize, bind to, and optionally cleave a DNA target site. The guide polynucleotide can be a single molecule or a double molecule. The guide polynucleotide sequence can be a RNA sequence (referred to as guide RNA, gRNA), a DNA sequence, or a combination thereof (a RNA-DNA combination sequence). Optionally, the guide polynucleotide can comprise at least one nucleotide, phosphodiester bond or linkage modification such as, but not limited, to Locked Nucleic Acid (LNA), 5-methyl dC, 2,6-Diaminopurine, 2'-Fluoro A, 2'-Fluoro U, 2'-O-Methyl RNA, phosphorothioate bond, linkage to a cholesterol molecule, linkage to a polyethylene glycol molecule, linkage to a spacer 18 (hexaethylene glycol chain) molecule, or 5' to 3' covalent linkage resulting in circularization. A guide polynucleotide that solely comprises ribonucleic acids is also referred to as a "guide RNA" or "gRNA" (See also U.S. Patent Application US 2015-0082478 A1, published on March 19, 2015 and US 2015-0059010 A1, published on February 26, 2015).

The guide polynucleotide can be a double molecule (also referred to as duplex guide polynucleotide) comprising a crNucleotide sequence and a tracrNucleotide sequence. The crNucleotide includes a first nucleotide sequence domain (referred to as Variable Targeting domain or VT domain) that can hybridize to a nucleotide sequence in a target DNA and a second nucleotide sequence (also referred to as a tracr mate sequence) that is part of a Cas endonuclease recognition (CER) domain. The tracr mate sequence can hybridized to a tracrNucleotide along a region of complementarity and together form the Cas endonuclease recognition domain or CER domain. The CER domain is capable of interacting with a Cas endonuclease polypeptide. The crNucleotide and the tracrNucleotide of the duplex guide polynucleotide can be RNA, DNA, and/or RNA-DNA- combination sequences. In some aspects of the disclosure, the crNucleotide molecule of the duplex guide polynucleotide is referred to as "crDNA" (when composed of a contiguous stretch of DNA nucleotides) or "crRNA" (when composed of a contiguous stretch of RNA nucleotides), or "crDNA-RNA" (when composed of a combination of DNA and RNA nucleotides). The crNucleotide can comprise a fragment of the crRNA naturally occurring in Bacteria and Archaea. The size of the fragment of the crRNA naturally occurring in Bacteria and Archaea that can be present in a crNucleotide disclosed herein can range from, but is not limited to, 2, 3, 4, 5, 6, 7, 8, 9,10, 11, 12, 13, 14, 15, 16, 17, 18, 19, 20 or more nucleotides. In some aspects of the disclosure the tracrNucleotide is referred to as "tracrRNA" (when composed of a contiguous stretch of RNA nucleotides) or "tracrDNA" (when composed of a contiguous stretch of DNA nucleotides) or "tracrDNA-RNA" (when composed of a combination of DNA and RNA nucleotides. In one aspect of the disclosure, the RNA that guides the RNA/ Cas9 endonuclease complex is a duplexed RNA comprising a duplex crRNA-tracrRNA.

The tracrRNA (trans-activating CRISPR RNA) contains, in the 5'-to-3' direction, (i) a sequence that anneals with the repeat region of CRISPR type II crRNA and (ii) a stem loop-containing portion (Deltcheva et al., Nature 471:602-607). The duplex guide polynucleotide can form a complex with a Cas endonuclease, wherein said guide polynucleotide/Cas endonuclease complex (also referred to as a guide polynucleotide/Cas endonuclease system) can direct the Cas endonuclease to a genomic target site, enabling the Cas endonuclease to recognize, bind to, and optionally nick or cleave (introduce a single or double strand break) into the target site. (See also U.S. Patent Application US 2015-0082478 A1, published on March 19, 2015 and US 2015-0059010 A1, published on February 26, 2015.)

The guide polynucleotide can also be a single molecule (also referred to as single guide polynucleotide) comprising a crNucleotide sequence linked to a tracrNucleotide sequence. The single guide polynucleotide comprises a first nucleotide sequence domain (referred to as Variable Targeting domain or VT domain) that can hybridize to a nucleotide sequence in a target DNA and a Cas endonuclease recognition domain (CER domain), that interacts with a Cas endonuclease polypeptide. By "domain" it is meant a contiguous stretch of nucleotides that can be RNA, DNA, and/or RNA-DNA-combination sequence. The VT domain and /or the CER domain of a single guide polynucleotide can comprise a RNA sequence, a DNA sequence, or a RNA-DNA-combination sequence. The single guide polynucleotide being comprised of sequences from the crNucleotide and the tracrNucleotide may be referred to as "single guide RNA" (when composed of a contiguous stretch of RNA nucleotides) or "single guide DNA" (when composed of a contiguous stretch of DNA nucleotides) or "single guide RNA-DNA" (when composed of a combination of RNA and DNA nucleotides). The single guide polynucleotide can form a complex with a Cas endonuclease, wherein said guide polynucleotide/Cas endonuclease complex (also referred to as a guide polynucleotide/Cas endonuclease system) can direct the Cas endonuclease to a genomic target site, enabling the Cas endonuclease to recognize, bind to, and optionally nick or cleave (introduce a single or double strand break) the target site. (See also U.S. Patent Application US 2015-0082478 A1, published on March 19, 2015 and US 2015-0059010 A1, published on February 26, 2015.)

The term "variable targeting domain" or "VT domain" is used interchangeably herein and includes a nucleotide sequence that can hybridize (is complementary) to one strand (nucleotide sequence) of a double strand DNA target site. The % complementation between the first nucleotide sequence domain (VT domain ) and the target sequence can be at least 50%, 51%, 52%, 53%, 54%, 55%, 56%, 57%, 58%, 59%, 60%, 61%, 62%, 63%, 63%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. The variable targeting domain can be at least 12, 13, 14, 15, 16, 17, 18, 19, 20, 21, 22, 23, 24, 25, 26, 27, 28, 29 or 30 nucleotides in length. In some aspects of the disclosure, the variable targeting domain comprises a contiguous stretch of 12 to 30 nucleotides. The variable targeting domain can be composed of a DNA sequence, a RNA sequence, a modified DNA sequence, a modified RNA sequence, or any combination thereof.

The term "Cas endonuclease recognition domain" or "CER domain" (of a guide polynucleotide) is used interchangeably herein and includes a nucleotide sequence that interacts with a Cas endonuclease polypeptide. A CER domain comprises a tracrNucleotide mate sequence followed by a tracrNucleotide sequence. The CER domain can be composed of a DNA sequence, a RNA sequence, a modified DNA sequence, a modified RNA sequence (see for example US 2015-0059010 A1, published on February 26, 2015), or any combination thereof.

The nucleotide sequence linking the crNucleotide and the tracrNucleotide of a single guide polynucleotide can comprise a RNA sequence, a DNA sequence, or a RNA-DNA combination sequence. In one aspect of the disclosure, the nucleotide sequence linking the crNucleotide and the tracrNucleotide of a single guide polynucleotide can be at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 78, 79, 80, 81, 82, 83, 84, 85, 86, 87, 88, 89, 90, 91, 92, 93, 94, 95, 96, 97, 98, 99 or 100 nucleotides in length. In another aspect of the disclosure, the nucleotide sequence linking the crNucleotide and the tracrNucleotide of a single guide polynucleotide can comprise a tetraloop sequence, such as, but not limiting to a GAAA tetraloop sequence.

Nucleotide sequence modification of the guide polynucleotide, VT domain and/or CER domain can be selected from, but not limited to , the group consisting of a 5' cap, a 3' polyadenylated tail, a riboswitch sequence, a stability control sequence, a sequence that forms a dsRNA duplex, a modification or sequence that targets the guide poly nucleotide to a subcellular location, a modification or sequence that provides for tracking , a modification or sequence that provides a binding site for proteins , a Locked Nucleic Acid (LNA), a 5-methyl dC nucleotide, a 2,6-Diaminopurine nucleotide, a 2'-Fluoro A nucleotide, a 2'-Fluoro U nucleotide; a 2'-O-Methyl RNA nucleotide, a phosphorothioate bond, linkage to a cholesterol molecule, linkage to a polyethylene glycol molecule, linkage to a spacer 18 molecule, a 5' to 3' covalent linkage, or any combination thereof. These modifications can result in at least one additional beneficial feature, wherein the additional beneficial feature is selected from the group of a modified or regulated stability, a subcellular targeting, tracking, a fluorescent label, a binding site for a protein or protein complex, modified binding affinity to complementary target sequence, modified resistance to cellular degradation, and increased cellular permeability.

The terms "functional fragment ", "fragment that is functionally equivalent" and "functionally equivalent fragment" of a guide RNA, crRNA or tracrRNA are used interchangeably herein, and refer to a portion or subsequence of the guide RNA, crRNA or tracrRNA , respectively, in which the ability to function as a guide RNA, crRNA or tracrRNA, respectively, is retained.

The terms "functional variant", "Variant that is functionally equivalent" and "functionally equivalent variant" of a guide RNA, crRNA or tracrRNA (respectively) are used interchangeably herein, and refer to a variant of the guide RNA, crRNA or tracrRNA, respectively, in which the ability to function as a guide RNA, crRNA or tracrRNA, respectively, is retained.

The terms "single guide RNA" , "gRNA" and "sgRNA" are used interchangeably herein and relate to a synthetic fusion of two RNA molecules, a crRNA (CRISPR RNA) comprising a variable targeting domain (linked to a tracr mate sequence that hybridizes to a tracrRNA), fused to a tracrRNA (trans-activating CRISPR RNA). The single guide RNA can comprise a crRNA or crRNA fragment and a tracrRNA or tracrRNA fragment of the type II CRISPR/Cas system that can form a complex with a type II Cas endonuclease, wherein said guide RNA/Cas endonuclease complex can direct the Cas endonuclease to a DNA target site, enabling the Cas endonuclease to recognize, bind to, and optionally nick or cleave (introduce a single or double strand break) the DNA target site.

The terms "guide RNA/Cas endonuclease complex", "guide RNA/Cas endonuclease system", " guide RNA/Cas complex", "guide RNA/Cas system", "gRNA/Cas complex", "gRNA/Cas system", "RNA-guided endonuclease" , "RGEN" are used interchangeably herein and refer to at least one RNA component and at least one Cas endonuclease protein that are capable of forming a complex , wherein said guide RNA/Cas endonuclease complex can direct the Cas endonuclease to a DNA target site, enabling the Cas endonuclease to recognize, bind to, and optionally nick or cleave (introduce a single or double strand break) the DNA target site. A guide RNA/Cas endonuclease complex herein can comprise Cas protein(s) and suitable RNA component(s) of any of the known CRISPR systems (Zetsche et al., 2015, Cell 163, 1-13; Shmakov et al., 2015, Molecular_Cell 60, 1-13; Makarova et al. 2015, Nature Reviews Microbiology Vol. 13:1-15; Horvath and Barrangou, Science 327:167-170) such as a type I, II, or III CRISPR system. A guide RNA/Cas endonuclease complex can comprise a Type II Cas9 endonuclease and at least one RNA component (e.g., a crRNA and tracrRNA, or a gRNA). (See also U.S. Patent Application US 2015-0082478 A1, published on March 19, 2015 and US 2015-0059010 A1, published on February 26, 2015).

The Cas endonuclease can be introduced into a cell (provided to a cell) by any method known in the art, for example, but not limited to transient introduction methods, transfection, microinjection, and/or topical application or indirectly via recombination constructs. Plant cells differ from human and animal cells in that plant cells contain a plant cell wall which may act as a barrier to the direct delivery of the Cas9 endonuclease into the plant cell. Recombinant DNA constructs encoding a Cas9 endonuclease have been successfully introduced into plant cells (Svitashev et al., Plant Physiology, 2015, Vol. 169, pp. 931-945) to allow for genome editing at a target site. One possible disadvantage of stably introducing recombinant DNA constructs in plant cells is that the continued presence of Cas9 endonucleases may increase off-target effects.

As described herein, direct delivery of the Cas endonuclease into plant cells can be achieved through particle mediated delivery. Based on the experiments described herein, a skilled artesian can now envision that any other direct method of delivery, such as but not limiting to, polyethylene glycol (PEG)-mediated transfection to protoplasts, whisker mediated delivery, electroporation, particle bombardment, cell-penetrating peptides, or mesoporous silica nanoparticle (MSN)-mediated direct protein delivery can be successfully used for delivering the Cas9 endonuclease in plant cells.

Direct delivery of the Cas endonuclease (also referred to as DNA free delivery off the Cas endonuclease ) can be achieved by introducing the Cas protein, the mRNA encoding the Cas endonuclease, and/ or the RNA guided endonuclease ribonucleotide-protein complex (RGEN) itself (as a ribonucleotide-protein complex), into a cell using any method known in the art. Direct delivery of the Cas endonuclease, either via mRNA encoding the Cas endonuclease or via a polypeptide molecule is also referred to herein as DNA free delivery of the Cas endonuclease, as no DNA molecule is involved in the production of the Cas endonuclease protein. Similarly, direct delivery of the guide RNA as an RNA molecule is also referred to herein as DNA free delivery of the guide RNA. Similarly, direct delivery of the guide RNA/endonuclease complex itself (RGEN) as a ribonucleotide-protein complex, is also referred to herein as DNA free delivery of the RGEN.

Directly introducing the Cas endonuclease as a protein, or as an mRNA molecule together with a gRNA, or as a RGEN ribonucleotide-protein itself, allows for genome editing at the target site followed by rapid degradation of the RGEN complex, and only a transient presence of the complex in the cell which leads to reduced off-target effects (as described in Example 12).

Direct delivery of these components can be accompanied by direct delivery (co-delivery) of other mRNAs that can promote the enrichment and/or visualization of cells receiving the RGEN components. For example, delivery of mRNAs encoding screenable visual markers such as fluorescence proteins (for example but not limited to Red, green, yellow, blue or combinations thereof) can also be used in lieu of, or coupled with, direct selection of a repaired disrupted, non-functional gene product.

Described herein are methods to restore the function of a non-functional gene product by restoring the nucleotide sequence of a disrupted gene such that the restored nucleotide sequence encodes the functional gene product.

A disrupted gene refers to a gene that has been modified (disrupted) such that its gene product loses its function (referred to as a non-functional gene product) or has a reduced function when compared to the product of the corresponding gene that does not have the disruption (also referred to as the undisrupted gene). For example, a gene encoding for a functional polypeptide or protein can be disrupted (modified) such that the translation product of the disrupted gene results in a polypeptide that has lost its function or has a reduced function.

A functional gene product includes a functional protein or polypeptide that has a biological or non-biological function.

A non-functional gene product includes reference to the gene product of a disrupted gene. The non-functional gene product includes polypeptides that have lost their function (absent function) or have a reduced function when compared to the gene product of the corresponding undisrupted gene.

In one aspect of the disclosure, the method comprises a method for restoring function to a non-functional gene product in the genome of a cell, the method comprising introducing a guide RNA/Cas endonuclease complex into a cell comprising a disrupted gene in its genome, wherein said complex creates a double strand break, wherein said disrupted gene does not encode a functional gene product, wherein said disrupted gene is restored without the use of a polynucleotide modification template to a non-disrupted gene capable of encoding said functional gene product. The disrupted gene can comprise a base pair deletion of the 4^{th} nucleotide upstream (5') of a PAM sequence when compared to its corresponding non-disrupted gene, wherein said base pair deletion creates an amino acid frameshift in the gene product of the disrupted gene thereby rendering the gene product of the disrupted gene non-functional. The base pair deletion can be the first, second or third nucleotide of a codon sequence. The restoration can be accomplished by Non-Homologous-End -Joining (NHEJ) resulting in the insertion of a single base at the double strand break site, or can be accomplished by the insertion of a single base at the double strand break site without the use of Homologous Recombination or Homology Directed Repair.

Coincident with the restoration of gene function by NHEJ (through for example delivery of RGEN components or the RGEN complex itself to a cell), modification of other targets can be accomplished by the simultaneous addition of other guide-polynucleotides. Such other targets (other than the target for restoration of gene function by NHEJ) can be any target in the genome including a transgenic locus. The approach of simultaneous delivery of two or more gRNAs when one gRNA targets and activates a selectable marker through NHEJ, (such as but not limiting to conferring herbicide tolerance) and the other gRNA(s) promote DSB(s) at target site(s) different than the selectable marker (or other disrupted gene design ) and can facilitate either targeted mutagenesis, deletion, gene editing, or site-specific trait gene insertions can allow for completely transient targeted genome modifications as all other necessary components (Cas9, gRNAs) can be delivered in a form of protein and/or in vitro transcribed RNA molecules.

A disrupted gene includes reference to a marker gene (such as, but not limited to, a phenotypic marker gene and a selectable marker gene) that has been modified (disrupted) such that its gene product loses its function (for example, in case of a herbicide disrupted selectable marker gene, the disrupted gene does not confer herbicide resistance anymore).

A selectable marker and screenable marker are used interchangeably herein and includes reference to a DNA segment (such as a selectable marker gene) that allows one to identify, or select for or against a molecule or a cell that contains it, often under particular conditions. These markers can encode an activity, such as, but not limited to, production of RNA, peptide, or protein, or can provide a binding site for RNA, peptides, proteins, and inorganic and organic compounds or compositions. A selectable marker further includes a gene that when modified or knocked-out generates a property in a cell that allows one to identify, or select for (or against) a cell that contains said property.

In one aspect, the selectable marker allows for the selection of cells by applying selection schemes, in which for example, a selective agent (such as, but not limited to an antibiotic or herbicide) is used to inhibit or kill cells or tissues that do not comprise the selectable marker, and the cells or tissues that comprise the selectable marker continue to grow due to expression of the selectable marker gene.

In one aspect, the selectable marker allows for the visual selection of cells by applying selection schemes, in which for example, a visible marker (such as a fluorescent molecule) is used to select cells that comprise the visible marker.

Selectable marker genes include, but are not limited to, chlorosulfuron resistance genes, phosphomannose isomerase genes (PMI), bialaphos resistance genes (BAR), phosphinothricin acetyltransferase (PAT) genes, hygromycin resistance genes (NPTII), glyphosate resistance genes, DNA segments that comprise restriction enzyme sites; DNA segments that encode products which provide resistance against otherwise toxic compounds including antibiotics, such as, spectinomycin, ampicillin, kanamycin, tetracycline, Basta, neomycin phosphotransferase II (NEO) and hygromycin phosphotransferase (HPT)); DNA segments that encode products which are otherwise lacking in the recipient cell (e.g., tRNA genes, auxotrophic markers); DNA segments that encode products which can be readily identified (referred to as visible maker genes. Visible marker genes include reference to fluorescent markers genes, such as red fluorescent marker genes, blue fluorescent marker genes, green fluorescent marker genes, yellow fluorescent marker genes), genes encoding DsRED, RFP, red fluorescent protein, CFP, GFP, green fluorescent protein) and genes encoding phenotypic markers such as β-galactosidase, GUS; fluorescent proteins such as green fluorescent protein (GFP), cyan (CFP), yellow (YFP), red (RFP), and cell surface proteins. Selectable marker genes further include the generation of new primer sites for PCR (e.g., the juxtaposition of two DNA sequence not previously juxtaposed), the inclusion of DNA sequences not acted upon or acted upon by a restriction endonuclease or other DNA modifying enzyme, chemical, etc.; and, the inclusion of a DNA sequences required for a specific modification (e.g., methylation) that allows its identification.

Additional selectable markers include genes that confer resistance to herbicidal compounds, such as glufosinate ammonium, bromoxynil, imidazolinones, and 2,4-dichlorophenoxyacetate (2,4-D). See for example, Yarranton, (1992) Curr Opin Biotech 3:506-11; Christopherson et al., (1992) Proc. Natl. Acad. Sci. USA 89:6314-8; Yao et al., (1992) Cell 71:63-72; Reznikoff, (1992) Mol Microbiol 6:2419-22; Hu et al., (1987) Cell 48:555-66; Brown et al., (1987) Cell 49:603-12; Figge et al., (1988) Cell 52:713-22; Deuschle et al., (1989) Proc. Natl. Acad. Sci. USA 86:5400-4; Fuerst et al., (1989) Proc. Natl. Acad. Sci. USA 86:2549-53; Deuschle et al., (1990) Science 248:480-3; Gossen, (1993) Ph.D. Thesis, University of Heidelberg; Reines et al., (1993) Proc. Natl. Acad. Sci. USA 90:1917-21; Labow et al., (1990) Mol Cell Biol 10:3343-56; Zambretti et al., (1992) Proc. Natl. Acad. Sci. USA 89:3952-6; Baim et al., (1991) Proc. Natl. Acad. Sci. USA 88:5072-6; Wyborski et al., (1991) Nucleic Acids Res 19:4647-53; Hillen and Wissman, (1989) Topics Mol Struc Biol 10:143-62; Degenkolb et al., (1991) Antimicrob Agents Chemother 35:1591-5; Kleinschnidt et al., (1988) Biochemistry 27:1094-104; Bonin, (1993) Ph.D. Thesis, University of Heidelberg; Gossen et al., (1992) Proc. Natl. Acad. Sci. USA 89:5547-51; Oliva et al., (1992) Antimicrob Agents Chemother 36:913-9; Hlavka et al., (1985) Handbook of Experimental Pharmacology, Vol. 78 (Springer-Verlag, Berlin); Gill et al., (1988) Nature 334:721-4.

Phenotypic marker genes include genes encoding a screenable or selectable marker that includes visual markers, whether it is a positive or negative selectable marker. Any phenotypic marker can be used.

As described herein, a phenotypic and selectable marker gene can be modified to be introduced into plant cells as a disrupted gene encoding a non-functional gene product and used as targets by double strand break inducing endonucleases for restoration back to the non-disrupted gene encoding a functional gene product, by guide RNA introduction and DNA repair.

Phenotypic or selectable marker genes to be disrupted can be marker genes that were previously introduced into the cell and are stably incorporated into the genome of the cell. Such pre-integrated selectable marker genes can also be complemented with other genes, for example, cell developmental enhancing genes (ZmODP2 and ZmWUS, see for example PCT/US16/49144, filed August 26, 2016 and PCT/US16/49128 filed August 26, 2016).

As described herein, the phenotypic and selectable marker genes can be modified to be introduced into plant cells as disrupted genes (inactive forms) and used as targets by double strand break inducing endonucleases for restoration (re-activation) by guide RNA introduction and repair.

Described herein are expression markers genes (such as but not limiting to ALS, EPSPS, BAR) that confers resistance to a compound or allows the endogenous or previously integrated marker gene or its gene product to be used as a marker, that can be modified into inactive forms and then used as targets for re-activation by guide RNA introduction and repair as described herein (see for Example 3-4).

In one aspect of the disclosure, the method comprises a method for modifying a nucleotide sequence in the genome of a cell, the method comprising: introducing into at least one cell comprising a target site and a disrupted selectable marker gene, a first guide RNA, a Cas endonuclease, and at least a second guide RNA, wherein said first guide RNA and Cas endonuclease can form a first complex capable of introducing a double strand break in said disrupted selectable marker gene, wherein said disrupted selectable marker gene is restored without the use of a polynucleotide modification template to a non-disrupted selectable marker gene capable of encoding a functional selectable marker protein, wherein said second guide RNA and Cas endonuclease can form a second complex that is capable of recognizing, binding to, and nicking or cleaving said target site located in said nucleotide sequence; and, selecting a cell having a modification in said nucleotide sequence, wherein the selection is provided by or based upon said functional selectable marker protein . The selection can include providing a chemical to the cell (such as a plant cell, or plants derived from said plant cell) expressing said functional selectable marker protein, wherein said functional selectable marker protein makes the cell (such as a plant cell, or plants derived from said plant cell) resistant to said chemical. The chemical can be provided during any developmental stage of the cell (in case of a plant cell during any stage of plant cell or plant development) to select for the cells or organisms comprising the desired modification in their genome. The selection also includes screening of cells (such as a plant cell, or plants derived from said plant cell) for the presence of a visual marker that results from the expression of said functional selectable marker protein .

For example, as described herein, the method can be a method for modifying a nucleotide sequence in the genome of a cell, the method comprising: introducing into at least one cell comprising a target site and a disrupted resistant-ALS marker gene, a first guide RNA, a Cas endonuclease, and at least a second guide RNA, wherein said first guide RNA and Cas endonuclease can form a first complex capable of introducing a double strand break in said disrupted resistant- ALS marker gene, wherein said disrupted resistance-ALS marker gene is restored without the use of a polynucleotide modification template to a non-disrupted resistant ALS-marker gene capable of encoding a functional resistance-ALS protein that confers resistance to chlorsulfuron, wherein said second guide RNA and Cas endonuclease can form a second complex that is capable of recognizing, binding to, and nicking or cleaving said target site located in said nucleotide sequence; and, selecting a cell having a modification in said nucleotide sequence, wherein the selection is provided by or based upon said resistance-ALS protein that confers resistance to chlorsulfuron, The selection step can include providing chlorsulfuron to the plant cell or plants derived from said plant cell at any stage during plant cell or plant development to select for the plant cell or plants comprising the desired modification the their genome.

The introducing and selection step does not comprise the introduction of a selectable marker gene, such as a recombinant DNA construct comprising a selectable marker gene. The disrupted selectable marker gene can be any disrupted marker gene including a disrupted visible marker gene. The modification in the targeted nucleotide sequence can be selected from the group consisting of an insertion of at least one nucleotide, a deletion of at least one nucleotide, or a substitution of at least one nucleotide in said target site. The method can further comprise introducing a polynucleotide modification template into said cell, wherein said polynucleotide modification template comprises at least one nucleotide modification of said nucleotide sequence, or a donor DNA wherein said donor DNA comprises at least one polynucleotide of interest to be inserted into said target site.

Any guided endonuclease can be used in the methods disclosed herein. Such endonucleases include, but are not limited to Cas and Cpf1 endonucleases. Many endonucleases have been described to date that can recognize specific PAM sequences (see for example -US patent applications 62/162377 filed May 15, 2015 and 62/162353 filed May 15, 2015 and Zetsche B et al. 2015. Cell 163, 1013) and cleave the target DNA at a specific positions. It is understood that based on the methods and aspects of the disclosure described herein utilizing a guided Cas system one can now tailor these methods such that they can utilize any guided endonuclease system. For example, one can envision adapting the method for restoring function to a non-functional gene product in the genome of a cell described herein to a method comprising introducing a guided Cpf1 endonuclease complex instead of a guided Cas endonuclease complex to restore a disrupted gene and creating a functional gene product. Other guided endonucleases and nucleotide-protein complexes that find use in the methods disclosed herein include those described in WO 2013/088446.

Endonucleases are enzymes that cleave the phosphodiester bond within a polynucleotide chain, and include restriction endonucleases that cleave DNA at specific sites without damaging the bases. Restriction endonucleases include Type I, Type II, Type III, and Type IV endonucleases, which further include subtypes. In the Type I and Type III systems, both the methylase and restriction activities are contained in a single complex. Endonucleases also include meganucleases, also known as homing endonucleases (HEases), which like restriction endonucleases, bind and cut at a specific recognition site, however the recognition sites for meganucleases are typically longer, about 18 bp or more (patent application PCT/US12/30061, filed on March 22, 2012). Meganucleases have been classified into four families based on conserved sequence motifs, the families are the LAGLIDADG, GIY-YIG, H-N-H, and His-Cys box families. These motifs participate in the coordination of metal ions and hydrolysis of phosphodiester bonds. HEases are notable for their long recognition sites, and for tolerating some sequence polymorphisms in their DNA substrates. The naming convention for meganuclease is similar to the convention for other restriction endonuclease. Meganucleases are also characterized by prefix F-, I-, or PI- for enzymes encoded by free-standing ORFs, introns, and inteins, respectively. One step in the recombination process involves polynucleotide cleavage at or near the recognition site. This cleaving activity can be used to produce a double-strand break. For reviews of site-specific recombinases and their recognition sites, see, Sauer (1994) Curr Op Biotechnol 5:521-7; and Sadowski (1993) FASEB 7:760-7. In some examples the recombinase is from the Integrase or Resolvase families.

TAL effector nucleases are a new class of sequence-specific nucleases that can be used to make double-strand breaks at specific target sequences in the genome of a plant or other organism. (Miller et al. (2011) Nature Biotechnology 29:143-148). Zinc finger nucleases (ZFNs) are engineered double-strand break inducing agents comprised of a zinc finger DNA binding domain and a double-strand-break-inducing agent domain. Recognition site specificity is conferred by the zinc finger domain, which typically comprising two, three, or four zinc fingers, for example having a C2H2 structure, however other zinc finger structures are known and have been engineered. Zinc finger domains are amenable for designing polypeptides which specifically bind a selected polynucleotide recognition sequence. ZFNs include an engineered DNA-binding zinc finger domain linked to a nonspecific endonuclease domain, for example nuclease domain from a Type IIs endonuclease such as Fokl. Additional functionalities can be fused to the zinc-finger binding domain, including transcriptional activator domains, transcription repressor domains, and methylases. In some examples, dimerization of nuclease domain is required for cleavage activity. Each zinc finger recognizes three consecutive base pairs in the target DNA. For example, a 3 finger domain recognized a sequence of 9 contiguous nucleotides, with a dimerization requirement of the nuclease, two sets of zinc finger triplets are used to bind an 18 nucleotide recognition sequence.

DNA double strand break (DSB) technologies (ZFNs, TALENs and CRISPR-Cas) have wide-ranging applications in academic research, gene therapy, and animal and plant breeding programs. These technologies have been successfully used to introduce genome modifications in multiple plant species, including major crops such as maize, wheat, soybean and rice. Plant genome editing is limited by current transformation and gene modification methods, efficiency of DNA delivery, and low frequencies of plant regeneration. In contrast to human and animal systems, the presence of a thick wall surrounding every plant cell fundamentally impacts plant transformation and plant gene modification protocols. This cell wall makes it impossible to use transfection or electroporation, which are broadly used for nucleic acid and/or protein delivery in mammalian genome editing experiments. For this reason, plant transformation and plant genome modification primarily relies on Agrobacterium-mediated and biolistic delivery (ballistic delivery) of guide RNA/Cas endonuclease reagents on DNA vectors. As a result, gRNA and Cas9 expression cassettes frequently integrate into the genome and potentially lead to gene disruption, plant mosaicism, and potential off-site cutting. Although these undesired secondary changes can be segregated away by several rounds of backcrossing to the wild type parent plant, this process can be time consuming especially for crops with complex polyploid genomes and long breeding cycles such as, but not limited to, soybean and wheat. As described herein, delivery of Cas endonuclease and gRNAs in the form of RGEN complexes into plant cells can mitigate many of these side effects (Example 11-12). An unexpected high frequency of NHEJ-mediated mutagenesis facilitated by delivery of RGEN complexes in plants is described in Example 10. Given this high frequency of mutagenesis using a RGEN complex, DNA-free and selectable marker-free gene modification may become a practical approach to generate gene knock-outs. This DNA- and selectable marker-free approach might be less practical for gene editing and gene insertion (as compared to gene mutations by NHEJ) applications due to the low frequency of the HDR pathway in somatic plant cells. Moreover, DNA molecules often integrate into the targeted DSB sites, decreasing the efficiency of gene editing, and especially, gene insertion. It has been demonstrated that DNA vectors encoding for genes delivered into the plant cell (for example, Cas9, gRNA, selectable marker genes and trait gene) have tendency to co-integrate into the same DSB site dramatically reducing frequency of usable events with site-specific trait gene insertions. Limiting delivered DNA molecules to donor DNA (for example, trait gene with homology arms) can increase the probability of events with desirable genotype. Therefore, the concept, described herein, of disrupted (inactive) endogenous or pre-integrated selectable marker genes that can be activated upon RGEN delivery, can make the DNA- and selectable marker-free approach for gene editing and gene insertion become very practical.

The guide polynucleotide can be introduced into a cell directly, as single stranded polynucleotide or a double stranded polynucleotide, using any method known in the art such as, but not limited to, particle bombardment, whiskers mediated transformation, *Agrobacterium transformation* or topical applications. The guide RNA can also be introduced indirectly into a cell by introducing a recombinant DNA molecule (via methods such as, but not limited to, particle bombardment or *Agrobacterium transformation*) comprising a heterologous nucleic acid fragment encoding a guide RNA, operably linked to a specific promoter that is capable of transcribing the guide RNA in said cell. The specific promoter can be, but is not limited to, a RNA polymerase III promoter, which allow for transcription of RNA with precisely defined, unmodified, 5'- and 3'-ends (DiCarlo et al., Nucleic Acids Res. 41 : 4336-4343; Ma et al., Mol. Ther. Nucleic Acids 3:e161). As described herein, direct delivery of a sgRNA into plant cells can be achieved through particle mediated delivery. Based on the experiments described herein, a skilled artesian can now envision that any other direct method of delivery, such as but not limiting to, polyethylene glycol (PEG)-mediated transfection to protoplasts, whiskers mediated transformation, electroporation, particle bombardment, cell-penetrating peptides, or mesoporous silica nanoparticle (MSN)-mediated direct protein delivery can be successfully used for delivering gRNA in plant cells.

The guide polynucleotide can be produced by any method known in the art, including chemically synthesizing guide polynucleotides (such as but not limiting to Hendel et al. 2015, Nature Biotechnology 33, 985-989), in vitro generated guide polynucleotides, and/or self-splicing guide RNAs (such as but not limiting to Xie et al. 2015, PNAS 112:3570-3575).

The terms "target site", "target sequence", "target site sequence, "target DNA", "target locus", "genomic target site", "genomic target sequence", "genomic target locus" and "protospacer", are used interchangeably herein and refer to a polynucleotide sequence such as, but not limited to, a nucleotide sequence on a chromosome, episome, a transgenic locus, or any other DNA molecule in the genome (including chromosomal, choloroplastic, mitochondrial DNA, plasmid DNA) of a cell, at which a guide polynucleotide/Cas endonuclease complex can recognize, bind to, and optionally nick or cleave . The target site can be an endogenous site in the genome of a cell, or alternatively, the target site can be heterologous to the cell and thereby not be naturally occurring in the genome of the cell, or the target site can be found in a heterologous genomic location compared to where it occurs in nature. As used herein, terms "endogenous target sequence" and "native target sequence" are used interchangeable herein to refer to a target sequence that is endogenous or native to the genome of a cell and is at the endogenous or native position of that target sequence in the genome of the cell. Cells include, but are not limited to, human, non-human, animal, bacterial, fungal, insect, yeast, non-conventional yeast, and plant cells as well as plants and seeds produced by the methods described herein. An "artificial target site" or "artificial target sequence" are used interchangeably herein and refer to a target sequence that has been introduced into the genome of a cell. Such an artificial target sequence can be identical in sequence to an endogenous or native target sequence in the genome of a cell but be located in a different position (i.e., a non-endogenous or non-native position) in the genome of a cell.

An "altered target site", "altered target sequence", "modified target site", "modified target sequence" are used interchangeably herein and refer to a target sequence as disclosed herein that comprises at least one alteration when compared to non-altered target sequence. Such "alterations" include, for example:
(i) replacement of at least one nucleotide, (ii) a deletion of at least one nucleotide, (iii) an insertion of at least one nucleotide, or (iv) any combination of (i) - (iii).

The length of the target DNA sequence (target site) can vary, and includes, for example, target sites that are at least 12, 13, 14, 15, 16, 17, 18, 19, 20, 21, 22, 23, 24, 25, 26, 27, 28, 29, 30 or more nucleotides in length. It is further possible that the target site can be palindromic, that is, the sequence on one strand reads the same in the opposite direction on the complementary strand. The nick/cleavage site can be within the target sequence or the nick/cleavage site could be outside of the target sequence. In another variation, the cleavage could occur at nucleotide positions immediately opposite each other to produce a blunt end cut or, in other Cases, the incisions could be staggered to produce single-stranded overhangs, also called "sticky ends", which can be either 5' overhangs, or 3' overhangs. Active variants of genomic target sites can also be used. Such active variants can comprise at least 65%, 70%, 75%, 80%, 85%, 90%, 91%, 92%, 93%, 94%, 95%, 96%, 97%, 98%, 99% or more sequence identity to the given target site, wherein the active variants retain biological activity and hence are capable of being recognized and cleaved by an Cas endonuclease. Assays to measure the single or double-strand break of a target site by an endonuclease are known in the art and generally measure the overall activity and specificity of the agent on DNA substrates containing recognition sites.

A "protospacer adjacent motif" (PAM) herein refers to a short nucleotide sequence adjacent to a target sequence (protospacer) that is recognized (targeted) by a guide polynucleotide/Cas endonuclease system. The Cas endonuclease may not successfully recognize a target DNA sequence if the target DNA sequence is not followed by a PAM sequence. The sequence and length of a PAM herein can differ depending on the Cas protein or Cas protein complex used. The PAM sequence can be of any length but is typically 1, 2, 3, 4, 5, 6, 7, 8, 9, 10, 11, 12, 13, 14, 15, 16, 17, 18, 19 or 20 nucleotides long.

The terms "targeting", "gene targeting" and "DNA targeting" are used interchangeably herein. DNA targeting herein may be the specific introduction of a knock-out, edit, or knock-in at a particular DNA sequence, such as in a chromosome or plasmid of a cell. In general, DNA targeting can be performed herein by cleaving one or both strands at a specific DNA sequence in a cell with a Cas protein associated with a suitable polynucleotide component. Such DNA cleavage, if a double-strand break (DSB), can prompt NHEJ or HDR processes which can lead to modifications at the target site.

The terms "knock-out", "gene knock-out" and "genetic knock-out" are used interchangeably herein. A knock-out represents a DNA sequence of a cell that has been rendered partially or completely inoperative by targeting with a Cas protein; such a DNA sequence prior to knock-out could have encoded an amino acid sequence, or could have had a regulatory function (e.g., promoter), for example. A knock-out may be produced by an indel (insertion or deletion of nucleotide bases in a target DNA sequence through NHEJ), or by specific removal of sequence that reduces or completely destroys the function of sequence at or near the targeting site.

The guide polynucleotide/Cas endonuclease system can be used in combination with a co-delivered polynucleotide modification template to allow for editing (modification) of a genomic nucleotide sequence of interest. (See also U.S. Patent Application US 2015-0082478 A1, published on March 19, 2015 and WO2015/026886 A1, published on February 26, 2015.)

A "modified nucleotide" or "edited nucleotide" refers to a nucleotide sequence of interest that comprises at least one alteration when compared to its non-modified nucleotide sequence. Such "alterations" include, for example: (i) replacement of at least one nucleotide, (ii) a deletion of at least one nucleotide, (iii) an insertion of at least one nucleotide, or (iv) any combination of (i) - (iii).

The term "polynucleotide modification template" includes a polynucleotide that comprises at least one nucleotide modification when compared to the nucleotide sequence to be edited. A nucleotide modification can be at least one nucleotide substitution, addition or deletion. Optionally, the polynucleotide modification template can further comprise homologous nucleotide sequences flanking the at least one nucleotide modification, wherein the flanking homologous nucleotide sequences provide sufficient homology to the desired nucleotide sequence to be edited.

A polynucleotide modification template can be introduced into a cell by any method known in the art, such as, but not limited to, transient introduction methods, transfection, electroporation, microinjection, particle mediated delivery, topical application, whiskers mediated delivery, delivery via cell-penetrating peptides, or mesoporous silica nanoparticle (MSN)-mediated direct delivery.

The polynucleotide modification template can be introduced into a cell as a single stranded polynucleotide molecule, a double stranded polynucleotide molecule, or as part of a circular DNA (vector DNA). The polynucleotide modification template can also be tethered to the guide RNA and/or the Cas endonuclease. Tethered DNAs can allow for co-localizing target and template DNA, useful in genome editing and targeted genome regulation, and can also be useful in targeting post-mitotic cells where function of endogenous HR machinery is expected to be highly diminished (Mali et al. 2013 Nature Methods Vol. 10 : 957-963.) The polynucleotide modification template may be present transiently in the cell or it can be introduced via a viral replicon.

The nucleotide to be edited can be located within or outside a target site recognized and cleaved by a Cas endonuclease. In one aspect of the disclosure, the at least one nucleotide modification is not a modification at a target site recognized and cleaved by a Cas endonuclease. In another aspect of the disclosure, there are at least 1, 2, 3, 4, 5, 6, 7, 8, 9, 10, 11, 12, 13, 14, 15, 16, 17, 18, 19, 20, 21, 22, 23, 24, 25, 26, 27, 30, 40, 50, 100, 200, 300, 400, 500, 600, 700, 900 or 1000 nucleotides between the at least one nucleotide to be edited and the genomic target site.

Genome editing can be accomplished using any method of gene editing available. For example, gene editing can be accomplished through the introduction into a host cell of a polynucleotide modification template (sometimes also referred to as a gene repair oligonucleotide) containing a targeted modification to a gene within the genome of the host cell. The polynucleotide modification template for use in such methods can be either single-stranded or double-stranded. Examples of such methods are generally described, for example, in US Publication No. 2013/0019349.

In one aspect of the disclosure, the method comprises a method for modifying a nucleotide sequence in the genome of a cell, the method comprising: introducing into at least one cell comprising a target site and a disrupted selectable marker gene, at least one polynucleotide modification template, a first guide RNA, a Cas endonuclease, and at least a second guide RNA, wherein said first guide RNA and Cas endonuclease can form a first complex capable of introducing a double strand break in said disrupted selectable marker gene, wherein said disrupted selectable marker gene is restored without the use of a polynucleotide modification template to a non-disrupted selectable marker gene capable of encoding a functional selectable marker protein, wherein said polynucleotide modification template comprises at least one nucleotide modification of said nucleotide sequence, wherein said second guide RNA and Cas endonuclease can form a second complex that is capable of recognizing, binding to, and nicking or cleaving said target site located in said nucleotide sequence; and, selecting a cell having a modification in said nucleotide sequence, wherein the selection is provided by said functional selectable marker protein.. The introducing and selection step does not comprise the introduction of a selectable marker gene, such as a recombinant DNA construct comprising a selectable marker gene. The disrupted selectable marker gene can be any disrupted marker gene including a disrupted visible marker gene.

Based on the experiments described herein, a skilled artesian can now envision that any other direct method of delivery, such as but not limiting to, polyethylene glycol (PEG)-mediated transfection to protoplasts, whiskers mediated transformation, electroporation, particle bombardment, cell-penetrating peptides, or mesoporous silica nanoparticle (MSN)-mediated direct protein delivery can be successfully used for delivering a polynucleotide modification template in plant cells.

In some aspects of the disclosure, gene editing may be facilitated through the induction of a double-stranded break (DSB) in a defined position in the genome near the desired alteration. DSBs can be induced using any DSB-inducing agent available, including, but not limited to, TALENs, meganucleases, zinc finger nucleases, nucleic acid guided-endonuclease systems, e.g. Cas9-gRNA systems (based on bacterial CRISPR-Cas systems). In some aspects of the disclosure, the introduction of a DSB can be combined with the introduction of a polynucleotide modification template.

The process for editing a genomic sequence combining DSB and modification templates generally comprises: introducing to a host cell, a DSB-inducing agent, or a nucleic acid encoding a DSB-inducing agent, that recognizes a target sequence in the chromosomal sequence and is able to induce a DSB in the genomic sequence, and at least one polynucleotide modification template comprising at least one nucleotide alteration when compared to the nucleotide sequence to be edited. The polynucleotide modification template can further comprise nucleotide sequences flanking the at least one nucleotide alteration, in which the flanking sequences are substantially homologous to the chromosomal region flanking the DSB. Genome editing using DSB-inducing agents, such as Cas9-gRNA complexes, has been described, for example in U.S. Patent Application US 2015-0082478 A1, published on March 19, 2015, WO2015/026886 A1, published on February 26, 2015, US application 62/023246, filed on July 07, 2014, and US application 62/036,652, filed on August 13, 2014.

Described herein are methods to method for editing a nucleotide sequence in the genome of a cell without the use of a polynucleotide modification template.

Gene editing using guided Cas endonucleases systems and a polynucleotide modification templates to modify a target sequence or a nucleotide of interest near a target sequence relies on homologous recombination / homologous directed repair (HDR) mechanisms that occur at a lower frequency in plant cell when compared to the frequency of Non Homologous End Joining (NHEJ). It would be desirable to increase the frequency of gene editing by not having to rely on HDR type mechanisms. Described herein are methods that enable gene editing without the use of a polynucleotide modification template, by relying on the restoration of a disrupted gene, wherein the restoration is accomplished by Non-Homologous-End - Joining (NHEJ) resulting in the insertion of at least a single base at the double strand break site or wherein the restoration is accomplished by the insertion of at least a single base at the double strand break site without the use of Homologous Recombination or Homology Directed Repair.

In one aspect of the disclosure, the method comprises a method for editing a nucleotide sequence in the genome of a cell without the use of a polynucleotide modification template, the method comprising: a) introducing into at least one cell at least one guide RNA and at least one Cas endonuclease, wherein said guide RNA and Cas endonuclease can form a complex capable of introducing a double strand break in said nucleotide sequence; b) selecting a cell from (a) comprising at least one single nucleotide deletion in said nucleotide sequence, wherein said nucleotide deletion is located at a position to be edited; and, c) introducing into a cell of (b) at least one guide RNA and at least one Cas endonuclease, wherein said guide RNA and Cas endonuclease can form a complex capable of introducing a double strand break in said nucleotide sequence and insert a single nucleotide at the same position of the nucleotide deletion of (b) without the use of a polynucleotide modification template.

In one aspect of the disclosure, the method comprises a method for editing a nucleotide sequence in the genome of a plant without the use of a polynucleotide modification template or donor DNA, the method comprising: a) introducing into at least one plant cell at least one guide RNA and at least one Cas endonuclease, wherein said guide RNA and Cas endonuclease can form a complex capable of introducing a double strand break in said nucleotide sequence; b) selecting a plant cell from (a) comprising at least one single nucleotide deletion in said nucleotide sequence, wherein said nucleotide deletion is located at a position to be edited; c) regenerating a plant from the plant cell of (b); d) introducing into a cell from the plant of (c) at least one guide RNA and at least one Cas endonuclease, wherein said guide RNA and Cas endonuclease can form a complex capable of introducing a double strand break in said nucleotide sequence and inserting a single nucleotide at the same position of the nucleotide deletion of (b) without the use of a polynucleotide modification template; and, e) optimally, selecting a cell comprising the nucleotide insertion of (d).

The terms "knock-in", "gene knock-in , "gene insertion" and "genetic knock-in" are used interchangeably herein. A knock-in represents the replacement or insertion of a DNA sequence at a specific DNA sequence in a cell by targeting with a Cas protein (by HR, wherein a suitable donor DNA polynucleotide is also used). Examples of knock-ins are a specific insertion of a heterologous amino acid coding sequence in a coding region of a gene, or a specific insertion of a transcriptional regulatory element in a genetic locus.

Various methods and compositions can be employed to obtain a cell or organism having a polynucleotide of interest inserted in a target site for a Cas endonuclease. Such methods can employ homologous recombination to provide integration of the polynucleotide of Interest at the target site. In one method disclosed herein, a polynucleotide of interest is provided to the organism cell in a donor DNA construct.

As used herein, "donor DNA" includes reference to a DNA construct that comprises a polynucleotide of interest to be inserted into the target site of a Cas endonuclease. The donor DNA construct can further comprise a first and a second region of homology that flank the polynucleotide of Interest. The first and second regions of homology of the donor DNA share homology to a first and a second genomic region, respectively, present in or flanking the target site of the cell or organism genome. The donor DNA can be tethered to the guide polynucleotide and /or the Cas endonuclease. Tethered donor DNAs can allow for co-localizing target and donor DNA, useful in genome editing and targeted genome regulation, and can also be useful in targeting post-mitotic cells where function of endogenous HR machinery is expected to be highly diminished (Mali et al. 2013 Nature Methods Vol. 10 : 957-963.)

By "homology" is meant DNA sequences that are similar. For example, a "region of homology to a genomic region" that is found on the donor DNA is a region of DNA that has a similar sequence to a given "genomic region" in the cell or organism genome. A region of homology can be of any length that is sufficient to promote homologous recombination at the cleaved target site. For example, the region of homology can comprise at least 5-10, 5-15, 5-20, 5-25, 5-30, 5-35, 5-40, 5-45, 5- 50, 5-55, 5-60, 5-65, 5- 70, 5-75, 5-80, 5-85, 5-90, 5-95, 5-100, 5-200, 5-300, 5-400, 5-500, 5-600, 5-700, 5-800, 5-900, 5-1000, 5-1100, 5-1200, 5-1300, 5-1400, 5-1500, 5-1600, 5-1700, 5-1800, 5-1900, 5-2000, 5-2100, 5-2200, 5-2300, 5-2400, 5-2500, 5-2600, 5-2700, 5-2800, 5-2900, 5-3000, 5-3100 or more bases in length such that the region of homology has sufficient homology to undergo homologous recombination with the corresponding genomic region. "Sufficient homology" indicates that two polynucleotide sequences have sufficient structural similarity to act as substrates for a homologous recombination reaction. The structural similarity includes overall length of each polynucleotide fragment, as well as the sequence similarity of the polynucleotides. Sequence similarity can be described by the percent sequence identity over the whole length of the sequences, and/or by conserved regions comprising localized similarities such as contiguous nucleotides having 100% sequence identity, and percent sequence identity over a portion of the length of the sequences.

The amount of homology or sequence identity shared by a target and a donor polynucleotide can vary and includes total lengths and/or regions having unit integral values in the ranges of about 1-20 bp, 20-50 bp, 50-100 bp, 75-150 bp, 100-250 bp, 150-300 bp, 200-400 bp, 250-500 bp, 300-600 bp, 350-750 bp, 400-800 bp, 450-900 bp, 500-1000 bp, 600-1250 bp, 700-1500 bp, 800-1750 bp, 900-2000 bp, 1-2.5 kb, 1.5-3 kb, 2-4 kb, 2.5-5 kb, 3-6 kb, 3.5-7 kb, 4-8 kb, 5-10 kb, or up to and including the total length of the target site. These ranges include every integer within the range, for example, the range of 1-20 bp includes 1, 2, 3, 4, 5, 6, 7, 8, 9, 10, 11, 12, 13, 14, 15, 16, 17, 18, 19 and 20 bps. The amount of homology can also described by percent sequence identity over the full aligned length of the two polynucleotides which includes percent sequence identity of about at least 50%, 55%, 60%, 65%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. Sufficient homology includes any combination of polynucleotide length, global percent sequence identity, and optionally conserved regions of contiguous nucleotides or local percent sequence identity, for example sufficient homology can be described as a region of 75-150 bp having at least 80% sequence identity to a region of the target locus. Sufficient homology can also be described by the predicted ability of two polynucleotides to specifically hybridize under high stringency conditions, see, for example, Sambrook et al., (1989) Molecular Cloning: A Laboratory Manual, (Cold Spring Harbor Laboratory Press, NY); Current Protocols in Molecular Biology, Ausubel et al., Eds (1994) Current Protocols, (Greene Publishing Associates, Inc. and John Wiley & Sons, Inc.); and, Tijssen (1993) Laboratory Techniques in Biochemistry and Molecular BiologyHybridization with Nucleic Acid Probes, (Elsevier, New York).

As used herein, a "genomic region" is a segment of a chromosome in the genome of a cell that is present on either side of the target site or, alternatively, also comprises a portion of the target site. The genomic region can comprise at least 5-10, 5-15, 5-20, 5-25, 5-30, 5-35, 5-40, 5-45, 5- 50, 5-55, 5-60, 5-65, 5- 70, 5-75, 5-80, 5-85, 5-90, 5-95, 5-100, 5-200, 5-300, 5-400, 5-500, 5-600, 5-700, 5-800, 5-900, 5-1000, 5-1100, 5-1200, 5-1300, 5-1400, 5-1500, 5-1600, 5-1700, 5-1800, 5-1900, 5-2000, 5-2100, 5-2200, 5-2300, 5-2400, 5-2500, 5-2600, 5-2700, 5-2800. 5-2900, 5-3000, 5-3100 or more bases such that the genomic region has sufficient homology to undergo homologous recombination with the corresponding region of homology.

Polynucleotides of interest and/or traits can be stacked together in a complex trait locus as described in US-2013-0263324-A1, published 03 Oct 2013 and in PCT/US13/22891, published January 24, 2013. The guide polynucleotide/Cas9 endonuclease system described herein provides for an efficient system to generate double strand breaks and allows for traits to be stacked in a complex trait locus.

The guide polynucleotide/Cas endonuclease system can be used for introducing one or more polynucleotides of interest or one or more traits of interest into one or more target sites by introducing one or more guide polynucleotides, one Cas endonuclease, and optionally one or more donor DNAs to a plant cell. ((as described in US patent application US-2015-0082478-A1, published on March 19, 2015). A fertile plant can be produced from that plant cell that comprises an alteration at said one or more target sites, wherein the alteration is selected from the group consisting of (i) replacement of at least one nucleotide, (ii) a deletion of at least one nucleotide, (iii) an insertion of at least one nucleotide, and (iv) any combination of (i) - (iii). Plants comprising these altered target sites can be crossed with plants comprising at least one gene or trait of interest in the same complex trait locus, thereby further stacking traits in said complex trait locus (see also US-2013-0263324-A1, published October 3, 2013 and in PCT/US13/22891, published January 24, 2013).

The structural similarity between a given genomic region and the corresponding region of homology found on the donor DNA can be any degree of sequence identity that allows for homologous recombination to occur. For example, the amount of homology or sequence identity shared by the "region of homology" of the donor DNA and the "genomic region" of the organism genome can be at least 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity, such that the sequences undergo homologous recombination

The region of homology on the donor DNA can have homology to any sequence flanking the target site. While in some aspects of the disclosure the regions of homology share significant sequence homology to the genomic sequence immediately flanking the target site, it is recognized that the regions of homology can be designed to have sufficient homology to regions that may be further 5' or 3' to the target site. In still other aspects of the disclosure, the regions of homology can also have homology with a fragment of the target site along with downstream genomic regions. In one aspect of the disclosure, the first region of homology further comprises a first fragment of the target site and the second region of homology comprises a second fragment of the target site, wherein the first and second fragments are dissimilar.

Once a double-strand break is induced in the DNA, the cell's DNA repair mechanism is activated to repair the break. The Non-Homologous-End-Joining (NHEJ) pathways are the most common repair mechanism to bring the broken ends together (Bleuyard et al., (2006) DNA Repair 5:1-12). The structural integrity of chromosomes is typically preserved by the repair, but deletions, insertions, or other rearrangements are possible. The two ends of one double-strand break are the most prevalent substrates of NHEJ (Kirik et al., (2000) EMBO J 19:5562-6), however if two different double-strand breaks occur, the free ends from different breaks can be ligated and result in chromosomal deletions (Siebert and Puchta, (2002) Plant Cell 14:1121-31), or chromosomal translocations between different chromosomes (Pacher et al., (2007) Genetics 175:21-9). Error-prone DNA repair mechanisms can produce mutations at double-strand break sites. The Non-Homologous-End-Joining (NHEJ) pathways are the most common repair mechanism to bring the broken ends together (Bleuyard et al., (2006) DNA Repair 5:1-12).

Alternatively, the double-strand break can be repaired by homologous recombination (HR) between homologous DNA sequences. Once the sequence around the double-strand break is altered, for example, by exonuclease activities involved in the maturation of double-strand breaks, gene conversion pathways can restore the original structure if a homologous sequence is available, such as a homologous chromosome in non-dividing somatic cells, or a sister chromatid after DNA replication (Molinier et al., (2004) Plant Cell 16:342-52). Ectopic and/or epigenic DNA sequences may also serve as a DNA repair template for homologous recombination (Puchta, (1999) Genetics 152:1173-81). Episomal DNA molecules can also be ligated into the double-strand break, for example, integration of T-DNAs into chromosomal double-strand breaks (Chilton and Que, (2003) Plant Physiol 133:956-65; Salomon and Puchta, (1998) EMBO J 17:6086-95).

As used herein, "homologous recombination (HR)" includes the exchange of DNA fragments between two DNA molecules at the sites of homology. The frequency of homologous recombination is influenced by a number of factors. Different organisms vary with respect to the amount of homologous recombination and the relative proportion of homologous to non-homologous recombination. Generally, the length of the region of homology affects the frequency of homologous recombination events: the longer the region of homology, the greater the frequency. The length of the homology region needed to observe homologous recombination is also species-variable. In many cases, at least 5 kb of homology has been utilized, but homologous recombination has been observed with as little as 25-50 bp of homology. See, for example, Singer et al., (1982) Cell 31:25-33; Shen and Huang, (1986) Genetics 112:441-57; Watt et al., (1985) Proc. Natl. Acad. Sci. USA 82:4768-72, Sugawara and Haber, (1992) Mol Cell Biol 12:563-75, Rubnitz and Subramani, (1984) Mol Cell Biol 4:2253-8; Ayares et al., (1986) Proc. Natl. Acad. Sci. USA 83:5199-203; Liskay et al., (1987) Genetics 115:161-7.

Homology-directed repair (HDR) is a mechanism in cells to repair double-stranded and single stranded DNA breaks. Homology-directed repair includes homologous recombination (HR) and single-strand annealing (SSA) (Lieber. 2010 Annu. Rev. Biochem. 79:181-211).The most common form of HDR is called homologous recombination (HR), which has the longest sequence homology requirements between the donor and acceptor DNA. Other forms of HDR include single-stranded annealing (SSA) and breakage-induced replication, and these require shorter sequence homology relative to HR. Homology-directed repair at nicks (single-stranded breaks) can occur via a mechanism distinct from HDR at double-strand breaks (Davis and Maizels. PNAS (0027-8424), 111 (10), p. E924-E932.

Alteration of the genome of a plant cell, for example, through homologyrirected repair (HDR),, is a powerful tool for genetic engineering. Despite the low frequency of homologous recombination in higher plants, there are a few examples of successful homologous recombination of plant endogenous genes. The parameters for homologous recombination in plants have primarily been investigated by rescuing introduced truncated selectable marker genes. In these experiments, the homologous DNA fragments were typically between 0.3 kb to 2 kb. Observed frequencies for homologous recombination were on the order of 10⁻⁴ to 10⁻⁵. See, for example, Halfter et al., (1992) Mol Gen Genet 231:186-93; Offringa et al., (1990) EMBO J 9:3077-84; Offringa et al., (1993) Proc. Natl. Acad. Sci. USA 90:7346-50; Paszkowski et al., (1988) EMBO J 7:4021-6; Hourda and Paszkowski, (1994) Mol Gen Genet 243:106-11; and Risseeuw et al., (1995) Plant J 7:109-19.

DNA double-strand breaks appear to be an effective factor to stimulate homologous recombination pathways (Puchta et al., (1995) Plant Mol Biol 28:281-92; Tzfira and White, (2005) Trends Biotechnol 23:567-9; Puchta, (2005) J Exp Bot 56:1-14). Using DNA-breaking agents, a two- to nine-fold increase of homologous recombination was observed between artificially constructed homologous DNA repeats in plants (Puchta et al., (1995) Plant Mol Biol 28:281-92). In maize protoplasts, experiments with linear DNA molecules demonstrated enhanced homologous recombination between plasmids (Lyznik et al., (1991) Mol Gen Genet 230:209-18).

The donor DNA may be introduced by any means known in the art. For example, a plant having a target site is provided. The donor DNA may be provided by any delivery method known in the art including, for example, *Agrobacterium-*mediated transformation, whiskers mediated transformation, or biolistic particle bombardment. The donor DNA may be present transiently in the cell or it can be introduced via a viral replicon. In the presence of the Cas endonuclease and the target site, the donor DNA is inserted into the plant's genome.

As described herein, direct delivery of a donor DNA into plant cells can be achieved through particle mediated delivery. Based on the experiments described herein, a skilled artesian can now envision that any other direct method of delivery, such as but not limiting to, polyethylene glycol (PEG)-mediated transfection to protoplasts, electroporation, particle bombardment, whiskers mediated delivery, cell-penetrating peptides, or mesoporous silica nanoparticle (MSN)-mediated direct protein delivery can be successfully used for delivering a donor DNA in plant cells.

In one aspect of the disclosure, the method comprises a method for modifying a nucleotide sequence in the genome of a cell, the method comprising: introducing into at least one cell comprising a target site and a disrupted selectable marker gene, at least one donor DNA, a first guide RNA, a Cas endonuclease, and at least a second guide RNA, wherein said first guide RNA and Cas endonuclease can form a first complex capable of introducing a double strand break in said disrupted selectable marker gene, wherein said disrupted selectable marker gene is restored without the use of a polynucleotide modification template to a non-disrupted selectable marker gene capable of encoding a functional selectable marker protein, wherein said donor DNA comprises at least one polynucleotide of interest to be inserted into said target site, wherein said second guide RNA and Cas endonuclease can form a second complex that is capable of recognizing, binding to, and nicking or cleaving said target site located in said nucleotide sequence; and, selecting a cell having a modification in said nucleotide sequence, wherein the selection is provided by said functional selectable marker protein . The introducing and selection step does not comprise the introduction of a selectable marker gene, such as a recombinant DNA construct comprising a selectable marker gene. The disrupted selectable marker gene can be any disrupted marker gene including a disrupted visible marker gene.

Further uses for guide RNA/Cas endonuclease systems have been described (See U.S. Patent Application US 2015-0082478 A1, published on March 19, 2015, WO2015/026886 A1, published on February 26, 2015, US 2015-0059010 A1, published on February 26, 2015, US application 62/023246, filed on July 07, 2014, and US application 62/036,652, filed on August 13, 2014) and include but are not limited to modifying or replacing nucleotide sequences of interest (such as a regulatory elements), insertion of polynucleotides of interest, gene knock-out, gene-knock in, modification of splicing sites and/or introducing alternate splicing sites, modifications of nucleotide sequences encoding a protein of interest, amino acid and/or protein fusions, and gene silencing by expressing an inverted repeat into a gene of interest.

Polynucleotides of interest are further described herein and include polynucleotides reflective of the commercial markets and interests of those involved in the development of the crop. Crops and markets of interest change, and as developing nations open up world markets, new crops and technologies will emerge also. In addition, as our understanding of agronomic traits and characteristics such as yield and heterosis increase, the choice of genes for genetic engineering will change accordingly.

Further disclosed herein are methods for identifying at least one plant cell, comprising in its genome, a polynucleotide of interest integrated at the target site. A variety of methods are available for identifying those plant cells with insertion into the genome at or near to the target site without using a screenable marker phenotype. Such methods can be viewed as directly analyzing a target sequence to detect any change in the target sequence, including but not limited to PCR methods, sequencing methods, nuclease digestion, Southern blots, and any combination thereof. See, for example, US Patent Application 12/147,834. The method also comprises recovering a plant from the plant cell comprising a polynucleotide of Interest integrated into its genome. The plant may be sterile or fertile. It is recognized that any polynucleotide of interest can be provided, integrated into the plant genome at the target site, and expressed in a plant.

Polynucleotides/polypeptides of interest include, but are not limited to, herbicide-resistance coding sequences, insecticidal coding sequences, nematicidal coding sequences, antimicrobial coding sequences, antifungal coding sequences, antiviral coding sequences, abiotic and biotic stress tolerance coding sequences, or sequences modifying plant traits such as yield, grain quality, nutrient content, starch quality and quantity, nitrogen fixation and/or utilization, fatty acids, and oil content and/or composition. More specific polynucleotides of interest include, but are not limited to, genes that improve crop yield, polypeptides that improve desirability of crops, genes encoding proteins conferring resistance to abiotic stress, such as drought, nitrogen, temperature, salinity, toxic metals or trace elements, or those conferring resistance to toxins such as pesticides and herbicides, or to biotic stress, such as attacks by fungi, viruses, bacteria, insects, and nematodes, and development of diseases associated with these organisms. General categories of genes of interest include, for example, those genes involved in information, such as zinc fingers, those involved in communication, such as kinases, and those involved in housekeeping, such as heat shock proteins. More specific categories of transgenes, for example, include genes encoding important traits for agronomics, insect resistance, disease resistance, herbicide resistance, fertility or sterility, grain characteristics, and commercial products. Genes of interest include, generally, those involved in oil, starch, carbohydrate, or nutrient metabolism as well as those affecting kernel size and sucrose loading that can be stacked or used in combination with other traits, such as but not limited to herbicide resistance, described herein.

Agronomically important traits such as oil, starch, and protein content can be genetically altered in addition to using traditional breeding methods. Modifications include increasing content of oleic acid, saturated and unsaturated oils, increasing levels of lysine and sulfur, introducing essential amino acids, and also modification of starch. Hordothionin protein modifications are described in U.S. Patent Nos. 5,703,049, 5,885,801, 5,885,802, and 5,990,389.

Polynucleotide sequences of interest may encode proteins involved in introducing disease or pest resistance. By "disease resistance" or "pest resistance" is intended that the plants avoid the harmful symptoms that are the outcome of the plant-pathogen interactions. Pest resistance genes may encode resistance to pests that have great yield drag such as rootworm, cutworm, and European Corn Borer. Disease resistance and insect resistance genes such as lysozymes or cecropins for antibacterial protection, or proteins such as defensins, glucanases or chitinases for antifungal protection, or Bacillus thuringiensis endotoxins, protease inhibitors, collagenases, lectins, or glycosidases for controlling nematodes or insects are all examples of useful gene products. Genes encoding disease resistance traits include detoxification genes, such as against fumonisin (U.S. Patent No. 5,792,931); avirulence (avr) and disease resistance (R) genes (Jones et al. (1994) Science 266:789; Martin et al. (1993) Science 262:1432; and Mindrinos et al. (1994) Cell 78:1089). Insect resistance genes may encode resistance to pests that have great yield drag such as rootworm, cutworm, and European Corn Borer. Such genes include, for example, *Bacillus thuringiensis* toxic protein genes (U.S. Patent Nos. 5,366,892; 5,747,450; 5,736,514; 5,723,756; 5,593,881; and Geiser et al. (1986) Gene 48:109).

An "herbicide resistance protein" or a protein resulting from expression of an "herbicide resistance-encoding nucleic acid molecule" includes proteins that confer upon a cell the ability to tolerate a higher concentration of an herbicide than cells that do not express the protein, or to tolerate a certain concentration of an herbicide for a longer period of time than cells that do not express the protein. Herbicide resistance traits may be introduced into plants by genes coding for resistance to herbicides that act to inhibit the action of acetolactate synthase (ALS also called AHAS), in particular the sulfonylurea-type (UK: sulphonylurea) herbicides, genes coding for resistance to herbicides that act to inhibit the action of glutamine synthase, such as phosphinothricin or basta (e.g., the bar gene), glyphosate (e.g., the EPSP synthase gene and the GAT gene), HPPD inhibitors (e.g, the HPPD gene) or other such genes known in the art. See, for example, US Patent Nos. 7,626,077, 5,310,667, 5,866,775, 6,225,114, 6,248,876, 7,169,970, 6,867,293, and US Provisional Application No. 61/401,456. The bar gene encodes resistance to the herbicide basta, the *nptII* gene encodes resistance to the antibiotics kanamycin and geneticin, and the ALS-gene mutants encode resistance to the herbicide chlorsulfuron.

As used herein, a "sulfonylurea-tolerant polypeptide" comprises any polypeptide which when expressed in a plant confers tolerance to at least one sulfonylurea. Sulfonylurea herbicides inhibit growth of higher plants by blocking acetolactate synthase (ALS), also known as, acetohydroxy acid synthase (AHAS). Plants containing particular mutations in ALS (e.g., the S4 and/or HRA mutations) are tolerant to sulfonylurea herbicides. The production of sulfonylurea-tolerant plants is described more fully in U.S. Patent Nos. 5,605,011; 5,013,659; 5,141,870; 5,767,361; 5,731,180; 5,304,732; 4,761,373; 5,331,107; 5,928,937; and 5,378,824; and international publication WO 96/33270, and in Tan et al. 2005. Imidazolinone-tolerant crops: history, current status and future. Pest Manag Sci 61:246-257. The sulfonylurea-tolerant polypeptide can be encoded by, for example, the SuRA or SuRB locus of ALS. In specific aspects of the disclosure, the ALS inhibitor-tolerant polypeptide comprises the C3 ALS mutant, the HRA ALS mutant, the S4 mutant or the S4/HRA mutant or any combination thereof. Different mutations in ALS are known to confer tolerance to different herbicides and groups (and/or subgroups) of herbicides; see, e.g., Tranel and Wright (2002) Weed Science 50:700-712. See also, U.S. Patent No. 5,605,011, 5,378,824, 5,141,870, and 5,013,659. The HRA mutation in ALS finds particular use in one aspect of the disclosure. The mutation results in the production of an acetolactate synthase polypeptide which is resistant to at least one sulfonylurea compound in

A gene encoding a sulfonylurea-tolerant polypeptide is referred to as a sulfonyl tolerant gene or a sulfonyl resistant gene. The terms sulfonyl tolerant gene or sulfonyl resistant gene are used interchangeably herein.

A disrupted sulfonylurea resistant (ALS) gene refers to a disrupted gene, of which its corresponding undisrupted gene encodes a sulfonylurea-tolerant polypeptide, that is modified such that its gene product no longer encodes a functional sulfonylurea-tolerant polypeptide.

In one aspect of the disclosure, the method comprises a method for producing a sulfonylurea resistant plant comprising a modified target site, the method comprising: a) introducing into a plant cell comprising a disrupted sulfonylurea resistant (ALS) gene, a first guide RNA, a Cas9 endonuclease, at least a second guide RNA, wherein said first guide RNA and Cas9 endonuclease can form a first complex capable of introducing a double strand in said disrupted sulfonylurea resistant (ALS) gene, wherein said second guide RNA and Cas9 endonuclease can form a second complex capable of introducing a double strand break at said target site; and, b) obtaining a sulfonylurea resistant plant from said plant cell, wherein said sulfonylurea resistant plant comprises a modification at said target, wherein said modification is selected from the group consisting of (i) a replacement of at least one nucleotide, (ii) a deletion of at least one nucleotide, (iii) an insertion of at least one nucleotide, and (iv) any combination of (i) - (iii).

Components of a sulfonylurea-responsive repressor system (as described in US 8,257,956, issued on September 4, 2012) can also be introduced into plant genomes to generate a repressor/operator/inducer systems into said plant where polypeptides can specifically bind to an operator, wherein the specific binding is regulated by a sulfonylurea compound.

Sterility genes can also be encoded in an expression cassette and provide an alternative to physical detasseling. Examples of genes used in such ways include male fertility genes such as MS26 (see for example U.S. Patents 7,098,388, 7,517,975, 7,612,251), MS45 (see for example U.S. Patents 5,478,369, 6,265,640) or MSCA1 (see for example U.S. Patent 7,919,676). Maize plants (Zea mays L.) can be bred by both self-pollination and cross-pollination techniques. Maize has male flowers, located on the tassel, and female flowers, located on the ear, on the same plant. It can self-pollinate ("selfing") or cross pollinate. Natural pollination occurs in maize when wind blows pollen from the tassels to the silks that protrude from the tops of the incipient ears. Pollination may be readily controlled by techniques known to those of skill in the art. The development of maize hybrids requires the development of homozygous inbred lines, the crossing of these lines, and the evaluation of the crosses. Pedigree breeding and recurrent selections are two of the breeding methods used to develop inbred lines from populations. Breeding programs combine desirable traits from two or more inbred lines or various broad-based sources into breeding pools from which new inbred lines are developed by selfing and selection of desired phenotypes. A hybrid maize variety is the cross of two such inbred lines, each of which may have one or more desirable characteristics lacked by the other or which complement the other. The new inbreds are crossed with other inbred lines and the hybrids from these crosses are evaluated to determine which have commercial potential. The hybrid progeny of the first generation is designated F1. The F1 hybrid is more vigorous than its inbred parents. This hybrid vigor, or heterosis, can be manifested in many ways, including increased vegetative growth and increased yield.

Hybrid maize seed can be produced by a male sterility system incorporating manual detasseling. To produce hybrid seed, the male tassel is removed from the growing female inbred parent, which can be planted in various alternating row patterns with the male inbred parent. Consequently, introducing that there is sufficient isolation from sources of foreign maize pollen, the ears of the female inbred will be fertilized only with pollen from the male inbred. The resulting seed is therefore hybrid (F1) and will form hybrid plants.

Field variation impacting plant development can result in plants tasseling after manual detasseling of the female parent is completed. Or, a female inbred plant tassel may not be completely removed during the detasseling process. In any event, the result is that the female plant will successfully shed pollen and some female plants will be self-pollinated. This will result in seed of the female inbred being harvested along with the hybrid seed which is normally produced. Female inbred seed does not exhibit heterosis and therefore is not as productive as F1 seed. In addition, the presence of female inbred seed can represent a germplasm security risk for the company producing the hybrid.

Alternatively, the female inbred can be mechanically detasseled by machine. Mechanical detasseling is approximately as reliable as hand detasseling, but is faster and less costly. However, most detasseling machines produce more damage to the plants than hand detasseling. Thus, no form of detasseling is presently entirely satisfactory, and a need continues to exist for alternatives which further reduce production costs and to eliminate self-pollination of the female parent in the production of hybrid seed.

Mutations that cause male sterility in plants have the potential to be useful in methods for hybrid seed production for crop plants such as maize and can lower production costs by eliminating the need for the labor-intensive removal of male flowers (also known as de-tasseling) from the maternal parent plants used as a hybrid parent. Mutations that cause male sterility in maize have been produced by a variety of methods such as X-rays or UV-irradiations, chemical treatments, or transposable element insertions (ms23, ms25, ms26, ms32) (Chaubal et al. (2000) Am J Bot 87:1193-1201). Conditional regulation of fertility genes through fertility/sterility "molecular switches" could enhance the options for designing new male-sterility systems for crop improvement (Unger et al. (2002) Transgenic Res 11:455-465).

Furthermore, it is recognized that the polynucleotide of interest may also comprise antisense sequences complementary to at least a portion of the messenger RNA (mRNA) for a targeted gene sequence of interest. Antisense nucleotides are constructed to hybridize with the corresponding mRNA. Modifications of the antisense sequences may be made as long as the sequences hybridize to and interfere with expression of the corresponding mRNA. In this manner, antisense constructions having 70%, 80%, or 85% sequence identity to the corresponding antisense sequences may be used. Furthermore, portions of the antisense nucleotides may be used to disrupt the expression of the target gene. Generally, sequences of at least 50 nucleotides, 100 nucleotides, 200 nucleotides, or greater may be used.

In addition, the polynucleotide of interest may also be used in the sense orientation to suppress the expression of endogenous genes in plants. Methods for suppressing gene expression in plants using polynucleotides in the sense orientation are known in the art. The methods generally involve transforming plants with a DNA construct comprising a promoter that drives expression in a plant operably linked to at least a portion of a nucleotide sequence that corresponds to the transcript of the endogenous gene. Typically, such a nucleotide sequence has substantial sequence identity to the sequence of the transcript of the endogenous gene, generally greater than about 65% sequence identity, about 85% sequence identity, or greater than about 95% sequence identity. See, U.S. Patent Nos. 5,283,184 and 5,034,323.

The polynucleotide of interest can also be a phenotypic marker.

The recombinant DNA molecules, DNA sequences of interest, and polynucleotides of interest can comprise one or more DNA sequences for gene silencing. Methods for gene silencing involving the expression of DNA sequences in plant are known in the art include, but are not limited to, cosuppression, antisense suppression, double-stranded RNA (dsRNA) interference, hairpin RNA (hpRNA) interference, intron-containing hairpin RNA (ihpRNA) interference, transcriptional gene silencing, and micro RNA (miRNA) interference

As used herein, "nucleic acid" means a polynucleotide and includes a single or a double-stranded polymer of deoxyribonucleotide or ribonucleotide bases. Nucleic acids may also include fragments and modified nucleotides. Thus, the terms "polynucleotide", "nucleic acid sequence", "nucleotide sequence" and "nucleic acid fragment" are used interchangeably to denote a polymer of RNA and/or DNA that is single- or double-stranded, optionally containing synthetic, non-natural, or altered nucleotide bases. Nucleotides (usually found in their 5'-monophosphate form) are referred to by their single letter designation as follows: "A" for adenosine or deoxyadenosine (for RNA or DNA, respectively), "C" for cytosine or deoxycytosine, "G" for guanosine or deoxyguanosine, "U" for uridine, "T" for deoxythymidine, "R" for purines (A or G), "Y" for pyrimidines (C or T), "K" for G or T, "H" for A or C or T, "I" for inosine, and "N" for any nucleotide.

"Open reading frame" is abbreviated ORF.

The terms "subfragment that is functionally equivalent" and "functionally equivalent subfragment" are used interchangeably herein. These terms refer to a portion or subsequence of an isolated nucleic acid fragment in which the ability to alter gene expression or produce a certain phenotype is retained whether or not the fragment or subfragment encodes an active enzyme. For example, the fragment or subfragment can be used in the design of genes to produce the desired phenotype in a transformed plant. Genes can be designed for use in suppression by linking a nucleic acid fragment or subfragment thereof, whether or not it encodes an active enzyme, in the sense or antisense orientation relative to a plant promoter sequence.

The term "conserved domain" or "motif" means a set of amino acids conserved at specific positions along an aligned sequence of evolutionarily related proteins. While amino acids at other positions can vary between homologous proteins, amino acids that are highly conserved at specific positions indicate amino acids that are essential to the structure, the stability, or the activity of a protein. Because they are identified by their high degree of conservation in aligned sequences of a family of protein homologues, they can be used as identifiers, or "signatures", to determine if a protein with a newly determined sequence belongs to a previously identified protein family.

Polynucleotide and polypeptide sequences, variants thereof, and the structural relationships of these sequences can be described by the terms "homology", "homologous", "substantially identical", "substantially similar" and "corresponding substantially" which are used interchangeably herein. These refer to polypeptide or nucleic acid fragments wherein changes in one or more amino acids or nucleotide bases do not affect the function of the molecule, such as the ability to mediate gene expression or to produce a certain phenotype. These terms also refer to modification(s) of nucleic acid fragments that do not substantially alter the functional properties of the resulting nucleic acid fragment relative to the initial, unmodified fragment. These modifications include deletion, substitution, and/or insertion of one or more nucleotides in the nucleic acid fragment.

Substantially similar nucleic acid sequences encompassed may be defined by their ability to hybridize (under moderately stringent conditions, e.g., 0.5X SSC, 0.1% SDS, 60°C) with the sequences exemplified herein, or to any portion of the nucleotide sequences disclosed herein and which are functionally equivalent to any of the nucleic acid sequences disclosed herein. Stringency conditions can be adjusted to screen for moderately similar fragments, such as homologous sequences from distantly related organisms, to highly similar fragments, such as genes that duplicate functional enzymes from closely related organisms. Post-hybridization washes determine stringency conditions.

The term "selectively hybridizes" includes reference to hybridization, under stringent hybridization conditions, of a nucleic acid sequence to a specified nucleic acid target sequence to a detectably greater degree (e.g., at least 2-fold over background) than its hybridization to non-target nucleic acid sequences and to the substantial exclusion of non-target nucleic acids. Selectively hybridizing sequences typically have about at least 80% sequence identity, or 90% sequence identity, up to and including 100% sequence identity (i.e., fully complementary) with each other.

The term "stringent conditions" or "stringent hybridization conditions" includes reference to conditions under which a probe will selectively hybridize to its target sequence in an *in vitro* hybridization assay. Stringent conditions are sequence-dependent and will be different in different circumstances. By controlling the stringency of the hybridization and/or washing conditions, target sequences can be identified which are 100% complementary to the probe (homologous probing). Alternatively, stringency conditions can be adjusted to allow some mismatching in sequences so that lower degrees of similarity are detected (heterologous probing). Generally, a probe is less than about 1000 nucleotides in length, optionally less than 500 nucleotides in length.

Typically, stringent conditions will be those in which the salt concentration is less than about 1.5 M Na ion, typically about 0.01 to 1.0 M Na ion concentration (or other salt(s)) at pH 7.0 to 8.3, and at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1 X SSC at 60 to 65°C.

"Sequence identity" or "identity" in the context of nucleic acid or polypeptide sequences refers to the nucleic acid bases or amino acid residues in two sequences that are the same when aligned for maximum correspondence over a specified comparison window.

The term "percentage of sequence identity" refers to the value determined by comparing two optimally aligned sequences over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the results by 100 to yield the percentage of sequence identity. Useful examples of percent sequence identities include, but are not limited to, 50%, 55%, 60%, 65%, 70%, 75%, 80%, 85%, 90% or 95%, or any integer percentage from 50% to 100%. These identities can be determined using any of the programs described herein.

Sequence alignments and percent identity or similarity calculations may be determined using a variety of comparison methods designed to detect homologous sequences including, but not limited to, the MegAlign^{™} program of the LASERGENE bioinformatics computing suite (DNASTAR Inc., Madison, WI). Within the context of this application it will be understood that where sequence analysis software is used for analysis, that the results of the analysis will be based on the "default values" of the program referenced, unless otherwise specified. As used herein "default values" will mean any set of values or parameters that originally load with the software when first initialized.

The "Clustal V method of alignment" corresponds to the alignment method labeled Clustal V (described by Higgins and Sharp, (1989) CABIOS 5:151-153; Higgins et al., (1992) Comput Appl Biosci 8:189-191) and found in the MegAlign^{™} program of the LASERGENE bioinformatics computing suite (DNASTAR Inc., Madison, WI). For multiple alignments, the default values correspond to GAP PENALTY=10 and GAP LENGTH PENALTY=10. Default parameters for pairwise alignments and calculation of percent identity of protein sequences using the Clustal method are KTUPLE=1, GAP PENALTY=3, WINDOW=5 and DIAGONALS SAVED=5. For nucleic acids these parameters are KTUPLE=2, GAP PENALTY=5, WINDOW=4 and DIAGONALS SAVED=4. After alignment of the sequences using the Clustal V program, it is possible to obtain a "percent identity" by viewing the "sequence distances" table in the same program.

The "Clustal W method of alignment" corresponds to the alignment method labeled Clustal W (described by Higgins and Sharp, (1989) CABIOS 5:151-153; Higgins et al., (1992) Comput Appl Biosci 8:189-191) and found in the MegAlign^{™} v6.1 program of the LASERGENE bioinformatics computing suite (DNASTAR Inc., Madison, WI). Default parameters for multiple alignment (GAP PENALTY=10, GAP LENGTH PENALTY=0.2, Delay Divergen Seqs (%)=30, DNA Transition Weight=0.5, Protein Weight Matrix=Gonnet Series, DNA Weight Matrix=IUB). After alignment of the sequences using the Clustal W program, it is possible to obtain a "percent identity" by viewing the "sequence distances" table in the same program.

Unless otherwise stated, sequence identity/similarity values provided herein refer to the value obtained using GAP Version 10 (GCG, Accelrys, San Diego, CA) using the following parameters: % identity and % similarity for a nucleotide sequence using a gap creation penalty weight of 50 and a gap length extension penalty weight of 3, and the nwsgapdna.cmp scoring matrix; % identity and % similarity for an amino acid sequence using a GAP creation penalty weight of 8 and a gap length extension penalty of 2, and the BLOSUM62 scoring matrix (Henikoff and Henikoff, (1989) Proc. Natl. Acad. Sci. USA 89:10915). GAP uses the algorithm of Needleman and Wunsch, (1970) J Mol Biol 48:443-53, to find an alignment of two complete sequences that maximizes the number of matches and minimizes the number of gaps. GAP considers all possible alignments and gap positions and creates the alignment with the largest number of matched bases and the fewest gaps, using a gap creation penalty and a gap extension penalty in units of matched bases.

"BLAST" is a searching algorithm provided by the National Center for Biotechnology Information (NCBI) used to find regions of similarity between biological sequences. The program compares nucleotide or protein sequences to sequence databases and calculates the statistical significance of matches to identify sequences having sufficient similarity to a query sequence such that the similarity would not be predicted to have occurred randomly. BLAST reports the identified sequences and their local alignment to the query sequence.

It is well understood by one skilled in the art that many levels of sequence identity are useful in identifying polypeptides from other species or modified naturally or synthetically wherein such polypeptides have the same or similar function or activity. Useful examples of percent identities include, but are not limited to, 50%, 55%, 60%, 65%, 70%, 75%, 80%, 85%, 90% or 95%, or any integer percentage from 50% to 100%. Indeed, any integer amino acid identity from 50% to 100% may be useful in describing the present disclosure, such as 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%.

"Gene" includes a nucleic acid fragment that expresses a functional molecule such as, but not limited to, a specific protein, including regulatory sequences preceding (5' non-coding sequences) and following (3' non-coding sequences) the coding sequence. "Native gene" refers to a gene as found in nature with its own regulatory sequences.

A "mutated gene" is a gene that has been altered through human intervention. Such a "mutated gene" has a sequence that differs from the sequence of the corresponding non-mutated gene by at least one nucleotide addition, deletion, or substitution. In certain aspects of the disclosure, the mutated gene comprises an alteration that results from a guide polynucleotide/Cas endonuclease system as disclosed herein. A mutated plant is a plant comprising a mutated gene.

As used herein, a "targeted mutation" is a mutation in a native gene that was made by altering a target sequence within the native gene using a method involving a double-strand-break-inducing agent that is capable of inducing a double-strand break in the DNA of the target sequence as disclosed herein or known in the art.

The guide RNA/Cas endonuclease induced targeted mutation can occur in a nucleotide sequence that is located within or outside a genomic target site that is recognized and cleaved by a Cas endonuclease.

The term "genome" as it applies to a plant cells encompasses not only chromosomal DNA found within the nucleus, but organelle DNA found within subcellular components (e.g., mitochondria, or plastid) of the cell.

A "codon-modified gene" or "codon-preferred gene" or "codon-optimized gene" is a gene having its frequency of codon usage designed to mimic the frequency of preferred codon usage of the host cell.

An "allele" is one of several alternative forms of a gene occupying a given locus on a chromosome. When all the alleles present at a given locus on a chromosome are the same, that plant is homozygous at that locus. If the alleles present at a given locus on a chromosome differ, that plant is heterozygous at that locus.

"Coding sequence" refers to a polynucleotide sequence which codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to: promoters, translation leader sequences, 5' untranslated sequences, 3' untranslated sequences, introns, polyadenylation target sequences, RNA processing sites, effector binding sites, and stem-loop structures.

"A plant-optimized nucleotide sequence" is nucleotide sequence that has been optimized for increased expression in plants. For example, a plant-optimized nucleotide sequence can be synthesized by modifying a nucleotide sequence encoding a protein such as, for example, double-strand-break-inducing agent (*e.g*., an endonuclease) as disclosed herein, using one or more plant-preferred codons for improved expression. *See*, for example, Campbell and Gowri (1990) Plant Physiol. 92:1-11 for a discussion of host-preferred codon usage.

Methods are available in the art for synthesizing plant-preferred genes. See, for example, U.S. Patent Nos. 5,380,831, and 5,436,391, and Murray et al. (1989) Nucleic Acids Res. 17:477-498. Additional sequence modifications are known to enhance gene expression in a plant host. These include, for example, elimination of: one or more sequences encoding spurious polyadenylation signals, one or more exon-intron splice site signals, one or more transposon-like repeats, and other such well-characterized sequences that may be deleterious to gene expression. The G-C content of the sequence may be adjusted to levels average for a given plant host, as calculated by reference to known genes expressed in the host plant cell. When possible, the sequence is modified to avoid one or more predicted hairpin secondary mRNA structures. Thus, "a plant-optimized nucleotide sequence" of the present disclosure comprises one or more of such sequence modifications.

A promoter is a region of DNA involved in recognition and binding of RNA polymerase and other proteins to initiate transcription. The promoter sequence consists of proximal and more distal upstream elements, the latter elements often referred to as enhancers. An "enhancer" is a DNA sequence that can stimulate promoter activity, and may be an innate element of the promoter or a heterologous element inserted to enhance the level or tissue-specificity of a promoter. Promoters may be derived in their entirety from a native gene, or be composed of different elements derived from different promoters found in nature, and/or comprise synthetic DNA segments. It is understood by those skilled in the art that different promoters may direct the expression of a gene in different tissues or cell types, or at different stages of development, or in response to different environmental conditions. It is further recognized that since in most cases the exact boundaries of regulatory sequences have not been completely defined, DNA fragments of some variation may have identical promoter activity. Promoters that cause a gene to be expressed in most cell types at most times are commonly referred to as "constitutive promoters".

It has been shown that certain promoters are able to direct RNA synthesis at a higher rate than others. These are called "strong promoters". Certain other promoters have been shown to direct RNA synthesis at higher levels only in particular types of cells or tissues and are often referred to as "tissue specific promoters", or "tissue-preferred promoters" if the promoters direct RNA synthesis preferably in certain tissues but also in other tissues at reduced levels. Since patterns of expression of a chimeric gene (or genes) introduced into a plant are controlled using promoters, there is an ongoing interest in the isolation of novel promoters which are capable of controlling the expression of a chimeric gene or (genes) at certain levels in specific tissue types or at specific plant developmental stages.

A plant promoter can include a promoter capable of initiating transcription in a plant cell, for a review of plant promoters, see, Potenza et al., (2004) In Vitro Cell Dev Biol 40:1-22. Constitutive promoters include, for example, the core promoter of the Rsyn7 promoter and other constitutive promoters disclosed in WO99/43838 and U.S. Patent No. 6,072,050; the core CaMV 35S promoter (Odell et al., (1985) Nature 313:810-2); rice actin (McElroy et al., (1990) Plant Cell 2:163-71); ubiquitin (Christensen et al., (1989) Plant Mol Biol 12:619-32; Christensen et al., (1992) Plant Mol Biol 18:675-89); pEMU (Last et al., (1991) Theor Appl Genet 81:581-8); MAS (Velten et al., (1984) EMBO J 3:2723-30); and ALS promoter (U.S. Patent No. 5,659,026). Other constitutive promoters are described in, for example, U.S. Patent Nos. 5,608,149; 5,608,144; 5,604,121; 5,569,597; 5,466,785; 5,399,680; 5,268,463; 5,608,142 and 6,177,611. In some examples an inducible promoter may be used. Pathogen-inducible promoters induced following infection by a pathogen include, but are not limited to those regulating expression of PR proteins, SAR proteins, beta-1,3-glucanase, chitinase, *etc.*

Chemical-regulated promoters can be used to modulate the expression of a gene in a plant through the application of an exogenous chemical regulator. The promoter may be a chemical-inducible promoter, where application of the chemical induces gene expression, or a chemical-repressible promoter, where application of the chemical represses gene expression. Chemical-inducible promoters include, but are not limited to, the maize In2-2 promoter, activated by benzene sulfonamide herbicide safeners (De Veylder et al., (1997) Plant Cell Physiol 38:568-77), the maize GST promoter (GST-II-27, WO93/01294), activated by hydrophobic electrophilic compounds used as pre-emergent herbicides, and the tobacco PR-1a promoter (Ono et al., (2004) Biosci Biotechnol Biochem 68:803-7) activated by salicylic acid. Other chemical-regulated promoters include steroid-responsive promoters (see, for example, the glucocorticoid-inducible promoter (Schena et al., (1991) Proc. Natl. Acad. Sci. USA 88:10421-5; McNellis et al., (1998) Plant J 14:247-257); tetracycline-inducible and tetracycline-repressible promoters (Gatz et al., (1991) Mol Gen Genet 227:229-37; U.S. Patent Nos. 5,814,618 and 5,789,156).

Tissue-preferred promoters can be utilized to target enhanced expression within a particular plant tissue. Tissue-preferred promoters include, for example, Kawamata et al., (1997) Plant Cell Physiol 38:792-803; Hansen et al., (1997) Mol Gen Genet 254:337-43; Russell et al., (1997) Transgenic Res 6:157-68; Rinehart et al., (1996) Plant Physiol 112:1331-41; Van Camp et al., (1996) Plant Physiol 112:525-35; Canevascini et al., (1996) Plant Physiol 112:513-524; Lam, (1994) Results Probl Cell Differ 20:181-96; and Guevara-Garcia et al., (1993) Plant J 4:495-505. Leaf-preferred promoters include, for example, Yamamoto et al., (1997) Plant J 12:255-65; Kwon et al., (1994) Plant Physiol 105:357-67; Yamamoto et al., (1994) Plant Cell Physiol 35:773-8; Gotor et al., (1993) Plant J 3:509-18; Orozco et al., (1993) Plant Mol Biol 23:1129-38; Matsuoka et al., (1993) Proc. Natl. Acad. Sci. USA 90:9586-90; Simpson et al., (1958) EMBO J 4:2723-9; Timko et al., (1988) Nature 318:57-8. Root-preferred promoters include, for example, Hire et al., (1992) Plant Mol Biol 20:207-18 (soybean root-specific glutamine synthase gene); Miao et al., (1991) Plant Cell 3:11-22 (cytosolic glutamine synthase (GS)); Keller and Baumgartner, (1991) Plant Cell 3:1051-61 (root-specific control element in the GRP 1.8 gene of French bean); Sanger et al., (1990) Plant Mol Biol 14:433-43 (root-specific promoter of *A. tumefaciens* mannopine synthase (MAS)); Bogusz et al., (1990) Plant Cell 2:633-41 (root-specific promoters isolated from *Parasponia andersonii* and *Trema tomentosa*); Leach and Aoyagi, (1991) Plant Sci 79:69-76 (A. *rhizogenes* rolC and rolD root-inducing genes); Teeri et al., (1989) EMBO J 8:343-50 (*Agrobacterium* wound-induced TR1' and TR2' genes); VfENOD-GRP3 gene promoter (Kuster et al., (1995) Plant Mol Biol 29:759-72); and rolB promoter (Capana et al., (1994) Plant Mol Biol 25:681-91 ; phaseolin gene (Murai et al., (1983) Science 23:476-82; Sengopta-Gopalen et al., (1988) Proc. Natl. Acad. Sci. USA 82:3320-4). See also, U.S. Patent Nos. 5,837,876; 5,750,386; 5,633,363; 5,459,252; 5,401,836; 5,110,732 and 5,023,179.

Seed-preferred promoters include both seed-specific promoters active during seed development, as well as seed-germinating promoters active during seed germination. See, Thompson et al., (1989) BioEssays 10:108. Seed-preferred promoters include, but are not limited to, Cim1 (cytokinin-induced message); cZ19B1 (maize 19 kDa zein); and milps (myo-inositol-1-phosphate synthase); (WO00/11177; and U.S. Patent 6,225,529). For dicots, seed-preferred promoters include, but are not limited to, bean β-phaseolin, napin, β-conglycinin, soybean lectin, and cruciferin. For monocots, seed-preferred promoters include, but are not limited to, maize 15 kDa zein, 22 kDa zein, 27 kDa gamma zein, waxy, shrunken 1, shrunken 2, globulin 1, oleosin, and nuc1. See also, WO00/12733, where seed-preferred promoters from *END1* and *END2* genes are disclosed.

The term "inducible promoter" refers to promoters that selectively express a coding sequence or functional RNA in response to the presence of an endogenous or exogenous stimulus, for example by chemical compounds (chemical inducers) or in response to environmental, hormonal, chemical, and/or developmental signals. Inducible or regulated promoters include, for example, promoters induced or regulated by light, heat, stress, flooding or drought, salt stress, osmotic stress, phytohormones, wounding, or chemicals such as ethanol, abscisic acid (ABA), jasmonate, salicylic acid, or safeners.

An example of a stress-inducible is RD29A promoter (Kasuga et al. (1999) Nature Biotechnol. 17:287-91). One of ordinary skill in the art is familiar with protocols for simulating drought conditions and for evaluating drought tolerance of plants that have been subjected to simulated or naturally-occurring drought conditions. For example, one can simulate drought conditions by giving plants less water than normally required or no water over a period of time, and one can evaluate drought tolerance by looking for differences in physiological and/or physical condition, including (but not limited to) vigor, growth, size, or root length, or in particular, leaf color or leaf area size. Other techniques for evaluating drought tolerance include measuring chlorophyll fluorescence, photosynthetic rates and gas exchange rates. Also, one of ordinary skill in the art is familiar with protocols for simulating stress conditions such as osmotic stress, salt stress and temperature stress and for evaluating stress tolerance of plants that have been subjected to simulated or naturally-occurring stress conditions.

Another example of an inducible promoter useful in plant cells has been described in US patent application, US 2013-0312137A1, published on November 21, 2013. US patent application US 2013-0312137A1 describes a ZmCAS1 promoter from a CBSU-Anther_Subtraction library (CAS1) gene encoding a mannitol dehydrogenase from maize, and functional fragments thereof. The ZmCAS1 promoter (also refered to as "CAS1 promoter", "mannitol dehydrogenase promoter " , "mdh promoter") can be induced by a chemical or stress treatment. The chemical can be a safener such as, but not limited to, N-(aminocarbonyl)-2-chlorobenzenesulfonamide (2-CBSU). The stress treatment can be a heat treatment such as, but not limited to, a heat shock treatment (see also US provisional patent application, 62/120421, filed on February 25, 2015.

New promoters of various types useful in plant cells are constantly being discovered; numerous examples may be found in the compilation by Okamuro and Goldberg, (1989) In The Biochemistry of Plants, Vol. 115, Stumpf and Conn, eds (New York, NY: Academic Press), pp. 1-82.

"Translation leader sequence" refers to a polynucleotide sequence located between the promoter sequence of a gene and the coding sequence. The translation leader sequence is present in the mRNA upstream of the translation start sequence. The translation leader sequence may affect processing of the primary transcript to mRNA, mRNA stability or translation efficiency. Examples of translation leader sequences have been described (e.g., Turner and Foster, (1995) Mol Biotechnol 3:225-236).

"3' non-coding sequences", "transcription terminator" or "termination sequences" refer to DNA sequences located downstream of a coding sequence and include polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht et al., (1989) Plant Cell 1:671-680.

"RNA transcript" refers to the product resulting from RNA polymerase-catalyzed transcription of a DNA sequence. When the RNA transcript is a perfect complimentary copy of the DNA sequence, it is referred to as the primary transcript or pre-mRNA. A RNA transcript is referred to as the mature RNA or mRNA when it is a RNA sequence derived from post-transcriptional processing of the primary transcript pre mRNA. "Messenger RNA" or "mRNA" refers to the RNA that is without introns and that can be translated into protein by the cell. "crDNA" refers to a DNA that is complementary to, and synthesized from, an mRNA template using the enzyme reverse transcriptase. The cDNA can be single-stranded or converted into double-stranded form using the Klenow fragment of DNA polymerase I. "Sense" RNA refers to RNA transcript that includes the mRNA and can be translated into protein within a cell or *in vitro.* "Antisense RNA" refers to an RNA transcript that is complementary to all or part of a target primary transcript or mRNA, and that blocks the expression of a target gene (see, e.g., U.S. Patent No. 5,107,065). The complementarity of an antisense RNA may be with any part of the specific gene transcript, i.e., at the 5' non-coding sequence, 3' non-coding sequence, introns, or the coding sequence. "Functional RNA" refers to antisense RNA, ribozyme RNA, or other RNA that may not be translated but yet has an effect on cellular processes. The terms "complement" and "reverse complement" are used interchangeably herein with respect to mRNA transcripts, and are meant to define the antisense RNA of the message.

The term "operably linked" refers to the association of nucleic acid sequences on a single nucleic acid fragment so that the function of one is regulated by the other. For example, a promoter is operably linked with a coding sequence when it is capable of regulating the expression of that coding sequence (i.e., the coding sequence is under the transcriptional control of the promoter). Coding sequences can be operably linked to regulatory sequences in a sense or antisense orientation. In another example, the complementary RNA regions can be operably linked, either directly or indirectly, 5' to the target mRNA, or 3' to the target mRNA, or within the target mRNA, or a first complementary region is 5' and its complement is 3' to the target mRNA.

Standard recombinant DNA and molecular cloning techniques used herein are well known in the art and are described more fully in Sambrook et al., Molecular Cloning: A Laboratory Manual; Cold Spring Harbor Laboratory: Cold Spring Harbor, NY (1989). Transformation methods are well known to those skilled in the art and are described *infra.*

"PCR" or "polymerase chain reaction" is a technique for the synthesis of specific DNA segments and consists of a series of repetitive denaturation, annealing, and extension cycles. Typically, a double-stranded DNA is heat denatured, and two primers complementary to the 3' boundaries of the target segment are annealed to the DNA at low temperature, and then extended at an intermediate temperature. One set of these three consecutive steps is referred to as a "cycle".

The term "recombinant" refers to an artificial combination of two otherwise separated segments of sequence, e.g., by chemical synthesis, or manipulation of isolated segments of nucleic acids by genetic engineering techniques.

The terms "plasmid", "vector" and "cassette" refer to an extra chromosomal element often carrying genes that are not part of the central metabolism of the cell, and usually in the form of double-stranded DNA. Such elements may be autonomously replicating sequences, genome integrating sequences, phage, or nucleotide sequences, in linear or circular form, of a single- or double-stranded DNA or RNA, derived from any source, in which a number of nucleotide sequences have been joined or recombined into a unique construction which is capable of introducing a polynucleotide of interest into a cell. "Transformation cassette" refers to a specific vector containing a gene and having elements in addition to the gene that facilitates transformation of a particular host cell. "Expression cassette" refers to a specific vector containing a gene and having elements in addition to the gene that allow for expression of that gene in a host.

The terms "recombinant DNA molecule", "recombinant construct", "expression construct", " construct", "construct", and "recombinant DNA construct" are used interchangeably herein. A recombinant construct comprises an artificial combination of nucleic acid fragments, e.g., regulatory and coding sequences that are not all found together in nature. For example, a construct may comprise regulatory sequences and coding sequences that are derived from different sources, or regulatory sequences and coding sequences derived from the same source, but arranged in a manner different than that found in nature. Such a construct may be used by itself or may be used in conjunction with a vector. If a vector is used, then the choice of vector is dependent upon the method that will be used to transform host cells as is well known to those skilled in the art. For example, a plasmid vector can be used. The skilled artisan is well aware of the genetic elements that must be present on the vector in order to successfully transform, select and propagate host cells. The skilled artisan will also recognize that different independent transformation events may result in different levels and patterns of expression (Jones et al., (1985) EMBO J 4:2411-2418; De Almeida et al., (1989) Mol Gen Genetics 218:78-86), and thus that multiple events are typically screened in order to obtain lines displaying the desired expression level and pattern. Such screening may be accomplished standard molecular biological, biochemical, and other assays including Southern analysis of DNA, Northern analysis of mRNA expression, PCR, real time quantitative PCR (qPCR), reverse transcription PCR (RT-PCR), immunoblotting analysis of protein expression, enzyme or activity assays, and/or phenotypic analysis.

The term "expression", as used herein, refers to the production of a functional end-product (e.g., an mRNA, guide RNA, or a protein) in either precursor or mature form.

The term "introducing" includes reference to introducing, providing, contacting a compound, such as but not limited to, a nucleic acid (e.g., expression construct) or peptide, polypeptide or protein into a cell. Introducing includes the direct delivery of polynucleotides (such as RNA, DNA, RNA-DNA hibrids, single or double stranded oligonucleotides, linear or circular polynucleotides) and/or includes the direct delivery of proteins (polypeptides). Introducing includes reference to the incorporation of a nucleic acid or polypeptide into a eukaryotic or prokaryotic cell where the nucleic acid may be incorporated into the genome of the cell, and includes reference to the transient introduction of a nucleic acid or protein into the cell. Introducing includes reference to stable or transient transformation methods, transfection, transduction, microinjection, electroporation, viral methods, Agrobacterium-mediated transformation, ballistic particle acceleration, whiskers mediated transformation, as well as sexually crossing. Thus, "introducing" in the context of inserting a nucleic acid fragment (e.g., a recombinant DNA construct/expression construct, guide RNA, guide DNA, template DNA, donor DNA) into a cell, includes "transfection" or "transformation" or "transduction" and includes reference to the incorporation of a nucleic acid fragment into a eukaryotic or prokaryotic cell where the nucleic acid fragment may be incorporated into the genome of the cell (e.g., chromosome, plasmid, plastid, or mitochondrial DNA), converted into an autonomous replicon, or transiently expressed (e.g., transfected mRNA).

A variety of methods are known for introducing, contacting and/or providing a composition into an organisms including stable transformation methods, transient transformation methods, virus-mediated methods, sexual crossing and sexual breeding. Stable transformation indicates that the introduced polynucleotide integrates into the genome of the organism and is capable of being inherited by progeny thereof. Transient transformation indicates that the introduced composition is only temporarily expressed or present in the organism.

Protocols for contacting, providing, introducing polynucleotides and polypeptides into cells or organisms are known and include microinjection (Crossway et al., (1986) Biotechniques 4:320-34 and U.S. Patent No. 6,300,543), meristem transformation (U.S. Patent No. 5,736,369), electroporation (Riggs et al., (1986) Proc. Natl. Acad. Sci. USA 83:5602-6, Agrobacterium-mediated transformation (U.S. Patent Nos. 5,563,055 and 5,981,840), whiskers mediated transformation (Ainley et al. 2013, Plant Biotechnology Journal 11:1126-1134; Shaheen A. and M. Arshad 2011 Properties and Applications of Silicon Carbide (2011), 345-358 Editor(s): Gerhardt, Rosario. Publisher: InTech, Rijeka, Croatia. CODEN: 69PQBP; ISBN: 978-953-307-201-2) direct gene transfer (Paszkowski et al., (1984) EMBO J 3:2717-22), and ballistic particle acceleration (U.S. Patent Nos. 4,945,050; 5,879,918; 5,886,244; 5,932,782; Tomes et al., (1995) "Direct DNA Transfer into Intact Plant Cells via Microprojectile Bombardment" in Plant Cell, Tissue, and Organ Culture: Fundamental Methods, ed. Gamborg & Phillips (Springer-Verlag, Berlin); McCabe et al., (1988) Biotechnology 6:923-6; Weissinger et al., (1988) Ann Rev Genet 22:421-77; Sanford et al., (1987) Particulate Science and Technology 5:27-37 (onion); Christou et al., (1988) Plant Physiol 87:671-4 (soybean); Finer and McMullen, (1991) In Vitro Cell Dev Biol 27P:175-82 (soybean); Singh et al., (1998) Theor Appl Genet 96:319-24 (soybean); Datta et al., (1990) Biotechnology 8:736-40 (rice); Klein et al., (1988) Proc. Natl. Acad. Sci. USA 85:4305-9 (maize); Klein et al., (1988) Biotechnology 6:559-63 (maize); U.S. Patent Nos. 5,240,855; 5,322,783 and 5,324,646; Klein et al., (1988) Plant Physiol 91:440-4 (maize); Fromm et al., (1990) Biotechnology 8:833-9 (maize); Hooykaas-Van Slogteren et al., (1984) Nature 311:763-4; U.S. Patent No. 5,736,369 (cereals); Bytebier et al., (1987) Proc. Natl. Acad. Sci. USA 84:5345-9 (*Liliaceae*); De Wet et al., (1985) in The Experimental Manipulation of Ovule Tissues, ed. Chapman et al., (Longman, New York), pp. 197-209 (pollen); Kaeppler et al., (1990) Plant Cell Rep 9:415-8) and Kaeppler et al., (1992) Theor Appl Genet 84:560-6 (whisker-mediated transformation); D'Halluin et al., (1992) Plant Cell 4:1495-505 (electroporation); Li et al., (1993) Plant Cell Rep 12:250-5; Christou and Ford (1995) Annals Botany 75:407-13 (rice) and Osjoda et al., (1996) Nat Biotechnol 14:745-50 (maize via *Agrobacterium tumefaciens).*

Alternatively, polynucleotides may be introduced into cells or organisms by contacting cells or organisms with a virus or viral nucleic acids. Generally, such methods involve incorporating a polynucleotide within a viral DNA or RNA molecule. In some examples a polypeptide of interest may be initially synthesized as part of a viral polyprotein, which is later processed by proteolysis *in vivo* or *in vitro* to produce the desired recombinant protein. Methods for introducing polynucleotides into plants and expressing a protein encoded therein, involving viral DNA or RNA molecules, are known, see, for example, U.S. Patent Nos. 5,889,191, 5,889,190, 5,866,785, 5,589,367 and 5,316,931. Transient transformation methods include, but are not limited to, the introduction of polypeptides, such as a double-strand break inducing agent, directly into the organism, the introduction of polynucleotides such as DNA and/or RNA polynucleotides, and the introduction of the RNA transcript, such as an mRNA encoding a double-strand break inducing agent, into the organism. Such methods include, for example, microinjection or particle bombardment. See, for example Crossway et al., (1986) Mol Gen Genet 202:179-85; Nomura et al., (1986) Plant Sci 44:53-8; Hepler et al., (1994) Proc. Natl. Acad. Sci. USA 91:2176-80; and, Hush et al., (1994) J Cell Sci 107:775-84.

Nucleic acids and proteins can be provided to a cell by any method including methods using molecules to facilitate the uptake of anyone or all components of a guided Cas system (protein and/or nucleic acids), such as cell-penetrating peptides and nanocariers. See also US20110035836 Nanocarier based plant transfection and transduction, and EP 2821486 A1 Method of introducing nucleic acid into plant cells.

Introducing a guide RNA/Cas endonuclease complex into a cell includes introducing the individual components of said complex either separately or combined into the cell, and either directly (direct delivery as RNA for the guide and protein for the Cas endonuclease) or via recombination constructs expressing the components (guide RNA, Cas endonuclease). Introducing a guide RNA/Cas endonuclease complex into a cell includes introducing the guide RNA/Cas endonuclease complex as a ribonucleotide-protein into the cell. The ribonucleotide-protein can be assembled prior to being introduced into the cell as described herein.

Plant cells differ from human and animal cells in that plant cells contain a plant cell wall which may act as a barrier to the direct delivery of the RGEN ribonucleoproteins and/or of the direct delivery of the RGEN components.

As described herein, direct delivery of the RGEN ribonucleoproteins into plant cells can be achieved through particle mediated delivery (particle bombardment. Based on the experiments described herein, a skilled artesian can now envision that any other direct method of delivery, such as but not limiting to, polyethylene glycol (PEG)-mediated transfection to protoplasts, electroporation, cell-penetrating peptides, or mesoporous silica nanoparticle (MSN)-mediated direct protein delivery, can be successfully used for delivering RGEN ribonucleoproteins into plant cells.

Direct delivery of the RGEN ribonucleoprotein, as described herein, allows for genome editing at a target site in the genome of a cell which can be followed by rapid degradation of the complex, and only a transient presence of the complex in the cell. This transient presence of the RGEN complex may lead to reduced off-target effects. In contrast, delivery of RGEN components (guide RNA, Cas9 endonuclease) via plasmid DNA sequences can result in constant expression of RGENs from these plasmids which can intensify off target effects (Cradick, T. J. et al (2013) Nucleic Acids Res 41:9584-9592; Fu, Y et al (2014) Nat. Biotechnol. 31:822-826.

Direct delivery can be achieved by combining any one component of the RNA guided endonuclease (guide RNA, Cas protein, mRNA encoding the gRNA or Cas endonuclease) or the RGEN complex itself, with a particle delivery matrix comprising a microparticle such as but not limited to of a gold particle, tungsten particle, and silicon carbide whisker particle. Examples of combination methods described herein for combining microparticles to plasmid DNA and DNA of interest can also be used for coating guide RNA molecules, mRNA molecules, Cas proteins and RGEN complexes to the microparticles.

These coated microparticles can be introduced into the cells by any direct method known in the art such as the particle bombardment method described in Example 8. Microparticles and RGEN components or RGEN complex can be combined (mixed) in any matter to allow for coating of the RGEN components to the mirco particles. For example, RGEN components can be precipitated onto gold pellets of a diameter ranging from at least 0.1 µm, 0.2 µm, 0.3 µm , 0.4 µm ,0.5 µm,0.6 µm ,0.7 µm ,0.8 µm ,0.9 µm or 1.0 µm in diameter using any suitable buffer (such as but not limiting to a water-soluble cationic lipid such as but not limiting to TransIT-2020 Transfection Reagent (Cat# MIR 5404, Mirus, USA). RGEN component solutions can prepared on ice (or at any temperature suitable to enable mircoparticle bounding) using at least 0.1 µg, 0.2 µg, 0.3 µg, 0.4 µg ,0.5 µg ,0.6 µg, 0.7 µg ,0.8 µg ,0.9 µg, 1.0 µg, 2.0 µg , 3.0 µg , 4.0 µg ,5.0 µg ,6.0 µg ,7.0 µg ,8.0 µg 9.0 µg or 10 µg of RNA (guided RNA or mRNA) or Cas endonuclease protein. To the pre-mixed RGEN components of RGEN complexes, at least 1 µl to 20 µl of prepared mircoparticles can be added and mixed carefully.

In one aspect of the disclosure, the method comprises a method of delivering a guide RNA /Cas endonuclease complex into a cell, the method comprising combining at least one guide RNA molecule and at least one Cas endonuclease protein to form a ribonucleotide-protein and combining said ribonucleotide-protein with a particle delivery matrix to allow for said ribonucleotide-protein and matrix to bind and form a ribonucleotide-protein - matrix complex; and, introducing said ribonucleotide-protein - matrix complex into said cell. The particle delivery matrix can comprise microparticles combined with a cationic lipid.

The term "cationic lipid" includes reference to a water soluble cationic lipid, such as but not limiting to TransIT-2020, or a cationic lipid solution such as but not limiting to a cationic lipid solution comprising N,N,N',N'-tetramethyl-N, N'-bis(2-hydroxylethyl)-2,3-di( oleoyloxy )-1 ,4-butanediammonium iodide, and L-dioleoyl phosphatidylethanolamine (DOPE).(see also US2007/0178593, published on August 2, 2007), 5. The method of claim.

The particle delivery matrix can comprise microparticles selected from the group consisting of gold particles, tungsten particles, and silicon carbide whisker particles.

The particle delivery matrix can further comprise a compound selected from the group consisting of Tfx-10^{™}, Tfx-20^{™}, Tfx-50^{™}, Lipofectin^{™}, Lipofectamine^{™}, Cellfectin^{™}, Effectene^{™}, Cytofectin GSV^{™}, Perfect Lipids^{™}, DOTAP^{™}, DMRIE-C^{™}, FuGENE-6^{™}, Superfect^{™}, Polyfeet^{™}, polyethyleneimine, chitosan, protamine Cl, histone H1, histone CENH3, poly-L lysine, and DMSA.(US2007/0178593, published on August 2, 2007)

RGEN components can also be combined prior to be coated on microparticles by combining least 0.1 µg, 0.2 µg, 0.3 µg, 0.4 µg ,0.5 µg ,0.6 µg ,0.7 µg ,0.8 µg ,0.9 µg, 1.0 µg, 2.0 µg , 3.0 µg , 4.0 µg ,5.0 µg ,6.0 µg ,7.0 µg ,8.0 µg 9.0 µg or 10 µg of guide RNA with at least 0.1 µg, 0.2 µg, 0.3 µg, 0.4 µg ,0.5 µg ,0.6 µg ,0.7 µg ,0.8 µg ,0.9 µg, 1.0 µg, 2.0 µg, 3.0 µg , 4.0 µg ,5.0 µg ,6.0 µg ,7.0 µg ,8.0 µg 9.0 µg or 10 µg of Cas endonuclease in a solution suitable to allow for complex formation (such as but not limiting to a Cas9 buffer (NEB)), at any temperature to allow for complex formation such as a temperature ranging from 1°C, 2°C , 3°C, 4°C ,5°C, 6°C ,7°C ,8°C, 9°C ,10°C, 11°C, 12°C , 13°C , 14°C, 15°C, 16°C, 17°C ,18°C, 19°C, 20°C, 21°C, 22°C, 23.0°C, 24°C, 25°C, 26°C, 27°C, 28°C, 29°C, 30°C, 31°C, 32°C, 33.0°C, 34°C, 35°C, 36°C, 37°C, 38°C, 39°C and 40°C.

In one aspect of the disclosure, the method comprises a method of delivering guide RNA/Cas endonuclease components into a cell, the method comprising introducing at least one guide RNA molecule and at least one Cas endonuclease protein into a cell, and growing said cell under suitable conditions to allow said guide RNA and said Cas endonuclease protein to form a complex inside said cell.

In one aspect of the disclosure, the method comprises a method of delivering guide RNA/Cas endonuclease components into a cell, the method comprising introducing at least one guide RNA molecule and at least one mRNA encoding a Cas endonuclease protein into a cell, and growing said cell under suitable conditions to allow said mRNA to translate said Cas endonuclease protein and form a complex with said guide RNA

In one aspect of the disclosure, the method comprises a method of delivering a guide RNA/Cas endonuclease complex into a cell, the method comprising combining at least one guide RNA molecule and at least one Cas endonuclease protein to form a ribonucleotide-protein and combining said ribonucleotide-protein with a particle delivery matrix to allow for said ribonucleotide-protein and matrix to bind and form a ribonucleotide-protein - matrix complex; and, introducing said ribonucleotide-protein - matrix complex together with at least one a polynucleotide template into said cell, wherein said polynucleotide modification template comprises at least one nucleotide modification of a nucleotide sequence in the genome of said cell, wherein said at least one nucleotide modification of said polynucleotide modification template is selected from the group consisting of (i) a replacement of at least one nucleotide, (ii) a deletion of at least one nucleotide, (iii) an insertion of at least one nucleotide, and (iv) any combination of (i) - (iii).

In one aspect of the disclosure, the method comprises a method of delivering a guide RNA/Cas endonuclease complex into a cell, the method comprising combining at least one guide RNA molecule and at least one Cas endonuclease protein to form a ribonucleotide-protein and combining said ribonucleotide-protein with a particle delivery matrix to allow for said ribonucleotide-protein and matrix to bind and form a ribonucleotide-protein - matrix complex; and, introducing said ribonucleotide-protein - matrix complex together with a donor DNA into said cell,, wherein said donor DNA comprises at least one polynucleotide of interest.

Suitable conditions for growing cells are well known in the art and the skilled artesian can use any growing condition based on the type of cell (such as conditions suitable for plant cells). As described in Example 8, plant embryos or cells can be incubated in any plant maintenance medium known in the art (such as, but not limiting to 560P, Example 8) for 12 to 48 hours at temperatures ranging from 26°C to 37°C, and then placed at 26°C. After 5 to 7 days the embryos/cells are transferred to any selection medium known in the art (such as, but not limiting to 560R, Example 8), and subcultured thereafter.

RGEN components (including guide RNA, Cas endonuclease protein) can be combined to form a ribonucleotide-protein complex (RNP) prior to be coated on (combined with) microparticles by combining least 0.1 µg, 0.2 µg, 0.3 µg, 0.4 µg, 0.5 µg ,0.6 µg ,0.7 µg ,0.8 µg ,0.9 µg, 1.0 µg, 2.0 µg, 3.0 µg, 4.0 µg, 5.0 µg, 6.0 µg, 7.0 µg, 8.0 µg, 9.0 µg or 10 µg of guide RNA with at least 0.1 µg, 0.2 µg, 0.3 µg, 0.4 µg, 0.5 µg, 0.6 µg, 0.7 µg, 0.8 µg, 0.9 µg, 1.0 µg, 2.0 µg, 3.0 µg, 4.0 µg, 5.0 µg, 6.0 µg, 7.0 µg, 8.0 µg, 9.0 µg or 10 µg of Cas endonuclease in a solution suitable to allow for complex formation (such as but not limiting to a Cas9 buffer (NEB)), at any temperature to allow for complex formation such as a temperature ranging from 1°C, 2°C , 3°C, 4°C ,5°C, 6°C,7°C,8°C, 9°C ,10°C, 11°C, 12°C, 13°C, 14°C, 15°C, 16°C, 17°C ,18°C, 19°C, 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C and 40°C.

"Mature" protein refers to a post-translationally processed polypeptide (i.e., one from which any pre- or propeptides present in the primary translation product have been removed). "Precursor" protein refers to the primary product of translation of mRNA (i.e., with pre- and propeptides still present). Pre- and propeptides may be but are not limited to intracellular localization signals.

"Stable transformation" refers to the transfer of a nucleic acid fragment into a genome of a host organism, including both nuclear and organellar genomes, resulting in genetically stable inheritance. In contrast, "transient transformation" refers to the transfer of a nucleic acid fragment into the nucleus, or other DNA-containing organelle, of a host organism resulting in gene expression without integration or stable inheritance. Host organisms containing the transformed nucleic acid fragments are referred to as "transgenic" organisms.

The commercial development of genetically improved germplasm has also advanced to the stage of introducing multiple traits into crop plants, often referred to as a gene stacking approach. In this approach, multiple genes conferring different characteristics of interest can be introduced into a plant. Gene stacking can be accomplished by many means including but not limited to co-transformation, retransformation, and crossing lines with different genes of interest.

Cells include, but are not limited to, human, non-human, animal, bacterial, fungal, insect, yeast, and plant cells as well as plants and seeds produced by the methods described herein. Plant cells include cells selected from the group consisting of maize, rice, sorghum, rye, barley, wheat, millet, oats, sugarcane, turfgrass, or switchgrass, soybean, canola, alfalfa, sunflower, cotton, tobacco, peanut, potato, tomato, tobacco, *Arabidopsis,* and safflower cells.

The term "plant" includes reference to whole plants, plant organs, plant tissues, seeds, and plant cells, and progeny of the same. Plant cells include, without limitation, cells from seeds, suspension cultures, embryos, meristematic regions, callus tissue, leaves, roots, shoots, gametophytes, sporophytes, pollen and microspores. Plant parts include differentiated and undifferentiated tissues including, but not limited to roots, stems, shoots, leaves, pollens, seeds, tumor tissue and various forms of cells and culture (e.g., single cells, protoplasts, embryos, and callus tissue). The plant tissue may be in plant or in a plant organ, tissue or cell culture. The term "plant organ" refers to plant tissue or a group of tissues that constitute a morphologically and functionally distinct part of a plant. The term "genome" refers to the entire complement of genetic material (genes and non-coding sequences) that is present in each cell of an organism, or virus or organelle; and/or a complete set of chromosomes inherited as a (haploid) unit from one parent. "Progeny" comprises any subsequent generation of a plant.

A transgenic plant includes, for example, a plant which comprises within its genome a heterologous polynucleotide introduced by a transformation step. The heterologous polynucleotide can be stably integrated within the genome such that the polynucleotide is passed on to successive generations. The heterologous polynucleotide may be integrated into the genome alone or as part of a recombinant DNA construct. A transgenic plant can also comprise more than one heterologous polynucleotide within its genome. Each heterologous polynucleotide may confer a different trait to the transgenic plant. A heterologous polynucleotide can include a sequence that originates from a foreign species, or, if from the same species, can be substantially modified from its native form. Transgenic can include any cell, cell line, callus, tissue, plant part or plant, the genotype of which has been altered by the presence of heterologous nucleic acid including those transgenics initially so altered as well as those created by sexual crosses or asexual propagation from the initial transgenic. The alterations of the genome (chromosomal or extra-chromosomal) by conventional plant breeding methods, by the genome editing procedure described herein that does not result in an insertion of a foreign polynucleotide, or by naturally occurring events such as random cross-fertilization, non-recombinant viral infection, non-recombinant bacterial transformation, non-recombinant transposition, or spontaneous mutation are not intended to be regarded as transgenic.

In certain aspects of the disclosure, a fertile plant is a plant that produces viable male and female gametes and is self-fertile. Such a self-fertile plant can produce a progeny plant without the contribution from any other plant of a gamete and the genetic material contained therein. Other aspects of the disclosure can involve the use of a plant that is not self-fertile because the plant does not produce male gametes, or female gametes, or both, that are viable or otherwise capable of fertilization. As used herein, a "male sterile plant" is a plant that does not produce male gametes that are viable or otherwise capable of fertilization. As used herein, a "female sterile plant" is a plant that does not produce female gametes that are viable or otherwise capable of fertilization. It is recognized that male-sterile and female-sterile plants can be female-fertile and male- fertile, respectively. It is further recognized that a male fertile (but female sterile) plant can produce viable progeny when crossed with a female fertile plant and that a female fertile (but male sterile) plant can produce viable progeny when crossed with a male fertile plant.

Non-conventional yeast herein refers to any yeast that is not a *Saccharomyces* (e.g., *S*. *cerevisiae*) or *Schizosaccharomyces* yeast species. Non-conventional yeast are described in Non-Conventional Yeasts in Genetics, Biochemistry and Biotechnology: Practical Protocols (K. Wolf, K.D. Breunig, G. Barth, Eds., Springer-Verlag, Berlin, Germany, 2003). Non-conventional yeast in certain aspects of the disclosure may additionally (or alternatively) be yeast that favor non-homologous end-joining (NHEJ) DNA repair processes over repair processes mediated by homologous recombination (HR). Definition of a non-conventional yeast along these lines - preference of NHEJ over HR - is further disclosed by Chen et al. (*PLoS ONE* 8:e57952). Preferred non-conventional yeast herein are those of the genus *Yarrowia* (e.g., *Yarrowia lipolytica*)*.* The term "yeast" herein refers to fungal species that predominantly exist in unicellular form. Yeast can alternative be referred to as "yeast cells" herein (see also US provisional application 62/036,652, filed on August 13, 2014).

A "centimorgan" (cM) or "map unit" is the distance between two linked genes, markers, target sites, loci, or any pair thereof, wherein 1% of the products of meiosis are recombinant. Thus, a centimorgan is equivalent to a distance equal to a 1% average recombination frequency between the two linked genes, markers, target sites, loci, or any pair thereof.

The present disclosure finds use in the breeding of plants comprising one or more introduced traits. Most commonly, transgenic traits are randomly inserted throughout the plant genome as a consequence of transformation systems based on *Agrobacterium,* biolistics, or other commonly used procedures. More recently, gene targeting protocols have been developed that enable directed transgene insertion. One important technology, site-specific integration (SSI) enables the targeting of a transgene to the same chromosomal location as a previously inserted transgene. Custom-designed meganucleases and custom-designed zinc finger meganucleases allow researchers to design nucleases to target specific chromosomal locations, and these reagents allow the targeting of transgenes at the chromosomal site cleaved by these nucleases.

The currently used systems for precision genetic engineering of eukaryotic genomes, e.g. plant genomes, rely upon homing endonucleases, meganucleases, zinc finger nucleases, and transcription activator-like effector nucleases (TALENs), which require de novo protein engineering for every new target locus. The highly specific, RNA-directed DNA nuclease, guide RNA/ Cas9 endonuclease system described herein, is more easily customizable and therefore more useful when modification of many different target sequences is the goal.

The guide RNA/Cas system described herein is especially useful for genome engineering, especially plant genome engineering, in circumstances where nuclease off-target cutting can be toxic to the targeted cells. In one aspect of the disclosure of the guide RNA/Cas system described herein, an expression-optimized Cas9 gene, is stably integrated into the target genome, e.g. plant genome. Expression of the Cas9 gene is under control of a promoter, e.g. plant promoter, which can be a constitutive promoter, tissue-specific promoter or inducible promoter, e.g. temperature-inducible, stress-inducible, developmental stage inducible, or chemically inducible promoter. In the absence of the guide RNA or crRNA, the Cas9 protein is not able to cut DNA and therefore its presence in the plant cell should have little or no consequence. Hence a key advantage of the guide RNA/Cas system described herein is the ability to create and maintain a cell line or transgenic organism capable of efficient expression of the Cas9 protein with little or no consequence to cell viability. In order to induce cutting at desired genomic sites to achieve targeted genetic modifications, guide RNAs or crRNAs can be introduced by a variety of methods into cells containing the stably-integrated and expressed cas9 gene. For example, guide RNAs or crRNAs can be chemically or enzymatically synthesized, and introduced into the Cas9 expressing cells via direct delivery methods such a particle bombardment or electroporation. Alternatively, genes capable of efficiently expressing guide RNAs or crRNAs in the target cells can be synthesized chemically, enzymatically or in a biological system, and these genes can be introduced into the Cas9 expressing cells via direct delivery methods such a particle bombardment, electroporation or biological delivery methods such as *Agrobacterium* mediated DNA delivery.

A guide RNA/Cas system mediating gene targeting can be used in methods for directing transgene insertion and / or for producing complex transgenic trait loci comprising multiple transgenes in a fashion similar as disclosed in WO2013/0198888 (published August 1, 2013) where instead of using a double strand break inducing agent to introduce a gene of interest, a guide RNA/Cas system as disclosed herein is used. A complex trait locus includes a genomic locus that has multiple transgenes genetically linked to each other. By inserting independent transgenes within 0.1, 0.2, 0.3, 0.4, 0.5 , 1.0, 2, or even 5 centimorgans (cM) from each other, the transgenes can be bred as a single genetic locus (see, for example, U.S. patent application 13/427,138) or PCT application PCT/US2012/030061. After selecting a plant comprising a transgene, plants containing (at least) one transgenes can be crossed to form an F1 that contains both transgenes. In progeny from these F1 (F2 or BC1) 1/500 progeny would have the two different transgenes recombined onto the same chromosome. The complex locus can then be bred as single genetic locus with both transgene traits. This process can be repeated to stack as many traits as desired.

Chromosomal intervals that correlate with a phenotype or trait of interest can be identified. A variety of methods well known in the art are available for identifying chromosomal intervals. The boundaries of such chromosomal intervals are drawn to encompass markers that will be linked to the gene controlling the trait of interest. In other words, the chromosomal interval is drawn such that any marker that lies within that interval (including the terminal markers that define the boundaries of the interval) can be used as a marker for northern leaf blight resistance. In one aspect of the disclosure, the chromosomal interval comprises at least one QTL, and furthermore, may indeed comprise more than one QTL. Close proximity of multiple QTLs in the same interval may obfuscate the correlation of a particular marker with a particular QTL, as one marker may demonstrate linkage to more than one QTL. Conversely, e.g., if two markers in close proximity show co-segregation with the desired phenotypic trait, it is sometimes unclear if each of those markers identifies the same QTL or two different QTL. The term "quantitative trait locus" or "QTL" refers to a region of DNA that is associated with the differential expression of a quantitative phenotypic trait in at least one genetic background, e.g., in at least one breeding population. The region of the QTL encompasses or is closely linked to the gene or genes that affect the trait in question. An "allele of a QTL" can comprise multiple genes or other genetic factors within a contiguous genomic region or linkage group, such as a haplotype. An allele of a QTL can denote a haplotype within a specified window wherein said window is a contiguous genomic region that can be defined, and tracked, with a set of one or more polymorphic markers. A haplotype can be defined by the unique fingerprint of alleles at each marker within the specified window.

A variety of methods are available to identify those cells having an altered genome at or near a target site without using a screenable marker phenotype. Such methods can be viewed as directly analyzing a target sequence to detect any change in the target sequence, including but not limited to PCR methods, sequencing methods, nuclease digestion, Southern blots, and any combination thereof.

Proteins may be altered in various ways including amino acid substitutions, deletions, truncations, and insertions. Methods for such manipulations are generally known. For example, amino acid sequence variants of the protein(s) can be prepared by mutations in the DNA. Methods for mutagenesis and nucleotide sequence alterations include, for example, Kunkel, (1985) Proc. Natl. Acad. Sci. USA 82:488-92; Kunkel et al., (1987) Meth Enzymol 154:367-82; U.S. Patent No. 4,873,192; Walker and Gaastra, eds. (1983) Techniques in Molecular Biology (MacMillan Publishing Company, New York) and the references cited therein. Guidance regarding amino acid substitutions not likely to affect biological activity of the protein is found, for example, in the model of Dayhoff et al., (1978) Atlas of Protein Sequence and Structure (Natl Biomed Res Found, Washington, D.C.). Conservative substitutions, such as exchanging one amino acid with another having similar properties, may be preferable. Conservative deletions, insertions, and amino acid substitutions are not expected to produce radical changes in the characteristics of the protein, and the effect of any substitution, deletion, insertion, or combination thereof can be evaluated by routine screening assays. Assays for double-strand-break-inducing activity are known and generally measure the overall activity and specificity of the agent on DNA substrates containing target sites.

The term "dicot" refers to the subclass of angiosperm plants also knows as "dicotyledoneae" and includes reference to whole plants, plant organs (e.g., leaves, stems, roots, etc.), seeds, plant cells, and progeny of the same. Plant cell, as used herein includes, without limitation, seeds, suspension cultures, embryos, meristematic regions, callus tissue, leaves, roots, shoots, gametophytes, sporophytes, pollen, and microspores.

The term "crossed" or "cross" or "crossing" in the context of this disclosure means the fusion of gametes via pollination to produce progeny (i.e., cells, seeds, or plants). The term encompasses both sexual crosses (the pollination of one plant by another) and selfing (self-pollination, i.e., when the pollen and ovule (or microspores and megaspores) are from the same plant or genetically identical plants).

The term "introgression" refers to the transmission of a desired allele of a genetic locus from one genetic background to another. For example, introgression of a desired allele at a specified locus can be transmitted to at least one progeny plant via a sexual cross between two parent plants, where at least one of the parent plants has the desired allele within its genome. Alternatively, for example, transmission of an allele can occur by recombination between two donor genomes, e.g., in a fused protoplast, where at least one of the donor protoplasts has the desired allele in its genome. The desired allele can be, e.g., a transgene, a modified (mutated or edited) native allele, or a selected allele of a marker or QTL.

Standard DNA isolation, purification, molecular cloning, vector construction, and verification/characterization methods are well established, see, for example Sambrook et al., (1989) Molecular Cloning: A Laboratory Manual, (Cold Spring Harbor Laboratory Press, NY). Vectors and constructs include circular plasmids, and linear polynucleotides, comprising a polynucleotide of interest and optionally other components including linkers, adapters, regulatory or analysis. In some examples a recognition site and/or target site can be contained within an intron, coding sequence, 5' UTRs, 3' UTRs, and/or regulatory regions.

Further disclosed herein are expression constructs for expressing in a plant, plant cell, or plant part a guide RNA/Cas system that is capable of binding to and creating a double strand break in a target site. In one aspect of the disclosure, the expression constructs of the disclosure comprise a promoter operably linked to a nucleotide sequence encoding a Cas gene and a promoter operably linked to a guide RNA of the present disclosure. The promoter is capable of driving expression of an operably linked nucleotide sequence in a plant cell.

Any plant can be used, including monocot and dicot plants. Examples of monocot plants that can be used include, but are not limited to, corn (Zea *mays*), rice (*Oryza sativa*), rye (*Secale cereale*)*,* sorghum (*Sorghum bicolor, Sorghum vulgare*), millet (e.g., pearl millet (*Pennisetum glaucum*), proso millet (*Panicum miliaceum),* foxtail millet *(Setaria italica),* finger millet *(Eleusine coracana)),* wheat *(Triticum aestivum),* sugarcane *(Saccharum spp.),* oats *(Avena),* barley *(Hordeum),* switchgrass *(Panicum virgatum),* pineapple *(Ananas comosus),* banana *(Musa spp.),* palm, ornamentals, turfgrasses, and other grasses. Examples of dicot plants that can be used include, but are not limited to, soybean *(Glycine max),* canola (*Brassica napus* and *B*. *campestris*), alfalfa (*Medicago sativa*), tobacco (*Nicotiana tabacum*), *Arabidopsis* (*Arabidopsis thaliana*), sunflower (*Helianthus annuus*), cotton (*Gossypium arboreum*), and peanut (*Arachis hypogaea*), tomato (*Solanum lycopersicum*)*,* potato (*Solanum tuberosum*) *etc.*

The meaning of abbreviations is as follows: "sec" means second(s), "min" means minute(s), "h" means hour(s), "d" means day(s), "µL" means microliter(s), "mL" means milliliter(s), "L" means liter(s), "µM" means micromolar, "mM" means millimolar, "M" means molar, "mmol" means millimole(s), "µmole" mean micromole(s), "g" means gram(s), "µg" means microgram(s), "ng" means nanogram(s), "U" means unit(s), "bp" means base pair(s) and "kb" means kilobase(s).

### EXAMPLES

In the following Examples, unless otherwise stated, parts and percentages are by weight and degrees are Celsius.

### EXAMPLE 1

### Modifying target DNA sequences in the genome of a plant cell by delivering Cas9 endonuclease and guide RNA expression cassettes

The Cas9 gene from *Streptococcus pyogenes* M1 GAS (SF370) (SEQ ID NO: 1) was maize codon optimized using standard techniques known in the art and the potato ST-LS1 intron (SEQ ID NO: 2) was introduced in order to eliminate its expression in *E.coli* and *Agrobacterium.* To facilitate nuclear localization of the Cas9 protein in maize cells, *Simian virus* 40 (SV40) monopartite amino terminal nuclear localization signal (MAPKKKRKV, SEQ ID NO: 3) and *Agrobacterium tumefaciens* bipartite VirD2 T-DNA border endonuclease carboxyl terminal nuclear localization signal (KRPRDRHDGELGGRKRAR, SEQ ID NO: 4) were incorporated at the amino and carboxyl-termini of the Cas9 open reading frame, respectively. The maize optimized Cas9 gene was operably linked to a maize constitutive promoter (Ubiquitin) by standard molecular biology techniques. Transcription is terminated by the addition of the 3' sequences from the potato proteinase inhibitor II gene (Pinll) to generate UBI:Cas9:Pinll vector. The sequence of the Ubiquitin driven maize optimized Cas9 expression cassette is shown in SEQ ID NO: 5.

Single guide RNAs (gRNAs) were designed using the methods described by Mali et al., 2013 (Science 339:823-26). A maize U6 polymerase III promoter and terminator were isolated and used to direct initiation and termination of gRNAs, respectively. Two *Bbs*I restriction endonuclease sites were introduced in an inverted tandem orientation with cleavage orientated in an outward direction as described in Cong et al., 2013 (Science 339:819-23) to facilitate the rapid introduction of maize genomic DNA target sequences into the gRNA expression constructs. Only target sequences starting with a G nucleotide were used to promote favorable polymerase III expression of the gRNA. The gRNA expression cassettes were subcloned into Bluescript SK vector (SEQ ID NO: 6).

To test whether the maize optimized Cas9-gRNA complex could recognize, cleave, and facilitate targeted mutations in maize chromosomal DNA through non-homologous end joining (NHEJ) repair pathway, 5 maize loci (three different genomic sequences in each locus) were targeted for cleavage (see Table 2) and examined by amplicon deep sequencing for the presence of mutations.

**Table 2. Maize genomic sites targeted by the Cas9-gRNA system**

| Locus | Location | Target Site Designation | Maize Genomic Target Site Sequence | PAM Sequenc e | SEQ ID NO: |
|---|---|---|---|---|---|
| MS26 | Chr. 1: 51.81cM | MS26Cas-1 | GTACTCCATCCGCCCCATCGAGTA | GGG | 7 |
| | | MS26Cas-2 | GCACGTACGTCACCATCCCGC | CGG | 8 |
| | | MS26Cas-3 | GACGTACGTGCCCTACTCGAT | GGG | 9 |
| LIG | Chr. 2: 28.45cM | LIGCas-1 | GTACCGTACGTGCCCCGGCGG | AGG | 10 |
| | | LIGCas-2 | GGAATTGTACCGTACGTGCCC | CGG | 11 |
| | | LIGCas-3 | GCGTACGCGTACGTGTG | AGG | 12 |
| MS45 | Chr. 9: 119.15cM | MS45Cas-1 | GCTGGCCGAGGTCGACTAC | CGG | 13 |
| | | MS45Cas-2 | GGCCGAGGTCGACTACCGGC | CGG | 14 |
| | | MS45Cas-3 | GGCGCGAGCTCGTGCTTCAC | CGG | 15 |
| ALS1 and ALS2 | 1 - Chr. 4: 107.73cM | ALSCas-1 | GGTGCCAATCATGCGTCG | CGG | 16 |
| | | ALSCas-2 | GGTCGCCATCACGGGAC | AGG | 17 |
| | 2 - Chr. 5: 115.49cM | ALSCas-3 | GTCGCGGCACCTGTCCCGTGA | TGG | 18 |

MS26=Male Sterility Gene 26, LIG=*Liguleless*-1 Gene Promoter, MS45=Male Sterility Gene 45, ALS1=Acetolactate Synthase Gene 1 (Chr.4), ALS1=Acetolactate Synthase Gene 2 (Chr.5).

The maize optimized Cas9 endonuclease and gRNA expression cassettes containing the specific maize variable targeting domains were co-delivered to 60-90 Hi-II immature maize embryos by particle bombardment (see Example 8) with selectable and visible marker (UBI:MoPAT:DsRED fusion) and developmental genes ZmODP-2 (BBM) and ZmWUS2 (WUS) (see Example 9). Hi-II maize embryos transformed with only the Cas9 or gRNA expression cassette served as negative controls. After 7 days, 20-30 most uniformly transformed embryos from each treatment (based on transient expression of the DsRED fluorescent protein) were pooled and total genomic DNA was extracted. The region surrounding the intended target site was PCR amplified with Phusion^{®} High Fidelity PCR Master Mix (New England Biolabs, M0531L) adding sequences necessary for amplicon-specific barcodes and Illumnia sequencing using "tailed" primers through two rounds of PCR. The primers used in the primary PCR reaction are shown in Table 3.

**Table 3. PCR primer sequences**

| Target Site | Primer | Primary PCR Primer Sequence | SEQ ID NO: |
|---|---|---|---|
| MS26Cas-1 | Forward | | 19 |
| MS26Cas-1 | Reverse | | 20 |
| MS26Cas-2 | Forward | | 21 |
| MS26Cas-2 | Reverse | | 22 |
| MS26Cas-3 | Forward | | 23 |
| MS26Cas-3 | Reverse | | 20 |
| LIGCas-1 | Forward | | 24 |
| LIGCas-1 | Reverse | | 25 |
| LIGCas-2 | Forward | | 26 |
| LIGCas-2 | Reverse | | 25 |
| LIGCas-3 | Forward | | 27 |
| LIGCas-3 | Reverse | | 28 |
| MS45Cas-1 | Forward | | 29 |
| MS45Cas-1 | Reverse | | 30 |
| MS45Cas-2 | Forward | | 31 |
| MS45Cas-2 | Reverse | | 30 |
| MS45Cas-3 | Forward | | 32 |
| MS45Cas-3 | Reverse | | 30 |
| ALSCas-1 | Forward | | 33 |
| ALSCas-1 | Reverse | | 34 |
| ALSCas-2 | Forward | | 35 |
| ALSCas-2 | Reverse | | 34 |
| ALSCas-3 | Forward | | 36 |
| ALSCas-3 | Reverse | | 34 |

Primers used in the secondary PCR reaction were AATGATACGGCGACCACCGAGATCTACACTCTTTCCCTACACG (forward, SEQ ID NO: 37) and CAAGCAGAAGACGGCATA (reverse, SEQ ID NO: 38).

The resulting PCR amplifications were purified with a Qiagen PCR purification spin column, concentration measured with a Hoechst dye-based fluorometric assay, combined in an equimolar ratio, and single read 100 nucleotide-length deep sequencing was performed on Illumina's MiSeq Personal Sequencer with a 30-40% (v/v) spike of PhiX control v3 (Illumina, FC-110-3001) to off-set sequence bias. Only those reads with a ≥1 nucleotide indel arising within the 10 nucleotide window centered over the expected site of cleavage and not found in a similar level in the negative control were classified as mutations. Mutant reads with the same mutation were counted and collapsed into a single group and the top 10 most prevalent mutations were visually confirmed as arising within the expected site of cleavage. The total numbers of mutations were then used to calculate the percentage of mutant reads based on the total number of reads of an appropriate length containing a perfect match to the barcode and forward primer.

The mutation frequencies revealed by amplicon deep sequencing for the Cas9-gRNA system targeting all 15 sites are shown in Table 4.

**Table 4. Percent of mutant reads at 5 target loci (15 target sites)**

| Target | DSB Reagents | Total Number of Reads | Number of Mutant Target Gene Reads | Percentage of Mutant Reads in Target Gene |
|---|---|---|---|---|
| LIG (Chr. 2) | LIG-CR1 gRNA+Cas9 | 716,854 | 33,050 | 4.61% |
| | LIG-CR2 gRNA+Cas9 | 711,047 | 16,675 | 2.35% |
| | LIG-CR3 gRNA+Cas9 | 713,183 | 27,959 | 3.92% |
| MS26 (Chr. 1) | MS26-CR1 gRNA+Cas9 | 575,671 | 10,073 | 1.75% |
| | MS26-CR2 gRNA+Cas9 | 543,856 | 16,930 | 3.11% |
| | MS26-CR3 gRNA+Cas9 | 538,141 | 13,879 | 2.58% |
| MS45 (Chr. 9) | MS45-CR1 gRNA+Cas9 | 812,644 | 3,795 | 0.47% |
| | MS45-CR2 gRNA+Cas9 | 785,183 | 14,704 | 1.87% |
| | MS45-CR3 gRNA+Cas9 | 728,023 | 9,203 | 1.26% |
| ALS1 (Chr. 4) and ALS2 (Chr. 5) | ALS-CR1 gRNA+Cas9 | 434,452 | 9,669 | 2.23% |
| | ALS-CR2 gRNA+Cas9 | 472,351 | 6,352 | 1.35% |
| | ALS-CR3 gRNA+Cas9 | 497,786 | 8,535 | 1.72% |
| Controls | Cas9 only | 640,063 | 1 | 0.00% |
| | LIG-CR1 gRNA only | 646,774 | 1 | 0.00% |

Further analysis demonstrated, that the most common type of mutations promoted by Cas9-gRNA system was single nucleotide insertions (for example, see Figure 1, SEQ ID NOs: 49-58). Similar results were observed for the majority of gRNAs tested (Table 5).

**Table 5. Frequency of a single nucleotide insertions and deletions in 15 target sites promoted by the Cas9-gRNA system**

| Target Locus | DSB Reagents | % Single nt Insertion of Total Number of Mutant Reads | % Single nt Deletion of Total Number of Mutant Reads |
|---|---|---|---|
| LIG | LIG-CR1 gRNA+Cas9 | 86% | 5% |
| | LIG-CR2 gRNA+Cas9 | 49% | 25% |
| | LIG-CR3 gRNA+Cas9 | 62% | 20% |
| MS26 | MS26-CR1 gRNA+Cas9 | 46% | 16% |
| | MS26-CR2 gRNA+Cas9 | 78% | 8% |
| | MS26-CR3 gRNA+Cas9 | 45% | 18% |
| MS45 | MS45-CR1 gRNA+Cas9 | 45% | 17% |
| | MS45-CR2 gRNA+Cas9 | 41% | 23% |
| | MS45-CR3 gRNA+Cas9 | 20% | 24% |
| ALS1 (Chr. 4) and ALS2 (Chr. 5) | ALS-CR1 gRNA+Cas9 | 22% | 76% |
| | ALS-CR2 gRNA+Cas9 | 60% | 27% |
| | ALS-CR3 gRNA+Cas9 | 84% | 12% |

This example demonstrates that RNA guided Cas9 generates double strand breaks resulting in high frequency of mutations. Analysis of mutations in multiple target sites showed that although various size deletions and/or insertions were observed, a single nucleotide insertion and a single nucleotide deletion were the most prevalent types of mutations generated by the Cas9-gRNA technology for the majority of the target sites tested in maize.

### EXAMPLE 2

### Edited Acetolactate Synthase Gene Confers Resistance to Chlorsulfuron

This example demonstrates that specific change(s) introduced into the nucleotide sequence of the native maize acetolactate synthase (ALS) gene result in resistance to sulfonylurea class herbicides, specifically, chlorsulfuron.

There are two ALS genes in maze, ALS1 (SEQ ID NO: 39) and ALS2 (SEQ ID NO: 40), located on chromosomes 4 and 5, respectively, with 94% sequence identity at the DNA level.

The ALS protein contains N-terminal transit and the mature protein is formed following transport into the chloroplast and subsequent cleavage of the transit peptide. The mature protein starts at residue S41, resulting in a mature protein of 598 amino acids with a predicted molecular weight of 65 kDa (SEQ ID NO: 41).

Modification of a nucleotide sequence of either ALS1 or ALS2 resulting in a single amino acid residue (P165A or P165S, boxed in grey) change in comparison to the endogenous maize acetolactate synthase protein provides resistance to herbicides in maize.

As acetolactate synthase is a critical enzyme for cell function in plants, simultaneous bi-allelic knockouts of ALS1 and ALS2 genes would not be expected to survive. Therefore, based on polymorphism between ALS1 and ALS2 nucleotide sequences, ALS2-specific ALSCas-4 target site was identified and tested. ALSCas-1 guide RNA expressing construct targeting both ALS1 and ALS2 genes was used as control. Table 6 presents information about ALSCas-1 and ALSCas-4 target sites.

**Table 6. ALSCas-1 and ALSCas-4 (ALS2-specific) target sites**

| Loci | Location | Target Site Designatio n | Maize Genomic Target Site Sequence | PAM Sequence | SEQ ID NO: |
|---|---|---|---|---|---|
| ALS1 and ALS2 | Chr. 4: 107.73cM and Chr. 5: 115.49cM | ALSCas-1 | GGTGCCAATCATGCGTCG | CGG | 16 |
| | | ALSCas-4 | | TGG | 42 |

Underlined nucleotides in the ALSCas-4 target site and PAM are different in the ALS1 gene.

Mutation frequencies at the ALSCas-1 and ALSCas-4 were determined by amplicon deep sequencing as described in Example 1 and shown in Table 7.

**Table 7. Frequencies of mutations at ALSCas-1 and ALSCas-4 target sites recovered by amplicon deep sequencing.**

| Target Site | Total Reads | Mutant reads (ALS1) | Mutant reads (ALS2) |
|---|---|---|---|
| ALSCas-1 | 204,230 | 2704 (1.3%) | 5072 (2.5%) |
| ALSCas-4 | 120,766 | 40 (0.03%) | 3294 (2.7%) |

These results demonstrated that ALSCas-4 gRNA/Cas9 system mutated the ALS2 gene with approximately 90 times higher efficiency than the ALS1 gene. Therefore, the ALSCas-4 target site and the corresponding ALS-CR4 gRNA were selected for the ALS gene editing experiment.

To generate ALS2 edited alleles, a 794 bp polynucleotide modification template comprising a fragment of homology (SEQ ID NO: 43) was cloned into a plasmid vector and two 127 nt single-stranded polynucleotide modification templates (also referred to as DNA oligos, Oligo1, SEQ ID NO: 44, and Oligo2, SEQ ID NO: 45) were tested as polynucleotide modification templates (Figure 2). The 794 bp fragment had the same sequence modifications as Oligo1. The polynucleotide modification templates (repair templates) contained several nucleotide changes in comparison to the native sequence. Single-stranded Oligo1 and the 794 bp repair templates included a single nucleotide change which would direct editing of DNA sequences corresponding to the proline at amino acid position 165 to a serine (P165S), as well as three additional changes within the ALS-CR4 target site and PAM sequence. Modification of the PAM sequence within the repair template altered the methionine codon (AUG) to isoleucine (AUU), which naturally occurs in the ALS1 gene. A second 127 nt single-stranded oligo repair template (Oligo2) was also tested which preserved the methionine at position 157 but contained three additional single nucleotide changes in the sequence which would influence base pairing with the ALS-CR4 gRNA (Figure 2).

Approximately 1,000 immature embryos per treatment were bombarded with the two oligo or single plasmid repair templates, Cas9, ALS-CR4 gRNA, and MoPAT-DsRED in DNA expression cassettes and placed on media to select for bialaphos resistance conferred by PAT. Five weeks post-transformation, two hundred (per treatment) randomly selected independent young callus sectors growing on selective media were separated from the embryos and transferred to fresh bialaphos plates. The remaining embryos (> 800 per treatment) with developing callus events were transferred to the plates containing 100 ppm of chlorosulfuron as direct selection for an edited ALS2 gene. A month later, a total of 384 randomly picked callus sectors growing on bialaphos (approximately 130 events for each repair template) and 7 callus sectors that continued growing on media with chlorsulfuron were analyzed by PCR amplification and sequencing. Edited ALS2 alleles were detected in nine callus sectors: two derived from the callus sectors growing on bialaphos and generated using the 794 bp repair DNA template, and the remaining 7 derived from chlorosulfuron resistant callus sectors edited using the 127 nt single-stranded oligos, three by Oligo1 and four by Oligo2. The second ALS2 allele in these callus sectors was mutated as a result of NHEJ repair. Analysis of the ALS1 gene revealed only wild-type sequence confirming high specificity of the ALS-CR4 gRNA.

Plants were regenerated from 7 out of 9 callus sectors containing edited ALS2 alleles for additional molecular analysis and progeny testing. DNA sequence analysis of ALS2 alleles confirmed the presence of the P165S modifications (ALS2-P165S) as well as the other nucleotide changes associated with the respective repair templates. T1 and T2 progeny of two T0 plants generated from different callus events (794 bp repair DNA and Oligo2) were analyzed to evaluate the inheritance of the edited ALS2 alleles. Progeny plants derived from crosses using pollen from wild type Hi-II plants were analyzed by sequencing and demonstrated sexual transmission of the edited alleles observed in the parent plant with expected 1:1 segregation ratio (57:56 and 47:49, respectively). To test whether the edited ALS sequence confers herbicide resistance, selected four-week old segregating T1 plants with edited and wild-type ALS2 alleles were sprayed with four different concentrations of chlorsulfuron (50, 100 (1x), 200, and 400 mg/liter). Three weeks after treatment, plants with an edited allele showed normal phenotype (Figure 3 - left), while plants with only wild-type alleles demonstrated strong signs of senescence (Figure 3-right).

In addition to resistance to sulfonylurea class herbicides (specifically, chlorsulfuron), ALS genes can be modified to confer resistance to other classes of AHAS inhibitors including triazolopy-rimidines, pyrimidinylthio-benzoates, and Imidazolinone herbicides (Tan S, Evans RR, Dahmer ML, Singh BK, Shaner DL (2005) Imidazolinone-tolerant crops: history, current status and future. Pest Management Science 61: 246-257). Thus, modifications to ALS genes should not be limited to changes describe herein and conferring chlorsulfuron resistance.

These experiments demonstrate that Cas9-gRNA can stimulate HDR-dependent targeted sequence modifications in maize resulting in plants with an edited endogenous gene which properly transmits to subsequent generations. The data also indicate that a single edited ALS2 allele under endogenous promoter provides herbicide resistance in maize.

### EXAMPLE 3

### ALS2 as Endogenous Selectable Marker Gene

This example demonstrates how specifically edited ALS2 gene can be used to generate a selectable marker in a cell replacing delivery of exogenous marker genes currently used in plant transformation.

Due to the relatively low frequencies of plant transformation (transgenic event recovery), selectable marker genes providing resistance to various herbicides are routinely co-delivered with trait genes. To confer resistance, these selectable marker genes need to be stably integrated into the plant genome and have to be excised or bred out in consecutive generations. Some native plant genes can be specifically modified (edited) to confer resistance to herbicides. As described in Example 2, the ALS2 gene with a single amino acid change provides resistance to chlorsulfuron. Therefore, it may be anticipated that gene mutagenesis, gene editing or co-delivery of a trait gene and coincident ALS2 gene editing can be used without an exogenously supplied marker gene. Giving high mutation frequency as the result of NHEJ repair of DSB generated by Cas9-gRNA system (Example 1), this approach might be useful for gene mutagenesis. In this case, the frequency of mutated events would be anticipated to be dependent on HDR-mediated ALS gene editing. With respect to gene editing, it is likely that the combination of two low frequency HDR-dependent genome editing processes (one for ALS gene repair for selection and another for endogenous gene editing or trait gene integration) in plant cells would make the approach using coincident ALS2 gene editing rather impractical.

The following example describes a method that allows overcoming this low efficiency (and impracticality) and improving the likelihood of selecting for plant cells resistant to selective agents. The method does not rely on HDR-dependent gene editing but rather relies on the restoration of the gene function by targeted mutagenesis through NHEJ DNA repair, which is more common (than HDR) in plant somatic cells. As described in Example 2, there are two ALS genes in maize, ALS1 and ALS2, located on chromosomes 4 and 5, respectively. Specific editing of either one of the two ALS genes will confer herbicide resistance. These genes play an essential role in plant metabolism, consequently, targeting and mutating both of them at the same time leads to the cell death. Therefore, in this example, modifications only involve the ALS2 gene by using an ALS2-specific gRNA that does not target the ALS1 gene, hence ALS1 remains wild type. Specifically, two modifications are introduced into the ALS2 gene; first, specific nucleotide(s) change, for example, C to T at the nucleotide position 493 (Figure 2, oligo1) or C to T and C to G at the nucleotide positions 493 and 495, respectively (Figure 2, oligo2) to convert Proline to Serine at amino acid position 165 (named ALS2-P165S) conferring resistance to chlorsulfuron (see Example 2 for details). Second, removal of a single nucleotide, for example a G at the nucleotide position 165 (Figure 4A-4B) resulting in the translational frameshift (Figure 4B) and, hence, loss of ALS2-mediated chlorsulfuron resistance (named ALS2-P165S-CCΔ). While many designs are anticipated, for the highest frequency of ALS2 gene repair, the single nucleotide position (example shown in Figure 4A-4C) should preferably be : i) the 4^{th} nucleotide upstream (5') from the PAM sequence in a gRNA/Cas endonuclease target site, and ii) the 3^{rd} nucleotide in the codon (Figure 4A). This 3^{rd} position is flexible for most amino acids and can be occupied by any of the four nucleotides in 8 out of 20 amino acids (Table 8). Given such flexibility, a higher frequency of proper repair is anticipated at the 3^{rd} position, when compared to the 1^{st} or 2^{nd} position.

**Table 8. Genetic code.**

| **Amino Acid** | **Codons** | **Compressed Codons** |
|---|---|---|
| Alanine / Ala | GCU, GCC, GCA, GCG | GCN |
| Arginine / Arg | CGU, CGC, CGA, CGG, AGA, AGG | CGN, MGR |
| Glycine / Gly | GGU, GGC, GGA, GGG | GGN |
| Leucine / Leu | CUU, CUC, CUA, CUG, UUA, UUG | CUN, YUR |
| Proline / Pro | CCU, CCC, CCA, CCG | CCN |
| Serine / Ser | UCU, UCC, UCA, UCG, AGU, AGC | UCN, AGY |
| Threonine / Thr | ACU, ACC, ACA, ACG | ACN |
| Valine / Val | GUU, GUC, GUA, GUG | GUN |
| Isoleucine / Ile | AUU, AUC, AUA | AUH |
| Asparagine / Asn | AAU, AAC | AAY |
| Aspartic Acid / Asp | GAU, GAC | GAY |
| Cysteine / Cys | UGU, UGC | UGY |
| Glutamine / Gln | CAA, CAG | CAR |
| Glutamic Acid / Glu | GAA, GAG | GAR |
| Histidine / His | CAU, CAC | CAY |
| Lysine / Lys | AAA, AAG | AAR |
| Phenylalanine / Phe | UUU, UUC | UUY |
| Tyrosine / Tyr | UAU, UAC | UAY |
| Methionine / Met | AUG | - |
| Tryptophan / Trp | UGG | - |

Four different target sites satisfying the above criteria and the corresponding gRNAs were selected. Besides the above stated preferred single nucleotide position, the corresponding gRNA should promote high frequency of mutations with high percentage of mutations representing a single nucleotide insertion at the cleavage site. Only one of the four target sites tested satisfied all the described preferences and, therefore, suitable for this experiment, was identified (referred to as ALSCas-7; Table 9).

**Table 9. ALS2-specific ALSCas-7 target site and ALS-CR7- gRNA evaluation by amplicon deep sequencing.**

| Target | Target Site Sequence | % of Mutant Reads | % of Mutant Reads with 1 bp Insertion | % of Mutant Reads with 1 bp Deletion | SEQ ID NO: |
|---|---|---|---|---|---|
| ALSCas-4 (control) | GCTGCTCGATTCCGTCCCCA | 2.73% | - | - | 42 |
| ALSCas-7 | GCTCCCCCGGCCACCCCGCTC | 2.99% | 2.23% (75%) | 0.14% (5%) | 79 |

Then, the ALS2-P165S gene conferring resistance to chlorsulfuron was further modified (resulting in a disrupted gene): the proline codon encoded by CCG (underlined in Table 9) in the ALSCas-7 target site was altered by removal of the G nucleotide at the wobble position (3^{rd} nucleotide position in the codon) resulting in the translational frameshift and a disrupted gene (referred to as ALS2-P165S-CCΔ; Figure 4 A-4B). As demonstrated in Example 1, repair of DSBs generated by Cas9-gRNA system in maize, and repaired through NHEJ, often results in a single nucleotide insertion at the cleavage site. Therefore, the function of the ALS2-P165S-CCΔ gene, and consecutively, cell resistance to chlorsulfuron, can be restored by generating a double strand break (DSB) at the modified ALSCas-7 site, referred to as ALSCas-7-1 (GCTCCCCCGGCCACCCCCTC; SEQ ID NO: 80) and its repair through NHEJ (see Figure 4B-4C).

Based on this disclosure, one can envision simultaneous delivery of two or more gRNAs when one gRNA targets and activates the disrupted ALS2-P165S-CCΔ gene through NHEJ (thus conferring herbicide resistance) and the other gRNA(s) promote DSB(s) at a site(s) different than ALS2 and facilitate desired genome modifications, for example, targeted mutagenesis, deletion, gene editing, or site-specific trait gene insertions. This approach can allow for completely transient targeted genome modifications as all other necessary components (Cas9, gRNAs) can be delivered in a form of protein and/or *in vitro* transcribed RNA molecules.

To test this approach, maize plants (Hi-II genotype) with specifically modified the ALS2 gene described above were generated. First, ALS2 sequence was modified at the amino acid position 165 to confer resistance to chlorsulfuron as described in Example 2. Immature embryos from plants homozygous for the edit were then bombarded with Cas9 and ALS-CR7 gRNA targeting ALSCas-7 target site, selectable marker (UBI:MoPAT-DSRED fusion) and cell developmental enhancing genes (for details, see Examples 8 and 9). Regenerants from bialaphos resistant callus sectors were analyzed by sequencing. Several T0 plants with a single nucleotide deletion (a G in the nucleotide position 165) were identified (Figure 4A-4B). This deletion resulted in the translational frameshift (Figure 4B) and, hence, loss of ALS2-P165S-mediated chlorsulfuron resistance. Plants homozygous for both edits (ALS2-P165S-CCΔ) were regenerated, confirmed by sequencing and tested for the loss of herbicide resistance by spraying with chlorsulfuron.

Embryos from homozygous plants with specifically modified endogenous ALS2 gene (ALS2-P165S-CCΔ) were used in a prove-of-concept experiment. In order to demonstrate that an edited disrupted ALS2-P165S-CCΔ gene can be repaired as described above (so that it encodes a functional protein) and work as selectable marker, DNA vectors encoding for Cas9, gRNA targeting the ALSCas-7-1 site (refed as ALS-CR7-1), cell development enhancing genes (ZmODP2 and ZmWUS), and MS45-CR2 gRNA were co-delivered into maize (Hi-II) immature embryo cells. One week after bombardment, embryos were transferred to the media with 100ppm chlorsulfuron for selection. Approximately 30% of embryos (84 out of 290) developed herbicide resistant callus events, which were analyzed by sequencing. The vast majority of the events (79 events) demonstrated a single nucleotide insertion at the expected ALSCas-7-1 DSB site (Figure 4C) and complete restoration of the ALS2-P165S gene. Four events had no insertion but either 2 or 5 bp deletions putting the gene back in frame, and a single event that seemed to be an escape. Fifty out of 83 events (60%) also demonstrated mutations at the MS45Cas-2 target site.

This example demonstrates prove-of-concept and utility of a specifically modified, inactive ALS2 as endogenous selectable marker gene by the use of a guided Cas endonuclease system. Based on the results described herein, one skilled in the art can use and expand the described approach to any similarly modified endogenous or pre-integrated exogenous gene(s) replacing co-delivery of a selectable marker gene currently used in plant genome editing experiments.

### EXAMPLE 4

### Alternative Designs to Restore Function to a Non-functional Protein Encoded by a Disrupted Gene.

In the previous example, sequence alterations were incorporated within the coding region or ALS2-P165S. It is anticipated that others sequence changes which create a disrupted gene (that does not encode a functional protein) can also be designed to be used as re-activation sequences. This example describes generation of a re-activation sequence that does not depend upon the restoration of a codon within a coding sequence, but rather the elimination of a start codon which is upstream and out-of-frame of the primary translational start codon of ALS2-P165S.

According to a scanning model of eukaryotic translation initiation the first AUG codon relative to the 5' cap of an mRNAs is used to initiate protein synthesis (Kozak M. 1989. The scanning model for translation: an update. The Journal of Cell Biology 108: 229-241). Thus, if an AUG codon within the non-coding leader of the 15 RNA transcript is upstream and out-of-frame of the primary start codon, protein synthesis of the polypeptide encoded by the mRNA is abolished. To take advantage of this rule and apply to a strategy of reactivation of gene expression or function, an endogenous ALS2-P165S allele can be generated to contain an upstream out-of-frame translational start codon (Figure 5A-5B). This allele contains a Cas9 PAM recognition site and an ALS-CRX targeting spacer. Cutting by Cas9 between nucleotides 3 and 4, located 5' of the PAM site in this example can promote nucleotide deletion(s) or addition(s) which can result in the loss of the ATG codon. Loss of this upstream out-of-frame ATG by any combination of deletion or addition due to NHEJ repair can result in translation initiation at the primary ALS2-P165S start codon conferring herbicide resistance.

It is anticipated that the re-activation strategy using an upstream out-of-frame ATG not be limited to the design in Figure 5A-5B. PAM and targeting spacer can also be placed at various positions relative to the upstream out-of-frame ATG, as long as targeted cutting by Cas9 results in the loss of this start codon. For example, the PAM can be present on the antisense strand, 5' of the start codon. Other designs can be contemplated; the out-of-frame ATG start codon can also be placed at different positions within the 5' leader sequence. The PAM sequence can be recognized by other Cas9 proteins like *Streptococcus pyogenes* which recognize nGG PAMs or non-nGG PAM sequences for example *Streptococcus thermophiles* CR1 (PAM sequence recognition nnAGAAn) and others having PAM sequences. The utility of other Cas9 proteins would satisfy the re-activation design of this example as well as Example 3 described above.

Other designs for gene activation are anticipated. As mentioned earlier, in addition to chlorosulfuron resistance, modifications to the ALS gene which confer resistance to other herbicides can be used for reactivation. Furthermore, the phosphomannose isomerase gene (PMI), bialaphos resistance gene (BAR), phosphinothricin acetyltransferase (PAT), hygromycin resistance gene (NPTII), selectable marker genes, fluorescent marker genes (such as but not limiting to RFP, red fluorescent protein, CFP, GFP, green fluorescent protein) and glyphosate resistance genes can be modified to be introduced into plant cells as inactive forms and used as targets for re-activation by guide RNA introduction and repair by NHEJ as described in Example 3. It would be also anticipated that having multiple inactive genes can serve as targets. For example, guide RNA multiplexing has been demonstrated to simultaneously modify multiple genes in a single experiment, thus targeted reactivation of chlorsulfuron and bialaphos resistance, but not limited to these genes, can be an additionally designed for this approach. As described above, coincident with restoration of gene function by NHEJ, modification of other targets would be accomplished simultaneously by the addition of other guide-polynucleotides.

In addition, it would be anticipated that similar approach can be applied to any native or introduced gene sequence and used as an efficient gene switch mechanism.

### EXAMPLE 5

### Specific gene editing without introducing polynucleotide modification templates for homology directed repair.

Example 2 described sequence alterations within the coding region of the ALS2 gene (P165S) using specifically designed polynucleotide modification templates (repair templates). The example below describes a different approach to generating an edited gene of interest that does not depend upon the HDR mechanism, but rather on NHEJ.

As described in Example 1, two of the most prevalent types of mutations facilitated by NHEJ repair of DSBs generated by Cas9 nuclease are 1 bp insertion and 1 bp deletion (Table 4). Based on these observations described herein, methods were developed for gene editing that can be accomplished in two consecutive steps or into a single step, as described below.

The first step includes targeting a gene or polynucleotide of interest, containing a target site that is recognized by a Cas endonuclease, using the RNA guided Cas nuclease system described herein, resulting in the generation of a cell or an organism with a specific nucleotide deletion due to NHEJ repair of the cleaved DNA (illustrated in Figure 6A). The second step requires re-targeting the mutated site and selecting events with insertion of a desired nucleotide (without the use of a polynucleotide modification template (repair template), hence, specifically changing the corresponding amino acid and the gene function. In general, the idea is illustrated in Figure 6A-6C. This method can also be used to edit non-coding DNA fragments.

Alternatively, both steps can be combined into a single step. Two different gRNAs, one recognizing the original target site and the second one the same site but with a 1 bp deletion can be used. In this case, one can envision a consecutive cutting and repair of the endogenous site resulting in a 1 bp deletion followed by cutting of the altered site and its repair with a 1 bp insertion. Then, an event with an insertion of a desired nucleotide can be selected. In the case of editing coding DNA sequences, this process accomplishes two goals - restoring the translational reading frame and replacement of an amino acid of interest. It would be anticipated that combinations of different endonuclease could be used in this two-step or one-step system. For example, the introduction of a first double strand break leading to a single base deletion in a polynucleotide of interest can be accomplished by a first endonuclease, whereas the introduction of a second double strand break leading to a single base insertion (and editing of the polynucleotide of interest) can be accomplished by a second endonuclease that is different from the first endonuclease. The differences between the endonucleases may include, but are not limited to, different PAM recognition sequences, different target recognition sequences, different cleavage activity (blunt-end, 5' or 3' overhang, single strand, double strand), different DNA or amino acid sequences, originating from different organism, or any one combination thereof.

The ability to edit a specific nucleotide in a genome of interest may depend on the endonuclease system of choice and its ability to recognize and cut a particular target site. The discovery of novel guided endonucleases (See for example US patent application 62/162377 filed May 15 2015), and/or modifications of guided endonucleases with various PAM sequences, will further increase the density of target sites that can be recognized and/or cleaved by these endonuclease ultimately resulting in the ability to target any given nucleotide position in the genome using the methods described herein.

### EXAMPLE 6

### Maize Lines with Stably Integrated Cas9 Endonuclease

This example describes generation and validation of maize lines with stably integrated Cas9 expression cassette.

Two *Agrobacterium* vectors (Figure 7, containing maize-codon optimized Cas9 under the transcriptional control of a constitutive (maize UBI, SEQ ID NO: 46) or a temperature regulated (maize MDH, SEQ ID NO: 47) promoter were introduced into Hi-II embryo cells to establish lines containing pre-integrated genomic copies of Cas9 endonuclease. These vectors also contained an embryo-preferred END2 promoter regulating the expression of a blue-fluorescence gene (AmCYAN) as a visible marker and an interrupted copy of the DsRED gene transcriptionally regulated by a maize Histone 2B promoter. Part of the DsRED sequence was duplicated in a direct orientation (369 bp fragment) and consisted of two fragments of the DsRED (RF-FP) gene which were separated by a 347 bp spacer that could be targeted by gRNAs. DSBs within the spacer region promote intramolecular recombination restoring function to the disrupted DsRED gene which results in red fluorescing cells. Maize plants with single-copy T-DNA inserts containing either UBI:Cas9 or MDH:Cas9 were used as a source of immature embryos. Blue-fluorescing embryos containing pre-integrated Cas9 were excised and incubated at 28⁰C (UBI:Cas9) or at 37⁰C (MDH:Cas9) for 24 hours. Post-bombardment, embryos with MDH:Cas9 were incubated at 37⁰C for 24 hours and then moved to 28⁰C. In contrast to control (no gRNA), UBI:Cas9 and MDH:Cas9 containing embryos bombarded with two DNA-expressed gRNAs that targeted sequences within the 347 bp spacer, readily produced red fluorescing foci.

These results demonstrate that described above maize lines poses single copies of functional Cas9 endonuclease.

### EXAMPLE 7

### Transient gRNA Delivery into Embryo Cells with Pre-Integrated Cas9 Generates Mutations in Maize

This Example demonstrates that delivery of gRNA in a form of *in vitro* transcribed RNA molecules into maize immature embryo cells with pre-integrated Cas 9 generates mutations at targeted sites.

Maize plants described in Example 6 containing either UBI:Cas9 or MDH:Cas9 were used as a source of immature embryos for delivery of gRNAs as *in vitro* transcribed RNA or as DNA expression cassettes as control. To measure mutation frequencies at the LIG and MS26 endogenous target sites, LIG-CR3 and MS26-CR2 gRNAs as RNA molecules (100 ng/shot) or as DNA vectors (25 ng/shot) were delivered into UBI:Cas9 and MDH:Cas9 containing embryo cells with temperature treatments described in Example 6. In these experiments, embryos were harvested two days post-bombardment and analyzed by amplicon deep sequencing. Similar frequencies were detected for gRNAs delivered as DNA vectors and as RNA molecules, particularly in the case of Cas9 regulated by the Ubiquitin promoter (Table 10).

**Table 10. Percentage of mutant reads at maize LIG and MS26 target sites produced by transient gRNA delivery into embryos with pre-integrated Cas9 under constitutive (UBI) or regulated (MDH) promoters**

| Target Site | Embryos | Transform ation | Percentage of Mutant Reads (2 days post-bombardment) |
|---|---|---|---|
| LIG | UBI:Cas9 | gRNA (DNA) | 1.22% |
| | | gRNA (RNA) | 1.86% |
| | MDH:Cas9 event 1 | gRNA (DNA) | 0.25% |
| | | gRNA (RNA) | 0.12% |
| | MDH:Cas9 event 2 | gRNA (DNA) | 0.57% |
| | | gRNA (RNA) | 0.26% |
| | MDH:Cas9 event 3 | gRNA (DNA) | 0.46% |
| | | gRNA (RNA) | 0.35% |
| MS26 | MDH:Cas9 event 2 | gRNA (DNA) | 0.58% |
| | | gRNA (RNA) | 0.17% |

Together, these data demonstrate that delivery of gRNA in the form of RNA directly into maize cells containing pre-integrated Cas9 is a viable alternative to DNA delivery for the generation of mutations in plant cells.

### EXAMPLE 8

### Transformation of Maize Immature Embryos

Transformation can be accomplished by various methods known to be effective in plants, including particle-mediated delivery, *Agrobacterium*-mediated transformation, PEG-mediated delivery, and electroporation.

### a. Particle-mediated delivery

Transformation of maize immature embryos using particle delivery is performed as follows. Media recipes follow below.

The ears are husked and surface sterilized in 30% Clorox bleach plus 0.5% Micro detergent for 20 minutes, and rinsed two times with sterile water. The immature embryos are isolated and placed embryo axis side down (scutellum side up), 25 embryos per plate, on 560Y medium for 4 hours and then aligned within the 2.5-cm target zone in preparation for bombardment. Alternatively, isolated embryos are placed on 560L (Initiation medium) and placed in the dark at temperatures ranging from 26°C to 37°C for 8 to 24 hours prior to placing on 560Y for 4 hours at 26°C prior to bombardment as described above.

Plasmids containing the double strand brake inducing agent and template or donor DNA are constructed using standard molecular biology techniques and co-bombarded with plasmids containing the developmental genes ODP2 (AP2 domain transcription factor ODP2 (Ovule development protein 2); US20090328252 A1) and WUSCHEL (US2011/0167516).

The plasmids and DNA of interest are precipitated onto 0.6 µm (average diameter) gold pellets using a water-soluble cationic lipid *Trans*IT-2020 Transfection Reagent (Cat# MIR 5404, Mirus, USA) as follows. DNA or DNA and RNA solution is prepared on ice using a total of 1 µg of DNA and/or RNA constructs (10 shots). To the pre-mixed DNA, 20 µl of prepared gold particles (15 mg/ml) and 1 µl *Trans*IT-2020 are added and mixed carefully. Gold particles are pelleted in a microfuge at 10,000 rpm for 1 min and supernatant is removed. 105 µl of 100% EtOH is added and the particles are resuspended by brief sonication. Then, 10 µl is spotted onto the center of each macrocarrier and allowed to dry before bombardment. The sample plates are bombarded using Biorad Helium Gun (shelf #3) at 425 PSI.

Following bombardment, the embryos are incubated on 560P (maintenance medium) for 12 to 48 hours at temperatures ranging from 26C to 37C, and then placed at 26C. After 5 to 7 days the embryos are transferred to 560R selection medium containing 3 mg/liter Bialaphos, and subcultured every 2 weeks at 26C. After approximately 10 weeks of selection, selection-resistant callus clones are transferred to 288J medium to initiate plant regeneration. Following somatic embryo maturation (2-4 weeks), well-developed somatic embryos are transferred to medium for germination and transferred to a lighted culture room. Approximately 7-10 days later, developing plantlets are transferred to 272V hormone-free medium in tubes for 7-10 days until plantlets are well established. Plants are then transferred to inserts in flats (equivalent to a 2.5" pot) containing potting soil and grown for 1 week in a growth chamber, subsequently grown an additional 1-2 weeks in the greenhouse, then transferred to Classic 600 pots (1.6 gallon) and grown to maturity. Plants are monitored and scored for transformation efficiency, and/or modification of regenerative capabilities.

Initiation medium (560L) comprises 4.0 g/l N6 basal salts (SIGMA C-1416), 1.0 ml/l Eriksson's Vitamin Mix (1000X SIGMA-1511), 0.5 mg/l thiamine HCl, 20.0 g/l sucrose, 1.0 mg/l 2,4-D, and 2.88 g/l L-proline (brought to volume with D-I H2O following adjustment to pH 5.8 with KOH); 2.0 g/l Gelrite (added after bringing to volume with D-I H2O); and 8.5 mg/l silver nitrate (added after sterilizing the medium and cooling to room temperature).

Maintenance medium (560P) comprises 4.0 g/l N6 basal salts (SIGMA C-1416), 1.0 ml/l Eriksson's Vitamin Mix (1000X SIGMA-1511), 0.5 mg/l thiamine HCl, 30.0 g/l sucrose, 2.0 mg/l 2,4-D, and 0.69 g/l L-proline (brought to volume with D-I H2O following adjustment to pH 5.8 with KOH); 3.0 g/l Gelrite (added after bringing to volume with D-I H2O); and 0.85 mg/l silver nitrate (added after sterilizing the medium and cooling to room temperature).

Bombardment medium (560Y) comprises 4.0 g/l N6 basal salts (SIGMA C-1416), 1.0 ml/l Eriksson's Vitamin Mix (1000X SIGMA-1511), 0.5 mg/l thiamine HCl, 120.0 g/l sucrose, 1.0 mg/l 2,4-D, and 2.88 g/l L-proline (brought to volume with D-I H2O following adjustment to pH 5.8 with KOH); 2.0 g/l Gelrite (added after bringing to volume with D-I H2O); and 8.5 mg/l silver nitrate (added after sterilizing the medium and cooling to room temperature).

Selection medium (560R) comprises 4.0 g/l N6 basal salts (SIGMA C-1416), 1.0 ml/l Eriksson's Vitamin Mix (1000X SIGMA-1511), 0.5 mg/l thiamine HCl, 30.0 g/l sucrose, and 2.0 mg/l 2,4-D (brought to volume with D-I H2O following adjustment to pH 5.8 with KOH); 3.0 g/l Gelrite (added after bringing to volume with D-I H2O); and 0.85 mg/l silver nitrate and 3.0 mg/l bialaphos (both added after sterilizing the medium and cooling to room temperature).

Plant regeneration medium (288J) comprises 4.3 g/l MS salts (GIBCO 11117-074), 5.0 ml/l MS vitamins stock solution (0.100 g nicotinic acid, 0.02 g/l thiamine HCL, 0.10 g/l pyridoxine HCL, and 0.40 g/l glycine brought to volume with polished D-I H2O) (Murashige and Skoog (1962) Physiol. Plant. 15:473), 100 mg/l myo-inositol, 0.5 mg/l zeatin, 60 g/l sucrose, and 1.0 ml/l of 0.1 mM abscisic acid (brought to volume with polished D-I H2O after adjusting to pH 5.6); 3.0 g/l Gelrite (added after bringing to volume with D-I H2O); and 1.0 mg/l indoleacetic acid and 3.0 mg/l bialaphos (added after sterilizing the medium and cooling to 60°C).

Hormone-free medium (272V) comprises 4.3 g/l MS salts (GIBCO 11117-074), 5.0 ml/l MS vitamins stock solution (0.100 g/l nicotinic acid, 0.02 g/l thiamine HCL, 0.10 g/l pyridoxine HCL, and 0.40 g/l glycine brought to volume with polished D-I H2O), 0.1 g/l myo-inositol, and 40.0 g/l sucrose (brought to volume with polished D-I H2O after adjusting pH to 5.6); and 6 g/l bacto-agar (added after bringing to volume with polished D-I H2O), sterilized and cooled to 60°C.

### b. Agrobacterium-mediated transformation

Agrobacterium-mediated transformation was performed essentially as described in Djukanovic et al. (2006) Plant Biotech J 4:345-57. Briefly, 10-12 day old immature embryos (0.8 -2.5 mm in size) were dissected from sterilized kernels and placed into liquid medium (4.0 g/L N6 Basal Salts (Sigma C-1416), 1.0 ml/L Eriksson's Vitamin Mix (Sigma E-1511), 1.0 mg/L thiamine HCl, 1.5 mg/L 2, 4-D, 0.690 g/L L-proline, 68.5 g/L sucrose, 36.0 g/L glucose, pH 5.2). After embryo collection, the medium was replaced with 1 ml *Agrobacterium* at a concentration of 0.35-0.45 OD550. Maize embryos were incubated with *Agrobacterium* for 5 min at room temperature, then the mixture was poured onto a media plate containing 4.0 g/L N6 Basal Salts (Sigma C-1416), 1.0 ml/L Eriksson's Vitamin Mix (Sigma E-1511), 1.0 mg/L thiamine HCl, 1.5 mg/L 2, 4-D, 0.690 g/L L-proline, 30.0 g/L sucrose, 0.85 mg/L silver nitrate, 0.1 nM acetosyringone, and 3.0 g/L Gelrite, pH 5.8. Embryos were incubated axis down, in the dark for 3 days at 20°C, then incubated 4 days in the dark at 28°C, then transferred onto new media plates containing 4.0 g/L N6 Basal Salts (Sigma C-1416), 1.0 ml/L Eriksson's Vitamin Mix (Sigma E-1511), 1.0 mg/L thiamine HCl, 1.5 mg/L 2, 4-D, 0.69 g/L L-proline, 30.0 g/L sucrose, 0.5 g/L MES buffer, 0.85 mg/L silver nitrate, 3.0 mg/L Bialaphos, 100 mg/L carbenicillin, and 6.0 g/L agar, pH 5.8. Embryos were subcultured every three weeks until transgenic events were identified. Somatic embryogenesis was induced by transferring a small amount of tissue onto regeneration medium (4.3 g/L MS salts (Gibco 11117), 5.0 ml/L MS Vitamins Stock Solution, 100 mg/L myo-inositol, 0.1 µM ABA, 1 mg/L IAA, 0.5 mg/L zeatin, 60.0 g/L sucrose, 1.5 mg/L Bialaphos, 100 mg/L carbenicillin, 3.0 g/L Gelrite, pH 5.6) and incubation in the dark for two weeks at 28°C. All material with visible shoots and roots were transferred onto media containing 4.3 g/L MS salts (Gibco 11117), 5.0 ml/L MS Vitamins Stock Solution, 100 mg/L myo-inositol, 40.0 g/L sucrose, 1.5 g/L Gelrite, pH 5.6, and incubated under artificial light at 28°C. One week later, plantlets were moved into glass tubes containing the same medium and grown until they were sampled and/or transplanted into soil.

### EXAMPLE 9

### Transient Expression of ZmODP-2 and ZmWUS Enhances Transformation

Parameters of the transformation protocol can be modified to ensure that the BBM activity is transient. One such method involves precipitating the BBM-containing plasmid in a manner that allows for transcription and expression, but precludes subsequent release of the DNA, for example, by using the chemical PEI.

In one example, the BBM plasmid is precipitated onto gold particles with PEI, while the transgenic expression cassette (UBI:MoPAT-GFPm:Pinll; MoPAT is the maize optimized PAT gene) to be integrated is precipitated onto gold particles using the standard calcium chloride method.

Briefly, gold particles were coated with PEI as follows. First, the gold particles were washed. Thirty-five mg of gold particles, 1.0 in average diameter (A.S.I. #162-0010), were weighed out in a microcentrifuge tube, and 1.2 ml absolute EtOH was added and vortexed for one minute. The tube was incubated for 15 minutes at room temperature and then centrifuged at high speed using a microfuge for 15 minutes at 4oC. The supernatant was discarded and a fresh 1.2 ml aliquot of ethanol (EtOH) was added, vortexed for one minute, centrifuged for one minute, and the supernatant again discarded (this is repeated twice). A fresh 1.2 ml aliquot of EtOH was added, and this suspension (gold particles in EtOH) was stored at -20oC for weeks. To coat particles with polyethylimine (PEI; Sigma #P3143), 250 µl of the washed gold particle/EtOH mix was centrifuged and the EtOH discarded. The particles were washed once in 100 µl ddH2O to remove residual ethanol, 250 µl of 0.25 mM PEI was added, followed by a pulse-sonication to suspend the particles and then the tube was plunged into a dry ice/EtOH bath to flash-freeze the suspension, which was then lyophilized overnight. At this point, dry, coated particles could be stored at -80oC for at least 3 weeks. Before use, the particles were rinsed 3 times with 250 µl aliquots of 2.5 mM HEPES buffer, pH 7.1, with 1x pulse-sonication, and then a quick vortex before each centrifugation. The particles were then suspended in a final volume of 250 µl HEPES buffer. A 25 µl aliquot of the particles was added to fresh tubes before attaching DNA. To attach uncoated DNA, the particles were pulse-sonicated, then 1 µg of DNA (in 5 µl water) was added, followed by mixing by pipetting up and down a few times with a Pipetteman and incubated for 10 minutes. The particles were spun briefly (i.e. 10 seconds), the supernatant removed, and 60 µl EtOH added. The particles with PEI-precipitated DNA-1 were washed twice in 60 µl of EtOH. The particles were centrifuged, the supernatant discarded, and the particles were resuspended in 45 µl water. To attach the second DNA (DNA-2), precipitation using *Trans*IT-2020 was used. The 45 µl of particles/DNA-1 suspension was briefly sonicated, and then 5 µl of 100 ng/µl of DNA-2 and 1 µl of *Trans*IT-2020 were added. The solution was placed on a rotary shaker for 10 minutes, centrifuged at 10,000g for 1 minute. The supernatant was removed, and the particles resuspended in 60 µl of EtOH. The solution was spotted onto macrocarriers and the gold particles onto which DNA-1 and DNA-2 had been sequentially attached were delivered into scutellar cells of 10 DAP Hi-II immature embryos using a standard protocol for the PDS-1000. For this experiment, the DNA-1 plasmid contained a UBI:RFP:Pinll expression cassette, and DNA-2 contained a UBI:CFP:Pinll expression cassette. Two days after bombardment, transient expression of both the CFP and RFP fluorescent markers was observed as numerous red & blue cells on the surface of the immature embryo. The embryos were then placed on non-selective culture medium and allowed to grow for 3 weeks before scoring for stable colonies. After this 3-week period, 10 multicellular, stably-expressing blue colonies were observed, in comparison to only one red colony. This demonstrated that PEI-precipitation could be used to effectively introduce DNA for transient expression while dramatically reducing integration of the PEI-introduced DNA and thus reducing the recovery of RFP-expressing transgenic events. In this manner, PEI-precipitation can be used to deliver transient expression of BBM and/or WUS2.

For example, the particles are first coated with UBI:BBM:Pinll using PEI, then coated with UBI:MoPAT-YFP using *Trans*IT-2020, and then bombarded into scutellar cells on the surface of immature embryos. PEI-mediated precipitation results in a high frequency of transiently expressing cells on the surface of the immature embryo and extremely low frequencies of recovery of stable transformants (relative to the *Trans*IT-2020 method). Thus, it is expected that the PEI-precipitated BBM cassette expresses transiently and stimulates a burst of embryogenic growth on the bombarded surface of the tissue (i.e. the scutellar surface), but this plasmid will not integrate. The MoPAT-GFP plasmid released from the Ca++/gold particles is expected to integrate and express the selectable marker at a frequency that results in substantially improved recovery of transgenic events. As a control treatment, PEI-precipitated particles containing a UBI:GUS:Pinll (instead of BBM) are mixed with the MoPAT-GFP/Ca++ particles. Immature embryos from both treatments are moved onto culture medium containing 3mg/l bialaphos. After 6-8 weeks, it is expected that GFP+, bialaphos-resistant calli will be observed in the PEI/BBM treatment at a much higher frequency relative to the control treatment (PEI/GUS).

As an alternative method, the BBM plasmid is precipitated onto gold particles with PEI, and then introduced into scutellar cells on the surface of immature embryos, and subsequent transient expression of the BBM gene elicits a rapid proliferation of embryogenic growth. During this period of induced growth, the explants are treated with Agrobacterium using standard methods for maize (see Example 1), with T-DNA delivery into the cell introducing a transgenic expression cassette such as UBI:MoPAT-GFPm:Pinll. After co-cultivation, explants are allowed to recover on normal culture medium, and then are moved onto culture medium containing 3 mg/l bialaphos. After 6-8 weeks, it is expected that GFP+, bialaphos-resistant calli will be observed in the PEI/BBM treatment at a much higher frequency relative to the control treatment (PEI/GUS).

It may be desirable to "kick start" callus growth by transiently expressing the BBM and/or WUS2 polynucleotide products. This can be done by delivering BBM and WUS2 5'-capped polyadenylated RNA, expression cassettes containing BBM and WUS2 DNA, or BBM and/or WUS2 proteins. All of these molecules can be delivered using a biolistics particle gun. For example 5'-capped polyadenylated BBM and/or WUS2 RNA can easily be made in vitro using Ambion's mMessage mMachine kit. RNA is co-delivered along with DNA containing a polynucleotide of interest and a marker used for selection/screening such as UBI:MoPAT-GFPm:Pinll. It is expected that the cells receiving the RNA will immediately begin dividing more rapidly and a large portion of these will have integrated the agronomic gene. These events can further be validated as being transgenic clonal colonies because they will also express the PAT~GFP fusion protein (and thus will display green fluorescence under appropriate illumination). Plants regenerated from these embryos can then be screened for the presence of the polynucleotide of interest.

### EXAMPLE 10

### Direct Delivery of gRNA and Cas9 as a guide RNA/Cas endonuclease Ribonucleotide-protein Complex (RGEN) into Embryo Cells Generates Mutations in Maize

This example demonstrates that direct delivery of Cas9 in the form of protein and gRNA in the form of *in vitro* transcribed or chemically synthesized RNA molecules, into maize immature embryo cells generates mutations at the corresponding targeted sites.

To generate gRNA in the form of RNA molecules, the maize-optimized U6 polymerase III gRNA expression cassettes were amplified by PCR using a 5' oligonucleotide primer that also contained the sequence of the T7 polymerase promoter and transcriptional initiation signal just 5' of the spacer to gene. T7 *in vitro* transcription was carried out with the AmpliScribe T7-Flash Kit (Epicentre) according to the manufacturer's recommendations, and products were purified using NucAway Spin Columns (Invitrogen; Life Technologies Inc) followed by ethanol precipitation.

To generate a guide RNA/Cas9 endonuclease protein complex (RGEN) (also referred to as a guide RNA/Cas9 endonuclease ribonucleotide-protein ), 7 µg of Cas9 *(Streptococcus pyogenes Cas9)* protein and 3 µg of gRNA molecules (1:2 molar ratio) were mixed in 1x Cas9 buffer (NEB) in a total volume of 20 µl and incubated at room temperature for 15 minutes. Together with the RGEN, plasmids containing Ubiquitin promoter regulated selectable and visible markers (MoPAT-DsRed fusion), Ubiquitin promoter regulated mays Ovule development protein 2, ZmODP2 (see US20090328252, published December 31, 2009) and maize IN2 promoter (Hershey et al. 1991, Plant Mol. Biol 17:679-690) regulated WUSCHEL, ZmWUS (see US20110167516, published July 7, 2011) were mixed with a particle delivery matrix comprising commercially available gold particles (0.6µm, Bio-Rad) and a water soluble cationic lipid *Trans*IT-2020 (Mirus, USA) . The particle delivery matrix comprising the guide RNA/Cas endonuclease ribonucleotide-protein complexes were delivered into maize embryo cells using particle mediated delivery (see Particle-mediated delivery described in Example 8) with some modifications. Specifically, after gold particles were pelleted in a microfuge at 10,000 rpm for 1 min and supernatant was removed, the particles were resuspended in 105 µl of sterile water instead of 100% ethanol. Then, 10 µl was spotted onto the center of each macrocarrier and allowed to dry before bombardment.

Wild type maize plants were used as a source of immature embryos for co-delivery of Cas9 and gRNA in the form of a guide RNA/Cas endonuclease ribonucleotide-protein complexes (RGEN) along with selectable and visible marker (UBI:MoPAT-DsRED) and developmental genes (UBI:ZmODP2 and IN2:ZmWUS). To measure mutation frequencies at the LIGCas-3, MS26Cas-2, MS45Cas-2 and ALSCas-4 endogenous target sites, embryos were harvested two days post-bombardment and analyzed by amplicon deep sequencing. Untreated embryos and embryos bombarded with the Cas9 protein only served as negative controls while embryos bombarded with DNA vectors expressing Cas9 and gRNA were used as positive controls. Similar frequencies were detected for Cas9-gRNA components delivered as DNA vectors and as guide RNA/Cas endonuclease ribonucleotide-protein complexes (Table 11).

**Table 11. Percentage of mutant reads at LIG, MS26, MS45, and ALS target sites produced by direct delivery of RGEN complexes into maize embryo cells.**

| Target Sites | Molecules delivered | Total Number of Reads | Number of Mutant Reads | Percentage of Mutant Reads |
|---|---|---|---|---|
| LIGCas-3 (Chr. 2) | Untreated embryos (control 1) | 915,198 | 38 | 0.004% |
| | Cas9 protein only (control 2) | 408,348 | 17 | 0.004% |
| | Cas9-Lig-CR3 RGEN | 439,827 | 2,510 | 0.57% |
| | Cas9 (DNA)+Lig-CR3 (DNA) | 369,443 | 2,058 | 0.56% |
| MS26Cas-2 (Chr. 1) | Untreated embryos (control 1) | 245,476 | 8 | 0.003% |
| | Cas9 protein only (control 2) | 429,388 | 20 | 0.004% |
| | Cas9-MS26-CR2 RGEN | 252,519 | 533 | 0.21% |
| | Cas9 (DNA)+MS26-CR2 (DNA) | 186,857 | 812 | 0.43% |
| MS45Cas-2 (Chr. 9) | Untreated embryos (control 1) | 255,877 | 12 | 0.005% |
| | Cas9 protein only (control 2) | 487,876 | 12 | 0.002% |
| | Cas9-MS45-CR2 RGEN | 241,287 | 2,075 | 0.86% |
| | Cas9 (DNA)+MS45-CR2 (DNA) | 304,622 | 1591 | 0.52% |
| ALS2Cas-4 (Chr. 5) | Cas9 protein only (control 2) | 807,014 | 125 | 0.02% |
| | Cas9-ALS-CR4 RGEN | 791,084 | 3,613 | 0.45% |
| | Cas9 (DNA)+ALS-CR4 (DNA) | 833,130 | 4251 | 0.51% |

To measure the mutation frequency at the plant level, 60 embryos co-bombarded with Cas9-MS45-gRNA complex, ZmODP2, ZmWUS and MOPAT-DSRED were placed on media containing bialaphos as selective agent. Multiple plants were regenerated from each of the 36 herbicide-resistant callus sectors and screened for mutations. Out of the 36 events, 17 (47%) contained mutant alleles (10 single and 7 biallelic) while 19 (53%) revealed only wild type *MS45* alleles. Among plants with mutations, the number of sequencing reads for each allele was similar indicating plants were not chimeric.

To demonstrate that direct RGEN delivery is also sufficient to generate specific edits in endogenous genes in plants, the maize ALS2 gene was targeted (ALS2-specific ALSCas-4 target site) as described in Example 2. A 127 nt single-stranded DNA Oligo2, (SEQ ID NO: 45) as a repair template was co-delivered with Cas9/ALS-CR4 RGEN complex in a similar manner as described above. Embryos were harvested two days post-bombardment and analyzed by amplicon deep sequencing (Table 12).

**Table 12. Percentage of mutant reads and reads with edits at ALS target site produced by delivery of RGEN complex and donor DNA template into maize embryo cells.**

| Target Site | Molecules delivered | Total Number of Reads | Number of Mutant Reads | % of Mutant Reads | Number of Reads with Edits | % of Reads with Edits |
|---|---|---|---|---|---|---|
| ALS2 | Cas9 protein only | 807,014 | 105 | 0.01% | - | - |
| | Cas9-ALS-CR4 RGEN + ss Oligo2 | 791,084 | 3,613 | 0.45% | 209 | 0.02% |

In addition, in two independent experiments, 40 to 50 bombarded embryos were transferred to plates containing 100 ppm of chlorsulfuron as direct selection for an edited ALS2 gene. Six weeks later, two callus sectors (one from each experiment) that continued growing on media with chlorsulfuron were analyzed by sequencing. In both events, one ALS2 allele was specifically edited while the second allele remained wild type. Plants regenerated from these callus sectors contained edited ALS2 alleles and were resistant to chlorsulfuron when sprayed with the herbicide.

These data demonstrate that direct delivery of Cas9 and gRNA, in the form of a guide RNA/Cas endonuclease ribonucleotide-protein complex (with or without a polynucleotide modification template DNA) into maize immature embryo cells, is a viable alternative to DNA delivery (such as recombinant DNA, plasmid DNA) for targeted mutagenesis and gene editing in plants.

### EXAMPLE 11

### Direct Delivery of Cas9 in the form on mRNA and gRNA into Embryo Cells Generates Mutations in Maize

This example demonstrates that direct delivery of Cas9, in the form of mRNA molecules and gRNA in the form of *in vitro* transcribed or chemically synthesized RNA molecules, into maize immature embryo cells generates mutations at the corresponding targeted sites.

In our previous experiment (Svitashev et al., Plant Physiology, 2015, Vol. 169, pp. 931-945), co-delivery of gRNA in the form of *in vitro* synthesized RNA molecules with the Cas9 expressing DNA vector yielded approximately 100-fold lower mutation frequency then in experiments where both Cas9 and gRNA were delivered as DNA vectors. One possible explanation for this difference may be that the requirement for coincident function of Cas9 and gRNA was not met when gRNA was delivered as RNA and Cas9 was delivered as a DNA vector. To overcome this problem, Cas9 can be delivered as mRNA molecules that will shorten the time from the moment of delivery to functional Cas9 protein expression. Commercially available Cas9 mRNA (TriLink Biotechnologies) was used in the experiment.

To test this idea, maize embryo cells were co-bombarded with Cas9 mRNA (200ng), gRNA in the form of *in vitro* synthesized RNA molecules (100ng), DNA vectors containing Ubiquitin regulated MoPAT-DsRED fusion (25ng) and developmental genes, Ubiquitin promoter regulated ODP2 and IN2 promoter regulated WUS (12ng each) per shot. Commercially available Cas9 mRNA (TriLink Biotechnologies) and RNA molecules, *in vitro* synthesized as described above, were used in the experiment. Analysis for mutation frequency was performed by amplicon deep sequencing on embryos collected 2 days post-transformation (Table 13).

**Table 13. Percentage of mutant reads at MS45 target site produced by transient delivery of Cas9 as mRNA and gRNA as RNA molecules into maize embryo cells.**

| Target Sites | Molecules delivered | Total Number of Reads | Number of Mutant Reads | Percentage of Mutant Reads |
|---|---|---|---|---|
| MS45 | Cas9 mRNA only | 1,097,279 | 799 | 0.07% |
| | Cas9 mRNA+Ms45 CR2 gRNA | 1,260,332 | 2,304 | 0.18% |
| | Cas9 (DNA)+Ms45 CR2 (DNA) | 1,106,125 | 3,490 | 0.31% |

These data demonstrate that delivery of both Cas9 and gRNA, in the form of RNA molecules, improves frequencies of targeted mutations and, along with Cas9-gRNA delivery as the RGEN complex, is a viable alternative to DNA delivery for targeted mutagenesis and gene editing in plants.

### EXAMPLE 12

### Direct Delivery of Cas9 and gRNA as a guide RNA/Cas endonuclease Ribonucleotide-protein Complex (RGEN) into Embryo Cells without the Use of a Selectable Marker Generates Mutations in Maize

This example demonstrates that delivery of Cas9 in the form of protein and gRNA in the form of *in vitro* transcribed or chemically synthesized RNA molecules, into maize immature embryo cells without co-delivery of selectable marker gene(s) is sufficient to regenerate plants with mutations at the corresponding targeted sites with practical frequencies.

The necessity of selectable markers to provide a growth advantage to transformed or modified cells has been the long standing paradigm in plant transformation and genome modification protocols. Therefore, in all mutation, gene editing and gene integration experiments, selectable markers are used to select for genome edited events. Taking into consideration the unexpected high activity (mutation frequency) of RGEN complexes in the experiments described in Example 10, a completely DNA-free (vector free) genome editing without a selectable marker was attempted. Maize embryo cells were bombarded with guide RNA/Cas endonuclease ribonucleotide-protein (RGEN) complexes targeting three different genes: *liguleless1* (*LIG*), *MS26* and *MS45.* Cas9 endonuclease and MS45-gRNA on DNA vectors were delivered in parallel experiments which served as controls. Plants were regenerated and analyzed by sequencing for targeted mutations. In all experiments, mutant plants were recovered at surprisingly high frequencies ranging from 2.4 to 9.7% (Table 14).

**Table 14. Mutation frequencies at LIG, MS26 and MS45 target sites upon delivery of Cas9 and gRNAs in the form of DNA vectors and direct delivery of RGEN complexes into maize immature embryo cells. Analysis was performed on TO plants regenerated without selection (without use of selectable markers).**

| Target site | Cas9 and gRNA delivery method | Plants analyzed | Plants with mutated alleles | SEQ ID NO: |
|---|---|---|---|---|
| LIGCas-3 | RGEN | 756 | 73 (9.7%) | 12 |
| MS26Cas-2 | RGEN | 756 | 18 (2.4%) | 14 |
| MS45 Cas-2 | RGEN | 1,880 | 70 (3.7%) | 8 |
| MS45 Cas-2 | Vector DNA | 940 | 38 (4.0%) | 8 |

Further, regenerated TO plants were crossed with wild type Hi-II plants and the progeny plants were used for segregation analysis. Sexual transmission of mutated *ms45* alleles at the expected Mendelian segregation (1:1) was demonstrated in all progeny plants analyzed.

To assess the potential of RGEN delivery to reduce off-target cleavage in maize, the mutation frequency at the MS45 off-site was evaluated using DNA vectors and RGEN delivery. Searches for a site with close homology to the on-target site were performed by aligning the protospacer region of the MS45Cas-2 target site (the region of the target site that base pairs with the guide RNA spacer) with the maize B73 reference genome (B73 RefGen_v3, Maize Genetics and Genomics Database) using Bowtie sequence aligner (Langmead, B., Trapnell, C., Pop, M. & Salzberg, S.L. Ultrafast and memory-efficient alignment of short DNA sequences to the human genome. Genome Biol. 10:R25, 2009) permitting up to two mismatches with the on-target sequence. Potential off-target sites were then examined for the presence of a NGG protospacer adjacent motif (PAM) sequence immediately 3' of the identified protospacer off-target. Only a single off-target site (5'-CGCCGAGGGCGACTACCGGC-3', SEQ ID NO:81) was identified using these search criteria. It contained a 2 bp mismatch with the MS45 protospacer target and an AGG PAM (Table 15). To confirm the site was cleaved *in vivo,* it was analyzed by deep sequencing for the presence of mutations in maize embryos transformed with DNA vectors expressing Cas9 and MS45-CR2 gRNA. As shown in Table 15, mutational activity of the off-target site was at a frequency of 2% compared to a 4% frequency observed for the on-target site. As shown in Table 15, RGEN off-target activity was greatly reduced relative to Cas9 and gRNA delivery on DNA vectors (from 2% to 0%).

**Table 15. Mutation frequencies at the MS45 off-target site upon delivery of Cas9 and gRNAs in the form of DNA vectors and RGEN complexes into maize immature embryo cells. Analysis performed on TO plants regenerated without selection.**

| Target Site | Cas9 and gRNA delivery method | Target site sequence with PAM* | Plants Analyzed | Plants with Mutations (number) | Plants with Mutation s (%) | SEQ ID NO: |
|---|---|---|---|---|---|---|
| MS45Cas-2 | | | 904 | 38 | 4% | 14 |
| MS45 off-site | DNA | | 940 | 19 | 2.0% | 82 |
| MS45 off-site | RGEN | | 1,880 | 0 | 0.0% | 82 |

| | | | | | | |
|---|---|---|---|---|---|---|
| *PAM - protospacer adjacent motif is a 3 nt sequence immediately 3' of the target site. Two nucleotides different in the MS45 off-target site in comparison to the intended site are shown in bold and underlined. | | | | | | |

This example demonstrates that generation of plants with targeted mutations using RNA-guided endonucleases, does not require co-delivery of selectable or screenable marker genes, thus increasing specificity and exactness of the introduced modifications. Regenerated plants contained only targeted mutations or targeted gene edits (if a repair template was included to modify DNA sequence) without random integration of DNA vectors. This method of delivery provides a completely DNA-free approach to gene mutagenesis in plant cells of major crop species including but not limited to maize, soybean, wheat, rice, millet, sorghum and canola.

## Claims

1. A method comprising introducing through particle mediated delivery a ribonucleoprotein complex comprising a Cas endonuclease protein and a guide RNA into a plant cell containing a plant cell wall, wherein the guide RNA comprises a polynucleotide sequence complementary to a genomic target site sequence in the genome of the plant cell; wherein the Cas endonuclease protein and the guide RNA are applied to a particle delivery matrix comprising a microparticle; and wherein the Cas endonuclease protein and the guide RNA bind to the genomic target site sequence in the genome of the plant cell.

2. The method of Claim 1, wherein the plant cell is selected from the group consisting of:
maize, soybean, cotton, canola, wheat, rice, Arabidopsis, alfalfa, tobacco, and sorghum.

3. A method comprising:
(a) introducing through particle mediated delivery into a plant cell containing a plant cell wall a microparticle coated with a ribonucleoprotein complex comprising a Cas endonuclease protein and a guide RNA, and
(b) incubating the plant cell to allow the ribonucleoprotein complex to bind to a target sequence in the genome of the plant cell;
wherein the ribonucleoprotein complex modifies the target sequence by the insertion of at least one nucleotide, the deletion of at least one nucleotide, or the substitution of at least one nucleotide.

4. The method of Claim 3, wherein the plant cell is selected from the group consisting of:
maize, soybean, cotton, canola, wheat, rice, Arabidopsis, alfalfa, tobacco, and sorghum.

5. The method of Claim 1 or 3, wherein the Cas endonuclease protein is catalytically inactive.

6. The method of Claim 1 or 3, wherein the microparticle is selected from the group consisting of a gold particle, a tungsten particle, and a silicon carbide whisker particle.

7. The method of Claim 3, further comprising introducing into the plant cell:
(i) a polynucleotide modification template, wherein said polynucleotide modification template comprises at least one nucleotide modification as compared to the target site sequence in the genome of said plant cell; or
(ii) a donor DNA molecule, wherein the donor DNA molecule comprises at least one polynucleotide of interest to be inserted into said target site in the genome of said plant cell.

8. A composition comprising a plant cell containing a plant cell wall and a particle delivery matrix, wherein the particle delivery matrix comprises:
(a) a microparticle; and
(b) a ribonucleoprotein complex comprising a Cas endonuclease protein and a guide RNA molecule comprising a polynucleotide sequence complementary to a genomic target site sequence in the genome of the plant cell;
wherein the microparticle is associated with the ribonucleoprotein complex.

9. The composition of Claim 8, wherein the ribonucleoprotein complex binds to a target site in the genome of the plant cell.

10. The composition of Claim 8, wherein the cell is selected from the group consisting of:
maize, soybean, cotton, canola, wheat, rice, Arabidopsis, alfalfa, tobacco, and sorghum.

11. The composition of Claim 8, wherein the Cas endonuclease protein is catalytically inactive.

12. The composition of claim 8, wherein the microparticle is selected from the group consisting of a gold particle, a tungsten particle, and a silicon carbide whisker particle.

13. The composition of Claim 8, further comprising:
(i) a polynucleotide modification template, wherein the polynucleotide modification template comprises at least one nucleotide modification as compared to the target site sequence in the genome of said plant cell; or
(ii) a donor DNA molecule, wherein the donor DNA molecule comprises at least one polynucleotide of interest to be inserted into said target site in the genome of said plant cell.

14. The composition of Claim 8, wherein the Cas endonuclease is Cas9 endonuclease.

15. The method of Claim 1 or 3, wherein the Cas endonuclease is Cas9 endonuclease.

## Patentansprüche

1. Verfahren, umfassend das Einbringen eines Ribonukleoproteinkomplexes, der ein Cas-Endonukleaseprotein und eine Leit-RNA umfasst, durch teilchenvermittelte Abgabe in eine Pflanzenzelle, die eine Pflanzenzellwand enthält, wobei die Leit-RNA eine Polynukleotidsequenz umfasst, die zu einer genomischen Zielstellensequenz in dem Genom der Pflanzenzelle komplementär ist; wobei das Cas-Endonukleaseprotein und die Leit-RNA auf eine Teilchenabgabematrix aufgebracht werden, die ein Mikroteilchen umfasst; und wobei das Cas-Endonukleaseprotein und die Leit-RNA an die genomische Zielstellensequenz in dem Genom der Pflanzenzelle binden.

2. Verfahren nach Anspruch 1, wobei die Pflanzenzelle aus der Gruppe ausgewählt ist, bestehend aus: Mais, Sojabohne, Baumwolle, Canola, Weizen, Reis, Arabidopsis, Alfalfa, Tabak und Sorghum.

3. Verfahren, umfassend:
(a) Einbringen eines Mikroteilchens, das mit einem Ribonukleoproteinkomplex beschichtet ist, der ein Cas-Endonukleaseprotein und eine Leit-RNA umfasst, durch teilchenvermittelte Abgabe in eine Pflanzenzelle, die eine Pflanzenzellwand enthält, und
(b) Inkubieren der Pflanzenzelle, damit der Ribonukleoproteinkomplex an eine Zielsequenz in dem Genom der Pflanzenzelle binden kann;
wobei der Ribonukleoproteinkomplex die Zielsequenz durch die Einsetzen von wenigstens einem Nukleotid, die Deletion von wenigstens einem Nukleotid oder die Substitution von wenigstens einem Nukleotid modifiziert.

4. Verfahren nach Anspruch 3, wobei die Pflanzenzelle aus der Gruppe ausgewählt ist, bestehend aus: Mais, Sojabohne, Baumwolle, Canola, Weizen, Reis, Arabidopsis, Alfalfa, Tabak und Sorghum.

5. Verfahren nach Anspruch 1 oder 3, wobei das Cas-Endonukleaseprotein katalytisch inaktiv ist.

6. Verfahren nach Anspruch 1 oder 3, wobei das Mikroteilchen aus der Gruppe ausgewählt ist, bestehend aus einem Goldteilchen, einem Wolframteilchen und einem Siliciumcarbid-Whiskerteilchen.

7. Verfahren nach Anspruch 3, das ferner das Einbringen in die Pflanzenzelle umfasst von:
(i) einer Polynukleotidmodifikationstemplate, wobei die Polynukleotidmodifikationstemplate wenigstens eine Nukleotidmodifikation im Vergleich zu der Zielstellensequenz in dem Genom der Pflanzenzelle umfasst; oder
(ii) einem Donor-DNA-Molekül, wobei das Donor-DNA-Molekül wenigstens ein Polynukleotid von Interesse umfasst, das in die Zielstelle in dem Genom der Pflanzenzelle eingefügt werden soll.

8. Zusammensetzung, umfassend eine Pflanzenzelle, die eine Pflanzenzellwand und eine Teilchenabgabematrix enthält, wobei die Teilchenabgabematrix umfasst:
(a) ein Mikroteilchen; und
(b) einen Ribonukleoproteinkomplex, der ein Cas-Endonukleaseprotein und ein Leit-RNA-Molekül umfasst, das eine Polynukleotidsequenz umfasst, die zu einer genomischen Zielstellensequenz in dem Genom der Pflanzenzelle komplementär ist;
wobei das Mikroteilchen mit dem Ribonukleoproteinkomplex assoziiert ist.

9. Zusammensetzung nach Anspruch 8, wobei der Ribonukleoproteinkomplex an eine Zielstelle in dem Genom der Pflanzenzelle bindet.

10. Zusammensetzung nach Anspruch 8, wobei die Zelle aus der Gruppe ausgewählt ist, bestehend aus: Mais, Sojabohne, Baumwolle, Canola, Weizen, Reis, Arabidopsis, Alfalfa, Tabak und Sorghum.

11. Zusammensetzung nach Anspruch 8, wobei das Cas-Endonukleaseprotein katalytisch inaktiv ist.

12. Zusammensetzung nach Anspruch 8, wobei das Mikroteilchen aus der Gruppe ausgewählt ist, bestehend aus einem Goldteilchen, einem Wolframteilchen und einem Siliciumcarbid-Whiskerteilchen.

13. Zusammensetzung nach Anspruch 8, ferner umfassend:
(i) eine Polynukleotidmodifikationstemplate, wobei die Polynukleotidmodifikationstemplate wenigstens eine Nukleotidmodifikation im Vergleich zu der Zielstellensequenz in dem Genom der Pflanzenzelle umfasst; oder
(ii) ein Donor-DNA-Molekül, wobei das Donor-DNA-Molekül wenigstens ein Polynukleotid von Interesse umfasst, das in die Zielstelle in dem Genom der Pflanzenzelle eingefügt werden soll.

14. Zusammensetzung nach Anspruch 8, wobei die Cas-Endonuklease Cas9-Endonuklease ist.

15. Zusammensetzung nach Anspruch 1 oder 3, wobei die Cas-Endonuklease Cas9-Endonuklease ist.

## Revendications

1. Procédé comprenant l'introduction par le biais d'une délivrance médiée par particules d'un complexe ribonucléoprotéique comprenant une protéine endonucléase Cas et un ARN guide dans une cellule végétale contenant une paroi de cellule végétale, dans lequel l'ARN guide comprend une séquence polynucléotidique complémentaire d'une séquence de site cible génomique dans le génome de la cellule végétale ; dans lequel la protéine endonucléase Cas et l'ARN guide sont appliqués à une matrice de délivrance de particule comprenant une microparticule ; et dans lequel la protéine endonucléase Cas et l'ARN guide se lient à la séquence de site cible génomique dans le génome de la cellule végétale.

2. Procédé selon la revendication 1, dans lequel la cellule végétale est choisie dans le groupe constitué par : le maïs, le soja, le coton, le canola, le blé, le riz, Arabidopsis, la luzerne, le tabac, et le sorgho.

3. Procédé comprenant :
(a) l'introduction par le biais d'une délivrance médiée par particules dans une cellule végétale contenant une paroi de cellule végétale d'une microparticule enrobée avec un complexe ribonucléoprotéique comprenant une protéine endonucléase Cas et un ARN guide, et
(b) l'incubation de la cellule végétale pour permettre au complexe ribonucléoprotéique de se lier à une séquence cible dans le génome de la cellule végétale ;
dans lequel le complexe ribonucléoprotéique modifie la séquence cible par l'insertion d'au moins un nucléotide, la délétion d'au moins un nucléotide, ou la substitution d'au moins un nucléotide.

4. Procédé selon la revendication 3, dans lequel la cellule végétale est choisie dans le groupe constitué par : le maïs, le soja, le coton, le canola, le blé, le riz, Arabidopsis, la luzerne, le tabac, et le sorgho.

5. Procédé selon la revendication 1 ou 3, dans lequel la protéine endonucléase Cas est catalytiquement inactive.

6. Procédé selon la revendication 1 ou 3, dans lequel la microparticule est choisie dans le groupe constitué par une particule d'or, une particule de tungstène, et une particule de trichite de carbure de silicium.

7. Procédé selon la revendication 3, comprenant en outre l'introduction dans la cellule végétale :
(i) d'une matrice de modification polynucléotidique, dans lequel ladite matrice de modification polynucléotidique comprend au moins une modification nucléotidique en comparaison avec la séquence de site cible dans le génome de ladite cellule végétale ; ou
(ii) d'une molécule d'ADN donneur, dans lequel la molécule d'ADN donneur comprend au moins un polynucléotide d'intérêt à insérer dans ledit site cible dans le génome de ladite cellule végétale.

8. Composition comprenant une cellule végétale contenant une paroi de cellule végétale et une matrice de délivrance de particule, dans laquelle la matrice de délivrance de particule comprend :
(a) une microparticule ; et
(b) un complexe ribonucléoprotéique comprenant une protéine endonucléase Cas et une molécule d'ARN guide comprenant une séquence polynucléotidique complémentaire d'une séquence de site cible génomique dans le génome de la cellule végétale ;
dans laquelle la microparticule est associée au complexe ribonucléoprotéique.

9. Composition selon la revendication 8, dans laquelle le complexe ribonucléoprotéique se lie à un site cible dans le génome de la cellule végétale.

10. Composition selon la revendication 8, dans laquelle la cellule est choisie dans le groupe constitué par : le maïs, le soja, le coton, le canola, le blé, le riz, Arabidopsis, la luzerne, le tabac, et le sorgho.

11. Composition selon la revendication 8, dans laquelle la protéine endonucléase Cas est catalytiquement inactive.

12. Composition selon la revendication 8, dans laquelle la microparticule est choisie dans le groupe constitué par une particule d'or, une particule de tungstène, et une particule de trichite de carbure de silicium.

13. Composition selon la revendication 8, comprenant en outre :
(i) une matrice de modification polynucléotidique, dans laquelle ladite matrice de modification polynucléotidique comprend au moins une modification nucléotidique en comparaison avec la séquence de site cible dans le génome de ladite cellule végétale ; ou
(ii) une molécule d'ADN donneur, dans laquelle la molécule d'ADN donneur comprend au moins un polynucléotide d'intérêt à insérer dans ledit site cible dans le génome de ladite cellule végétale.

14. Composition selon la revendication 8, dans laquelle l'endonucléase Cas est l'endonucléase Cas9.

15. Procédé selon la revendication 1 ou 3, dans lequel l'endonucléase Cas est l'endonucléase Cas9.
